(19) Europäisches Patentamt
European Patent Office
Office européen des brevets

(11) EP 4 382 100 A1

(12) # EUROPEAN PATENT APPLICATION
published in accordance with Art. 153(4) EPC

(43) Date of publication:
**12.06.2024 Bulletin 2024/24**

(21) Application number: **22851497.2**

(22) Date of filing: **02.08.2022**

(51) International Patent Classification (IPC):
***A61K 31/175** (2006.01)* ***A61K 31/4965** (2006.01)*
***A61P 25/02** (2006.01)* ***A61P 29/00** (2006.01)*

(52) Cooperative Patent Classification (CPC):
**A61K 31/175; A61K 31/4965; A61P 25/02; A61P 29/00**

(86) International application number:
**PCT/BR2022/050303**

(87) International publication number:
**WO 2023/010191 (09.02.2023 Gazette 2023/06)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **02.08.2021 US 202163228472 P**

(71) Applicants:
- **Eurofarma Laboratórios S.A.**
  **06696-000 Itapevi - SP (BR)**
- **Universidade Federal Do Rio De Janeiro - UFRJ**
  **21941-901 Rio de Janeiro (BR)**

(72) Inventors:
- **BARREIRO, Gabriela**
  **06696-000 São Paulo - SP (BR)**
- **SANT´ANA, Danilo Pereira De**
  **06709-320 São Paulo SP (BR)**
- **GAMBA, Luis Eduardo Reina**
  **06696-000 São Paulo - SP (BR)**
- **FRAGA, Carlos Alberto Manssour**
  **21920-190 Rio de Janeiro - RJ (BR)**
- **BARREIRO, Eliezer Jesus De Lacerda**
  **21931-220 Rio de Janeiro RJ (BR)**
- **LIMA, Lídia Moreira**
  **21931-220 Rio de Janeiro RJ (BR)**

(74) Representative: **Engelhard, Markus**
**Boehmert & Boehmert**
**Anwaltspartnerschaft mbB**
**Pettenkoferstrasse 22**
**80336 München (DE)**

(54) **N-ACYLHYDRAZONE COMPOUNDS CAPABLE OF INHIBITING NAV1.7 AND/OR NAV1.8, PROCESSES FOR THE PREPARATION THEREOF, COMPOSITIONS, USES, METHODS FOR TREATMENT USING SAME, AND KITS**

(57) The present invention relates to *N*-acylhydrazone compounds that are Nav 1.7 and/or Nav 1.8 inhibitors. More specifically, the present invention relates to *N*-acylhydrazone compounds of Formula (I), wherein the substituents R1 to R6 are independently selected from the groups defined in the specification, as well as to the processes for the preparation thereof, compositions comprising at least one of said compounds, uses, treatment methods for treating or preventing pain-related pathologies, and kits. The present invention is applicable in the fields of medicinal chemistry and organic synthesis, as well as in the treatment of pain-related disorders.

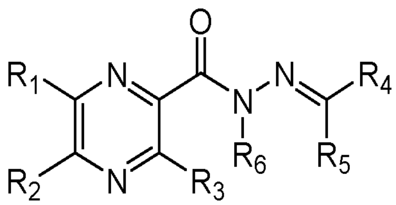

Formula (I)

## Description

## Field of invention

The present invention relates to *N*-acylhydrazone compounds that are Nav 1.7 and/or Nav 1.8 inhibitors, the processes for the preparation thereof, compositions containing these, uses, kits and treatment methods for treating or preventing pain-related pathologies. The present invention is applicable in the fields of medicinal chemistry, organic synthesis, as well as in the treatment of pain-related disorders.

## Background of invention

Physiological pain is an important protective mechanism developed to alert the body to actual or potential injuries that may endanger its integrity. In general terms, physiological pain can be classified into nociceptive pain and inflammatory pain. Nociceptive pain is characterised by presenting a high activation threshold, which remains until the stimulus that generated it is eliminated. Inflammatory pain, which arises as a response to tissue damage, is characterised by a low activation threshold and is a consequence of the activity of cellular and molecular mediators of the inflammatory process in sensitizing nociceptors (Schaible. Langenbecks Arch. Surg. 2004, 389, 237). When these nociceptive processes remain in the absence of harmful stimuli or in response to non-harmful stimuli, the protective and repair role of pain loses its functionality, constituting a maladaptive framework of neural plasticity and, as a consequence, a pathological state of chronic pain. Among the syndromes included in this classification, neuropathic pain has a high prevalence and impact today (Smith. Pain. 2020, 161, 1:S127; Cavalli. Int. J. Immunopathol. Pharmacol. 2019, 33:2058738419838383; Bouhassira. Rev Neurol (Paris). 2019, 175(1-2):16; Scholz. Nature Neurosci., 2002, 5,1062; Costigan. Annu. Rev. Neurosci., 2009, 32, 1).

Neuropathic pain is defined by the International Association for the Study of Pain (IASP) as pain initiated or caused by a primary dysfunction or injury in the central and/or peripheral nervous system (Dworkin. Clin. J. Pain, 2002, 18(6), 343). Central neuropathic pain comes from spinal cord injuries or diseases of the central nervous system such as multiple sclerosis or Parkinson's disease (Ducreux. Brain, 2006, 129, 963). Peripheral neuropathic pain, on the other hand, can be caused by trauma, metabolic disorders, chemical neurotoxicity, infection or tumor invasion, among others. Among the most common neuropathic pain syndromes are chemotherapy-induced neuropathic pain, complex regional pain, neuropathy related to viral infection, neuropathy secondary to tumor infiltration, diabetic neuropathy, phantom limb pain, postherpetic neuralgia, trigeminal neuralgia and postsurgical neuralgia (Pak. Curr. Pain Headache Rep., 2018, 22(2), 9).

Currently, there is no specific treatment for the control of pathologies related to neuropathic pain, however, the first-line alternative consists of the use of opioid analgesics, and - as adjuvants - local anesthetics, anticonvulsants and antidepressants. Nevertheless, adverse effects and poor efficacy drastically limit the use of these agents in the control of various pain-related pathologies (Kushnarev. 1. Expert Opinion. Investig. Drugs, 2020, 29(3), 259; Emery. Expert Opin. Ther. Targets, 2016, 20(8), 975).

Voltage-gated sodium channels (Nav) play a key role in the transmission of pain-related stimuli. These channels are activated in response to membrane depolarization allowing the generation and propagation of action potentials in neurons (and other electrically excitable cells) by controlling the flow of sodium ions across membranes. Structurally, voltage-gated sodium channels are heteromeric transmembrane proteins consisting of an $\alpha$ subunit and two helper $\beta$ subunits. The $\alpha$ subunit is organized into four homologous domains (I-IV) each with six transmembrane segments (S1-S6). The S4 segment of each domain is characterised by a conserved region of arginine residues, which act as sensors of the intra- and extracellular electrical environment of the neuron. This mechanism allows transforming alterations of the cellular electric field into specific conformational changes that, in turn, regulate the activation, deactivation and inactivation of voltage-gated sodium channels (Catterall. Nat. Chem. Biol., 2020, 16, 1314; Wisedchaisri. Cell., 2019, 178(4), 993; Clairfeuille. Science, 2019, 363, 1302).

In mammals, nine $\alpha$ subunits (Nav 1.1 - Nav 1.9) and four $\beta$ helper subunits ($\beta$1-$\beta$4) have been identified. The $\alpha$ subunits can also be classified as to their susceptibility to tetrodotoxin blockade (TTX), being classified as sensitive to tetrodotoxin (Nav 1.1, Nav 1.2, Nav 1.3, Nav 1.4, Nav 1.6 and Nav 1.7) or resistant to tetrodotoxin (Nav 1.5, Nav 1.8 and Nav 1.9) (Lera-Ruiz. J. Med. Chem., 2015, 58(18), 7093; Bagal. J. Med. Chem., 2013, 56(3), 593). Each of these $\alpha$ subunits exhibit a differentiated expression and function profile such that some of them are essential for the proper functioning of organs such as the heart and/or brain. Thus, the non-selective blockage of these channels is related to several types of adverse effects, such as migraine, epilepsy, paralysis and muscle and cardiac syndromes, among others (Bagal. J. Med. Chem., 2013, 56(3), 593; Bagal. Channels, 2015, 9(6), 360).

In general terms, sodium channels are distributed mainly in the central and peripheral nervous system, in neurons and glia. Nav channels 1.1, 1.2 and 1.3 are mainly expressed in the brain. Nav 1.4 and Nav 1.5 channels are mainly found in skeletal and cardiac muscles, respectively. Nav 1.6 channels are expressed in the central and peripheral

nervous system, while Nav 1.9 channels are selectively expressed in C-type nociceptive fibers in the dorsal root ganglion. On the other hand, Nav 1.7 and Nav 1.8 channels are mainly found in the peripheral nervous system and are directly related to pain transmission processes (Law. Drug Discovery Today, 2019, 24(7), 1389; Bagal. Channels (Austin), 2015, 9(6), 360; Lera-Ruiz. J. Med. Chem., 2015, 58(18), 7093).

Nav 1.7 sodium channels are expressed broadly in the olfactory epithelium, sympathetic ganglion, and dorsal root ganglion, predominantly in the C and Aδ nociceptivefibers. A large amount of evidence supports the important role of Nav 1.7 sodium channels in pain transmission processes. For example, gain-of-function-related mutations in the gene (SCN9A), which encodes the Nav 1.7 sodium channel, are associated with extreme pain disorders such as congenital pain hypersensitivity, paroxysmal extreme pain disorder, and primary erythromelalgia. On the other hand, mutations related to loss of gene function (SCN9A) are related to congenital insensitivity to pain in individuals who, in general terms, are free of motor or cognitive impairment (Vetter. Pharmacology & Therapeutics, 2017, 172, 73; Ahuja. Science, 2015, 350(6267), 1491; Kingwell. Nat. Rev. Drug Discov., 2019, 18, 321; Safina. J. Med. Chem., 2021, 64, 2953; Luo. J. Med. Chem., 2019, 62, 831; Bankar. Cell Reports, 2018, 24, 3133).

Nav 1.8 sodium channels show increased expression in the peripheral nervous system, largely (but not exclusively) in C-type nociceptive fibers in the dorsal root ganglion. Recent evidence that includes elevated Nav 1.8 expression levels in chronic pain states, Nav 1.8 *knockout* animal data, and analgesic activity of desensitizing oligodeoxynucleotides specific for Nav 1.8, among others (Brown. Bioorg. Med. Chem., 2019, 27(1), 230; Payne. Br. J. Pharmacol., 2015, 172(10), 2654; Bagal. Med. Chem. Lett. 2015, 6(6) 650; Kort. J. Med. Chem. 2008, 51, 407; Zhang. Neuropharmacology, 2010, 59, 201 and 207), support the role of the Nav 1.8 sodium channel in the development and process of pain-related pathologies (Kingwell. Nat. Rev. Drug Discov., 2019, 18, 321; Law. Drug Discovery Today, 2019, 24(7), 1389; Bagal. Channels (Austin), 2015, 9(6), 360; Lera-Ruiz. J. Med. Chem., 2015, 58(18), 7093).

In this way, the voltage-gated sodium channels Nav 1.7 and Nav 1.8 are considered promising therapeutic targets for the treatment of neuropathic pain-related dysfunctions (Kornecook. J. Pharmacol. Agency No. Ther., 2017, 362, 146; Kingwell. Nat. Rev. Drug Discov., 2019, 18, 321; Bagal. Channels (Austin), 2015, 9(6), 360; Lera-Ruiz. J. Med. Chem., 2015, 58(18), 7093; Deuis. Neuropharmacology, 2017, 127, 87 and 108; Kushnarev. Expert Opin. Investig. Drugs, 2020, 29(3), 259; McKerrall. Bioorganic & Medicinal Chemistry Lett., 2018, 28, 3141; Emery. Expert Opin. Ther. Targets, 2016, 20(8), 975; Bagal. Bioorganic & Medicinal Chemistry Lett., 2014, 24, 3690).

A large number of compounds have been described in the literature for their ability to act as blockers of Nav 1.7 and 1.8 sodium channels, however, they present a great structural diversity, a fact that does not allow the establishment of a common pharmacophoric group.

The patent literature contains several examples of compounds that act as sodium channel blockers. In particular, blockers of the selective sodium channels Nav 1.7 are described in US10550080, US9765029 and US10000475. Additionally, some documents describe selective blockers of Nav 1.8 sodium channels such as WO2020261114, WO2020092667, US9163042, WO2014120808, WO2014120815, WO2018213426, WO2019014352, WO2015006280 and US7928107. These documents disclose compounds having distinct structures of the present invention.

Still, there are patent documents describing dual inhibitors of Nav 1.7 and 1.8, including WO2018235851, US8629149, JP2017001991 that disclose, respectively, pyridyl amines, oxopiperazine derivatives and benzoxazolones. However, all these documents disclose compounds with structures and physicochemical characteristics distinct from the present invention.

In this context, it is advantageous to develop new alternatives of compounds that can act as inhibitors of Nav 1.7 and/or Nav 1.8 that have adequate pharmacological action and, preferably, provide mitigated adverse effects. Therefore, the present invention relates to N-acylhydrazone derivatives endowed with novel and inventive step as an alternative and/or complement to the treatment of pain-related diseases.

## Summary of Invention

The present invention discloses *N*-acylhydrazone compounds with inhibitory activity of Nav 1.7 and/or 1.8, against pain-related pathologies, in addition to compositions, uses, kits, treatment methods and related preparation processes.

The present invention relates to compound(s) of Formula (I):

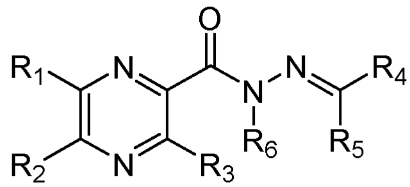

Formula (I)

or a pharmaceutically acceptable salt, hydrate, solvate, and isomer thereof, wherein:

-R1 is selected from hydrogen, halogen, branched or linear C1-6alkyl, branched or linearC1-6 alkoxy, morpholin, piperidinyl or substituted piperidinyl, pyridinyl or substituted pyridinyl, piperazin or substituted piperazin, C3-7 heterocycloalkyl, aryloxy, substituted aryloxy, or R7;
-R2 and R3 are independently selected from the group consisting of hydrogen, halogen, linear or branched C1-6 alkyl; linear or branched C1-6 alkoxy, amine, C1-6haloalkyl, aryl, substituted aryl, aryloxy, or substituted aryloxy;
-R4 is selected from the group consisting of hydrogen, halogen, linear or branched C1-6alkyl ; linear or branchedC1-6 alkoxy, aryl, pyridinyl or substituted pyridinyl, pyridone, saturated or unsaturated C3-7cycloalkyl saturated or unsaturated C1-5 alkylsubstituted C3-7cycloalkyl saturated or unsaturated halosubstituted C3-7 cycloalkyl, picolinamide orR13;
-R5 and R6 are independently selected from hydrogen or linear or branched C1-6 alkyl;
-R7 is:

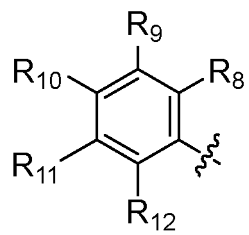

wherein,
-R8 is selected from the group consisting of hydrogen, halogen, linear or branched C1-6 alkyl, linear or branched C1-6 alkoxy, C1-6haloalkyl, morpholin,C1-6 haloalkyloxy, C1-6 alkylamino or (C1-6 alkylamino)C1-6alkoxy;
-R9, R11 andR12 are independently selected from the group consisting of hydrogen, halogen, linear or branched C1-6 alkyl, linear or branched C1-6 alkoxy; C1-6 haloalkyl or C3-6cycloalkyloxy;
-R10 is selected from the group consisting of hydrogen, halogen, linear or branched C1-6alkyl linear or branched C1-6 alkoxy, C3-6 cycloalkyloxy; (C3-6cycloalkyl) C1-6 alkoxy; -S-C1-6 alkyl;C1-6 alkylamino; haloalkyloxy C1-6,haloalkylC1-6, (C1-6 alkylamino)C1-6 alkoxy, (-S-C1-6alkyl) C1-6alkyloxy, C1-6alkylsulfonylamide, or-OCD3;
-R13 is:

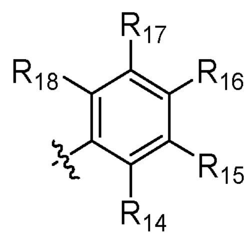

wherein,
- R14 and R15 are independently selected from the group consisting of hydrogen, halogen, linear or branched C1-6 alkyl; linear or branched C1-6 alkoxy, C1-6haloalkyl, C1-6alkylamino, C1-6 alkylsufonyl, C1-6 alkylsufonylamide, (C1-6 alkylsufonyl) C1-6 alkyl; C1-6 hydroxyalkyl,(C1-6 alkylamino ) C1-6 alkoxyamide,
-OCD3, or R14 and R15 are optionally substituted with an OC=N, N=CO or SC=N group forming together a thiazole or an oxazole;
-R16 andR17 are independently selected from the group consisting of hydrogen, halogen, linear or branched C1-6 alkyl; linear or branched C1-6 alkoxy, C1-6haloalkyl, C1-6alkylamino, C1-6alkylsufonylamide, (C1-6alkylamino) C1-6alkyl or-OCD3;
-R18 is selected from the group consisting of hydrogen, halogen, linear or branched C1-6alkyl or linear or branchedC1-6 alkoxy.

Additionally, the present invention also relates to compositions comprising one or more compound(s) of Formula (I) or a pharmaceutically acceptable salt, hydrate, solvate, and isomer thereof; and one or more pharmaceutically acceptable excipients.
Additionally, kits according to the present invention may comprise such compositions and application devices,

which may include ampoules, syringes, and others. Alternatively, kits according to the present invention comprise more than one compound of Formula (I) arranged in one or more dosage forms, including without limitation, tablets, accompanied by instructions for administration.

The present invention further relates to methods of treating, preventing, alleviating, suppressing and/or controlling diseases related to neuropathic pain. Also taught are uses of compound(s) of general Formula (I) for preparing a medicament for the treatment of neuropathic pain-related pathologies. Finally, the present invention teaches processes of obtaining compound(s) of general Formula (I).

## Detailed description of invention

The present invention features in a first embodiment, the compound of Formula (I):

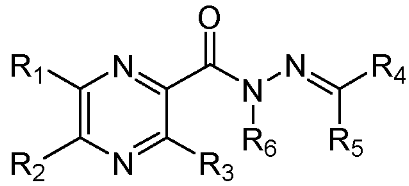

Formula (I)

or a pharmaceutically acceptable salt, hydrate, solvate, and isomer thereof, wherein:

- R1 is selected from hydrogen, halogen, branched or linear C1-6alkyl, branched or linear C1-6 alkoxy, morpholin, piperidinyl or substituted piperidinyl, pyridinyl or substituted pyridinyl, piperazin or substituted piperazin, C3-7 heterocycloalkyl, aryloxy, substituted aryloxy, or R7;
- R2 and R3 are independently selected from the group consisting of hydrogen, halogen, linear or branched C1-6 alkyl; linear or branched C1-6 alkoxy, amine, C1-6haloalkyl, aryl, substituted aryl, aryloxy, or substituted aryloxy;
- R4 is selected from the group consisting of hydrogen, halogen, linear or branched C1-6alkyl; linear or branchedC1-6 alkoxy, aryl, pyridinyl or substituted pyridinyl, pyridone, saturated or unsaturated C3-7cycloalkyl saturated or unsaturated C1-5 alkylsubstituted C3-7cycloalkyl saturated or unsaturated halosubstituted C3-7 cycloalkyl, picolinamide orR13;
- R5 and R6 are independently selected from hydrogen or linear or branched C1-6 alkyl;
- R7 is:

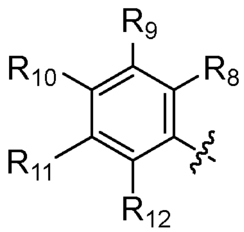

wherein,

- R8 is selected from the group consisting of hydrogen, halogen, linear or branched C1-6 alkyl, linear or branched C1-6 alkoxy, C1-6haloalkyl, morpholin,C1-6 haloalkyloxy, C1-6 alkylamino or (C1-6 alkylamino)C1-6alkoxy; -R9, R11 andR12 are independently selected from the group consisting of hydrogen, halogen, linear or branched C1-6 alkyl, linear or branched C1-6 alkoxy; C1-6haloalkyl or C3-6cycloalkyloxy;
- R10 is selected from the group consisting of hydrogen, halogen, linear or branched C1-6alkyl linear or branched C1-6 alkoxy, C3-6 cycloalkyloxy; (C3-6cycloalkyl) C1-6 alkoxy; -S-C1-6 alkyl;C1-6 alkylamino; haloalkyloxy C1-6,haloalkylC1-6, (C1-6 alkylamino)C1-6 alkoxy, (-S-C1-6alkyl) C1-6alkyloxy, C1-6alkylsulfonylamide, or-OCD3;
- R13 is:

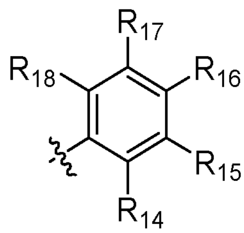

wherein,

- R14 and R15 are independently selected from the group consisting of hydrogen, halogen, linear or branched C1-6 alkyl; linear or branched C1-6 alkoxy, C1-6haloalkyl, C1-6alkylamino, C1-6 alkylsufonyl, C1-6 alkylsufonylamide, (C1-6 alkylsufonyl) C1-6 alkyl; C1-6 hydroxyalkyl, (C1-6 alkylamino ) C1-6 alkoxy,amide, -OCD3, or R14 and R15 are optionally substituted with an OC=N, N=CO or SC=N group forming together a thiazole or an oxazole;
- R16 andR17 are independently selected from the group consisting of hydrogen, halogen, linear or branched C1-6 alkyl; linear or branched C1-6 alkoxy, C1-6haloalkyl, C1-6alkylamino, C1-6alkylsufonylamide, (C1-6alkylamino) C1-6alkyl, or-OCD3;
- R18 is selected from the group consisting of hydrogen, halogen, linear or branched C1-6alkyl or linear or branchedC1-6 alkoxy.

In one embodiment, R1 is selected from the group consisting of hydrogen, halogen, branched or linear C1-6 alkyl, branched or linear C1-6 alkoxy, morpholin, piperidinyl or substituted piperidinyl, pyridinyl or substituted pyridinyl, substituted piperazin, C3-7 heterocycloalkyl, substituted aryloxy, or R7; R2 is selected from the group consisting of halogen, linear or branched C1-6 alkyl; linear or branched C1-6 alkoxy, amine, substituted C1-6 aryl haloalkyl, substituted aryloxy; R3 is selected from the group consisting of hydrogen, halogen, linear or branched C1-6 alkyl, linear or branched C1-6 alkoxy, aryl, substituted aryl, aryloxy, substituted aryloxy; R4 is independently selected from the group consisting of hydrogen, pyridone, pyridinyl or substituted pyridinyl, saturated or unsaturated C3-7 cycloalkyl, saturated or unsaturated C1-5 alkylsubstituted C3-7 cycloalkyl, saturated or unsaturated halosubstituted C3-7 cycloalkyl, picolinamide or R13; R5 and R6 are independently selected from the group consisting of hydrogen and linear or branched C1-6 alkyl; R8 is selected from the group consisting of hydrogen, halogen, linear or branched C1-6 alkyl, linear or branched C1-6 alkoxy, haloalkyl C1-6, morpholin, haloalkyloxy C1-6, C1-6 alkylamino or (C1-6 alkylamino)alkoxy C1-6; R9 is selected from the group consisting of hydrogen, halogen, linear or branched C1-6 alkyl, linear or branched C1-6 alkoxy, C1-6 haloalkyl, or C3-6 cycloalkyloxy; R10 is selected from the group consisting of hydrogen, halogen, linear or branched C1-6 alkyl, linear or branched C1-6 alkoxy, C3-6 cycloalkyloxy; (C3-6 cycloalkyl) C1-6 alkoxy; -S-C1-6 alkyl, C1-6 alkylamino, haloalkyloxy C1-6, haloalkyl C1-6, (C1-6 alkylamino) C1-6 alkoxy, (-S-C1-6 alkyl) C1-6 alkyloxy, C1-6 alkylsulfonylamide, or -OCD3; R11 is selected from the group consisting of hydrogen or halogen; R12 is selected from the group consisting of hydrogen or halogen; from the group consisting of hydrogen, halogen, C1-6 haloalkyl or C3-6 cycloalkyloxy; R14 is selected from the group consisting of hydrogen, halogen, linear or branched C1-6 alkyl, linear or branched C1-6 alkoxy, C1-6 haloalkyl, C1-6 alkylamino, C1-6 alkylsufonylamide, C1-6 alkylsufonylamide or(C1-6 alkylsufonyl) C1-6 alkyl; R15 is selected from the group consisting of hydrogen, halogen, linear or branched C1-6 alkyl, linear or branched C1-6 alkoxy, C1-6 haloalkyl, C1-6 alkylamino, C1-6 alkylsufonylamide, C1-6 hydroxyalkyl, (C1-6 alkylamino) C1-6 alkoxy, amide or -OCD3; or R14 and R15 are optionally substituted with an OC group =N, N=CO or SC=N forming together a thiazole or an oxazole; R16 is selected from the group consisting of hydrogen, halogen, linear or branched C1-6 alkyl, linear or branched C1-6 alkoxy, C1-6 haloalkyl, C1-6 alkylamino or C1-6 alkylsufonylamide; R17 is selected from the group consisting of hydrogen, halogen, linear or branched C1-6 alkyl; linear or branched C1-6 alkoxy, C1-6 haloalkyl, C1-6 alkylamino, (C1-6 alkylamino) C1-6 alkyl or -OCD3; R18 is hydrogen or linear or branched C1-6 alkoxy.

In one embodiment, R1 is selected from the group consisting of hydrogen, chloro, bromo, fluoro, methyl, ethyl, propyl, isopropyl, butyl, methoxy, ethoxy, propoxy, isopropoxy, morpholin, pyridin-2-yl, pyridin-4-yl, pyridin-3-yl, 6-methoxypyridin-3-yl, 5-methoxypyridin-2-yl, 3-methoxypyridin-2-yl, 4-methoxypyridin-2-yl, 2-methoxypyridin-3-yl, 4-methoxypyridin-3-yl, 5-methoxypyridin-3-yl, piperidin-1-yl, 4,4-difluoro-3-methylpiperidin-1-yl; 4,4-difluoropiperidin-1-yl, 4,4-difluoro-2-methylpiperidin-1-yl, 4,4-dichloro-3-methylpiperidin-1-yl; 4,4-dichloropiperidin-1-yl, 4,4-dichloro-2-methylpiperidin-1-yl, 4-propylpiperazin-1-yl, 4-methylpiperazin-1-yl, 4-ethylpiperazin-1-yl, 4,4-difluoroazepan-1-yl, 4-ethoxyphenoxy, 4-methoxyphenoxy, orR7. In a preferred embodiment, R1 is selected from the group consisting of hydrogen, chloro, methyl, ethyl, isopropyl, methoxy, morpholin, pyridin-2-yl, pyridin-4-yl, pyridin-3-yl, 6-methoxypyridin-3-yl, 5-methoxypyridin-2-yl, piperidin-1-yl, 4,4-difluoro-3-methylpiperidin-1-yl; 4,4-difluoropiperidin-1-yl, 4,4-dichloro-3-methylpiperidin-1-yl; 4,4-dichloropiperidin-1-yl, 4,4-dichloro-2-methylpiperidin-1-yl, 4-propylpiperazin-1-yl, 4,4-difluoroazepan-1-yl, 4-ethoxyphenoxy, 4-methoxyphenoxy or R7.

In one embodiment, R2 is selected from the group consisting of chloro, bromo, fluoro, methyl, ethyl, isopropyl,

amine, trifluoromethyl, methoxy, ethoxy, isopropoxy, 4-methoxyphenyl, or 4-methoxyphenoxy. In a preferred embodiment, R2 is independently selected from the group consisting of chloro, methyl, ethyl, isopropyl, methoxy, ethoxy, amine, trifluoromethyl, 4-methoxyphenyl or 4-methoxyphenoxy.

In one embodiment, R3 is selected from the group consisting of hydrogen, chloro, bromo, fluoro, methyl, ethyl, isopropyl, methoxy, ethoxy, isopropoxy, phenyl, 3-phenoxy, 4-methoxyphenyl, or 4-methoxyphenoxy. In a preferred embodiment, R3 is selected from the group consisting of hydrogen, chloro, methyl, ethyl, isopropyl, methoxy, phenyl, 3-phenoxy, 4-methoxyphenyl or 4-methoxyphenoxy.

In one embodiment, R4 is selected from the group consisting of hydrogen, pyridone, pyridin-3-yl, pyridin-4-yl, pyridin-2-yl, 2-methoxypyridin-4-yl, 4-methoxypyridin-2-yl, 5-methoxypyridin-3-yl, 6-methoxypyridin-2-yl, cyclohexyl, cycloheptyl, cyclopentyl, cyclobutyl, 2-methylcyclopent-1-en-1-yl, 2-methylcyclohex-1-en-1-yl, 2-methylcyclohept-1-en-1-yl, 4-difluorocyclohexyl, picolinamide, orR13. In a preferred embodiment, R4 is selected from the group consisting of pyridone, pyridin-3-yl, pyridin-4-yl, pyridin-2-yl, 2-methoxypyridin-4-yl, 4-methoxypyridin-2-yl, 5-methoxypyridin-3-yl, 6-methoxypyridin-2-yl, cyclohexyl, cycloheptyl, 2-methylcyclopent-1-en-1-yl, 2-methylcyclohex-1-en-1-yl, 2-methylcyclohept-1-en-1-yl, 4-difluorocyclohexyl, picolinamide orR13.

In one embodiment, R5 andR6 are each independently hydrogen or methyl. In a preferred embodiment, R5 is hydrogen or methyl andR6 is hydrogen. In a further preferred embodiment, R5 is hydrogen or methyl andR6 is methyl. In a further preferred embodiment, R5 is methyl andR6 is hydrogen.

In one embodiment, R8 is selected from the group consisting of hydrogen, chloro, fluoro, bromo, methyl, ethyl, isopropyl, methoxy, ethoxy, isopropoxy, butoxy, trifluoromethyl, difluoromethyl, morpholin, trifluoromethoxy, dimethylamino, or (dimethylamino)methoxy. In a preferred embodiment, R8 is selected from the group consisting of hydrogen, chloro, fluoro, methyl, methoxy, ethoxy, isopropoxy, butoxy, trifluoromethyl, difluoromethyl, morpholin, trifluoromethoxy, dimethylamino or (dimethylamino)methoxy.

In one embodiment, R9 is selected from the group consisting of hydrogen, chloro, fluoro, bromo, methyl, ethyl, isopropyl, methoxy, ethoxy, isopropoxy, or butoxy. In a preferred embodiment, R9 is selected from the group consisting of hydrogen, chlorine, fluorine, methyl or methoxy.

In one embodiment, R10 is selected from the group consisting of hydrogen, chloro, fluoro, bromo, methyl, propyl, tert-butyl, methoxy, ethoxy, isopropoxy, propoxy, butoxy, isobutoxy, tert-butoxy, cyclopropoxy, cyclopentyloxy, cyclohexyloxy, cyclobutyloxy, cyclopropylmethoxy, -S-ethyl, ethylamino, trifluoromethoxy, trifluoromethyl, difluoromethyl, (dimethylamino)ethoxy, (-S-methyl)methoxy, methylsufonylamide, or-OCD3. In a preferred embodiment, R10 is selected from the group consisting of hydrogen, chloro, fluoro, methyl, propyl, tert-butyl, methoxy, ethoxy, isopropoxy, propoxy, butoxy, isobutoxy , tert-butoxy, cyclopropoxy, cyclopentyloxy, cyclohexyloxy, cyclobutyloxy, cyclopropylmethoxy, -S-ethyl, ethylamino, trifluoromethoxy, difluoromethyl, (dimethylamino)ethoxy, (-S-methyl)methoxy, methylsufonylamide or-OCD3.

In a preferred embodiment, R11 is selected from the group consisting of hydrogen, chlorine or fluorine. In a preferred embodiment, R11 is hydrogen or chlorine.

In one embodiment, R12 is selected from the group consisting of hydrogen, chloro, bromo, fluoro, methyl, trifluoromethyl, or cyclopentyloxy. In one embodiment, R12 is selected from the group consisting of hydrogen, chloro, bromo, fluoro, methyl, trifluoromethyl, or cyclopentyloxy.

In one embodiment, R14 is selected from the group consisting of hydrogen, chloro, bromo, fluoro, methyl, *tert-butyl,* ethyl, isopropyl, methoxy, ethoxy, propoxy, isopropoxy, trifluoromethyl, dimethylamino, methylsufonyl, methylsufonylamide, or (methylsufonyl)methyl. In a preferred embodiment, R14 is selected from the group consisting of hydrogen, chloro, bromo, fluoro, methyl, *tert-butyl,* methoxy, ethoxy, propoxy, isopropoxy, trifluoromethyl, dimethylamino, methylsufonyl, methylsufonylamide or (methylsufonyl)methyl.

In one embodiment, R15 is selected from the group consisting of hydrogen, chloro, bromo, fluoro, methyl, *tert-butyl,* ethyl, isopropyl, methoxy, ethoxy, propoxy, isopropoxy, trifluoromethyl, amide, dimethylamino, methylsufonyl, methylsufonylamide, 2-hydroxyethyl; 1-hydroxyethyl or (dimethylamino)ethoxy. In a preferred embodiment, R15 is selected from the group consisting of hydrogen, chloro, bromo, fluoro, methyl, *tert-butyl,* methoxy, ethoxy, propoxy, isopropoxy, trifluoromethyl, amide, dimethylamino, methylsufonyl, methylsufonylamide, 2-hydroxyethyl; 1-hydroxyethyl, (dimethylamino)ethoxy or-OCD3.

In one embodiment, R14 andR15 are optionally substituted with an OC=N, N=CO or SC=N group forming together a thiazole or an oxazole.

In one embodiment, R16 is selected from the group consisting of hydrogen, chloro, bromo, fluoro, methyl, *tert-butyl,* ethyl, isopropyl, methoxy, ethoxy, propoxy, isopropoxy, trifluoromethyl, dimethylamino, or methylsufonylamide. In a preferred embodiment, R16 is selected from the group consisting of hydrogen, chloro, bromo, fluoro, methyl, *tert-butyl,* methoxy, ethoxy, propoxy, isopropoxy, trifluoromethyl, dimethylamino or methylsufonilamide.

In one embodiment, R17 is selected from the group consisting of hydrogen, chloro, bromo, fluoro, methyl, *tert-butyl,* ethyl, isopropyl, methoxy, ethoxy, propoxy, isopropoxy, trifluoromethyl, (dimethylamino)methyl, or dimethylamino. In a preferred embodiment, R17 is selected from the group consisting of hydrogen, chloro, bromo, fluoro, methyl, *tert-*

*butyl,* methoxy, ethoxy, propoxy, isopropoxy, trifluoromethyl, (dimethylamino)methyl, dimethylamino or-OCD3.

In one embodiment, R18 is selected from hydrogen, methoxy, ethoxy, propoxy, isopropoxy. In a preferred embodiment, R18 is hydrogen or methoxy.

In a preferred embodiment, the compound(s) of Formula (I) is selected from the group consisting of:

6-(4-chlorophenyl)-*N*'-[*(E)*-(3,5-dimethoxyphenyl)methylidene]pyrazine-2-carbohydrazide (Compound 1);
(*E*)-*N*'-(3,5-dimethoxybenzylidene)-6-(4-ethoxyphenyl)pyrazine-2-carbohydrazide (Compound 2);
(*E*)-*N*'-(2,5-dimethoxybenzylidene)-6-(4-ethoxyphenyl)pyrazine-2-carbohydrazide (Compound 3);
*(E)*-*N*'-(3-(2-(dimethylam ino)ethoxy)-5-methoxybenzylidene)-6-(4-ethoxyphenyl)pyrazine-2-carbohydrazide (Compound 4);
*(E)* -6-(4-ethoxyphenyl) -*N'*-((5-methoxybenzo[d]oxazol-7-yl)methylene)pyrazine-2-carbohydrazide (Compound 5);
(*E*) -6-(4-ethoxyphenyl) -*N'*-((6-methoxybenzo[d]oxazol-4-yl)methylene)pyrazine-2-carbohydrazide (Compound 6);
*(E)*-6-(4-ethoxyphenyl)-*N'*-((5-methoxybenzo[d]thiazol-7-yl)methylene)pyrazine-2-carbohydrazide (Compound 7);
*(E)*-6-(4-ethoxyphenyl)-*N'*-((5-methoxypyridin-3-yl)methylene)pyrazine-2-carbohydrazide (Compound 8);
*(E)*-6-(4-ethoxyphenyl)-*N'*-((4-methoxypyridin-2-yl)methylene)pyrazine-2-carbohydrazide (Compound 9);
*(E)*-6-(4-ethoxyphenyl)-*N'*-((2-methoxypyridin-4-yl)methylene)pyrazine-2-carbohydrazide (Compound 10);
*(E)*-6-(4-ethoxyphenyl)-*N'*-((6-methoxypyridin-2-yl)methylene)pyrazine-2-carbohydrazide (Compound 11);
(*E*)-*N'*-(3,5-dimethylbenzylidene)-6-(4-ethoxyphenyl)pyrazine-2-carbohydrazide (Compound 12);
*(E)*-6-(4-ethoxyphenyl)-N'-(2,3,5-trimethoxybenzylidene)pyrazine-2-carbohydrazide (Compound 13);
*(E)*-N'-(2-ethoxy-3,5-dimethoxybenzylidene)-6-(4-ethoxyphenyl)pyrazine-2-carbohydrazide (Compound 14);
*(E)*-6-(4-ethoxyphenyl)-N'-(3-fluoro-5-methoxybenzylidene)pyrazine-2-carbohydrazide (Compound 15);
*(E)*-6-(4-ethoxyphenyl)-*N'*-(3-methoxy-5-propoxybenzylidene)pyrazine-2-carbohydrazide (Compound 16);
*(E)*-6-(4-ethoxyphenyl)-*N'*-(4-fluoro-3,5-dimethoxybenzylidene)pyrazine-2-carbohydrazide (Compound 17);
*(E)*-*N*'-(2-chloro-5-methoxybenzylidene)-6-(4-ethoxyphenyl)pyrazine-2-carbohydrazide (Compound 18);
*(E)*-*N*'-(2-chloro-3-methoxybenzylidene)-6-(4-ethoxyphenyl)pyrazine-2-carbohydrazide (Compound 19);
*(E)*-6-(4-ethoxyphenyl)-*N'*-(2-fluoro-5-methoxybenzylidene)pyrazine-2-carbohydrazide (Compound 20);
*(E)*-6-(4-ethoxyphenyl)-*N'*-(2-fluoro-3-methoxybenzylidene)pyrazine-2-carbohydrazide (Compound 21);
*(E)*-6-(4-ethoxyphenyl)-*N'*-(3-methoxybenzylidene)pyrazine-2-carbohydrazide (Compound 22);
*(E)*-6-(4-ethoxyphenyl)-*N'*-(4-methoxybenzylidene)pyrazine-2-carbohydrazide (Compound 23);
*(E)*-6-(4-ethoxyphenyl)-*N'*-(pyridin-4-ylmethylene)pyrazine-2-carbohydrazide (Compound 24);
*(E)*-6-(4-ethoxyphenyl)-N'-(pyridin-2-ylmethylene)pyrazine-2-carbohydrazide (Compound 25);
*(E)* -6-(4-ethoxyphenyl) -*N'*-(2-methoxybenzylidene)pyrazine-2-carbohydrazide (Compound 26);
*(E)*-*N'*-(2,3-dimethoxybenzylidene)-6-(4-ethoxyphenyl)pyrazine-2-carbohydrazide (Compound 27);
*(E)*-*N'*-(3,4-dimethoxybenzylidene)-6-(4-ethoxyphenyl)pyrazine-2-carbohydrazide (Compound 28);
*(E)*-*N'*-(3,5-dimethoxybenzylidene)-6-(4-isopropoxyphenyl)pyrazine-2-carbohydrazide (Compound 29);
6-[4-(cyclopentyloxy)phenyl]-*N'*-[*(E)*-(3,5-dimethoxyphenyl)methylidene]pyrazine-2-carbohydrazide (Compound 30);
*N'*-[*(E)*-(3,5-dimethoxyphenyl)methylidene]-6-[4-(trifluoromethoxy)phenyl]pyrazine-2-carbohydrazide (Compound31);
*N'*-[*(E)*-(3,5-dimethoxyphenyl)methylidene]-6-(4-propoxyphenyl)pyrazine-2-carbohydrazide (Compound 32);
*N'*-[*(E)*-(3,5-dimethoxyphenyl)methylidene]-6-(4-methoxyphenyl)pyrazine-2-carbohydrazide (Compound 33);
*(E)*-6-(4-methoxyphenyl)-*N'*-((2-oxo-1,2-dihydropyridin-4-yl)methylene)pyrazine-2-carbohydrazide (Compound 34);
*(E)*-2-fluoro-5-((2-(6-(4-methoxyphenyl)pyrazine-2-carbonyl)hydrazono)methyl) benzamide (Compound 35);
*(E)*-4-((2-(6-(4-methoxyphenyl)pyrazine-2-carbonyl)hydrazono)methyl)picolinamide (Compound 36);
*(E)*-*N'*-(3-methoxybenzylidene)-6-(4-methoxyphenyl)pyrazine-2-carbohydrazide (Compound 37);
*(E)*-*N'*-(3-((dimethylamino)methyl)benzylidene)-6-(4-methoxyphenyl)pyrazine-2-carbohydrazide (Compound 38);
*(E)*-*N'*-(3-ethoxybenzylidene)-6-(4-methoxyphenyl)pyrazine-2-carbohydrazide (Compound 39);
*(E)*-6-(4-methoxyphenyl)-*N'*-(3-propoxybenzylidene)pyrazine-2-carbohydrazide (Compound 40);
*(E)*-*N'*-(3-isopropoxybenzylidene)-6-(4-methoxyphenyl)pyrazine-2-carbohydrazide (Compound 41);
*(E)*-*N'*-(2-methoxybenzylidene)-6-(4-methoxyphenyl)pyrazine-2-carbohydrazide (Compound 42);
*(E)*-*N'*-(2-ethoxybenzylidene)-6-(4-methoxyphenyl)pyrazine-2-carbohydrazide (Compound 43);
*(E)*-6-(4-methoxyphenyl)-*N'*-(2-propoxybenzylidene)pyrazine-2-carbohydrazide (Compound 44);
*(E)*-*N'*-(2-isopropoxybenzylidene)-6-(4-methoxyphenyl)pyrazine-2-carbohydrazide (Compound 45);
*(E)*-*N'*-(2-fluorobenzylidene)-6-(4-methoxyphenyl)pyrazine-2-carbohydrazide (Compound 46);
*(E)*-*N'*-(2-chlorobenzylidene)-6-(4-methoxyphenyl)pyrazine-2-carbohydrazide (Compound 47);
*(E)*-*N'*-(2-bromobenzylidene)-6-(4-methoxyphenyl)pyrazine-2-carbohydrazide (Compound 48);
*(E)*-6-(4-methoxyphenyl)-*N'*-(2-(trifluoromethyl)benzylidene)pyrazine-2-carbohydrazide (Compound 49);

*(E)*-*N'*-(2-(dimethylamino)benzylidene)-6-(4-methoxyphenyl)pyrazine-2-carbohydrazide (Compound 50);
*(E)*-6-(4-methoxyphenyl)-*N'*-(2-methylbenzylidene)pyrazine-2-carbohydrazide (Compound 51);
*(E)*-*N'*-(2-(tert-butyl)benzylidene)-6-(4-methoxyphenyl)pyrazine-2-carbohydrazide (Compound 52);
*(E)*-*N'*-(3-fluorobenzylidene)-6-(4-methoxyphenyl)pyrazine-2-carbohydrazide (Compound 53);
*(E)*-*N'*-(3-chlorobenzylidene)-6-(4-methoxyphenyl)pyrazine-2-carbohydrazide (Compound 54);
*(E)*-*N'*-(3-bromobenzylidene)-6-(4-methoxyphenyl)pyrazine-2-carbohydrazide (Compound 55);
*(E)*-6-(4-methoxyphenyl)-*N'*-(3-(trifluoromethyl)benzylidene)pyrazine-2-carbohydrazide (Compound 56);
*(E)*-*N'*-(3-(dimethylamino)benzylidene)-6-(4-methoxyphenyl)pyrazine-2-carbohydrazide (Compound 57);
*(E)*-6-(4-methoxyphenyl)-*N'*-(3-methylbenzylidene)pyrazine-2-carbohydrazide (Compound 58);
*(E)*-*N'*-(3-(tert-butyl)benzylidene)-6-(4-methoxyphenyl)pyrazine-2-carbohydrazide (Compound 59);
*(E)*-*N'*-(4-fluorobenzylidene)-6-(4-methoxyphenyl)pyrazine-2-carbohydrazide (Compound 60);
E)-*N'*-(4-chlorobenzylidene)-6-(4-methoxyphenyl)pyrazine-2-carbohydrazide (Compound 61);
*(E)*-*N'*-(4-bromobenzylidene)-6-(4-methoxyphenyl)pyrazine-2-carbohydrazide (Compound 62);
*(E)*-6-(4-methoxyphenyl)-*N'*-(4-(trifluoromethyl)benzylidene)pyrazine-2-carbohydrazide (Compound 63);
*(E)*-*N'*-(4-(dimethylamino)benzylidene)-6-(4-methoxyphenyl)pyrazine-2-carbohydrazide (Compound 64);
*(E)*-6-(4-methoxyphenyl)-*N'*-(4-methylbenzylidene)pyrazine-2-carbohydrazide (Compound 65);
*(E)*-*N'*-(4-(tert-butyl)benzylidene)-6-(4-methoxyphenyl)pyrazine-2-carbohydrazide (Compound 66);
*(E)*-*N'*-(3,5-diethoxybenzylidene)-6-(4-methoxyphenyl)pyrazine-2-carbohydrazide (Compound 67);
*(E)*-*N'*-(3,5-dipropoxybenzylidene)-6-(4-methoxyphenyl)pyrazine-2-carbohydrazide (Compound 68);
*(E)*-*N'*-(3,5-diisopropoxybenzylidene)-6-(4-methoxyphenyl)pyrazine-2-carbohydrazide (Compound 69);
*(E)*-*N'*-(3,5-difluorobenzylidene)-6-(4-methoxyphenyl)pyrazine-2-carbohydrazide (Compound 70);
*(E)*-*N'*-(3,5-dichlorobenzylidene)-6-(4-methoxyphenyl)pyrazine-2-carbohydrazide (Compound 71);
*(E)*-*N'*-(3,5-dibromobenzylidene)-6-(4-methoxyphenyl)pyrazine-2-carbohydrazide (Compound 72);
(*E*)-*N'*-(3,5-bis(trifluoromethyl)benzylidene)-6-(4-methoxyphenyl)pyrazine-2-carbohydrazide (Compound 73);
*(E)*-*N'*-(3,5-bis(dimethylamino)benzylidene)-6-(4-methoxyphenyl)pyrazine-2-carbohydrazide (Compound 74);
*(E)*-*N'*-(3,5-dimethylbenzylidene)-6-(4-methoxyphenyl)pyrazine-2-carbohydrazide (Compound 75);
*(E)*-*N'*-(*3,5-di-tert-butylbenzylidene*)-6-(4-methoxyphenyl)pyrazine-2-carbohydrazide (Compound 76);
*(E)*-6-(4-methoxyphenyl)-*N'*-(pyridin-3-ylmethylene)pyrazine-2-carbohydrazide (Compound 77);
*(E)*-*N'*-(2,4-dimethoxybenzylidene)-6-(4-methoxyphenyl)pyrazine-2-carbohydrazide (Compound 78);
*(E)*-*N'*-(2,6-dimethoxybenzylidene)-6-(4-methoxyphenyl)pyrazine-2-carbohydrazide (Compound 79);
*(E)*-6-(4-methoxyphenyl)-*N'*-(2,3,5-trimethoxybenzylidene)pyrazine-2-carbohydrazide (Compound 80);
*(E)*-6-(4-methoxyphenyl)-*N'*-(3,4,5-trimethoxybenzylidene)pyrazine-2-carbohydrazide (Compound 81);
*(E)*-6-(4-methoxyphenyl)-*N'*-(2,3,4-trimethoxybenzylidene)pyrazine-2-carbohydrazide (Compound 82);
6-(4-butoxyphenyl)-*N'*-[*(E)*-(3,5-dimethoxyphenyl)methylidene]pyrazine-2-carbohydrazide (Compound 83);
*(E)*-6-(4-butoxyphenyl)-*N'*-(3,5-dimethylbenzylidene)pyrazine-2-carbohydrazide (Compound 84);
*(E)*-*N'*-(3,5-dimethoxybenzylidene)-6-(4-isobutoxyphenyl)pyrazine-2-carbohydrazide (Compound 85);
*(E)*-*N'*-(3,5-dimethylbenzylidene)-6-(4-isobutoxyphenyl)pyrazine-2-carbohydrazide (Compound 86);
*(E)*-6-(4-(tert-butoxy)phenyl)-*N'*-(3,5-dimethoxybenzylidene)pyrazine-2-carbohydrazide (Compound 87);
*(E)*-6-(4-cyclopropoxyphenyl)-*N'*-(3,5-dimethoxybenzylidene)pyrazine-2-carbohydrazide (Compound 88);
*(E)*-6-(4-(cyclohexyloxy)phenyl)-*N'*-(3,5-dimethoxybenzylidene)pyrazine-2-carbohydrazide (Compound 89);
*(E)*-*N'*-(3,5-dimethoxybenzylidene)-3-(4-methoxyphenyl)pyrazine-2-carbohydrazide (Compound 90);
*(E)*-*N'*-(3,5-dimethoxybenzylidene)-5-(4-methoxyphenyl)pyrazine-2-carbohydrazide (Compound 91);
*(E)*-*N'*-(3,5-dimethoxybenzylidene)-6-(4-fluorophenyl)pyrazine-2-carbohydrazide (Compound 92);
*(E)*-6-(3-chlorophenyl)-N'-(3,5-dimethoxybenzylidene)pyrazine-2-carbohydrazide (Compound 93);
*(E)*-*N'*-(3,5-dimethoxybenzylidene)-6-(3-fluorophenyl)pyrazine-2-carbohydrazide (Compound 94);
*(E)*-6-(2-chlorophenyl)-*N'*-(3,5-dimethoxybenzylidene)pyrazine-2-carbohydrazide (Compound 95);
*(E)*-*N'*-(3,5-dimethoxybenzylidene)-6-(p-tolyl)pyrazine-2-carbohydrazide (Compound 96);
*(E)*-*N'*-(3,5-dimethoxybenzylidene)-6-phenylpyrazine-2-carbohydrazide (Compound 97);
*(E)*-*N'*-(3,5-dimethoxybenzylidene)-6-(2-fluorophenyl)pyrazine-2-carbohydrazide (Compound 98);
*(E)*-*N'*-(3,5-dimethoxybenzylidene)-6-(o-tolyl)pyrazine-2-carbohydrazide (Compound 99);
*(E)*-*N'*-(3,5-dimethoxybenzylidene)-6-(m-tolyl)pyrazine-2-carbohydrazide (Compound 100);
*(E)*-*N'*-(3,5-dimethoxybenzylidene)-6-(3-methoxyphenyl)pyrazine-2-carbohydrazide (Compound 101);
*(E)*-*N'*-(3,5-dimethoxybenzylidene)-6-(2-methoxyphenyl)pyrazine-2-carbohydrazide (Compound 102);
*(E)*-*N'*-(3,5-dimethoxybenzylidene)-6-(pyridin-4-yl)pyrazine-2-carbohydrazide (Compound 103);
*(E)*-*N'*-(3,5-dimethoxybenzylidene)-6-(pyridin-3-yl)pyrazine-2-carbohydrazide (Compound 104);
*(E)*-6-(4-ethoxy-2-methoxyphenyl)-*N'*-(3-fluoro-5-methoxybenzylidene)pyrazine-2-carbohydrazide (Compound 105);
*(E)*-6-(4-ethoxy-2-methoxyphenyl)-*N'*-(3-methoxy-5-propoxybenzylidene)pyrazine-2-carbohydrazide (Compound

106);
*(E)*-6-(4-ethoxy-2-methoxyphenyl)-*N'*-(4-fluoro-3,5-dimethoxybenzylidene)pyrazine-2-carbohydrazide (Compound 107);
*(E)*-6-(4-ethoxy-2-methoxyphenyl)-*N'*-(3-methoxybenzylidene)pyrazine-2-carbohydrazide (Compound 108);
*(E)*-*N'*-(3,5-dimethoxybenzylidene)-6-(4-methoxy-2-methylphenyl)pyrazine-2-carbohydrazide (Compound 109);
*(E)*-6-(2-chloro-4-ethoxyphenyl)-*N'*-(3,5-dimethoxybenzylidene)pyrazine-2-carbohydrazide (Compound 110);
*(E)*-6-(2-chloro-4-ethoxyphenyl)-*N'*-(3-methoxybenzylidene)pyrazine-2-carbohydrazide (Compound 111);
*(E)*-*N'*-(3,5-dimethoxybenzylidene)-6-(4-methoxy-2-(trifluoromethoxy)phenyl)pyrazine-2-carbohydrazide (Compound 112);
*(E)*-*N'*-(3,5-dimethoxybenzylidene)-6-(4-propylphenyl)pyrazine-2-carbohydrazide (Compound 113);
*(E)*-*N'*-(3,5-dimethoxybenzylidene)-6-(2-morpholinophenyl)pyrazine-2-carbohydrazide (Compound 114);
*(E)*-6-(2-butoxyphenyl)-*N'*-(3,5-dimethoxybenzylidene)pyrazine-2-carbohydrazide (Compound 115);
*(E)*-*N'*-(3,5-dimethoxybenzylidene)-6-(4-isobutylphenyl)pyrazine-2-carbohydrazide (Compound 116);
*(E)*-6-(3-chloro-4-ethoxyphenyl)-*N'*-(3,5-dimethoxybenzylidene)pyrazine-2-carbohydrazide (Compound 117);
*(E)*-*N'*-(3,5-dimethoxybenzylidene)-6-(4-(ethylthio)phenyl)pyrazine-2-carbohydrazide (Compound 118);
*(E)*-6-(4-ethoxy-2-fluorophenyl)-*N'*-(3-methoxybenzylidene)pyrazine-2-carbohydrazide (Compound 119);
*(E)*-6-(4-ethoxy-2-(trifluoromethyl)phenyl)-*N'*-(3-methoxybenzylidene)pyrazine-2-carbohydrazide (Compound 120);
*(E)*-*N'*-(3,5-dimethoxybenzylidene)-6-(2-fluoro-4-methoxyphenyl)pyrazine-2-carbohydrazide (Compound 121);
*(E)*-6-(2-chloro-4-methoxyphenyl)-*N'*-(3,5-dimethoxybenzylidene)pyrazine-2-carbohydrazide (Compound 122);
*(E)*-*N'*-(3,5-dimethoxybenzylidene)-6-(4-methoxy-2-(trifluoromethyl)phenyl)pyrazine-2-carbohydrazide (Compound 123);
*(E)*-*N'*-(3,5-dimethoxybenzylidene)-6-(2,4-dimethoxyphenyl)pyrazine-2-carbohydrazide (Compound 124);
*(E)*-*N'*-(3,5-dimethoxybenzylidene)-6-(pyridin-2-yl)pyrazine-2-carbohydrazide (Compound 125);
*(E)*-*N'*-(3,5-dimethoxybenzylidene)-6-morpholinopyrazine-2-carbohydrazide (Compound 126);
*(E)*-*N'*-(3,5-dimethoxybenzylidene)-6-(piperidin-1-yl)pyrazine-2-carbohydrazide (Compound 127);
*(E)*-*N'*-(3,5-dimethoxybenzylidene)-6-(4-propylpiperazin-1-yl)pyrazine-2-carbohydrazide (Compound 128);
*(E)*-*N'*-(3,5-dimethoxybenzylidene)-3-(4-methoxyphenoxy)pyrazine-2-carbohydrazide (Compound 129);
*(E)*-*N'*-(3,5-dimethoxybenzylidene)-5-(4-methoxyphenoxy)pyrazine-2-carbohydrazide (Compound 130);
*(E)*-*N'*-(3,5-dimethoxybenzylidene)-6-(4-ethoxyphenoxy)pyrazine-2-carbohydrazide (Compound 131);
*(E)*-*N'*-(3,5-dimethoxybenzylidene)-3-ethylpyrazine-2-carbohydrazide (Compound 132);
*(E)*-*N'*-(3,5-dimethoxybenzylidene)-6-ethylpyrazine-2-carbohydrazide (Compound 133);
*(E)*-*N'*-(3,5-dimethoxybenzylidene)-6-isopropylpyrazine-2-carbohydrazide (Compound 134);
*(E)*-*N'*-(3,5-dimethoxybenzylidene)-3-isopropylpyrazine-2-carbohydrazide (Compound 135);
*(E)*-*N'*-(3,5-dimethoxybenzylidene)-5-isopropylpyrazine-2-carbohydrazide (Compound 136);
(*E*)-*N*'-(3,5-dimethoxybenzylidene)-6-methylpyrazine-2-carbohydrazide (Compound 137);
*(E)*-*N'*-(3,5-dimethoxybenzylidene)-5-methylpyrazine-2-carbohydrazide (Compound 138);
*(E)*-*N'*-(3,5-dimethoxybenzylidene)-3-methylpyrazine-2-carbohydrazide (Compound 139);
*(E)*-*N'*-(3,5-dimethoxybenzylidene)-6-methoxypyrazine-2-carbohydrazide (Compound 140);
*(E)*-*N'*-(3,5-dimethoxybenzylidene)-3-methoxypyrazine-2-carbohydrazide (Compound 141);
*(E)*-*N'*-(3,5-dimethoxybenzylidene)-5-methoxypyrazine-2-carbohydrazide (Compound 142);
*(E)*-*N'*-(3,5-dimethoxybenzylidene)-6-(4-ethoxyphenyl)-3-methylpyrazine-2-carbohydrazide (Compound 143);
*(E)*-*N'*-(3,5-dimethoxybenzylidene)-6-(4-ethoxyphenyl)-3-ethylpyrazine-2-carbohydrazide (Compound 144);
*(E)*-*N'*-(3,5-dimethoxybenzylidene)-6-(4-ethoxyphenyl)-3-methoxypyrazine-2-carbohydrazide (Compound 145);
*(E)*-*N'*-(3,5-dimethoxybenzylidene)-6-(4-ethoxyphenyl)-3-phenoxypyrazine-2-carbohydrazide (Compound 146);
*(E)*-*N'*-(3,5-dimethoxybenzylidene)-6-(4-ethoxyphenyl)-3-phenylpyrazine-2-carbohydrazide (Compound 147);
*(E)*-6-(4-cyclobutoxyphenyl)-*N'*-(3,5-dimethoxybenzylidene)pyrazine-2-carbohydrazide (Compound 148);
*(E)*-*N'*-(3,5-dimethoxybenzylidene)-6-(4-((methylthio)methoxy)phenyl)pyrazine-2-carbohydrazide (Compound 149);
*(E)*-6-(4-(cyclopentyloxy)phenyl)-*N'*-(3,5-dimethylbenzylidene)pyrazine-2-carbohydrazide (Compound 150);
*(E)*-*N'*-(3,5-dimethoxybenzylidene)-6-(4-(2-(dimethylamino)ethoxy)phenyl)pyrazine-2-carbohydrazide (Compound 151);
*(E)* -6-(4-(cyclopropylmethoxy)phenyl) -*N'*-(3,5-dimethoxybenzylidene)pyrazine-2-carbohydrazide (Compound 152);
*(E)*-6-(2,4-diethoxyphenyl)-*N'*-(3-methoxybenzylidene)pyrazine-2-carbohydrazide (Compound 153);
*(E)*-6-(4-ethoxy-2-isopropoxyphenyl)-*N'*-(3-methoxybenzylidene)pyrazine-2-carbohydrazide (Compound 154);
*(E)*-*N'*-(3,5-dimethoxybenzylidene)-6-(2-methoxyphenyl)pyrazine-2-carbohydrazide (Compound 155);
*(E)*-*N'*-(3,5-dimethoxybenzylidene)-6-(2-isopropoxy-4-methoxyphenyl)pyrazine-2-carbohydrazide (Compound 156);

*(E)*-6-(2-chloro-4-(cyclopentyloxy)phenyl)-*N'*-(3,5-dimethoxybenzylidene)pyrazine-2-carbohydrazide (Compound 157);
*(E)*-6-(4-butoxy-2-chlorophenyl)-*N'*-(3,5-dimethoxybenzylidene)pyrazine-2-carbohydrazide (Compound 158);
*(E)*-6-(2-chloro-4-isobutoxyphenyl)-*N'*-(3,5-dimethoxybenzylidene)pyrazine-2-carbohydrazide (Compound 159);
*(E)*-*N'*-(3,5-dimethoxybenzylidene)-6-(2-(2-(dimethylamino)ethoxy)-4-methoxyphenol) pyrazine-2-carbohydrazide (Compound 160);
*(E)*-6-(2-(cyclopentyloxy)phenyl)-*N'*-(3,5-dimethoxybenzylidene)pyrazine-2-carbohydrazide (Compound 161);
*(E)*-*N'*-(3,5-dimethoxybenzylidene)-6-(4-(ethylamino)phenyl)pyrazine-2-carbohydrazide (Compound 162);
*(E)*-6-chloro-N'-(3,5-dimethoxybenzylidene)pyrazine-2-carbohydrazide (Compound 163);
*(E)*-5-chloro-N'-(3,5-dimethoxybenzylidene)pyrazine-2-carbohydrazide (Compound 164);
*(E)*-3-chloro-N'-(3,5-dimethoxybenzylidene)pyrazine-2-carbohydrazide (Compound 165);
*(E)*-*N'*-(1-(3,5-dimethoxyphenyl)ethylidene)-6-(4-ethoxyphenyl)pyrazine-2-carbohydrazide (Compound 166);
*(E)*-*N'*-(3,5-dimethoxybenzylidene)-5-ethylpyrazine-2-carbohydrazide (Compound 167);
*(E)*-*N'*-(3,5-dimethoxybenzylidene)-6-(4-methoxyphenyl)-N-methylpyrazine-2-carbohydrazide (Compound 168);
*(E)*-6-(4,4-difluoro-3-methylpiperidin-1-yl)-*N'*-(3,5-dimethoxybenzylidene)pyrazine-2-carbohydrazide (Compound 169);
*(E)*-*N'*-benzylidene-6-(4-ethoxyphenyl)pyrazine-2-carbohydrazide (Compound 170);
*(E)*-*N'*-(cyclohexylmethylene)-6-(4-ethoxyphenyl)pyrazine-2-carbohydrazide (Compound 171);
*(E)*-*N'*-((4,4-difluorocyclohexyl)methylene)-6-(4-ethoxyphenyl)pyrazine-2-carbohydrazide (Compound 172);
*(E)*-*N'*-(cyclohexylmethylene)-6-(4-methoxyphenyl)pyrazine-2-carbohydrazide (Compound 173);
*(E)*-*N'*-((4,4-difluorocyclohexyl)methylene)-6-(4-methoxyphenyl)pyrazine-2-carbohydrazide (Compound 174);
*(E)*-*N'*-(cyclohexylmethylene)-6-(4-isopropoxyphenyl)pyrazine-2-carbohydrazide (Compound 175);
*(E)*-*N'*-((4,4-difluorocyclohexyl)methylene)-6-(4-isopropoxyphenyl)pyrazine-2-carbohydrazide (Compound 176);
*(E)*-6-(4-(difluoromethyl)phenyl)-*N'*-(3,5-dimethoxybenzylidene)pyrazine-2-carbohydrazide (Compound 177);
*(E)*-*N'*-benzylidene-6-(4-methoxyphenyl)pyrazine-2-carbohydrazide (Compound 178);
*(E)*-6-(4,4-difluoroazepan-1-yl)-*N'*-(3,5-dimethoxybenzylidene)pyrazine-2-carbohydrazide (Compound 179);
*(E)*-6-(4,4-difluoropiperidin-1-yl)-*N'*-(3,5-dimethoxybenzylidene)pyrazine-2-carbohydrazide (Compound 180);
*(E)*-*N'*-(cycloheptylmethylene)-6-(4-methoxyphenyl)pyrazine-2-carbohydrazide (Compound 181);
*(E)*-*N'*-(cycloheptylmethylene)-6-(4-ethoxyphenyl)pyrazine-2-carbohydrazide (Compound 182);
*(E)*-*N'*-(cycloheptylmethylene)-6-(4-isopropoxyphenyl)pyrazine-2-carbohydrazide (Compound 183);
*(E)*-*N'*-(3,5-dimethoxybenzylidene)-6-(6-methoxypyridin-3-yl)pyrazine-2-carbohydrazide (Compound 184);
*(E)*-*N'*-(3,5-dimethoxybenzylidene)-6-(5-methoxypyridin-2-yl)pyrazine-2-carbohydrazide (Compound 185);
*(E)*-6-(4-(cyclopentyloxy)-2-(trifluoromethyl)phenyl)-*N'*-(3,5-dimethoxybenzylidene) pyrazine-2-carbohydrazide (Compound 186);
*(E)*-*N'*-(3,5-dimethoxybenzylidene)-6-(4-ethoxy-2-(trifluoromethyl)phenyl)pyrazine-2-carbohydrazide (Compound 187);
*(E)*-4-((2-(6-(4-ethoxyphenyl)pyrazine-2-carbonyl)hydrazinalidene)methyl)-*N*-(methyl sulfonyl)benzamide (Compound 188);
*(E)*-3-((2-(6-(4-ethoxyphenyl)pyrazine-2-carbonyl)hydrazinaylidene)methyl)-*N*-(methyl sulfonyl)benzamide (Compound 189);
*(E)*-4-(6-(2-(3,5-dimethoxybenzylidene)hydrazine-1-carbonyl)pyrazin-2-yl)-*N*-(methyl sulfonyl)benzamide (Compound 190);
*(E)*-6-(4-ethoxyphenyl)-*N'*-(2-((methylsulfonyl)methyl)benzylidene)pyrazine-2-carbohydrazide (Compound 191);
*(E)*-6-(4-ethoxyphenyl)-*N'*-(2-(methylsulfonyl)benzylidene)pyrazine-2-carbohydrazide (Compound 192);
*(E)*-*N*-(2-((2-(6-(4-ethoxyphenyl)pyrazine-2-carbonyl)hydrazinalidene)methyl)phenyl) methanesulfonamide (Compound 193);
*(E)*-6-(4-methoxyphenyl)-N'-((2-methylcyclopent-1-en-1-yl)methylene)pyrazine-2-carbohydrazide (Compound 194);
*(E)*-6-(4-ethoxyphenyl)-*N'*-((2-methylcyclopent-1-en-1-yl)methylene)pyrazine-2-carbohydrazide (Compound 195);
*(E)*-6-(4-isopropoxyphenyl)-*N'*-((2-methylcyclopent-1-en-1-yl)methylene)pyrazine-2-carbohydrazide (Compound 196);
*(E)*-6-(4-methoxyphenyl)-*N'*-((2-methylcyclohex-1-en-1-yl)methylene)pyrazine-2-carbohydrazide (Compound 197);
*(E)*-6-(4-ethoxyphenyl)-*N'*-((2-methylcyclohex-1-en-1-yl)methylene)pyrazine-2-carbohydrazide (Compound 198);
*(E)*-6-(4-isopropoxyphenyl)-*N'*-((2-methylcyclohex-1-en-1-yl)methylene)pyrazine-2-carbohydrazide (Compound 199);
*(E)*-6-(4-methoxyphenyl)-*N'*-((2-methylcyclohept-1-en-1-yl)methylene)pyrazine-2-carbohydrazide (Compound 200);
*(E)*-6-(4-ethoxyphenyl)-*N'*-((2-methylcyclohept-1-en-1-yl)methylene)pyrazine-2-carbohydrazide (Compound 201);

*(E)*-6-(4-isopropoxyphenyl)-*N'*-((2-methylcyclohept-1-en-1-yl)methylene)pyrazine-2-carbohydrazide (Compound 202);
*(E)*-*N'*-(3-(2-hydroxyethyl)-5-methoxybenzylidene)-6-(4-methoxyphenyl)pyrazine-2-carbohydrazide (Compound 203);
*(E)*-*N'*-(3-(1-hydroxyethyl)-5-methoxybenzylidene)-6-(4-methoxyphenyl)pyrazine-2-carbohydrazide (Compound 204);
*(E)*-6-(2,6-dichloro-4-ethoxyphenyl)-*N'*-(3,5-dimethoxybenzylidene)pyrazine-2-carbohydrazide (Compound 205);
*(E)*-5-amino-N'-(3,5-dimethoxybenzylidene)-6-(4-ethoxyphenyl)pyrazine-2-carbohydrazide (Compound 206);
*(E)*-*N'*-(3,5-dimethoxybenzylidene)-6-(4-ethoxyphenyl)-5-methylpyrazine-2-carbohydrazide (Compound 207);
*(E)*-*N'*-(3,5-dimethoxybenzylidene)-6-(4-ethoxyphenyl)-5-ethylpyrazine-2-carbohydrazide (Compound 208);
*(E)*-5-chloro-*N'*-(3,5-dimethoxybenzylidene)-6-(4-ethoxyphenyl)pyrazine-2-carbohydrazide (Compound 209);
*(E)*-*N'*-(3,5-dimethoxybenzylidene)-6-(4-ethoxyphenyl)-5-(trifluoromethyl)pyrazine-2-carbohydrazide (Compound 210);
*(E)*-*N'*-(3,5-dimethoxybenzylidene)-6-(4-ethoxyphenyl)-5-methoxypyrazine-2-carbohydrazide (Compound 211);
*(E)*-*N'*-(3,5-dimethoxybenzylidene)-5-ethoxy-6-(4-ethoxyphenyl)pyrazine-2-carbohydrazide (Compound 212);
*(E)*-6-(4-ethoxy-2,6-dimethylphenyl)-*N'*-(3-methoxybenzylidene)pyrazine-2-carbohydrazide (Compound 213);
*(E)*-*N'*-(3-(2-hydroxyethyl)-5-methoxybenzylidene)-6-(4-ethoxyphenyl)pyrazine-2-carbohydrazide (Compound 214); and,
*(E)*-*N'*-(3-(1-hydroxyethyl)-5-methoxybenzylidene)-6-(4-ethoxyphenyl)pyrazine-2-carbohydrazide (Compound 215).

In one embodiment, the Formula (I) compound (s) are selective inhibitors of the voltage-gated sodium channels Nav 1.7 and/or Nav 1.8. In a preferred embodiment, the compound(s) Formula (I) are dual inhibitors of the voltage-gated sodium channels Nav 1.7 and Nav 1.8.
In some embodiments, the compound(s) of Formula (I) may be basic in nature, and accordingly pharmaceutically acceptable salts may be obtained by the addition of organic or inorganic acids. Non-limiting examples of organic acids that may be used are fumaric, maleic, benzoic, lactic acids, among others. Among the inorganic acids may be mentioned hydrochloric acid, phosphoric acid, hydrobromic acid, sulfuric acid, nitric acid, among others.
In some embodiments, the compound (s) of Formula (I) may be obtained in the form of crystals, which may optionally be presented as pharmaceutically acceptable solvates, wherein the solvent is incorporated in stoichiometric proportions or not into the crystal lattice. In further embodiments, the crystallization solvent is water, resulting in pharmaceutically acceptable hydrates.
Finally, in some embodiments, the compound(s) of Formula (I) may exhibit more than one isomer, including without limitation, spatial isomerism such as geometric and optical isomerism.

## Definitions

For the purpose of clarity or elucidation of the terms used in the present invention, the following definitions are presented, and the scope is not limited thereto.
The term "straight or branched C1-6alkyl" refers to saturated straight or branched chain hydrocarbons such as methyl, ethyl, propyl, butyl, isopropyl, *tert-butyl*, pentyl, hexyl, not limited thereto.
The term "saturated or unsaturated C3-7cycloalkyl" refers to saturated or unsaturated alkyl chain cycles. Non-limiting examples are: cyclohexyl, cycloheptyl, cyclopropyl, cyclobutyl, cyclopentyl, cyclopentenyl, cycloheptenyl, cyclobutenyl, cyclopropenyl, and cyclohexenyl.
The term "C1-6 saturated or unsaturated alkylsubstituted or halosubstituted C3-7cycloalkyl " refers to groups such as 2-methylcyclopent-1-en-1-yl; 2-methylcyclohex-1-en-1-yl; 2-methylcyclohept-1-en-1-yl, 2-methylcyclopentyl; 2-methylcyclohexyl; 2-methylcycloheptyl, 2-ethylcyclopent-1-en-1-yl; 2-ethylcyclohex-1-en-1-yl; 2-ethylcyclohept-1-en-1-yl, 2-propylcyclopent-1-en-1-yl; 2-propylcyclohex-1-en-1-yl; 2-propylcyclohept-1-en-1-yl, 4,4-difluorocyclohexyl, 4,4-dichlorocyclohexyl, 3,3-difluorocyclopentyl, 3,3-difluorocyclobutyl, 3,3-difluorocyclopropyl, 3,3-dichlorocyclopentyl, 3,3-dichlorocyclobutyl, 2,2-dichlorocyclopropyl, 1-(trifluoromethyl)cyclopropyl, not limited thereto.
The term "linear or branched C1-6 alkoxy" refers to alkyl groups bonded to a radical oxygen, including methoxy, ethoxy, propoxy, butoxy, pentoxy, hexoxy, isoproxy, isobutoxy , *tert-butoxy*, and is not limited thereto.
The term "-OCD3" refers to alkyl groups bonded to a radical oxygen, wherein D refers to a deuterium atom.
The term "C1-6 alkylamino" refers to groups comprising an amine linked to an alkyl chain in its structure, such as: methylamino, dimethylamino, diethylamino, propylamino, butylamino, isopropylamino, not limited thereto.
The term "pyridinyl" refers to a pyridine ring and isomers thereof that can be used as substituents. Non-limiting examples are: pyridin-2-yl, pyridin-4-yl, and pyridin-3-yl. Additionally, the term "substituted pyridinyl" refers to substituted pyridine rings such as 6-methoxypyridin-3-yl, 5-methoxypyridin-3-yl, 5-methoxypyridin-2-yl, 4-methoxypyridin-2-yl, 2-methoxypyridin-4-yl, 6-methoxypyridin-2-yl, and is not limited thereto.

The terms "piperidinyl or substituted piperidinyl" refer to unsubstituted or substituted piperidine rings, which when substituted may have one or more substituents selected from halogens or one to six-carbon alkyl chains. Non-limiting examples are: piperidin-1-yl; piperidin-2-yl; piperidin-3-yl; piperidin-4-yl; 4,4-difluoro-3-methylpiperidin-1-yl; 4,4-difluoropiperidin-1-yl; 4,4-dichloro-3-methylpiperidin-1-yl, 4,4-dibromo-3-methylpiperidin-1-yl, among others, not limited thereto.

The terms "piperazin or substituted piperazin" refer to unsubstituted or substituted piperazine rings. Non-limiting examples are: piperazin-1-yl, piperazin-2-yl; piperazin-3-yl, 4-methylpiperazin-1-yl; 4-ethylpiperazin-1-yl; 4-propylpiperazin-1-yl; 4-butylpiperazin-1-yl; 4-pentylpiperazin-1-yl; 4-hexylpiperazin-1-yl; 4-isopropylpiperazin-yl and 4-isobutylpiperazin-1-yl.

The term "(C1-6 alkylamino) C1-6 alkoxy" refers to groups such as 2-(dimethylamino)methoxy; 2-(dimethylamino)ethoxy; 2-(dimethylamino)propoxy; 2-(dimethylamino)butoxy, not limited thereto.

The term "(C1-6 alkylamino) C1-6alkyl" refers to groups such as (dimethylamino)methyl, (methylamino)methyl, (ethylamino)methyl, (ethylamino)ethyl, (dimethylamino)ethyl, (dimethylamino)propyl, (dimethylamino)butyl, not limited thereto.

The term "C3-6 cycloalkyloxy" refers to groups such as cyclopropoxy; cyclopentyloxy; cyclohexyloxy; cyclobutyloxy, not limited thereto.

The term "-S-C1-6alkyl" refers to alkyl groups attached to a radical sulfur, including groups such as -S-methyl, -S-ethyl, -S-propyl, -S-butyl, -S-pentyl, -S-isopropyl, -S-isobutyl, -S-tert-butyl, -S-sec-butyl, not limited thereto.

The term "C1-6haloalkyl" refers to alkyl groups attached to a halogen, including groups such as trifluoromethyl, difluoromethyl, fluoro-methyl, chloromethyl, dichloromethyl, trichloromethyl, tribromomethyl, tribromobutyl, bromomethyl, not limited thereto.

The term "C1-6haloalkyloxy" refers to alkoxy groups bonded to a halogen, including groups such as trifluoromethoxy, difluoromethoxy, fluoromethoxy, chloromethoxy, dichloromethoxy, trichloromethoxy, 2,2,2-trifluoroethoxy, 2,2-difluoroethoxy, 2-fluoroethoxy, 2-chloroethoxy, 2,2-dichloroethoxy, 2,2,2-trichloroethoxy, 3,3,3-trifluoropropoxy, 3,3-difluoropropoxy, 3-fluoroproxy, 3-chloropropoxy, 3,3-dichloropropoxy, 3,3,3-trichloropropoxy, 4,4,4-trifluorobutoxy, 5,5,5-trifluoropentoxy, not limited thereto.

The term "(C3-6cycloalkyl) C1-6 alkoxy" refers to groups such as cyclopropylmethoxy, cyclopropylethoxy, cyclopropylpropoxy, cyclopropyl butoxy, cyclobutylmethoxy, cyclobutylethoxy, cyclobutylpropoxy, cyclobutylbutoxy, cyclopentylmethoxy, cyclopentylethoxy, cyclopentylpropoxy, cyclohexylmethoxy, not limited thereto.

The term "C1-6alkylsufonyl" refers to methylsufonyl, ethylsufonyl, propylsufonyl, isopropylsufonyl, butylsufonyl groups, not limited thereto.

The term "(C1-6 alkylsufonyl) C1-6alkyl" refers to groups such as (methylsulfonyl)methyl), (ethylsulfonyl)methyl), (propylsulfonyl)methyl), (butylsulfonyl)methyl), (methylsulfonyl)ethyl), (methylsulfonyl)propyl), (methylsulfonyl)butyl), not limited thereto.

The term "C1-6 hydroxyalkyl" refers to groups such as 2-hydroxyethyl; 1-hydroxyethyl, hydroxymethyl, 1-hydroxypropyl, 2-hydroxypropyl, 1-hydroxybutyl, 2-hydroxybutyl, not limited thereto.

The terms "aryl" or "substituted aryl" refer to aromatic groups such as phenyl, benzyl, 4-methoxyphenyl, 3-methoxyphenyl, 2-methoxyphenyl, 4-ethoxyphenyl, 3-ethoxyphenyl, 4-chlorophenyl, 3-chlorophenyl, 2-chlorophenyl, 4-fluorophenyl, 3-fluorophenyl, 4-bromophenyl, 3-bromophenyl, 3-methoxybenzyl, 2-methoxybenzyl, 4-ethoxybenzyl, 3-ethoxybenzyl, not limited thereto.

The terms "aryloxy" or "substituted aryloxy" refer to aromatic groups substituted with a radical oxygen, such as phenoxy, benzoxy, 4-ethoxyphenoxy; 4-methoxyphenoxy; 3-methoxyphenoxy, 3-ethoxyphenoxy, 4-chlorophenoxy, 3-chlorophenoxy, 4-fluorophenoxy, 3-fluorophenoxy, 4-bromophenoxy, 3-bromophenoxy, not limited thereto.

The term "C3-6 heterocycloalkyl" refers to group as 4,4-difluoroazepan-1-yl, 4-oxoazepan-1-yl, 1-oxido-1,4-thiazepan-4-yl, 1,1-dioxido-1,4-thiazepan-4-yl, 1,4-diazepan-1-yl, 1,4-oxazepan-4-yl, 1,4-thiazepan-4-yl, 4-methyl-1,4-diazepan-1-yl, 4,4-difluoro-3,5-dimethylcyclohexyl, 4,4-dichloro-3-methylcyclohexyl, 4,4-difluoro-3,5-dimethylpiperidin-1-yl, 4,4-dichloro-3-methylpiperidin-1-yl, 4,4-difluoro-3-methylpiperidin-1-yl, 6-azaspiro[2.5]octan-6-yl, 4,4-difluoropiperidin-1-yl, 4,4-dichloropiperidin-1-yl, 2-methyl-6-(trifluoromethyl)morpholino, 2-methyl-6-(trifluoromethyl)tetrahydro-2H-pyran-4-yl, 4-propylcyclohexyl, 4-ethoxycyclohexyl, 3-methoxybicyclo[1.1,1]pentan-1-yl, (2r,3R,4r,5S) -4-methoxycuban-1-yl), (1R,5S) -3-methoxybicyclo [3.1.1]heptan-6-yl, not limited thereto.

The term "(-S-C1-6-alkyl) C1-6 alkyloxy" refers to groups such as (-S-methyl)methoxy, (-S-ethyl)methoxy, (-S-methyl)ethoxy, (-S-methyl)propoxy, (-S-methyl)butoxy, (-S-ethyl)methoxy, and is not limited thereto.

In a second embodiment, the present invention features a composition comprising a therapeutically effective amount of compound(s) of Formula (I) of the present invention or a pharmaceutically acceptable salt, hydrate, solvate, and isomerthereof; and one or more pharmaceutically acceptable excipients.

Pharmaceutically acceptable excipients are considered to be any substance, other than the active pharmaceutical ingredient, that has been evaluated for safety and that is intentionally added to the dosage form. Such excipients

are selected according to the pharmaceutical dosage form of interest, its route of administration, physicochemical compatibility with the active ingredient, and the effect on efficacy.

Furthermore, said excipients are widely known in the state of art and are classified according to their function, including without limitation diluents, binders, disintegrants or disaggregants, lubricants, suspending agents, thickeners, solvents, surfactants, glidants, anti-caking or flowing agents, coating agents, plasticizers, sweeteners, sweeteners, isotonicity agents, dyes and pigments, preservatives, antioxidants, pH modifying or control agents, complexing agents, chelating agents, flavoring, flavoring, viscosity modifying agents, opacifiers, permeation promoters, among others.

The pharmaceutical compositions may be administered by various routes including, but not limited to, oral, sublingual, nasal, parenteral, injectable, submuscular, topical, transdermal, ocular, rectal.

In a third embodiment, the present invention features the use of compound(s) of Formula (I) or a pharmaceutically acceptable salt, hydrate, solvate, and isomer thereof to prepare a medicament for treating neuropathic pain-related pathologies.

In a preferred embodiment, said pathologies are selected from the group consisting of peripheral neuropathic pain, chemotherapy-induced neuropathy, complex regional pain, neuropathy related to viral infection, neuropathy secondary to tumor infiltration, diabetic neuropathy, phantom limb pain, postherpetic neuralgia, trigeminal neuralgia and postsurgical neuralgia.

In a fourth embodiment, the present invention features a method of treating, preventing, alleviating, suppressing, and/or controlling neuropathic pain-related pathologies comprising administering an effective amount of compound(s) of Formula (I) or a pharmaceutically acceptable salt, hydrate, solvate, and isomer thereof. In a preferred embodiment, the method is for the treatment, prevention, alleviation, suppression and/or control of peripheral neuropathic pain, chemotherapy-induced neuropathy, complex regional pain, neuropathy related to viral infection, neuropathy secondary to tumor infiltration, diabetic neuropathy, phantom limb pain, postherpetic neuralgia, trigeminal neuralgia and postsurgical neuralgia. In a further preferred embodiment, the administration of the at least one compound of Formula (I) is selected from the group comprising oral, sublingual, nasal, parenteral, injectable, submuscular, topical, transdermal, ocular, and rectal.

In a fifth embodiment of the invention, there are provided processes of obtaining compound(s) of Formula (I) comprising the following steps:

(a) Forming the intermediate of Formula III:

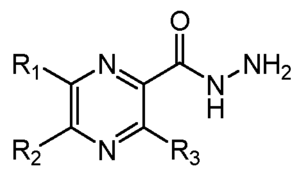

**Fórmula III**

from the hydrazinolysis reaction of an intermediate of Formula IV:

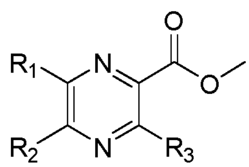

**Fórmula IV**

(b) obtaining a Formula I compound wherein R4 is hydrogen;

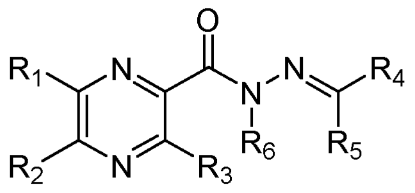

Formula (I)

from condensation of intermediates of Formula II:

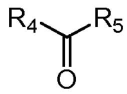

**Fórmula II**

and Formula III, with or without presence of catalyst and a suitable solvent; wherein,

- R1, R2, R3, R4, R5, R7, R8, R9, R10, R11, R12, R13, R14, R15, R16, R17 e R18 are independently selected from the groups previously described.

In one embodiment, the process further comprises a step (c) of forming compound(s) of Formula (I) whereinR6 is linear or branched C1-6alkyl from the nucleophilic substitution reaction of a compound obtained in step (b), with linear or branched C1-6alkyl halides in the presence of inorganic base and polar aprotic solvents.

In one embodiment of the process, in step (b) said catalyst is selected from concentrated hydrochloric acid, acetic acid, trifluoroacetic acid, formic acid, or combinations thereof and said solvent is selected from dimethylformamide, alcohols, or combinations thereof. In another embodiment, in step (c) said inorganic base may be selectedfrom K2CO3 or NaH.

The compound(s) of Formula (I) of the present invention were prepared from the synthetic route described in General Scheme 1. However, a person skilled in the art will readily appreciate that additional detailing and/or modifications to the arrangement of one or more steps may be performed without departing from the processes taught herein. Such variations may be, without limitation, combinations of solvents and catalysts, including stereoselectives, protecting groups, among others.

In the following General Scheme 1, the formation of the intermediates of Formula II, III and IV is described, in addition to the formation of the compound(s) of Formula (I), wherein Formula Ia corresponds to compounds wherein R4 is hydrogen and Formula Ib corresponds to compounds wherein R4 is a C1-6alkyl group (represented by R in the scheme), from these intermediates.

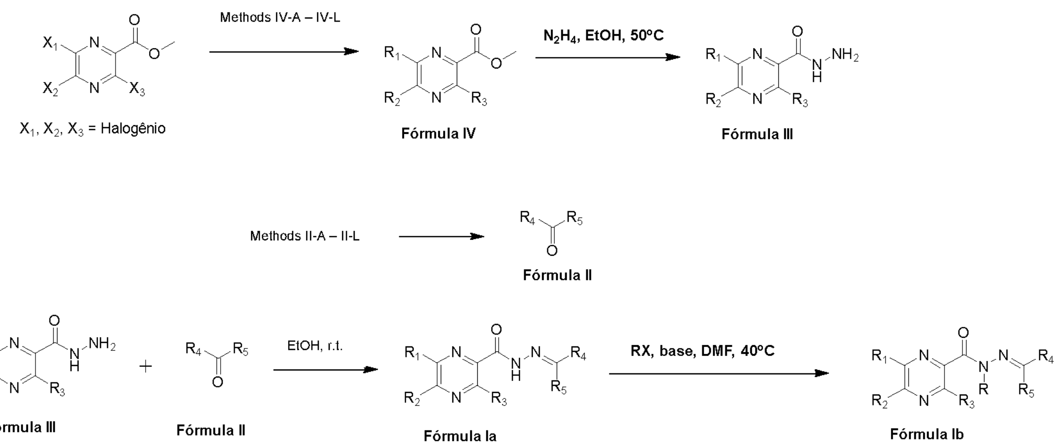


**General Scheme 1**

As illustrated in General Scheme 1, compounds of Formula Ia can be prepared from condensation reactions of the intermediates of Formula III with the intermediates of Formula II, without or upon acid catalysis using concentrated hydrochloric acid, acetic acid, trifluoroacetic acid or formic acid in suitable solvents such as DMF or alcohols such as methanol, ethanol or propanol. Compounds of Formula Ib (R = R6 being C1-6alkylgroup), can be obtained from an

additional step, starting from compounds of Formula Ia, by nucleophilic substitution reactions with corresponding alkyl halides, in the presence of an inorganic base suchas K2CO3 or NaH and polar aprotic solvents. In turn, intermediates of Formula III can be prepared by hydrazinolysis reaction from the corresponding esters (Formula IV) obtained following methods IV-A to IV-L. Intermediates of Formula II can be prepared from the corresponding reagents following methods II-A to II-L, some of which can be obtained commercially. The details of such methodologies are described below.

**Obtaining the intermediates of Formula IV:**

Following General Scheme 1, methods IV-A to IV-L for the formation of intermediates of Formula IV are presented, but such methods are not limiting.

**Method IV-A:**

*Intermediates IV-1 to IV-44 / : IV-82 to IV-93*

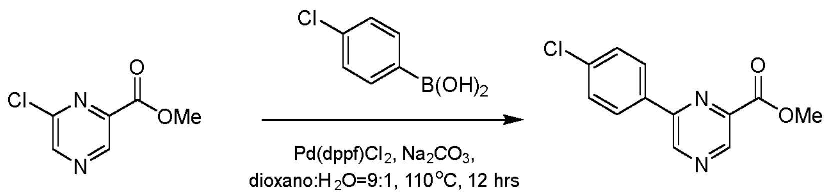


Into a round-bottom flask were added 14.4 mmol of methyl 6-chloropyrazine-2-carboxylate, 21.7 mmol of 4-chlorophenylboronic acid, 724 μmol of Pd(dppf)Cl2, 43.4 mmol of Na2CO3, 27 mL of dioxane, and 3 mL of H2O. Subsequently, the reaction mixture was de-gassed and allowed to stir at 110 °C for 12 hours under N2 atmosphere. Subsequently, the reaction mixture was concentrated under reduced pressure to obtain an oil, which was washed with distilled water (25 mL) and extracted with ethyl acetate (3 x 10 mL). The organic phases were pooled, dried over anhydrous magnesium sulfate, filtered and concentrated to obtain 1.8 g (50% yield) of intermediate IV-1, which was used for the next step without further purification. 1H-RMN (600 MHz, DMSO-d6) $\delta$ = 9,50 (s, 1H), 9,15 (s, 1H), 8,22 (d, J = 8,6 Hz, 2H), 7,64 (d, J = 8,6 Hz, 2H), 3,94 (s, 3H). This procedure is used to obtain the intermediates in Table 1.

For the synthesis of intermediates IV-15, IV-16 and IV-17, the solvent was replaced with DMSO and the base with K2CO3.

For the synthesis of intermediates IV-26 and IV-27, the temperature was maintained at 120 °C for 12 hours.

**Table 1.** Intermediates obtained, from the corresponding reagents, following Method IV-A.

| Intermediate | Structure | Intermediate | Structure |
|---|---|---|---|
| IV-1 | 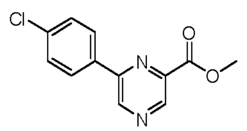 | IV-29 | 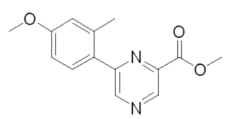 |
| IV-2 | 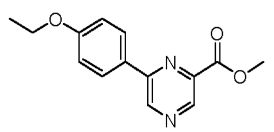 | IV-30 | 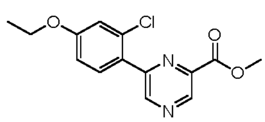 |
| IV-3 | 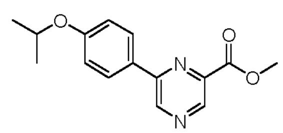 | IV-31 | 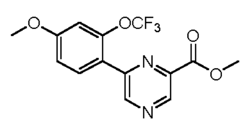 |

(continued)

| Intermediate | Structure | Intermediate | Structure |
|---|---|---|---|
| IV-4 | 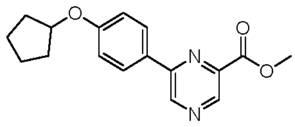 | IV-32 | 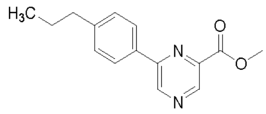 |
| IV-5 | 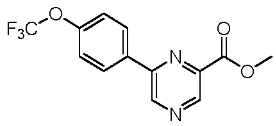 | IV-33 | 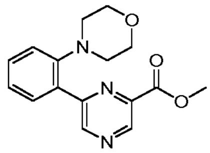 |
| IV-6 | 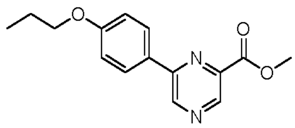 | IV-34 | 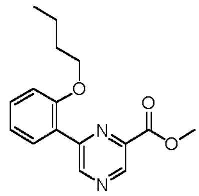 |
| IV-7 | 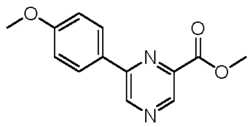 | IV-35 | 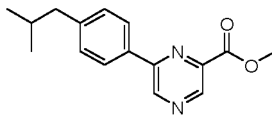 |
| IV-8 | 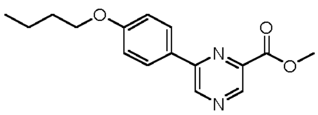 | IV-36 | 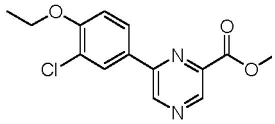 |
| IV-9 | 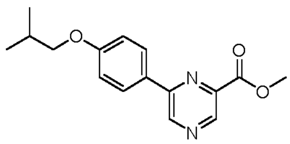 | IV-37 | 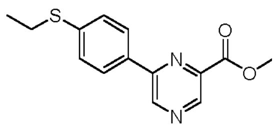 |
| IV-10 | 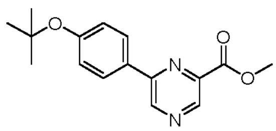 | IV-38 | 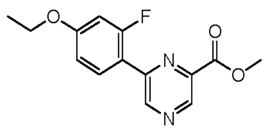 |
| IV-11 | 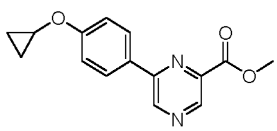 | IV-39 | 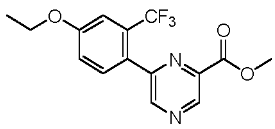 |
| IV-12 | 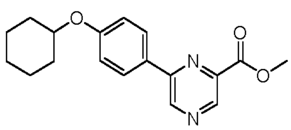 | IV-40 | 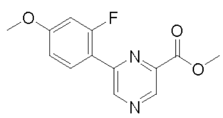 |
| IV-13 | 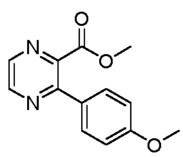 | IV-41 | 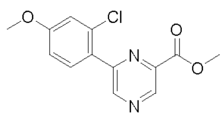 |

(continued)

| Intermediate | Structure | Intermediate | Structure |
|---|---|---|---|
| IV-14 | 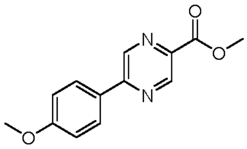 | IV-42 | 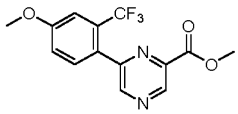 |
| IV-15 | 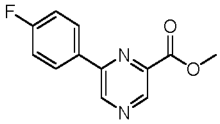 | IV-43 | 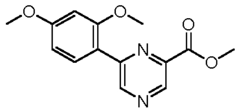 |
| IV-16 | 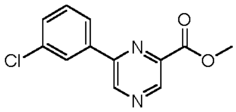 | IV-44 | 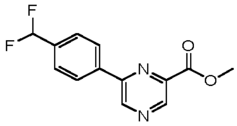 |
| IV-17 | 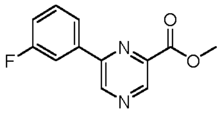 | IV-82 | 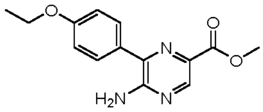 |
| IV-18 | 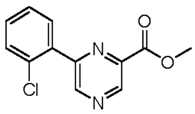 | IV-83 | 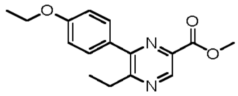 |
| IV-19 | 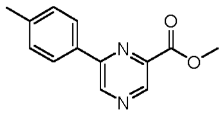 | IV-84 | 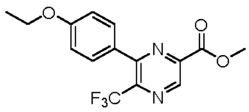 |
| IV-20 | 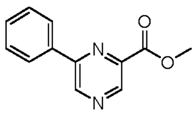 | IV-85 | 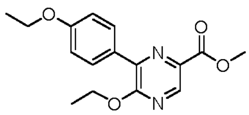 |
| IV-21 | 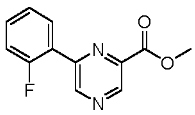 | IV-86 | 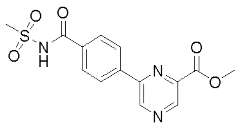 |
| IV-22 | 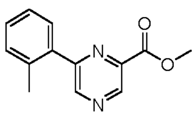 | IV-87 | 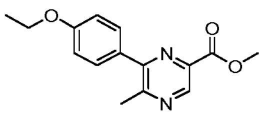 |
| IV-23 | 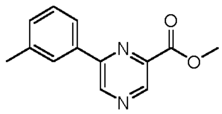 | IV-88 | 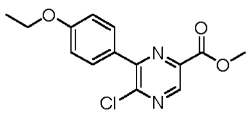 |
| IV-24 | 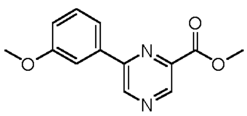 | IV-89 | 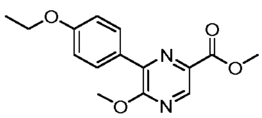 |

(continued)

| Intermediate | Structure | Intermediate | Structure |
|---|---|---|---|
| IV-25 | 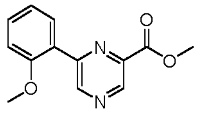 | IV-90 | 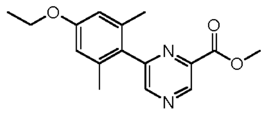 |
| IV-26 | 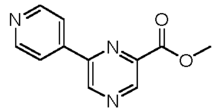 | IV-91 | 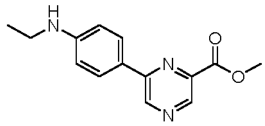 |
| IV-27 | 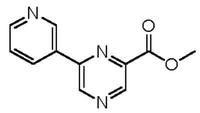 | IV-92 | 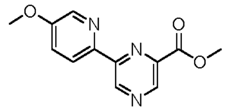 |
| IV-28 | 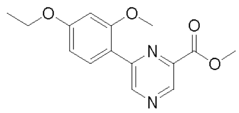 | IV-93 | 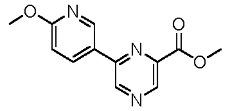 |

**Method IV-B**

*Intermediate IV-45:*

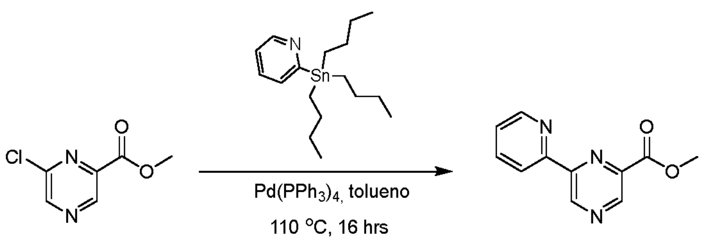


In a round bottom flask containing 5.79 mmol of methyl 6-chloropyrazine-2-carboxylate, 5.37 mmol of 2-(tributyltin)pyridine and 268 μmol of $Pd(PPh_3)_4$ were added. Subsequently, the reaction mixture was allowed to stir at 110 °C for 16 hours under N2 atmosphere. After total consumption of the starting reagent (monitored by LC-MS), the reaction mixture was filtered and concentrated at reduced pressure to obtain an oil, which was purified by preparative HPLC (neutral condition). 850 mg (74% yield) of intermediate IV-45 were obtained.

**Method IV-C:**

*Intermediates IV-46 to IV-48 / IV-92 to IV-94:*

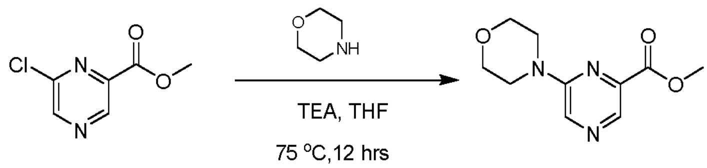


Into a round bottom flask were added 5.79 mmol methyl 6-chloropyrazine-2-carboxylate, 58 mmol morpholine,

86.9 mmol tea and 15 mL THF. Subsequently, the reaction mixture was allowed to stir at 75 oC for 12 hours. After total consumption of the starting reagent (monitored by CCF), the reaction mixture was diluted with H2O (15 mL) and extracted with AcOEt (10 mL x 3). The organic phases were washed with saturated sodium chloride (brine) solution (10 mL x 2), dried over Na2CO3, filtered and concentrated at reduced pressure until a residue was obtained, which was purified by column chromatography (petroleum ether/AcOEt: 50/1 → 0/1). 1.0 g (77% yield) of intermediate IV-46 was obtained. This procedure is also used to obtain the precursors of Table 2 from the corresponding reagents.

For the synthesis of intermediate IV-46 the solvent was replaced by acetonitrile (MeCN) and the base by Cs2CO3 while maintaining the temperature at 25 °C.

**Table 2.** Intermediates obtained, from the corresponding reagents, following Method IV-C.

| Intermediate | Structure |
|---|---|
| IV-46 | 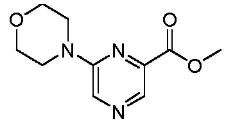 |
| IV-47 | 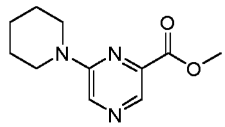 |
| IV-48 | 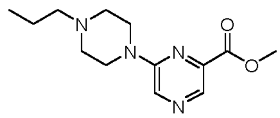 |
| IV-92 | 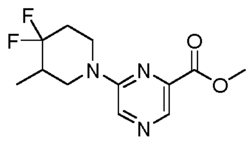 |
| IV-93 | 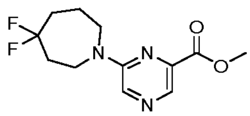 |
| IV-94 | 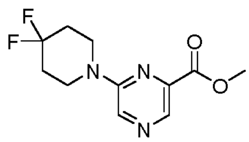 |

**Method IV-D:**

*Intermediates IV-49 to IV-51:*

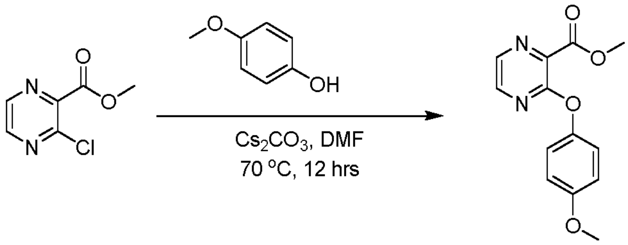


In a round bottom flask containing 11.6 mmol of methyl 3-chloropyrazine-2-carboxylate in 10 mL of DMF, 17.4

mmol of Cs2CO3and 13.9 mmol of 4-methoxyphenol were added. Subsequently, the reaction mixture was allowed to stir at 70 °C for 2 hours. After total consumption of the starting reagent (monitored by LC-MS), the reaction mixture was filtered and treated with ethyl acetate (AcOEt) (20 mL x 3). The obtained residue was triturated with 20 mL of AcOEt. 3 g (99% yield) of intermediate IV-49 were obtained.

**Table 3.** Intermediates obtained, from the corresponding reagents, following Method IV-D.

| Intermediate | Structure |
|---|---|
| IV-49 | 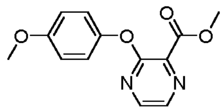 |
| IV-50 | 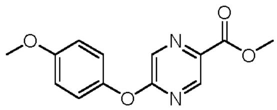 |
| IV-51 | 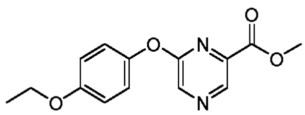 |

**Method IV-E:**

To obtain intermediates IV-52 to IV-57 (step IV-E-ii) an additional step (step IV-E-i) is required for formation of precursors IV-52i to IV-57i (Table 4).

Step *IV-E-i:*

*Precursors IV-52i to IV-57i:*

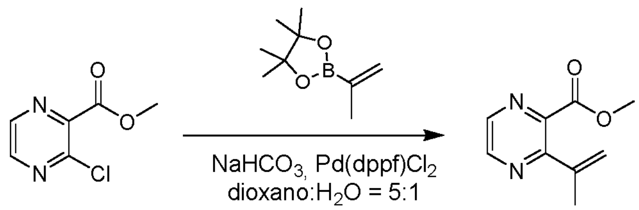


Into a round bottom flask were added 11.6 mmol of methyl 3-chloropyrazine-2-carboxylate, 13.9 mmol of 4,4,5,5-tetramethyl-2-(prop-1-en-2-yl)-1,3,2-dioxaborolane, 1.16 mmol of Pd(dppf)Cl2, 23.2 mmol ofNaHCO3, 15 mL of dioxane and 3 mL ofH2O. Subsequently, the reaction mixture was allowed to stir at 70 oC for 12 hours. After total consumption of the starting reagent (monitored by CCF), the reaction mixture was concentrated at reduced pressure to obtain an oil, which was purified by column chromatography (petroleum ether/AcOEt: 50/1 → 1/1). 1.9 g (70% yield) of the IV-52i precursor were obtained.

**Table 4.** Precursors obtained, from the corresponding reagents, following step *IV-E-i.*

| Precursor | Structure | Precursor | Structure |
|---|---|---|---|
| IV-52i | 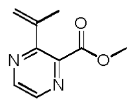 | IV-55i | 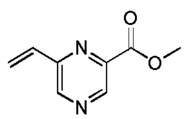 |

(continued)

| Precursor | Structure | Precursor | Structure |
|---|---|---|---|
| IV-53i | 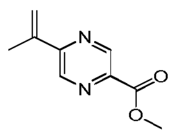 | IV-56i | 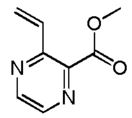 |
| IV-54i | 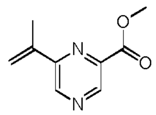 | IV-57i | 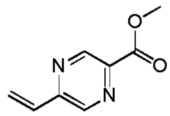 |

Step IV-E-ii:

*Intermediates IV-52 to IV-57:*

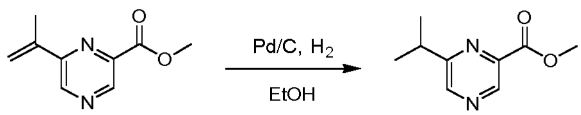


In a round bottom flask containing 7.3 mmol of methyl 6-(prop-1-en-2-yl)pyrazine-2-carboxylate (precursor IV-51i) in 10 mL of MeOH, 7.3 mmol of Pd/C (10% purity) was added under N2 atmosphere. Subsequently, the reaction mixture was de-gassed and purged withH2 repeatedly. The mixture was allowed to stir underH2 atmosphere (15 psi) at 20 °C for 0.25 hours. After total consumption of the starting reagent, the reaction mixture was filtered and concentrated to obtain the product, which was used for the next step without further purification. 1.1 g (91% yield) of intermediate IV-54 were obtained.

**Table 5.** Intermediates obtained, from the corresponding precursors, following step IV-E-ii.

| Intermediate | Structure |
|---|---|
| IV-52 | 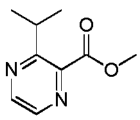 |
| IV-53 | 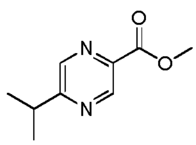 |
| IV-54 | 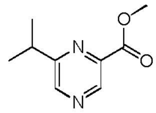 |
| IV-55 | 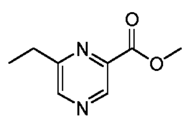 |

| Intermediate | Structure |
|---|---|
| IV-56 | 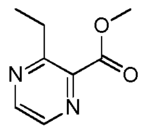 |
| IV-57 | 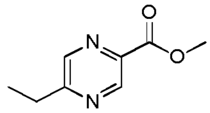 |

**Method IV-F:**

*Intermediates IV-58 to IV-60:*

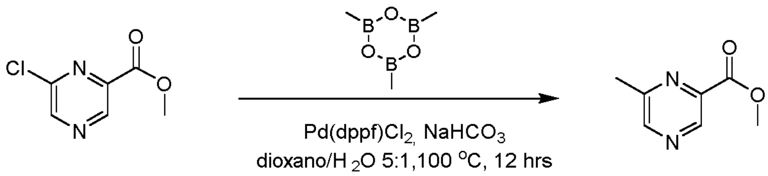


Into a round-bottom flask were added 11.6 mmol of methyl 6-chloropyrazine-2-carboxylate, 13.9 mmol of 2,4,6-trimethyl-1,3,5,2,4,6-trioxatriborinane, 1.16 mmol of Pd(dppf)Cl2, 23.2 mmol of NaHCO3, 15 mL of dioxane and 3 mL of H2O. Subsequently, the reaction mixture was allowed to stir at 100 °C for 12 hours. After total consumption of the starting reagent (monitored by CCF), the reaction mixture was concentrated at reduced pressure to obtain an oil, which was purified by column chromatography (petroleum ether/AcOEt: 50/1 → 1/1). 1.9 g (70% yield) of intermediate IV-58 were obtained.

**Table 6.** Intermediates obtained, from the corresponding reagents, following Method IV-F.

| Intermediate | Structure |
|---|---|
| IV-58 | 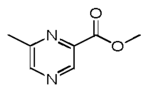 |
| IV-59 | 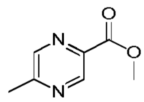 |
| IV-60 | 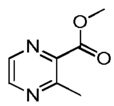 |

**Method IV-G:**

*Intermediates IV-61 to IV-63:*

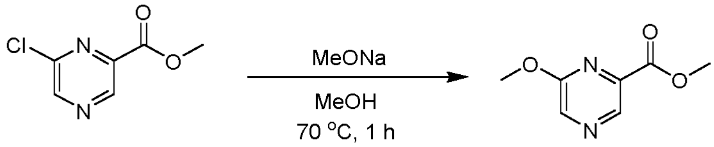


Into a round bottom flask were added 11.6 mmol of methyl 6-chloropyrazine-2-carboxylate, 23.2 mmol of MeONa and 10 mL of MeOH. Subsequently, the reaction mixture was allowed to stir at 70 °C for 2 hours. After total consumption of the starting reagent (monitored by LC-MS), the pH of the reaction mixture was adjusted to 6.0 with aqueous citric acid, to subsequently be extracted with AcOEt (50 mL x 3). The organic phases were pooled, dried over anhydrous magnesium sulfate, filtered and concentrated to a residue, which was treated with 10 mL of HCl/MeOH and kept stirring at 20 oC for 2 hours. Subsequently, the mixture containing intermediate IV-61, was filtered, concentrated and used for the next step without further purification.

**Table 7.** Intermediates obtained, from the corresponding reagents, following Method IV-G.

| Intermediaries | Structure |
|---|---|
| IV-61 | 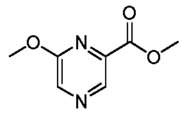 |
| IV-62 | 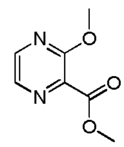 |
| IV-63 | 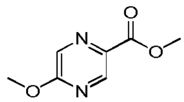 |

**Method IV-H:**

The precursor IV-63*i*, obtained from step IV-H-*i*, is used to obtain intermediates IV-63 to IV-67, according to the process described in steps IV-H-*iia* - IV-H-*iie*.

Step IV-H-*i*:

*Precursor IV-64i:*

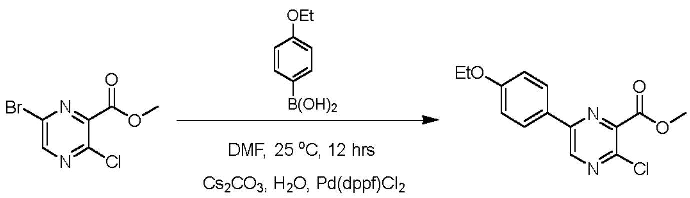


To a solution of methyl 6-bromo-3-chloropyrazine-2-carboxylate (59.6 mmol) in DMF (120 mL) was added 4-ethoxyphenylboronic acid (59.7 mmol), Cs2CO3 (149 mmol), H2O (596 mmol) and Pd(dppf)Cl2 (5.97 mmol). Then the reaction mixture was allowed to stir at 25 °C for 12 hours. After total consumption of the starting reagent (monitored by CCF), the reaction mixture was diluted with a saturated solution of NH4Cl (100 mL) and extracted with AcOEt (50 mL x 2). The organic phase was washed with H2O (50 mL) and saturated sodium chloride solution (brine) (20 mL). Subsequently, the organic phase was concentrated and purified by column chromatography (petroleum ether/AcOEt: 50/1 → 5/1). 10.5 g (60% yield) of intermediate IV-64i was obtained.

Step *IV-H-iia*:

*Intermediate IV-64:*

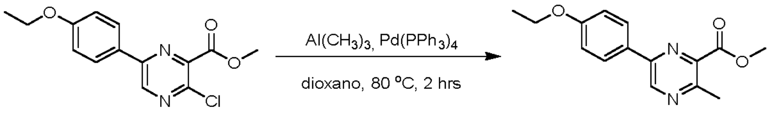


To a solution of methyl 3-chloro-6-(4-ethoxyphenyl)pyrazine-2-carboxylate (precursor IV-63i)(6.83 mmol) in dioxane (20 mL) was added a solution of trimethylaluminum (2 M, 4.1 mL) and subsequently Pd(PPh3)4 (683 μmmol). In continuation, the reaction mixture was allowed to stir at 80 °C for 2 hours. After total consumption of the starting reagent (monitored by CCF), the reaction mixture was concentrated and purified by column chromatography (petroleum ether/AcOEt: 50/1 → 5/1). 1.6 g (86% yield) of intermediate IV-64 were obtained.

Step IV-H-*iib*:

*Intermediate IV-65:*

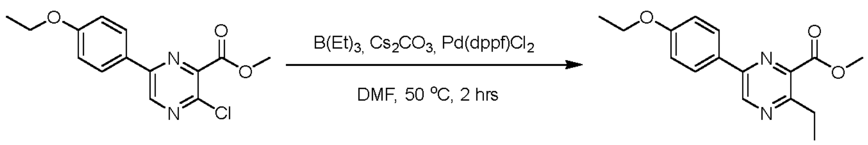


To a solution of methyl 3-chloro-6-(4-ethoxyphenyl)pyrazine-2-carboxylate (precursor IV-64i) (6.83 mmol) in DMF (20 mL) was added a solution of trimethylborane (1 M, 20.5 mL). Subsequently, Cs2CO3 (20.5 mmol) and Pd (dppf)Cl2 (1.02 mmol) were added. In continuation, the reaction mixture was allowed to stir at 80 °C for 2 hours. After total consumption of the starting reagent (monitored by CCF), the reaction mixture was concentrated and purified by column chromatography (petroleum ether/AcOEt: 50/1 → 5/1). 1.52 g (78% yield) of intermediate IV-65 were obtained.

Step IV-H-*iic*:

*Intermediate IV-66:*

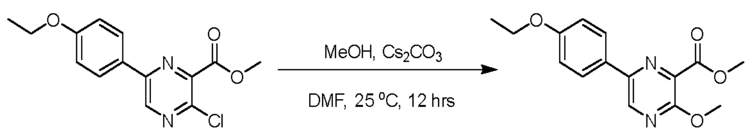


To a solution of methyl 3-chloro-6-(4-ethoxyphenyl)pyrazine-2-carboxylate (precursor IV-64i) (6.83 mmol) in DMF (20 mL) was added MeOH (13.7 mmol, 553 μL). Subsequently, Cs2CO3 (13.7 mmol) was added. Following, the reaction mixture was allowed to stir at 25 °C for 12 hours. After total consumption of the starting reagent (monitored by CCF), the reaction mixture was concentrated and purified by column chromatography (petroleum ether/AcOEt: 50/1 → 5/1). 910 mg (46% yield) of intermediate IV-66 were obtained.

*Step IV-H-iid:*

*Intermediate IV-67:*

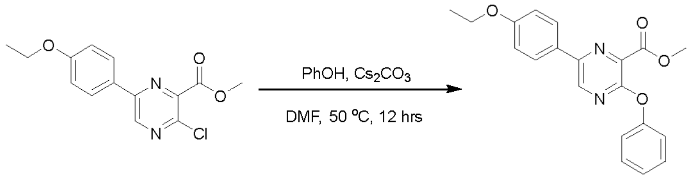


To a solution of methyl 3-chloro-6-(4-ethoxyphenyl)pyrazine-2-carboxylate (precursor IV-64i) (6.83 mmol) in DMF (20 mL) was added phenol (13.7 mmol) and subsequently Cs2CO3 (13.7 mmol). Then the reaction mixture was allowed to stir at 50 °C for 12 hours. After total consumption of the starting reagent (monitored by CCF), the reaction mixture was diluted with a saturated solution of NH4Cl (100 mL) and extracted with AcOEt (50 mL x 2). The organic phase was washed with H2O (50 mL) and saturated sodium chloride solution (brine) (20 mL). Subsequently, the organic phase was concentrated and purified by column chromatography (petroleum ether/AcOEt: 50/1 → 5/1). 1.64 g (69% yield) of intermediate IV-67 were obtained.

*Step IV-H-iie*:

Intermediate IV-68:

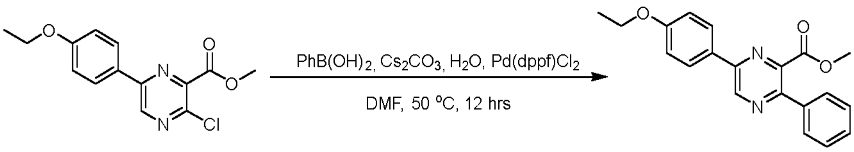


To a solution of methyl 3-chloro-6-(4-ethoxyphenyl)pyrazine-2-carboxylate (precursor IV-64i)(6.83 mmol) in DMF (20 mL) was added phenylboronic acid (20.5 mmol) and subsequently Cs2CO3 (20.5 mmol), H2O (68.3 mmol) and Pd(dppf)Cl2 (1.02 mmol). Then the reaction mixture was allowed to stir at 50 °C for 12 hours. After total consumption of the starting reagent (monitored by CCF), the reaction mixture was diluted with a saturated solution of NH4Cl (100 mL) and extracted with AcOEt (50 mL x 2). The organic phase was washed with H2O (50 mL) and saturated sodium chloride solution (brine) (20 mL). Subsequently, the organic phase was concentrated and purified by column chromatography (petroleum ether/AcOEt: 50/1 → 5/1). 1.01 g (44% yield) of intermediate IV-68 were obtained.

**Method IV-I:**

Method IV-I is used to obtain intermediates IV-69 to IV-73 (Table 8), by using the corresponding reagents.

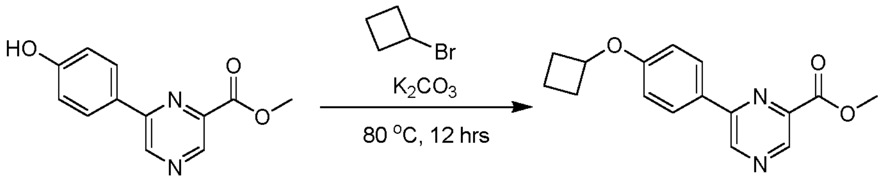


Into a round bottom flask were added 4.3 mmol of methyl 6-(4-hydroxyphenyl)pyrazine-2-carboxylate, 8.7 mmol of bromocyclobutane, 8.7 mmol of K2CO3, and 10 mL of DMF. Subsequently, the reaction mixture was de-gassed and allowed to stir at 80 °C for 12 hours. After total consumption of the starting reagent (monitored by CCF), the reaction mixture was diluted with H2O (50 mL) and extracted with AcOEt (30 mL x 3). The organic phase was washed with H2O (50 mL) and saturated sodium chloride solution (brine) (20 mL). Subsequently, the organic phase was concentrated and used for the next step without further purification. 1.2 g (75% yield) of intermediate IV-69 were obtained (Table 8).

**Table 8.** Intermediates obtained, from the corresponding reagents, following Method IV-I.

| Intermediate | Structure |
| --- | --- |
| IV-69 | 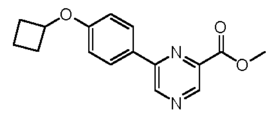 |
| IV-70 | 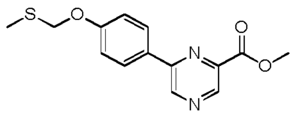 |
| IV-71 | 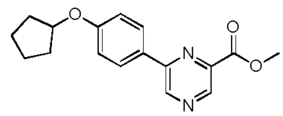 |
| IV-72 | 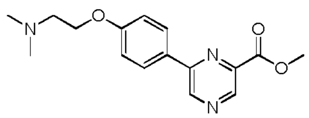 |
| IV-73 | 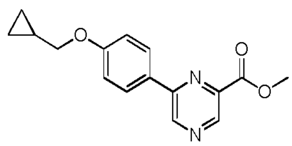 |

**Method IV-J:**

To obtain intermediates IV-74 to IV-77 (Table 11) by step *IV-J-iii* , the precursors of Tables 9 and 10 are first obtained by steps IV-J-i and *IV-J-ii*, as described below.

Step *IV-J-i*:

*Precursors IV-74i to IV-77i*:

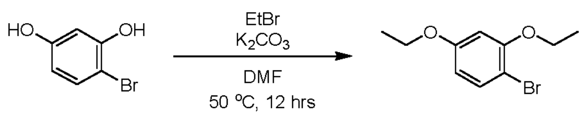


Into a round bottom flask were added 26.5 mmol of 4-bromobenzene-1,3-diol, 79.5 mmol of bromoethane, 39.8 mmol of K2CO3 and 60 mL of DMF. Subsequently, the reaction mixture was allowed to stir at 50 oC for 12 hours. After total consumption of the starting reagent (monitored by CCF), the reaction mixture was diluted with H2O (50 mL) and extracted with AcOEt (30 mL x 3). The organic phase was washed withH2O (50 mL) and saturated sodium chloride solution (brine) (20 mL). Subsequently, the organic phase was concentrated and purified by column chromatography (petroleum ether/AcOEt: 10/1 → 1/1). 3.30 g (54% yield) of precursor IV-74i was obtained.

For the synthesis of precursor IV-75i, the starting reagent was previously alkylated with an equivalent of isopropyl iodide, following the procedure described in step IV-J-i.

For the synthesis of precursors IV-76i and IV-77i, the starting reagent was replaced with 2-bromo-5-methoxyphenol.

**Table 9.** Precursors obtained, from the corresponding reagents, following step IV-J-i.

| Precursor | Structure |
|---|---|
| IV-74i | 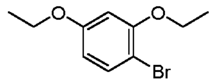 |
| IV-75i | 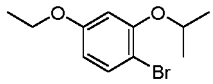 |
| IV-76i | 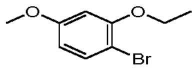 |
| IV-77i | 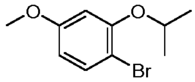 |

Step IV-J-*ii*:

*Precursors IV-74ii to IV-77ii:*

Using the precursors obtained in step IV-J-*i*, the other precursors IV-74*ii* to IV-75*ii* are obtained (Table 10).

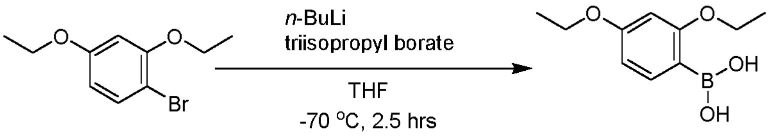


Into a round bottom flask were added 8.16 mmol of 1-bromo-2,4-diethoxybenzene (precursor*IV-74i*) and 15 mL of THF. Subsequently, a solution of *n*-BuLi (2.5 M, 6.53 mL) and triisopropyl borate (16.3 mmol) at -70 oC was added dropwise. The reaction mixture was heated to 20 °C and allowed to stir at that temperature for 2 hours under N2atmosphere. After total consumption of the starting reagent (monitored by LC-MS), the reaction mixture was diluted with H2Oand extracted with AcOEt (50 mL x 4). The organic phase was washed with H2O (50 mL) and saturated sodium chloride solution (brine) (20 mL). Subsequently, the organic phase was concentrated and used without further purification. 1.40 g (82% yield) of precursor IV-74ii were obtained.

**Table 10.** Precursors obtained, from the corresponding reagents, following step IV-J-*i*.

| Precursor | Structure |
|---|---|
| IV-74ii | 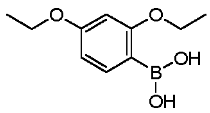 |
| IV-75ii | 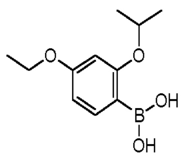 |
| IV-76ii | 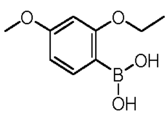 |

(continued)

| Precursor | Structure |
|---|---|
| IV-77ii | 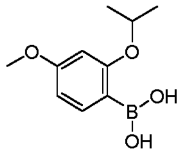 |

<u>Step IV-J-*iii*:</u>

*Intermediates IV-74 to IV-77:*

Using the compounds in Table 10, the method of obtaining intermediates IV-74 to IV-77 follows.

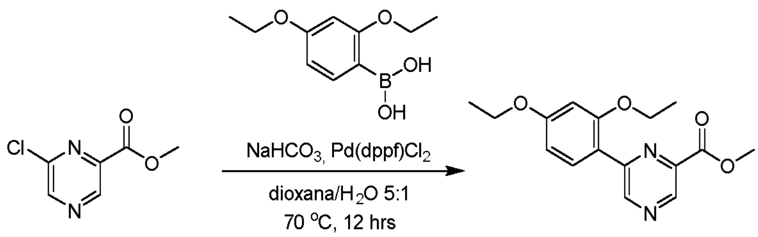


Into a round bottom flask were added 20.3 mmol of methyl 6-chloropyrazine-2-carboxylate, 24.3 mmol of (2,4-diethoxyphenyl)boronic acid (precursor IV-74ii), 1.0 mmol of Pd (dppf)Cl2, 40.6 mmol of NaHCO3, 30 mL of dioxane and 5 mL ofH2O. Subsequently, the reaction mixture was de-gassed and allowed to stir at 70 °C for 12 hours under N2 atmosphere. After total consumption of the starting reagent (monitored by LC-MS), the reaction mixture was diluted withH2Oand extracted with ethyl acetate (3 x 10 mL). Subsequently, the organic phase was concentrated and purified by column chromatography (petroleum ether/AcOEt: 20/1 → 1/1). 5.24 g (90% yield) of intermediate IV-74 were obtained.

**Table 11.** Intermediates obtained, from the corresponding reagents, following step IV-J-*iii*.

| Intermediate | Structure |
|---|---|
| IV-74 | 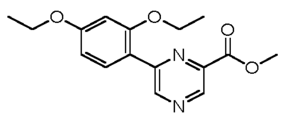 |
| IV-75 | 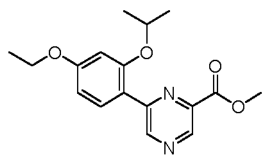 |
| IV-76 | 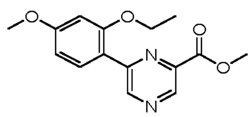 |
| IV-77 | 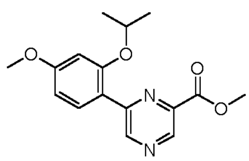 |

**Method IV-K:**

For the synthesis of intermediates IV-78 to IV-80 (Table 12), the synthesis of precursor IV-78-*iv* is required, which is obtained after four steps (IV-K-i to IV-K-iv), as described below.

Step *IV-K-i:*

*Precursor IV-78i:*

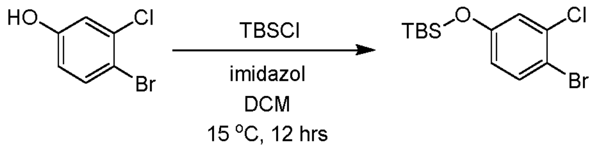


Into a round bottom flask were added 116 mmol 4-bromo-3-chlorophenol, 174 mmol imidazole, 150 mmol TBSCl and 250 mL DCM. Subsequently, the reaction mixture was allowed to stir at 15 °C for 12 hours. After total consumption of the starting reagent (monitored by CCF), the reaction mixture was diluted with H2O (100 mL) and extracted with DCM (200 mL x 3). The organic phases were pooled, dried over anhydrous magnesium sulfate, filtered and concentrated to obtain the product which was purified by column chromatography (petroleum ether/AcOEt: 1/0 → 10/1). 30 g (81% yield) of precursor IV-78i was obtained.

Step IV-K-ii:

*Precursor IV-78ii:*

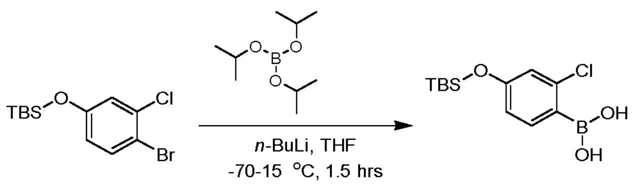


In a round bottom flask containing 31.1 mmol of (4-bromo-3-chlorophenoxy)(*tert-butyl*)dimethylsilane (precursor IV-78i), in 100 mL of THF, was added a solution of *n-BuLi*(2.5 M, 24.9 mL) at -70 °C. The reaction mixture was allowed to stir at -70 °C for 0.5 hours. Subsequently, 62.2 mmol of the triisopropyl borate was added, keeping the reaction mixture stirring at 15 °C for 2 hours. After total consumption of the starting reagent (monitored by CCF), the reaction mixture was diluted with H2O (25 mL) and extracted with EtOAc (3 x 100mL). Subsequently, the organic phase was concentrated and purified by column chromatography (petroleum ether/AcOEt: 1/0 → 3/1). 2.2 g (25% yield) of precursor IV-78ii were obtained.

Step IV-K-iii:

*Precursor IV-78iii:*

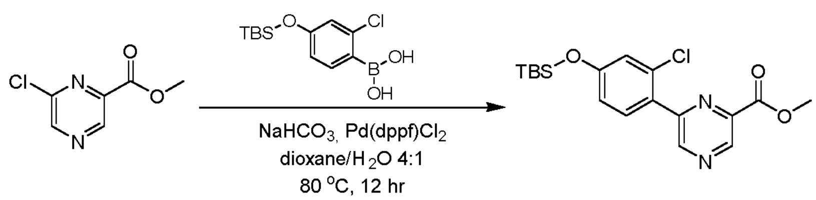

Into a round bottom flask were added 49.3 mmol of methyl 6-chloropyrazine-2-carboxylate, 73.9 mmol of (4-((tert-butyldimethylsilyl)oxy) -2-chlorophenyl)boronic acid (precursor IV-78ii), 4.9 mmol of Pd(dppf)Cl2, 73.9 mmol of NaHCO3, 85 mL of dioxane, and 17 mL ofH2O. Subsequently, the reaction mixture was de-gassed and allowed to stir at 80 °C for 12 hours under N2 atmosphere. After total consumption of the starting reagent (monitored by LC-MS), the reaction mixture was diluted with H2O and extracted with EtOAc (100 mL x 5). Subsequently, the organic phase was concentrated and purified by column chromatography (petroleum ether/AcOEt: 50/1 → 0/1). 2.30 g (48% yield) of precursor IV-78iii was obtained.

<u>Step IV-K-iv:</u>

*Precursor IV-78iv:*

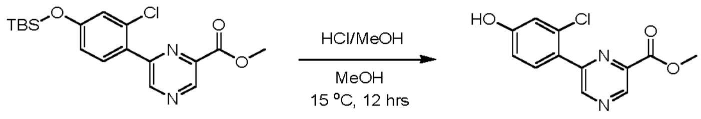


In a round bottom flask containing 6.07 mmol of methyl 6-(4- ((tertbutyldimethylsilyl)oxy)-2-chlorophenyl)pyrazine-2-carboxylate (precursor IV-78iii) and 20 mL of MeOH, a solution of HCl/MeOH (2N, 30 mL) was added. Subsequently, the reaction mixture was allowed to stir at 15 °C for 12 hours. After total consumption of the starting reagent (monitored by CCF), the reaction mixture was diluted with a saturated solution of NaHCO3 to pH 8 and extracted with AcOEt (50 mL x 3). Subsequently, the organic phase was concentrated and purified by column chromatography (petroleum ether/AcOEt: 1/0 → 1/1). 1.50 g (93% yield) of precursor IV-78iv was obtained.

<u>Step *IV-K-v:*</u>

*Intermediates IV-78 to IV-80:*

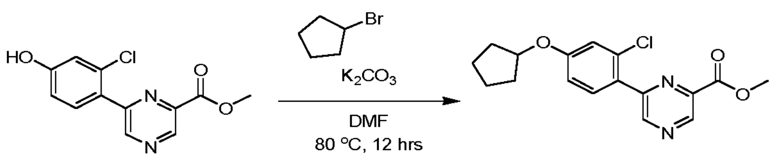


Into a round bottom flask were added 4.34 mmol of methyl 6-(2-chloro-4-hydroxyphenyl)pyrazine-2-carboxylate (*precursor* IV-78iv), 8.69 mmol of bromocyclobutane, 8.69 mmolof K2CO3 and10 mL of DMF. Subsequently, the reaction mixture was de-gassed and allowed to stir at 80 °C for 12 hours. After total consumption of the starting reagent (monitored by CCF), the reaction mixture was diluted with H2O (50 mL) and extracted with AcOEt (30 mL x 3). The organic phase was washed with water (50 mL) and saturated sodium chloride solution (brine) (40 mL). Subsequently, the organic phase was concentrated and used without further purification. 1.2 g (75% yield) of intermediate IV-78 were obtained.

**Table** 12. Intermediates obtained, from the corresponding precursors, following step IV-K-v.

| Intermediate | Structure |
|---|---|
| IV-78 | 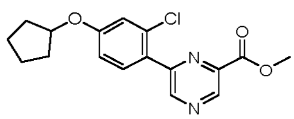 |
| IV-79 | 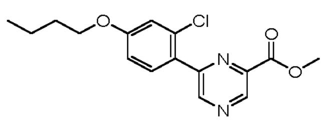 |

(continued)

| Intermediate | Structure |
| --- | --- |
| IV-80 | 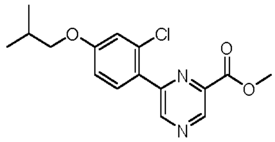 |

**Method IV-L:**

For the synthesis of intermediate IV-81, it is necessary to obtain the precursor IV-81iv, which is obtained after four steps (IV-L-ito IV-L-iv), as described below.

Step *IV-L-i:*

*Precursor IV-81i:*

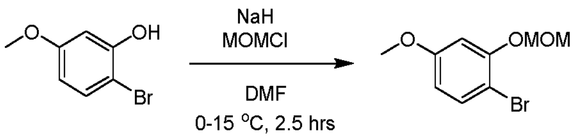


In a round bottom flask containing 9.9 mmol of the 2-bromo-5-methoxyphenol in 10 mL of DMF, 14.8 mmol of 0 oC NaH was added keeping the reaction mixture stirring at that temperature for 0.5 hours. Subsequently, MOMCl (19.8 mmol) was added keeping the reaction mixture stirring at 15 °C for 2 hours. After total consumption of the starting reagent (monitored by CCF), a saturated NH4Cl solution (20 mL) was added at 0 °C. The mixture was extracted with EtOAc (30 mL x 3). The organic phases were pooled, dried over anhydrous magnesium sulfate, filtered and concentrated to give the product, which was used for the next step without further purification. 2.5 g (Crude Product) of precursor IV-81i was obtained.

Step *IV-L-ii:*

*Precursor IV-81ii:*

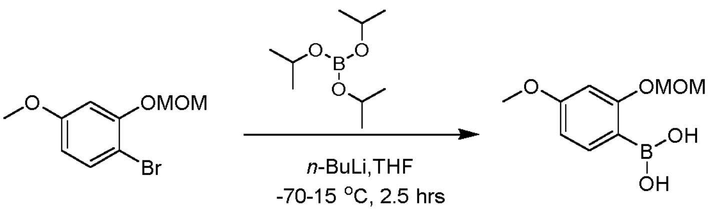


In a round bottom flask containing 10.1 mmol of the 1-bromo-4-methoxy-2-(methoxymethoxy)benzene (precursor IV-81i) in 25 mL of DMF, a solution of *n-BuLi* (2.5 M, 8.09 mL) at -70 °C under N2 atmosphere was added. The reaction mixture was allowed to stir at this temperature for 1 h. Subsequently, 20.2 mmol of triisopropyl borate was added. The mixture was allowed to stir at 15 °C for 2 hours. After total consumption of the starting reagent (monitored by LC-MS), the reaction mixture was diluted with H2O (20 mL) at 0 oC. The mixture was extracted with AcOEt (3 x 50 mL). The organic phases were pooled, dried over anhydrous magnesium sulfate, filtered and concentrated to give the product, which was used for the next step without further purification. 2.2 g of the precursor IV-81ii (crude product) were obtained.

Step IV-L-*iii:*

*Precursor IV-81iii:*

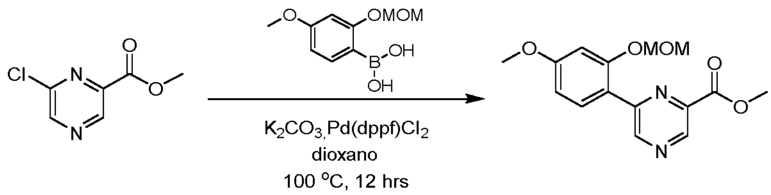


Into a round-bottom flask were added 8.7 mmol of methyl 6-chloropyrazine-2-carboxylate, 9.56 mmol of (4-methoxy-2-(methoxymethoxy)phenyl)boronic acid ( precursor IV-81-ii), 17.4 mmolofK2CO3, 435 µmol of Pd(PPh3)2Cl2, and 15 mL of dioxane. Subsequently, the reaction mixture was allowed to stir at 100 °C for 5 hours under N2 atmosphere. After total consumption of the starting reagent (monitored by CCF), the reaction mixture was concentrated at reduced pressure to obtain an oil, which was purified by column chromatography (petroleum ether/AcOEt: 1/0 → 3/1). 900 mg (34% yield) of precursor IV-81-iii was obtained.

Step IV-L-iv:

*Precursor IV-81iv:*

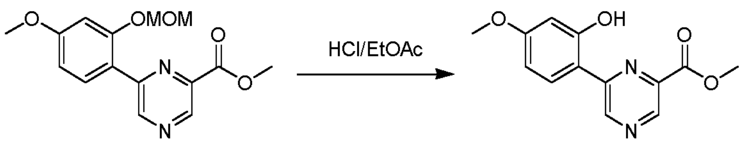


In a round bottom flask containing 3.3 mmol of methyl 6-(4-methoxy-2-(methoxymethoxy)phenyl)pyrazine-2-carboxylate (precursor *IV-81iii*) in 10 mL of DCM, a solution of HCl/dioxane (4M, 4.1 mL) was added. Subsequently, the reaction mixture was allowed to stir at 15 °C for 3 hours. After total consumption of the starting reagent (monitored by LC-MS), the reaction mixture was diluted with a saturated solution of NaHCO3 to pH 7-8 and extracted with DCM (20 mL x 3). The organic phases were combined, dried over anhydrous magnesium sulphate, filtered and concentrated to obtain the IV-81iv precursor (900 mg, crude product), which was used for the next step without further purification.

Step *IV-L-v:*

*Intermediate IV-81:*

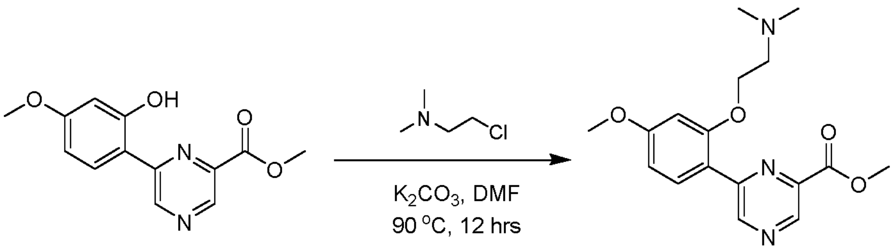


Into a round bottom flask were added 3.5 mmol of methyl 6-(2-hydroxy-4-methoxyphenyl)pyrazine-2-carboxylate (precursor IV-81iv), 6.92 mmolof 2-chloro-N, N-dimethyl-ethanamine, 6.92 mmol of K2CO3 and 10 mL of DMF. Subsequently, the reaction mixture was allowed to stir at 90 oC for 12 hours. After total consumption of the starting reagent

(monitored by CCF), the reaction mixture was diluted with H2O (30 mL) and extracted with AcOEt (30 mL x 3). The organic phases were pooled, dried over anhydrous magnesium sulfate, filtered and concentrated to obtain the product which was purified by column chromatography (petroleum ether/AcOEt: 1/0 → 0/1). 500 mg (43% yield) of intermediate IV-81 were obtained.

**Obtaining the intermediates of Formula III:**

Following General Scheme 1, an exemplary methodology for the formation of intermediates of Formula III is presented without limitation below.

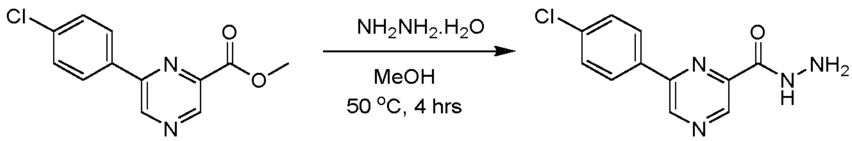


Into a flask containing 3.5 mmol of methyl4-(4-chlorophenyl)pyridine-2-carboxylate (intermediate IV-1), dissolved in 10 mL of MeOH, was added 10.8mmol ofN2H4.H2O(98% purity). The reaction medium was allowed to stir at 50 °C for 4 hours. After total consumption of the starting reagent (monitored by CCF), the solvent was concentrated and the solid formed was triturated with 20 mL of MeOH at 25 °C for 0.5 hours and subsequently filtered. 800 mg (80% yield) of the hydrazide of interest were obtained (Table 13).

**Table 13.** Intermediates obtained, from the corresponding reagents, following the method described in obtaining the compounds of Formula III.

| Intermediate | Structure | Intermediate | Structure |
|---|---|---|---|
| III-1 | 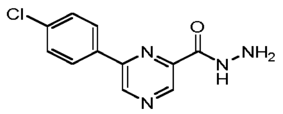 | III-52 | 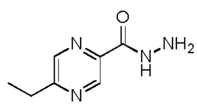 |
| III-2 | 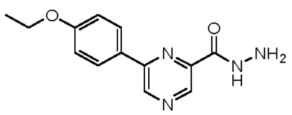 | III-54 | 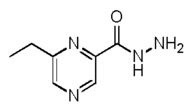 |
| III-3 | 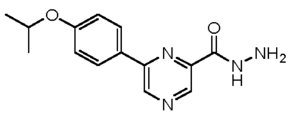 | III-55 | 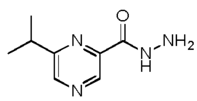 |
| III-4 | 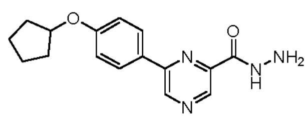 | III-56 | 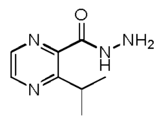 |
| III-5 | 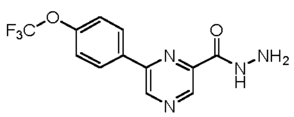 | III-57 | 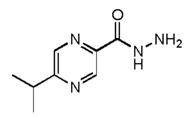 |
| III-6 | 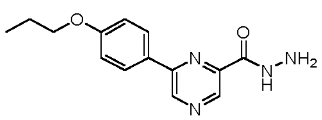 | III-58 | 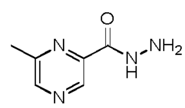 |
| III-7 | 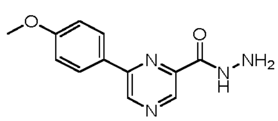 | III-59 | 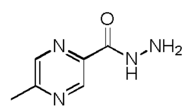 |

(continued)

| Intermediate | Structure | Intermediate | Structure |
|---|---|---|---|
| III-8 | 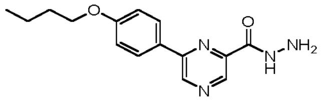 | III-60 | 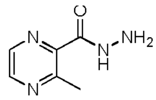 |
| III-9 | 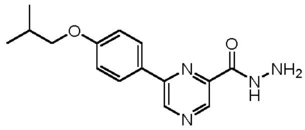 | III-61 | 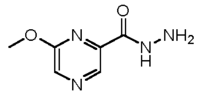 |
| III-10 | 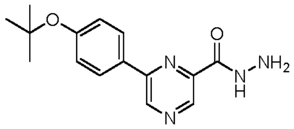 | III-62 | 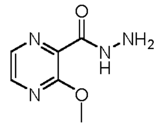 |
| III-11 | 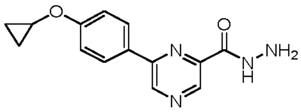 | III-63 | 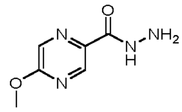 |
| III-12 | 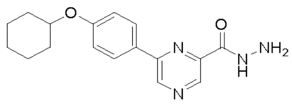 | III-64 | 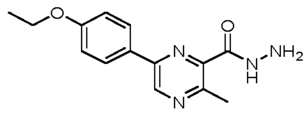 |
| III-13 | 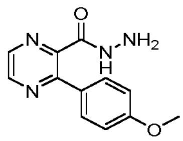 | III-65 | 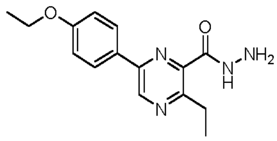 |
| III-14 | 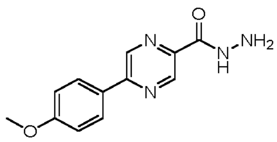 | III-66 | 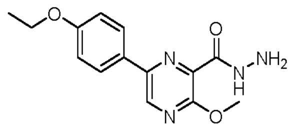 |
| III-15 | 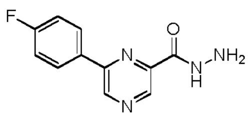 | III-67 | 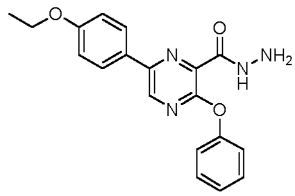 |
| III-16 | 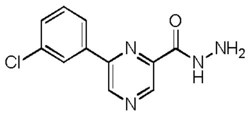 | III-68 | 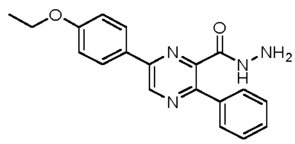 |
| III-17 | 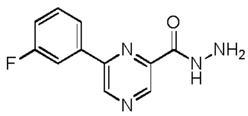 | III-69 | 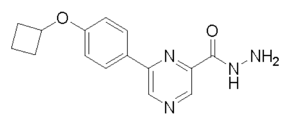 |
| III-18 | 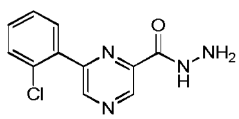 | III-70 | 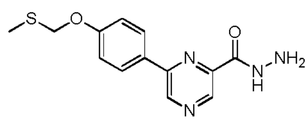 |

(continued)

| Intermediate | Structure | Intermediate | Structure |
|---|---|---|---|
| III-19 | 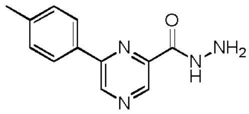 | III-71 | 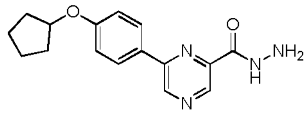 |
| III-20 | 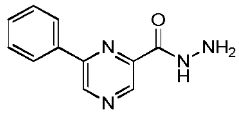 | III-72 | 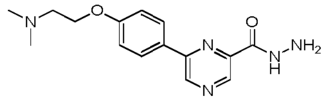 |
| III-21 | 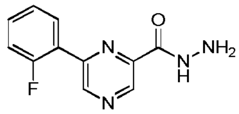 | III-73 | 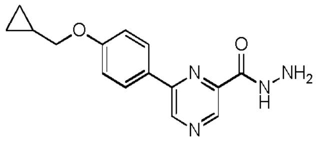 |
| III-22 | 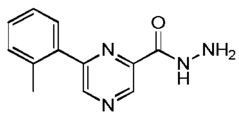 | III-74 | 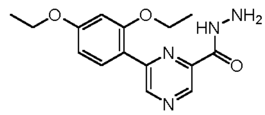 |
| III-23 | 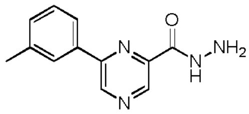 | III-75 | 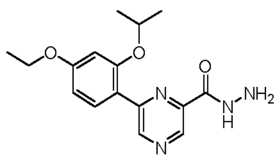 |
| III-24 | 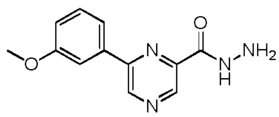 | III-76 | 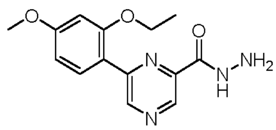 |
| III-25 | 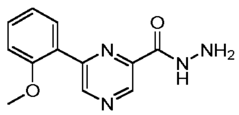 | III-77 | 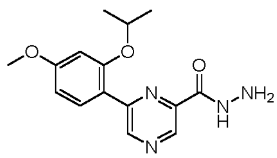 |
| III-26 | 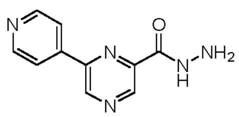 | III-78 | 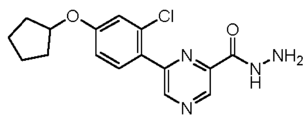 |
| III-27 | 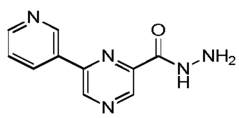 | III-79 | 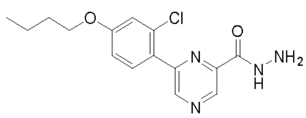 |
| III-28 | 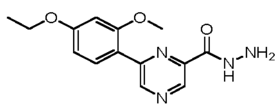 | III-80 | 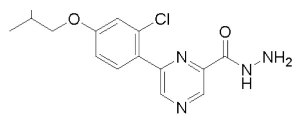 |
| III-29 | 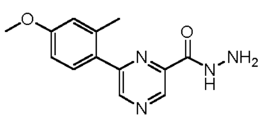 | III-81 | 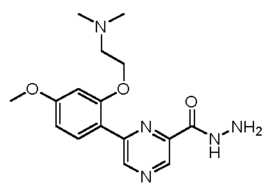 |

(continued)

| Intermediate | Structure | Intermediate | Structure |
|---|---|---|---|
| III-30 | 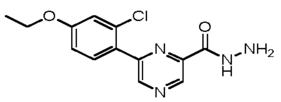 | III-82 | 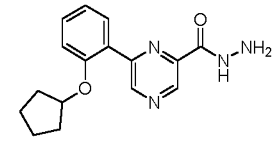 |
| III-31 | 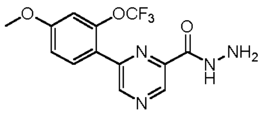 | III-83 | 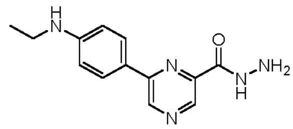 |
| III-32 | 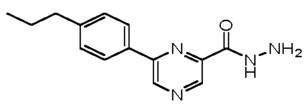 | III-84 | 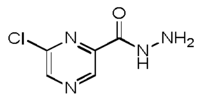 |
| III-33 | 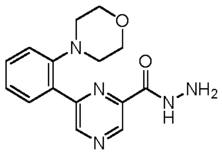 | III-85 | 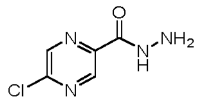 |
| III-34 | 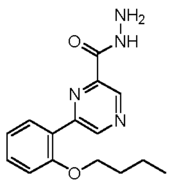 | III-86 | 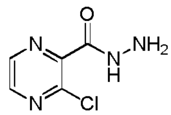 |
| III-35 | 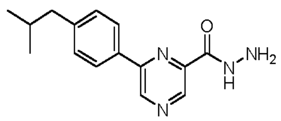 | III-87 | 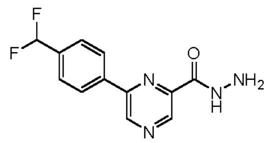 |
| III-36 | 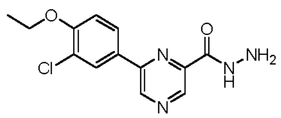 | III-88 | 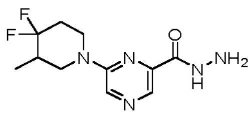 |
| III-37 | 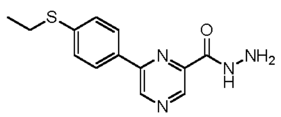 | III-89 | 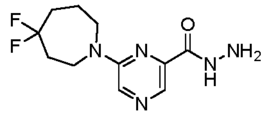 |
| III-38 | 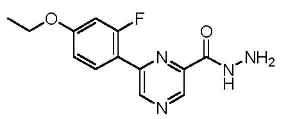 | III-90 | 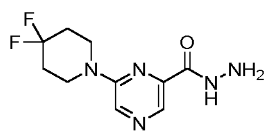 |
| III-39 | 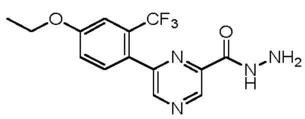 | III-91 | 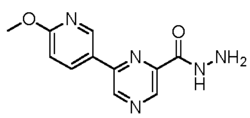 |
| III-40 | 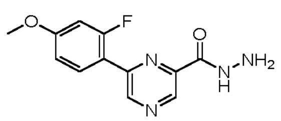 | III-92 | 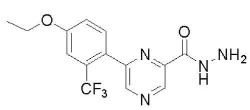 |

(continued)

| Intermediate | Structure | Intermediate | Structure |
|---|---|---|---|
| III-41 | 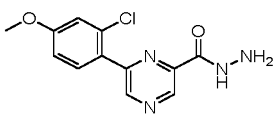 | III-93 | 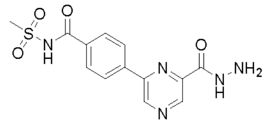 |
| III-42 | 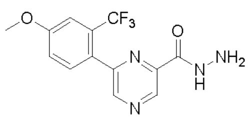 | III-94 | 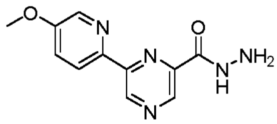 |
| III-43 | 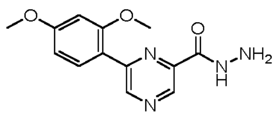 | III-95 | 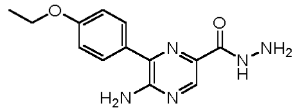 |
| III-44 | 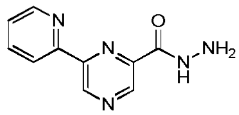 | III-96 | 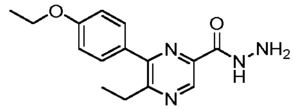 |
| III-45 | 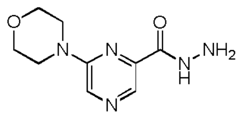 | III-97 | 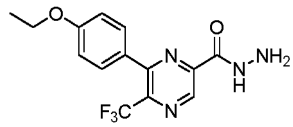 |
| III-46 | 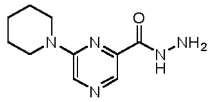 | III-98 | 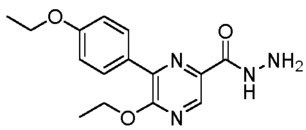 |
| III-47 | 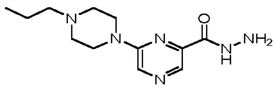 | III-99 | 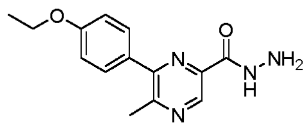 |
| III-48 | 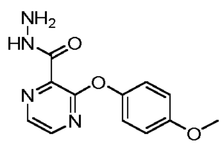 | III-100 | 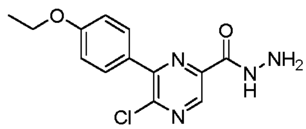 |
| III-49 | 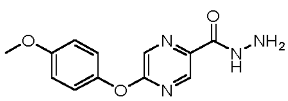 | III-101 | 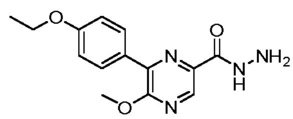 |
| III-50 | 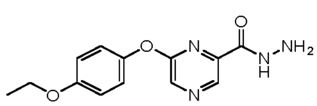 | III-102 | 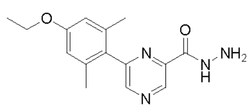 |
| III-51 | 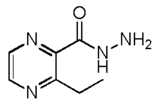 | | |

**Obtaining Compounds of Formula II:**

Following General Scheme 1, the following exemplary methods II-A to II-O for the formation of intermediates

of Formula II are presented without limitation.

**Method II-A:**

*Intermediate II-1:*

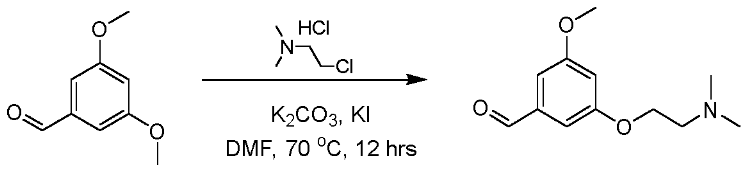


Into a round-bottom flask were added 6.97 mmol of 3-hydroxy-5-methoxybenzaldehyde, 13.93 mmol of 2-chloro-N, *N-dimethyl-ethanamine hydrochloride,* 697 μmol of KI, 45.3 mmol ofK2CO3, and 5 mL of DMF. Subsequently, the reaction mixture was allowed to stir at 70 °C for 12 hours. After total consumption of the starting reagent (monitored by CCF), the reaction mixture was diluted with H2O (100 mL) and extracted with AcOEt (300 mL). The organic phase was washed with H2O (50 mL) and saturated sodium chloride solution (brine) (20 mL). Subsequently, the organic phase was concentrated and purified by column chromatography (petroleum ether/AcOEt: 10/1 → 0/1). 320 mg (19% yield) of the intermediate II of interest were obtained. MS = 224.1.

**Method II-B:**

For the synthesis of intermediate II-2, it is necessary to obtain the precursor II-2*iii*, which is obtained after three steps (II-B-i to II-B-iii), as described below.

Step *II-B-i:*

*Precursor II-2i:*

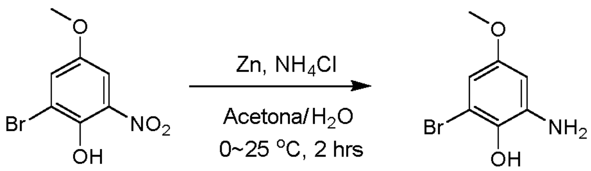


Into a round bottom flask containing 40.3 mmol of *2-bromo-4-methoxy-6-nitrophenol*were added 20 mL of H2O and 200 mL of acetone. Subsequently and slowly, 726 mmol of Zn at 25 °C was added. Subsequently, the reaction mixture was allowed to stir at 0 °C and 282 mmolNH4Clwas added. The reaction mixture was allowed to stir at 25 °C for an additional 2 hours. After total consumption of the starting reagent (monitored by CCF), the reaction mixture was diluted in EtOH (100 mL), filtered and concentrated. Subsequently, H2O (100 mL) was added, keeping the reaction mixture stirring for 10 minutes, to be filtered, washed withH2O (50 mL x 3) and concentrated. 8.7 g of the precursor II-2i was obtained, which was used for the next step without further purification.

Step *II-B-ii:*

*Precursor II-2ii:*

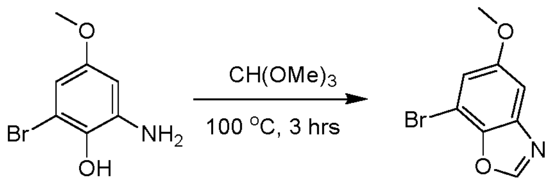


Into a round bottom flask were added 39.4 mmol of 2-amino-6-bromo-4-methoxyphenol (precursor *II-2i*) and 784 mmol of trimethoxymethane. Subsequently, the reaction mixture was allowed to stir at 100 oC for 3 hours. After total consumption of the starting reagent (monitored by CCF), the reaction mixture was concentrated and purified by column chromatography (petroleum ether/AcOEt: 30/1 → 5/1). 4.9 g (55% yield) of the precursor interessei-2ii were obtained.

<u>Step II-B-*iii*:</u>

*Precursor II-2iii:*

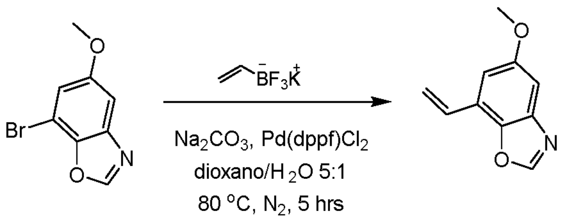


In a round bottom flask were dissolved 4.9 mmol of the 7-bromo-5-methoxybenzo[d]oxazole *(precursor* II-2ii) in 30 mL of dioxane and 5 mL ofH2O. Subsequently, 25.8 mmol of potassium trifluoro (vinyl)boranuide, 64.5 mmol of Na2CO3, and 2.15 mmol of Pd(dppf)Cl2 were added. The reaction mixture was allowed to stir at 80 °C underN2 atmosphere for 5 hours. After total consumption of the starting reagent (monitored by LC-MS), the reaction mixture was filtered (washing the solid residue with 50 mL AcOEt), concentrated, and purified by column chromatography (petroleum ether/AcOEt: 20/1 → 1/1). 3.7 g (98% yield) of precursor II-2iii was obtained.

<u>Step II-B-*iv*:</u>

*Intermediate II-2:*

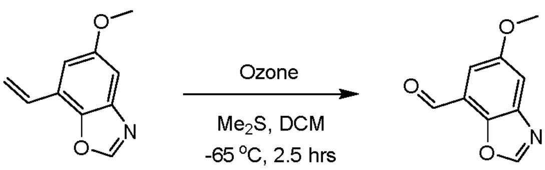


In a round bottom flask were dissolved 17 mmol of 5-methoxy-7-vinylbenzo [d]oxazole (precursor *II-2iii*) in 60 mL of DCM. Subsequently, ozone was bubbled at -65 °C for 30 min. Subsequently and under N2 atmosphere, 51.4 mmol of Me2S at -65 °C was added. The reaction was allowed to stir at -65 oC for 2 h. After majority consumption of the starting reagent (monitored by CCF), the reaction mixture was concentrated and the resulting product was purified by column chromatography (petroleum ether/AcOEt: 30/1 → 1/1). 510 mg (15% yield) of intermediate II-2 were obtained.

<u>**Method II-C:**</u>

For the synthesis of intermediate II-3, it is necessary to obtain the precursor II-3-*v*, which is obtained through five steps *(*II-C-i to II-C-*v*), as described below.

Step *II-C-i:*

*Precursor II-3i:*

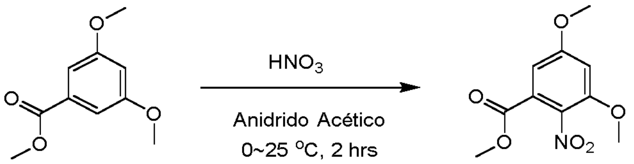


Into a round bottom flask were added 153 mmol of methyl 3,5-dimethoxybenzoate and 200 mL of acetic anhydride at 25 °C. Subsequently, 188 mmolHNO3 (added with a 70% w/v aqueous solution) was added dropwise at 0 °C. The reaction mixture was kept stirring at 25 °C for 2 hours. After total consumption of the starting reagent (monitored by CCF), the reaction mixture was diluted inH2O (500 mL), filtered and washed withH2O (100 mL x 3). 36 g (crude product) of precursor II-3i was obtained.

Step II-C-*ii*:

*Precursor II-3ii:*

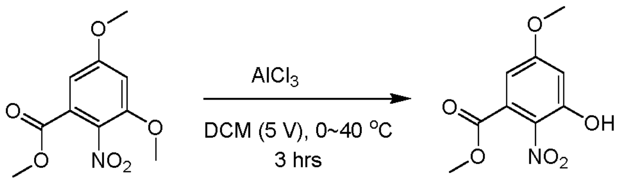


In a round bottom flask were dissolved 124 mmol of methyl 3,5-dimethoxy-2-nitrobenzoate (precursor II-3i) in 150 mL of DCM at 25 °C. Subsequently and slowly, 497 mmol of AlCl3 was added, after which the reaction mixture was allowed to stir at 40 °C for 3 hours. After total consumption of the starting reagent (monitored by CCF), HCl (12m) was added dropwise at 0 °C to pH 6-7. Subsequently, the mixture was extracted with DCM (100 mL x 3) and the organic phases were combined and concentrated. 23.5 g (crude product) of precursor II-3ii was obtained.

Step II-C-*iii*:

*Precursor II-3iii:*

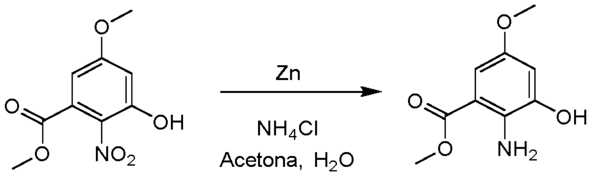


In a round bottom flask were dissolved 103 mmol of methyl 3-hydroxy-5-methoxy-2-nitrobenzoate (precursor II-3ii*)* in 470 mL of acetone and 47 mL of H2O. Subsequently, the reaction mixture was cooled to 0 °C and 724 mmol of NH4C!was added. The reaction mixture was kept stirring at 25 °C for 2 hours. After total consumption of the starting reagent (monitored by CCF), MeOH (300 mL) was added and the resulting mixture was filtered and washed with MeOH (100 mL x 3). Subsequent concentration of the residue at reduced pressure allowed to obtain 16.3 g (crude product) of the precursor *II-3iii.*

Step II-C-iv:

*Precursor II-3-iv:*

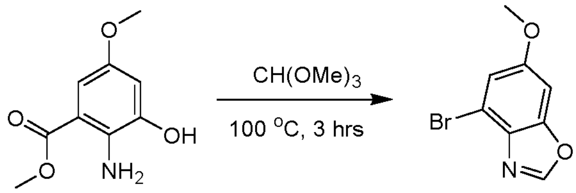


Into a round bottom flask were added 39.4 mmol of methyl 2-amino-3-hydroxy-5-methoxybenzoate (precursor II-3iii*)* and 784 mmol of trimethoxymethane. Subsequently, the reaction mixture was allowed to stir at 100 °C for 3 hours. After total consumption of the starting reagent (monitored by CCF), the reaction mixture was concentrated and purified by column chromatography (petroleum ether/AcOEt: 30/1 → 5/1). 4.9 g (55% yield) of the precursor interessei II-3-iv were obtained.

Step II-C-v:

*Precursor II-3-v:*

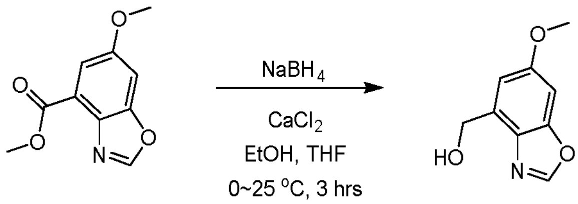


Into a round bottom flask were added 14.5 mmol of methyl6-methoxybenzo [d]oxazole-4-carboxylate (precursor II-3iv*)* and 60 mL of THF. Subsequently, the reaction mixture was cooled to 0 °C to add 29 mmolCaCl2 dissolved in 60 mL EtOH and 29 mmol NaHB4, respectively. The mixture was allowed to stir at 25 °C for 3 hours. After total consumption of the starting reagent (monitored by CCF), the reaction mixture was diluted with 50 mL of H2O, filtered, concentrated, and purified by column chromatography (petroleum ether/AcOEt: 30/1 → 1/1). 1.2 g (39% yield) of precursor II-3-v *wereobtained.*

Step II-C-*vi*:

*Intermediate II-3:*

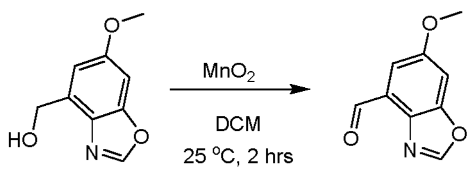


Into a round bottom flask was added 7.09 mmol of (6-methoxybenzo [d]oxazol-4-yl)methanol (precursor *II-3v*), 10 mL of DCM and 106 mmol of MnO2. Subsequently, the reaction mixture was allowed to 25 stir at 2 °C. After total consumption of the starting reagent (monitored by CCF), the reaction mixture was concentrated and purified by column chromatography (petroleum ether/AcOEt: 30/1 → 1/1). 460 mg (35% yield) of intermediate II-3 were obtained.

**Method II-D:**

For the synthesis of intermediate II-4, it is necessary to *obtain the* precursor II-4-v, which is obtained through five steps *(*II-D-i *to* II-D-*v)*, as described below.

Step II-D-*i*:

*Precursor II-4i:*

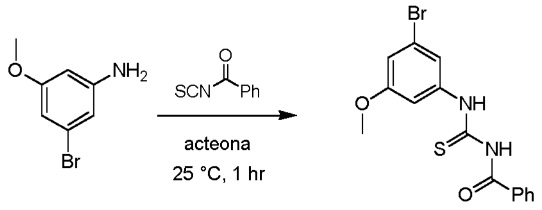


Into a round bottom flask were added 24.8 mmol of the 3-bromo-5-methoxyaniline and 100 mL of acetone, after which benzoyl isothiocyanate (27.2 mmol) was added at 25 °C. The reaction mixture was allowed to stir at 25 °C for 1 hour. After total consumption of the starting reagent (monitored by CCF), the reaction mixture was concentrated and triturated with petroleum ether at 25 °C for 0.5 hours. 9 g (99% yield) of precursor II-4i were obtained.

Step II-D-*ii*:

*Precursor II-4ii:*

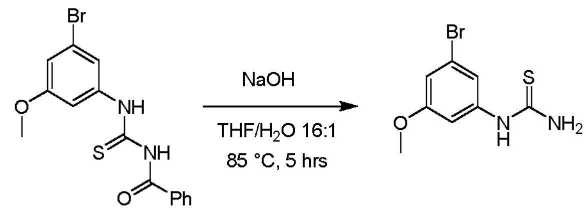


Into a round bottom flask were added 24.6 mmol of *N*-((3-bromo-5-methoxyphenyl)carbamothioyl)benzamide (precursor *II-4-i*) and 150 mL of THF, after which a solution of NaOH (123 mmol) in 10 mL of H2O was added. The reaction mixture was allowed to stir at 85 °C for 5 hours. After total consumption of the starting reagent (monitored by CCF), the reaction mixture was diluted with 50 mL of H2O and extracted with AcOEt (50 mL x 3). 4 g (62% yield) of precursor II-4ii were obtained.

Step II-O-*iii*:

*Precursor II-4iii:*

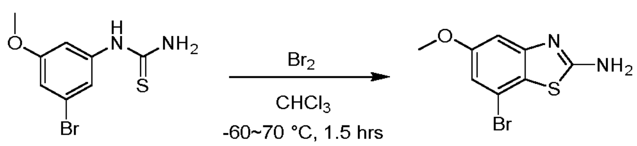


Into a round bottom flask were added 15.3 mmol of the 1-(3-bromo-5-methoxyphenyl)thiourea (precursor II-4ii) and 30 mL of CHCl3, after which a solution of Br2( 15.3 mmol) in 10 mL of CHCl3 was added. The reaction mixture was

allowed to stir at 25 °C for 0.5 hours, to subsequently be heated to 70 °C for 1 hour. After total consumption of the starting reagent (monitored by CCF), the reaction mixture was cooled and basified with a saturated solution of NH4OH. The mixture was extracted with AcOEt (30 mL x 3). The organic phase was filtered and concentrated to obtain 3.9 g (98% yield) of precursor *II-4iii.*

Step II-D-*iv*:

*Precursor II-4iv:*

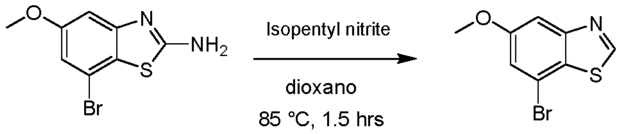


Into a round bottom flask and under N2 atmosphere, 15.1 mmol of the 7-bromo-5-methoxybenzo [d]thiazol-2-amine (precursor II-4iii) *and* 30 mL of dioxane were added, after which isopentyl nitrite (30.1 mmol) was added. The reaction mixture was allowed to stir at 85 °C for 5 hours. After total consumption of the starting reagent (monitored by CCF), the reaction mixture was concentrated and purified by column chromatography (petroleum ether/AcOEt:20/1 → 5/1). 2.5 (68% yield) of precursor II-4iv were obtained.

Step II-O-*v*:

*Precursor II-4v:*

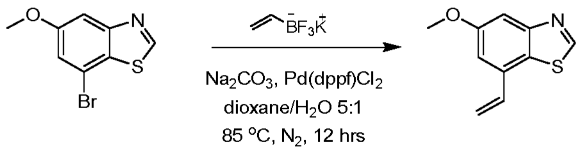


Into a round bottom flask, 8.2 mmol of the 7-bromo-5-methoxybenzo [d]thiazole (precursor II-4iv), 10 mL of dioxane and 2 mL of H2O were added, after which potassium trifluoro(vinyl)boranuide (9.83 mmol), Na2CO3 (24.6 mmol) and Pd(dppf)Cl2 (819 μmol) were added. The reaction mixture was allowed to stir at 85 °C for 12 hours. After total consumption of the starting reagent (monitored by CCF), the reaction mixture was concentrated and purified by column chromatography (petroleum ether/AcOEt: 20/1 → 5/1). 1.0 g (64% yield) of precursor II-4v was obtained.

Step II-D-*vi*:

*Intermediate II-4:*

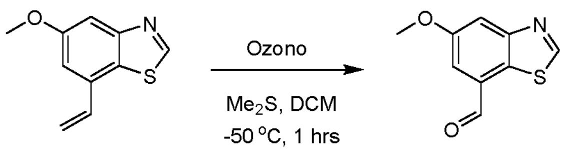


In a round bottom flask 5.2 mmol of the 5-methoxy-7-vinylbenzo [d]thiazole (precursor II-4v) was dissolved in 10 mL of DCM. Subsequently, ozone was bubbled at -50 °C for 30 min. Subsequently and under N2 atmosphere, 15.7 mmol of Me2S at- 50 °C was added. The reaction was allowed to stir at -50 °C for 0.5 hours. After total consumption of the starting reagent (monitored by CCF), the reaction mixture was concentrated and purified by column chromatography (petroleum ether/AcOEt: 20/1 → 5/1). 200 mg (20% yield) of intermediate II-4 were obtained.

**Method II-E:**

*Intermediate II-7:*

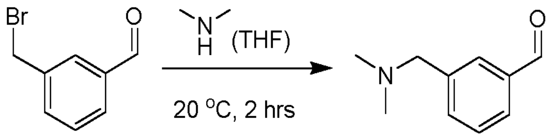


Into a round bottom flask were added 9.29 mmol of 3-(bromomethyl)benzaldehyde and 22.5 mL of N,N-dimethylamine (2M). Subsequently, the reaction mixture was allowed to stir at 20 °C for 2 hours. After total consumption of the starting reagent (monitored by CCF), the reaction mixture was diluted with H2O (20 mL) and extracted with AcOEt (20 mL x 2). The organic phase was dried over anhydrous magnesium sulfate and concentrated to obtain 1.5 g (98% yield) of intermediate II-7 which was used for the next step without further purification.

**Method II-F:**

For the synthesis of intermediate II-8, it is necessary to obtain precursor II-8i, as described in step *II-F-i.*

Step II-F-*i*:

*Precursor II-8i:*

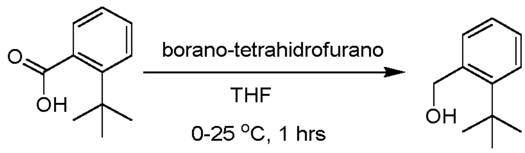


In a round bottom flask containing 9.3 mmol of 2-(*tert-butyl*)benzoic acid in 20 mL of THF, 37.3 mL of borane-tetrahydrofuran (1M) was added at 0 °C. The reaction mixture was allowed to stir at 25 °C for 1 hour. After total consumption of the starting reagent (monitored by LC-MS), the reaction mixture was diluted with 40 mL of a saturated aqueous NH4Cl solution and extracted with AcOEt (30 mL x 3). The organic phase was washed with saturated sodium chloride (brine) solution (25 mL x 2), dried over anhydrous magnesium sulfate and concentrated to obtain 1.7 g (Crude Product) of precursor II-8i *which* was used for the next step without further purification.

Step *II-F-i:*

*Intermediate II-8:*

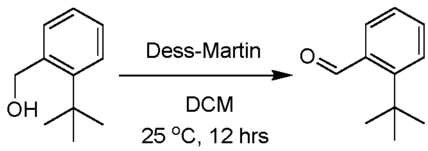


In a round bottom flask containing 10.4 mmol of (2-(*tert-butyl*)phenyl)methanol (precursor II-8i )in 20 mL of DCM, 15.5 mmol of the Dess-Martin reagent was added. Subsequently, the reaction mixture was allowed to stir at 25 °C for 12 hours. After total consumption of the starting reagent (monitored by LC-MS), the reaction mixture was treated with a saturated solution of K2CO3 (30 mL) to pH 10 and finally extracted with DCM (30 mL x 3). The organic phase was washed with saturated sodium chloride (brine) solution (30 mL x 2), dried over anhydrous magnesium sulfate and

concentrated to obtain 500 mg (30% yield) of the intermediate II of interest, which was used for the next step without further purification.

**Method II-G:**

*Intermediates II-9 to 11-11:*

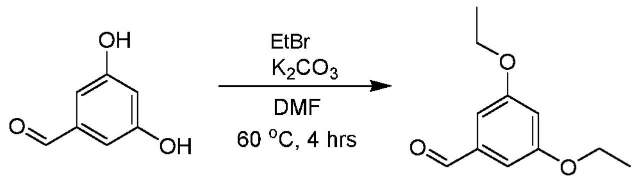


Into a round bottom flask containing 21.8 mmol of 3,5-dihydroxybenzaldehyde, 109 mmol of bromoethane and 30 mL of DMF, 43.4 mmol of K2CO3 was added. Subsequently, the reaction mixture was allowed to stir at 60 °C for 4 hours. After total consumption of the starting reagent (monitored by CCF), the reaction mixture was diluted with H2O (100 mL) and extracted with AcOEt (100 mL x 3). The organic phase was dried over anhydrous magnesium sulfate, concentrated and purified by column chromatography (petroleum ether/AcOEt: 10/1 → 0/1). 3.21 g (76% yield) of the intermediate II of interest were obtained (Table 14).

**Table 14.** Intermediates obtained, from the corresponding reagents, following Method II-G.

| Intermediate | Structure |
|---|---|
| II-9 | 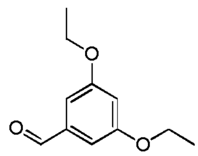 |
| II-10 | 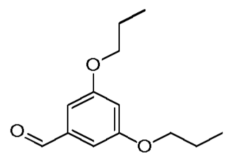 |
| II-11 | 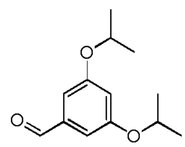 |

**Method II-H:**

For the synthesis of intermediate II-12, it is necessary to obtain the precursor II-12ii, which is obtained through two steps *(*II-H-i to II-H-ii), as described below.

Step II-H-*i*:

*Precursor 11-12i:*

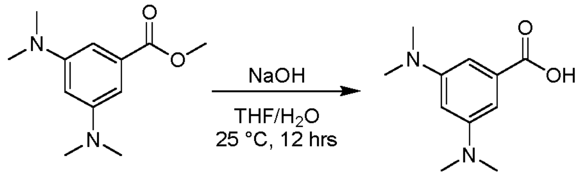


Into a round bottom flask containing 6.3 mmol of methyl 3,5-bis(dimethylamino)benzoate in 20 mL of MeOH and 8 mL of H2O, 12.6 mmol of NaOH was added. Subsequently, the reaction mixture was allowed to stir at 25 °C for 2 hours. After total consumption of the starting reagent (monitored by CCF), the reaction mixture was concentrated and the residue diluted with 20 mL of H2O. The pH was adjusted to 3-4 with a solution of HCl (10%), and the mixture was extracted with AcOEt (15 mL x 3). The organic phase was concentrated and used in the next step without further purification. 2.6 g (Crude Product) of precursor *II*-121 were obtained.

<u>Step II-H-*ii*:</u>

*Precursor 11-12ii:*

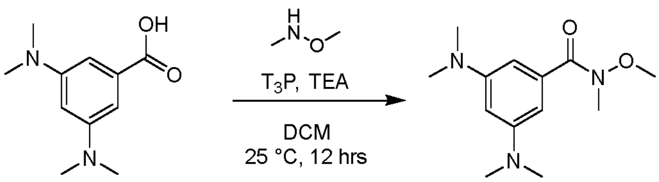


Into a round bottom flask containing 6.2 mmol 3,5-bis(dimethylamino)benzoic acid *(precursor* II-12i) and 9.4 mmol N-methoxymethanamine hydrochloride, in 20 mL DCM, was added 18.7 mmol DIEA and 8.12 mmol (4.83 mL, 50% purity) T3P. Subsequently, the reaction mixture was allowed to stir at 12 °C for 12 hours. After total consumption of the starting reagent (monitored by LC-MS), the reaction mixture was treated with an aqueous solution ofNaHCO3 (30 mL) and extracted with DCM (60 mL x 2). The organic phase was dried over anhydrous magnesium sulfate, filtered, concentrated and used for the next step without further purification. 1.7 g (Crude Product) of the II-12ii precursor *were-obtained.*

<u>Step II-H-*iii*:</u>

*Intermediate II-12:*

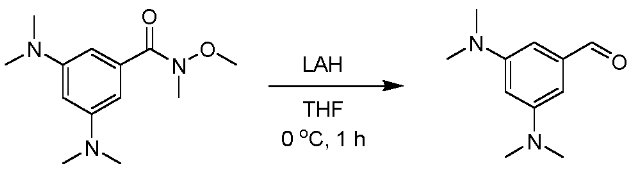


In a round bottom flask containing 8.0 mmol of *3,5-bis(dimethylamino)* - N-methoxy-N-methylbenzamide(precursor II-12ii) *in* 5 mL of THF, 1.76 mmol of LiAlH4 at 0 °C was added. The reaction mixture was allowed to stir at 0 °C for 1 hour. After total consumption of the starting reagent (monitored by CCF), 3 g of Na2SO4.10H2O was added to the reaction mixture. Then the mixture was filtered and concentrated. The resulting product was purified by column chromatography (petroleum ether/AcOEt: 5/1). 1.0 g (52% yield) of intermediate II-12 was obtained.

<u>**Method II-I:**</u>

For the synthesis of intermediate II-13, it is necessary to obtain the precursor II-13i, which is obtained through two steps (II-I-*i* to II-I-ii), as described in the continuation.

Step II-I-*i*:

*Precursor 11-13i:*

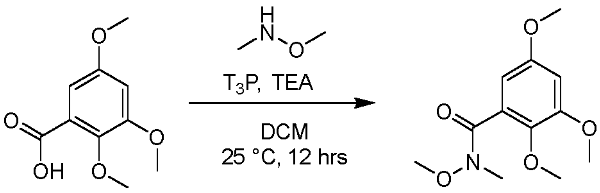


Into a round bottom flask containing 4.24 mmol 2,3,5-trimethoxybenzoic acid and 4.67 mmol N-methoxymethanamine *hydrochloride,* in 10 mL DCM, were added 12.7 mmol DIEA and 5.09 mmol T3P (50% purity) at 0 °C. Subsequently, the reaction mixture was allowed to stir at 25 °C for 12 hours. After total consumption of the starting reagent (monitored by LC-MS), the reaction mixture was diluted with a saturated solution of NaHCO3 (20 mL) and extracted with DCM (20 mL x 3). The organic phase was concentrated and used in the next step without further purification. 950 mg of the precursor II-13i were obtained (crude product).

Step II-I-*ii*:

*Intermediate II-13:*

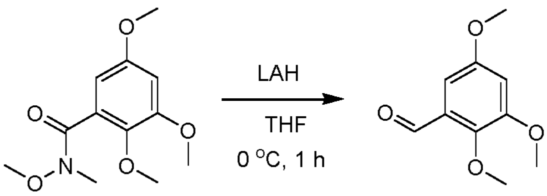


In a round bottom flask containing 3.5 mmol N,2,3,5-tetramethoxy-N-methylbenzamide (precursor II-13i) in 5 mL THF, 1.76 mmol LiAlH4 at 0 °C was added. The reaction mixture was allowed to stir at 0 °C for 1 hour. After total consumption of the starting reagent (monitored by CCF), Na2SO4.10H2O was added to the reaction mixture. The continuing mixture was filtered and concentrated. The resulting product was purified by column chromatography (petroleum ether/AcOEt: 5/1). 600 mg (87% yield) of intermediate II-13 were obtained.

**Method II-J:**

For the synthesis of intermediate II-14, it is necessary to obtain precursor II-14*iv*, which is obtained through four steps *(*II-J-*i* to II-J-*iv)*, as described below.

Step II-J-*i*:

*Precursor 11-14i:*

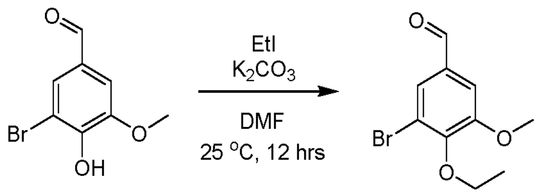


Into a round bottom flask was added 12.9 mmol of 3-bromo-4-hydroxy-5-methoxybenzaldehyde, 25.9 mmol ofK2CO3,15.6 mmol of ethyl iodide, and 30 mL of DMF. The reaction mixture was allowed to stir at 25 °C for 12 hours. After total consumption of the starting reagent (monitored by CCF), the reaction mixture was diluted with H2O (100 mL) and extracted with AcOEt (60 mL x 4). The organic phase was washed with H2O (50 mL) and saturated sodium chloride solution (brine) (20 mL). Subsequently, the organic phase was concentrated and purified by column chromatography (petroleum ether/AcOEt: 20/1 → 0/1). 2.7 g (80% yield) of precursor II-14i were obtained.

<u>Step II-J-*ii*:</u>

*Precursor 11-14ii:*

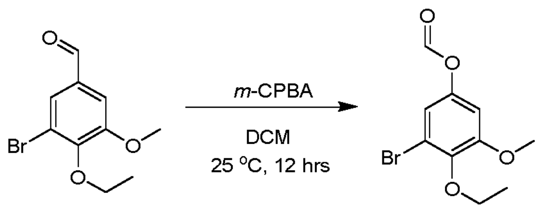


Into a round bottom flask containing 13.1 mmol of the 3-bromo-4-ethoxy-5-methoxybenzaldehyde (precursor *II-14i*) in 25 mL of DCM, was added 19.7 mmol m-CPBA at 0 °C. The reaction mixture was allowed to stir at 0 °C for 0.5 hours, then allowed to stir at 25 °C for 12 hours. After total consumption of the starting reagent (monitored by LC-MS), the reaction mixture was diluted with a saturated solution of Na2SO3, washed with a saturated solution of K2CO3 and finally filtered. Subsequently, the filtrate was extracted with DCM (30 mL x 4), and the organic phase was washed with H2O (50 mL) and saturated sodium chloride solution (brine) (40 mL). Subsequently, the organic phase was concentrated and used without further purification. 3.0 g (83% yield) of precursor II-14ii were obtained.

<u>Step II-J-*iii*:</u>

*Precursor 11-14iii:*

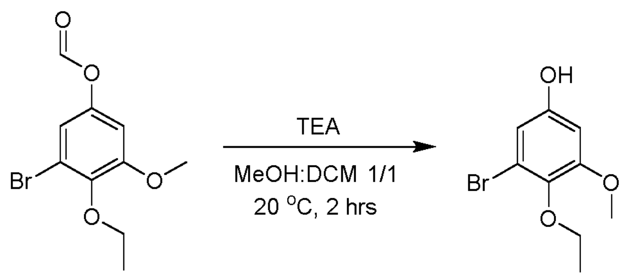


Into a round bottom flask was added 10.9 mmol of the 3-bromo-4-ethoxy-5-methoxyphenyl formate (precursor *II-14ii*), 10 mL of MeOH, 10 mL of DCM and 11.1 mmol of TEA. Subsequently, the reaction mixture was allowed to stir at 20 oC for 2 hours. After total consumption of the starting reagent (monitored by LC-MS), the reaction mixture was diluted withH2Oand acidified with a solution of HCl (1 N) to pH 6. The aqueous phase was extracted with AcOEt (50 mL x 4). Subsequently, the organic phase was concentrated and used without further purification. 2.8 g of the precursor II-14iii were obtained.

Step *II-J-iv:*

*Precursor II-14iv:*

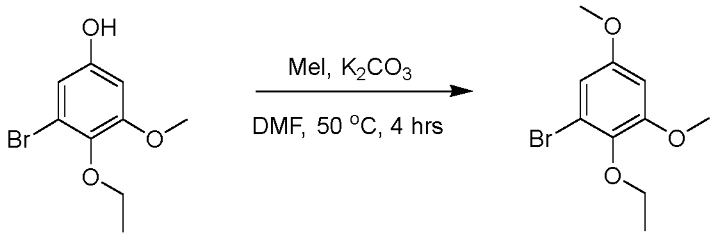


Into a round bottom flask containing 11.3 mmol of the 3-bromo-4-ethoxy-5-methoxyphenol *(precursor* II-14iii), 17 mmol of K2CO3 and 25 mL of DMF, was added 34 mmol of iodomethane. The reaction mixture was allowed to stir at 25 °C for 12 hours. After total consumption of the starting reagent (monitored by LC-MS), the reaction mixture was diluted with H2O and extracted with AcOEt (50 mL x 3). The organic phase was washed with saturated sodium chloride solution (brine) (30 mL x 2) and concentrated. The resulting product was purified by column chromatography (petroleum ether/AcOEt: 5/1 → 1/1). 2.5 g (85% yield) of precursor II-14iv were obtained.

Step *II-J-v:*

*Intermediate II-14:*

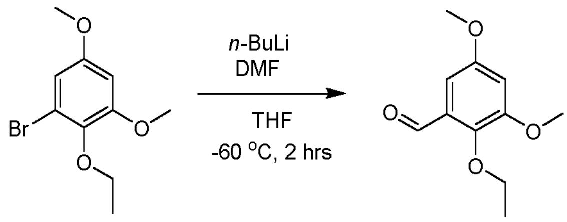


Into a round bottom flask containing 4.3 mmol of 1-bromo-2-ethoxy-3,5-dimethoxybenzene (precursor *11-14iv)* in 10 mL of THF was added a solution of n-BuLi (2.5 M, 3.4 mL) at -70 °C under N2 atmosphere. The reaction mixture was maintained at -70 °C for 1.5 hours. Subsequently, 6.38 mmol of DMF at -70 oC under N2 atmosphere was added. The reaction mixture was allowed to stir at - 70 °C for 0.5 hours. After total consumption of the starting reagent (monitored by LC-MS), the reaction mixture was diluted with H2O (10 mL) and extracted with AcOEt (10 mL x 4). The organic phase was washed with saturated sodium chloride solution (brine) (30 mL x 2) and concentrated. The resulting product was purified by column chromatography (petroleum ether/AcOEt: 20/1 → 0/1). 500 mg (25% yield) of intermediate II-14 were obtained.

**Method II-K:**

*Intermediate II-15:*

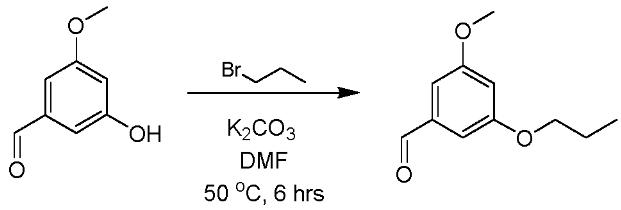


Into a round bottom flask were added 5.92 mmol of 3-hydroxy-5-methoxybenzaldehyde, 8.9 mmol of 1-bromopropane, 8.9 mmol of K2CO3 and10 mL of DMF. The reaction mixture was allowed to stir at 50 °C for 12 hours. After total consumption of the starting reagent (monitored by CCF), the reaction mixture was diluted with H2O (50 mL) and extracted with AcOEt (30 mL x 3). The organic phase was washed with H2O (50 mL) and saturated sodium chloride solution (brine) (20 mL). Subsequently, the organic phase was concentrated and used for the next step without further purification. 1 g (87% yield) of intermediate II-15 was obtained.

<u>Method II-L:</u>

*Intermediates II-16:*

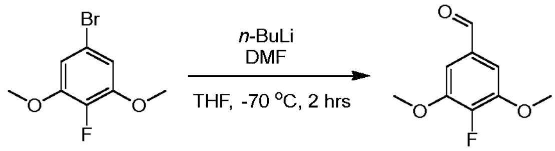


Into a round bottom flask were added 4.25 mmol of the 5-bromo-2-fluoro-1,3-dimethoxybenzene and 10 mL of THF. Subsequently, a solution of n-BuLi (2.5 M, 3.4 mL) at -70 °C under N2 atmosphere was added dropwise while maintaining stirring for 1.5 hours. Subsequently, under N2 atmosphere, 6.38 mmol of DMF was added and the reaction mixture was allowed to stir at -70 °C for 0.5 hours. After total consumption of the starting reagent (monitored by LC-MS), the reaction mixture was diluted with H2O (10 mL) and extracted with AcOEt (10 mL x 4). Subsequently, the organic phase was concentrated and purified by column chromatography (petroleum ether/AcOEt: 20/1 → 0/1). 490 mg (63% yield) of intermediate II-16 were obtained.

## <u>Examples</u>

The following examples, described in detail, serve to illustrate embodiments of the present invention without, however, having any limiting character to the scope of protection thereof.

The compound(s) of general Formula I of the present invention were obtained from condensation of the intermediates of Formula III with commercial aldehydes or with the intermediates of Formula II described above, synthesized according to the various methodologies, described above and schematized in General Scheme 1, but not limited thereto.

**Example 1.** 6-(4-chlorophenyl)-N'-[(E)-(3,5-dimethoxyphenyl)methylidene]pyrazine-2-carbohydrazide (Compound 1):

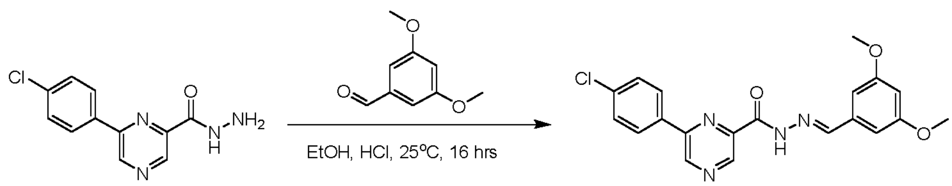


In a round bottom flask containing 6.0 mmol of 6-(4-chlorophenyl)pyrazine-2-carbohydrazide (intermediate III-1) in 20 mL of EtOH, 6.03 mmol of 3,5-dimethoxybenzaldehyde and one drop of concentrated HCl were added. The reaction medium was allowed to stir at 25 °C for 16 hours. After total starting reagent consumption (monitored by LC-MS), the solvent was concentrated and the residue was treated with 20 mL of H2O. The mixture was filtered and the

washed solid was with H2O (20 mL x 3). 2 g (84% yield) of compound 1 were obtained.

Compounds **2** to **167** and compounds **169** to **212** as per Table 15 are obtained by the procedure described for Example 1 (Compound 1) by altering the corresponding intermediates II and III.

As an exception to the conditions mentioned, for the synthesis of compound **166,** acetic acid was employed as catalyst and the temperature was adjusted to 80 °C for 6 hours.

**Table 15.** Compounds obtained, from the corresponding reagents, following method I-A.

| Compound No. | Structure | 1H-NMR/ MS m/z [M+H] + |
|---|---|---|
| 1 | 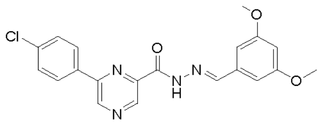 6-(4-chlorophenyl)-*N*'-[*(E)*-(3,5-dimethoxyphenyl) methylidene]pyra zine-2-carbohydrazide. | 1H-NMR, 400*MHz,* DMSO-d6 $\delta$ = 12.13 (s, 1H), 9.56 (s, 1H), 9.21 (s, 1H), 8.65 (s, 1H), 8.54 - 8.42 (m, 2H), 7.72 - 7.62 (m, 2H), 6.93 (d, J = 2.3 Hz, 2H), 6.62 (t, J = 2.3 Hz, 1H), 3.82 (s, 6H). [M+H] +: 397.1. |
| 2 | 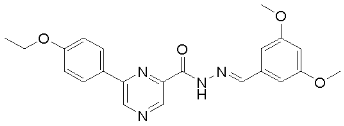 *(E)-N*'-(3,5-dimethoxybenzylidene)-6-(4-ethoxyphenyl) pyrazine-2-carbohydrazide. | 1H-NMR, 400*MHz,* DMSO-d6 $\delta$ = 12,06 (s, 1H), 9,46 (s, 1H), 9,10 (s, 1H), 8,66 (s, 1H), 8,42 - 8,39 (m, 2H), 7,13 - 7,11 (m, 2H), 6,93 (d, *J* = 2,3 Hz, 2H), 6,62 (t, *J* = 2,3 Hz, 1H), 4,15 (q, *J* = 6,97 Hz, 2 H), 3,82 (s, 6H), 1,38 (t, *J* = 6,97 Hz, 3 H). [M+H]+: 407,1. |
| 3 | 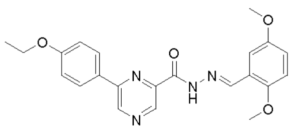 *(E)-N*'-(2,5- dimethoxybenzylidene)-6-(4-ethoxyphenyl) pyrazine-2-carbohydrazide. | 1H-NMR, 400MHz CDCl3 $\delta$ 10,91 (s, 1 H) 9,36 (s, 1 H) 9,20 (s, 1 H) 8,49 - 8,58 (m, 1 H) 8,39 (dd, J = 5,87, 2,08 Hz, 2 H) 8,01 (d, J = 8,80 Hz, 2 H) 7,80 - 7,90 (m, 1 H) 7,08 (d, J = 8,80 Hz, 2 H) 4,15 (q, J = 6,97 Hz, 2 H) 3,94 (s, 3 H) 1,49 (t, J = 6,97 Hz, 3 H). [M+H]+: 407,1. |
| 4 | 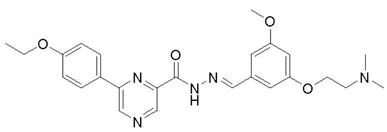 *(E)-N*'-(3-(2-(dimethylamino)ethoxy)- 5-methoxybenzylidene)-6-(4- ethoxyphenyl)pyrazine-2-carbohydrazide. | 1H-NMR, 400MHz CDCl3 $\delta$ 10,78 (br s, 1 H) 9,34 (s, 1 H) 9,17 (s, 1 H) 8,36 (s, 1 H) 8,00 (br d, J = 8,38 Hz, 2 H) 7,07 (br d, J = 8,38 Hz, 2 H) 7,01 (br s, 1 H) 6,96 (br s, 1 H) 6,58 (br s, 1 H) 4,14 (br d, J = 6,39 Hz, 3 H) 4,02 - 4,26 (m, 1 H) 3,84 (s, 3 H) 2,80 (br s, 2 H) 2,39 (s, 6 H) 1,48 (br t, J = 6,84 Hz, 3 H). [M+H]+: 464,3. |
| 5 | 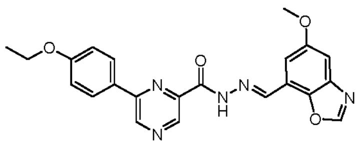 *(E)*-6-(4-ethoxyphenyl)-*N*'-((5- methoxybenzo[d]oxazol-7-yl)methylene)pyrazine-2- carbohydrazide. | 1H-NMR, 400MHz CDCl3 $\delta$ 10,97 (s, 1 H) 9,36 (s, 1 H) 9,19 (s, 1 H) 8,92 (s, 1 H) 8,18 (s, 1 H) 7,96 - 8,08 (m, 2 H) 7,50 (s, 1 H) 7,37 (s, 1 H) 7,08 (br d, J = 8,38 Hz, 2 H) 4,15 (q, J = 6,84 Hz, 2 H) 3,92 (s, 3 H) 1,49 (br t, J = 6,95 Hz, 3 H). [M+H]+: 418,1. |

(continued)

| Compound No. | Structure | 1H-NMR/ MS m/z [M+H] + |
|---|---|---|
| 6 | 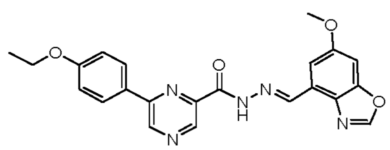 *(E)*-6-(4-ethoxyphenyl)-*N'*-((6- methoxybenzo[d]oxazol-4-yl)methylene)pyrazine-2- carbohydrazide. | 1H-NMR, 400MHz CDCl3 $\delta$ 11,00 (s, 1 H) 9,37 (s, 1 H) 9,20 (s, 1 H) 9,02 (s, 1 H) 8,09 (s, 1 H) 8,04 (d, J = 8,82 Hz, 2 H) 7,79 (d, J = 2,20 Hz, 1 H) 7,20 (d, J = 2,20 Hz, 1 H) 7,09 (d, J = 8,82 Hz, 2 H) 4,16 (q, J = 7,06 Hz, 2 H) 3,95 (s, 2 H) 3,90 - 3,98 (m, 1 H) 1,50 (t, J = 6,95 Hz, 3 H). [M+H]+: 418,1. |
| 7 | 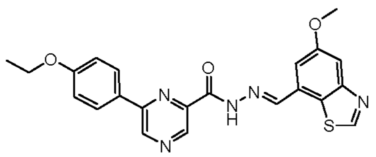 *(E)*-6-(4-ethoxyphenyl)-*N'*-((5- methoxybenzo[d]oxazol-7-yl)methylene)pyrazine-2- carbohydrazide. | 1H-NMR, 400MHz CDCl3 $\delta$ 10,98 (s, 1 H) 9,33 (s, 1 H) 9,14 (br d, J = 17,64 Hz, 2 H) 8,79 (s, 1 H) 8,00 (br d, J = 8,38 Hz, 2 H) 7,69 (s, 1 H) 7,26 (br s, 1 H) 7,07 (br d, J = 8,38 Hz, 2 H) 4,13 (q, J = 6,69 Hz, 2 H) 3,94 (s, 3 H) 1,48 (br t, J =6,95 Hz, 3 H). [M+H] +: 434,1. |
| 8 | 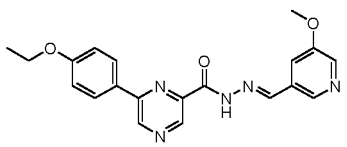 *(E)*-6-(4-ethoxyphenyl)-*N'*-((5- methoxypyridin-3- yl) methylene)pyrazine-2-carbohydrazide. | 1H-NMR, 400MHz CDCl3 $\delta$ 10,91 (s, 1 H) 9,36 (s, 1 H) 9,20 (s, 1 H) 8,49 - 8,58 (m, 1 H) 8,39 (dd, J = 5,87, 2,08 Hz, 2 H) 8,01 (d, J = 8,80 Hz, 2 H) 7,80 - 7,90 (m, 1 H) 7,08 (d, J = 8,80 Hz, 2 H) 4,15 (q, J = 6,97 Hz, 2 H) 3,94 (s, 3 H) 1,49 (t, J = 6,97 Hz, 3 H). [M+H]+: 378,1. |
| 9 | 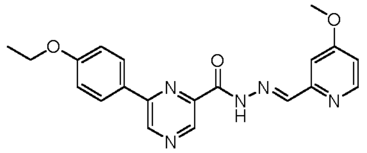 *(E)*-6-(4-ethoxyphenyl)-*N'*-((4-methoxypyridin-2-yl) methylene)pyrazine-2-carbohydrazide. | 1H-NMR, 400MHz, CDCl3 $\delta$ = 10,95 (s, 1H), 9,37 (s, 1H), 9,21 (s, 1H), 8,47 - 8,38 (m, 2H), 8,02 (d, J = 8,8 Hz, 2H), 7,76 (d, J = 2,6 Hz, 1H), 7,08 (d, J =9,0 Hz, 2H), 6,88 (dd, J = 2,6, 5,7 Hz, 1H), 4,16 (q, J = 6,9 Hz, 2H), 3,96 (s, 3H), 1,50 (t, J = 7,1 Hz, 3H). [M+H]+: 378,1. |
| 10 | 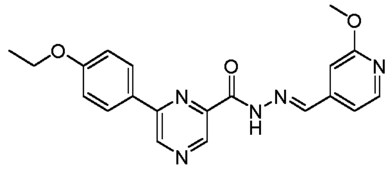 *(E)*-6-(4-ethoxyphenyl)-*N'*-((2-methoxypyridin-4-yl) methylene)pyrazine-2-carbohydrazide. | 1H-NMR, 400MHz, CDCl3 $\delta$ = 10,90 (s, 1H), 9,36 (s, 1H), 9,20 (s, 1H), 8,51 (s, 1H), 8,24 (d, J =5,4 Hz, 1H), 8,01 (d, J =8,8 Hz, 2H), 7,41 (d, J =4,3 Hz, 1H), 7,16 - 6,97 (m, 3H), 4,15 (q, J =7,0 Hz, 2H), 3,99 (s, 3H), 1,49 (t, J =7,0 Hz, 3H). [M+H]+: 378,1. |
| 11 | 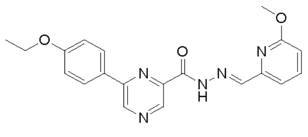 *(E)*-6-(4-ethoxyphenyl)-*N'*-((6-methoxypyridin-2-yl) methylene)pyrazine-2-carbohydrazide. | 1H-NMR, 400MHz, CDCl3 $\delta$ = 11,12-10,68 (m, 1H), 9,37 (s, 1H), 9,20 (s, 1H), 8,42 (s, 1H), 8,02 (d, J = 8,8 Hz, 2H), 7,83 (d, J = 7,3 Hz, 1H), 7,66 (t, J = 7,8 Hz, 1H), 7,08 (d, J = 8,8 Hz, 2H), 6,80 (d, J = 8,1 Hz, 1H), 4,15 (q, J = 7,0 Hz, 2H), 3,99 (s, 3H), 1,49 (t, J = 7,0 Hz, 3H). [M+H]+: 378,1. |

(continued)

| Compound No. | Structure | 1H-NMR/ MS m/z [M+H] + |
|---|---|---|
| 12 | 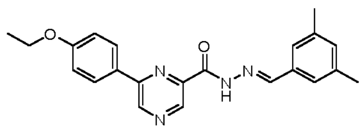 *(E)-N'*-(3,5- dimethoxybenzylidene)-6-(4-ethoxyphenyl) pyrazine-2-carbohydrazide. | 1H-NMR, 400MHz, CDCl3 $\delta$ 12,03 (s, 1H), 9,46 (s, 1H), 9,11 (s, 1H), 8,64 (s, 1H), 8,40 (d, J = 8,8 Hz, 2H), 7,39 (s, 2H), 7,12 (d, J = 8,7 Hz, 3H), 4,15 (q, J = 6,8 Hz, 2H), 2,34 (s, 6H), 1,38 (t, J = 7,0 Hz, 3H). [M+H]+: 375,2. |
| 13 | 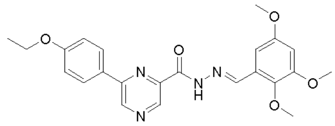 *(E)*-6-(4-ethoxyphenyl)-*N'*-(2,3,5-trimethoxybenzylidene) pyrazine-2-carbohydrazide. | 1H-NMR, 400*MHz,* DMSO-d6 $\delta$ 12,18 (s, 1H), 9,43 (s, 1H), 9,08 (s, 1H), 8,95 (s, 1H), 8,38 (d, J = 7,9 Hz, 2H), 7,11 (d, J = 8,2 Hz, 2H), 6,96 (d, J = 2,0 Hz, 1H), 6,71 (d, J = 2,0 Hz, 1H), 4,12 (q, J = 7,1 Hz, 2H), 3,85 - 3,72 (m, 9H), 1,36 (t, J = 6,9 Hz, 3H). [M+H]+: 437,1. |
| 14 | 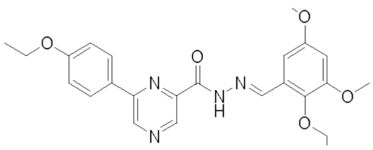 *(E)-N'*-(2-ethoxy-3,5-dimethoxybenzylidene)-6-(4-ethoxyphenyl)pyrazine-2-carbohydrazide. | 1H-NMR, 400*MHz,* DMSO-d6 $\delta$ 12.20 (s, 1H), 9.43 (s, 1H), 9.07 (s, 1H), 8.94 (s, 1H), 8.37 (d, J = 8.8 Hz, 2H), 7.11 (d, J = 8.8 Hz, 2H), 6.97 (d, J = 2.9 Hz, 1H), 6.70 (d, J = 2.9 Hz, 1H), 4.12 (q, J = 6.8 Hz, 2H), 3.95 (q, J = 7.1 Hz, 2H), 3.79 (d, J = 9.7 Hz, 6H), 1.38 - 1.27 (m, 6H). [M+H]+: 451,2. |
| 15 | 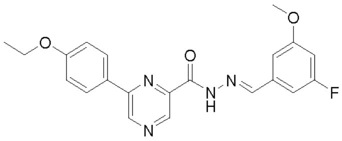 *(E)*-6-(4-ethoxyphenyl)-*N'*-(3-fluoro-5-methoxybenzylidene)pyrazine-2-carbohydrazide. | 1H-NMR, 400*MHz,* DMSO-d6 $\delta$ 12,17 (s, 1H), 9,47 (s, 1H), 9,11 (s, 1H), 8,69 (s, 1H), 8,40 (d, J = 8,9 Hz, 2H), 7,20 - 7,15 (m, 2H), 7,12 (d, J = 8,8 Hz, 2H), 6,96 (td, J = 2,3, 10,8 Hz, 1H), 4,15 (q, J = 7,0 Hz, 2H), 3,85 (s, 3H), 1,39 (t, J = 6,9 Hz, 3H). [M+H]+: 395,1. |
| 16 | 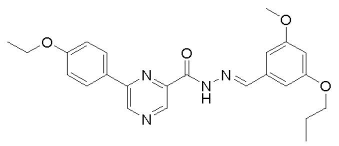 *(E)*-6-(4-ethoxyphenyl)-*N'*-(3-methoxy-5-propoxybenzylidene)pyrazine-2-carbohydrazide. | 1H-NMR, 400*MHz,* DMSO-d6 $\delta$ 12,04 (s, 1H), 9,43 (s, 1H), 9,07 (s, 1H), 8,61 (s, 1H), 8,37 (d, J = 8,8 Hz, 2H), 7,09 (d, J = 8,8 Hz, 2H), 6,88 (dd, J = 2,1, 3,4 Hz, 2H), 6,57 (t, J = 2,2 Hz, 1H), 4,12 (q, J = 6,9 Hz, 2H), 3,95 (t, J = 6,5 Hz, 2H), 3,78 (s, 3H), 1,72 (sxt, J = 7,0 Hz, 2H), 1,35 (t, J = 6,9 Hz, 3H), 0,97 (t, J = 7,3 Hz, 3H). [M+H]+: 435,2. |
| 17 | 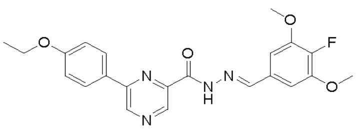 *(E)*-6-(4-ethoxyphenyl)-*N'*-(4-fluoro-3,5-dimethoxybenzylidene)pyrazine-2-carbohydrazide. | 1H-NMR, 400*MHz,* DMSO-d6 $\delta$ 12,10 (s, 1H), 9,47 (s, 1H), 9,11 (s, 1H), 8,68 (s, 1H), 8,41 (d, J = 8,8 Hz, 2H), 7,18 (d, J = 7,2 Hz, 2H), 7,12 (d, J = 8,8 Hz, 2H), 4,16 (q, J = 6,9 Hz, 2H), 3,92 (s, 6H), 1,39 (t, J = 6,9 Hz, 3H). [M+H]+: 425,1. |

(continued)

| Compound No. | Structure | 1H-NMR/ MS m/z [M+H] + |
|---|---|---|
| 18 | 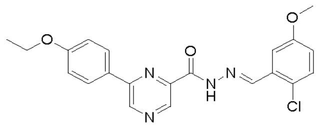 *(E)-N'*-(2-chloro-5-methoxybenzylidene)-6-(4-ethoxyphenyl)pyrazine-2-carbohydrazide. | 1H-NMR, 400MHz,DMSO-d6 $\delta$ = 12,39 (s, 1H), 9,46 (s, 1H), 9,10 (d, J = 5,0 Hz, 2H), 8,53 - 8,28 (m, 2H), 7,55 (d, J = 2,9 Hz, 1H), 7,47 (d, J = 8,9 Hz, 1H), 7,24 - 6.96 (m, 3H), 4,14 (q, J = 7,0 Hz, 2H), 3,84 (s, 3H), 1,38 (t, J =7,0 Hz, 3H). [M+H]+: 411,1. |
| 19 | 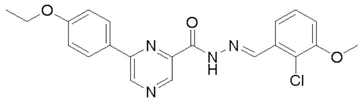 *(E)-N'*-(2-chloro-3-methoxybenzylidene)-6-(4-ethoxyphenyl)pyrazine-2-carbohydrazide. | 1H-NMR, 400MHz,DMSO-d6 $\delta$ = 12,37 (s, 1H), 9,46 (s, 1H), 9,16 (s, 1H), 9,11 (s, 1H), 8,46 - 8,34 (m, 2H), 7,67 (dd, J = 1,2, 7,9 Hz, 1H), 7,42 (t, J = 8,0 Hz, 1H), 7,25 (dd, J = 1,2, 8,3 Hz, 1H), 7,16 - 7,10 (m, 2H), 4,15 (q, J = 7,0 Hz, 2H), 3,97 - 3,84 (m, 3H), 1,38 (t, J = 7,0 Hz, 3H). [M+H]+: 411,1. |
| 20 | 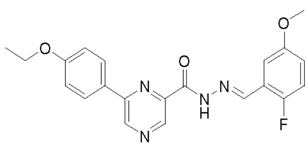 *(E)*-6-(4-ethoxyphenyl)-*N'*-(2-fluoro-5-methoxybenzylidene)pyrazine-2-carbohydrazide. | 1H-NMR, 400MHz,DMSO-d6 $\delta$ = 12,23 (s, 1H), 9,46 (s, 1H), 9,11 (s, 1H), 8,97 (s, 1H), 8,47 - 8,35 (m, 2H), 7,43 (dd, J = 3,2, 5,6 Hz, 1H), 7,28 (t, J = 9,5 Hz, 1H), 7,16 - 7,05 (m, 3H), 4,14 (q, J = 7,0 Hz, 2H), 3,82 (s, 3H), 1,38 (t, J = 7,0 Hz, 3H). [M+H]+: 395,2. |
| 21 | 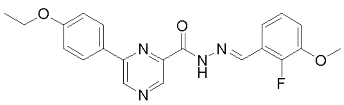 *(E)*-6-(4-ethoxyphenyl)-*N'*-(2-fluoro-3-methoxybenzylidene)pyrazine-2-carbohydrazide. | 1H-NMR, 400*MHz,* DMSO-d6 $\delta$ = 12,23 (s, 1H), 9,46 (s, 1H), 9,11 (s, 1H), 9,00 (s, 1H), 8,45 - 8,38 (m, 2H), 7,58 - 7,49 (m, 1H), 7,31 - 7,22 (m, 2H), 7,13 (d, J = 8,9 Hz, 2H), 4,15 (q, J = 7,0 Hz, 2H), 3,89 (s, 3H), 1,38 (t, J = 7,0 Hz, 3H). [M+H]+: 395,2. |
| 22 | 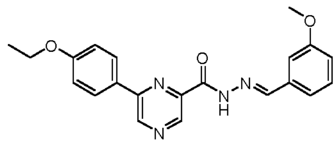 *(E)*-6-(4-ethoxyphenyl)-*N'*-(3-methoxybenzylidene) pyrazine-2-carbohydrazide. | 1H-NMR, 400*MHz,* DMSO-d6 $\delta$ 12,06 (s, 1H), 9,45 (s, 1H), 9,10 (s, 1H), 8,70 (s, 1H), 8,44 - 8,35 (m, 2H), 7,41 (t, J = 8,0 Hz, 1H), 7,34 (dt, J = 7,6, 1,6 Hz, 2H), 7,15 - 7,07 (m, 2H), 7,07 - 7,01 (m, 1H), 4,14 (q, J = 7,0 Hz, 2H), 4,05 (dt, J = 1,8, 0,9 Hz, 1H), 3,83 (s, 3H), 1,37 (t, J = 7,0 Hz, 3H). [M+H] +: 377,17. |
| 23 | 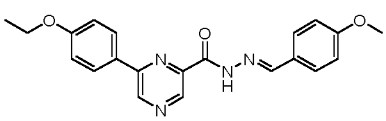 *(E)*-6-(4-ethoxyphenyl)-*N'*-(4-methoxybenzylidene) pyrazine-2-carbohydrazide. | 1H-NMR, 400*MHz,* DMSO-d6 $\delta$ 11,67 (s, 1H), 9,35 (s, 1H), 9,07 (s, 1H), 8,64 (s, 1H), 8,31 (d, J = 8,4 Hz, 2H), 7,71 (d, J = 8,2 Hz, 2H), 7,17 - 7,07 (m, 2H), 7,03 (d, J = 8,6 Hz, 2H), 4,16 (q, J = 7,0 Hz, 2H), 3,83 (s, 3H), 1,38 (t, J = 7,0 Hz, 3H). [M+H]+: 377,17. |

(continued)

| Compound No. | Structure | 1H-NMR/ MS m/z [M+H] + |
|---|---|---|
| 24 | 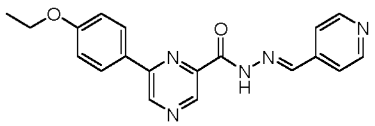<br>*(E)*-6-(4-ethoxyphenyl)-*N'*-(pyridin-4-ylmethylene) pyrazine-2-carbohydrazide. | 1H-RMN, 600 MHz, DMSO-d6 $\delta$ 12,31 (s, 1H), 9,48 (s, 1H), 9,12 (s, 1H), 8,74 (s, 1H), 8,70 (d, J = 5,5 Hz, 2H), 8,40 (d, J = 8,5 Hz, 2H), 7,72 (d, J = 5,8 Hz, 2H), 7,12 (d, J = 8,5 Hz, 2H), 4,15 (q, J = 6,9 Hz, 2H), 1,38 (t, J = 6,9 Hz, 3H). [M+H]+: 348,11. |
| 25 | 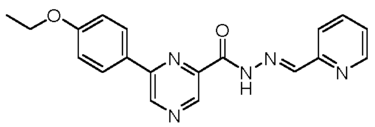<br>*(E)*-6-(4-ethoxyphenyl)-N'-(pyridin-2-ylmethylene) pyrazine-2-carbohydrazide. | 1H-RMN, 600 MHz, DMSO-d6 $\delta$ 9,51 (s, 1H), 9,17 (s, 1H), 8,84 (dd, J = 4,9, 1,7 Hz, 1H), 8,40 - 8,34 (m, 2H), 8,15 (td, J = 7,7, 1,7 Hz, 1H), 7,88 (d, J = 7,8 Hz, 1H), 7,83 (s, 1H), 7,69 (ddd, J = 7,6, 4,9, 1,2 Hz, 1H), 7,29 - 7,22 (m, 2H), 4,19 (q, J = 7,0 Hz, 2H), 1,41 (t, J = 7,0 Hz, 3H). [M+H]+: 348,09. |
| 26 | 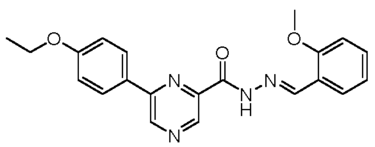<br>*(E)*-6-(4-ethoxyphenyl)-*N'-(2*-methoxybenzylidene) pyrazine-2-carbohydrazide. | 1H-RMN, 600 MHz, DMSO-d6 12,15 (s, 1H), 9,45 (s, 1H), 9,10 (s, 1H), 9,06 (s, 1H), 8,47 - 8,37 (m, 2H), 7,93 (dd, J = 7,7, 1,8 Hz, 1H), 7,46 (ddd, J = 8,3, 7,3, 1,8 Hz, 1H), 7,17 - 7,10 (m, 3H), 7,08 - 7,04 (m, 1H), 4,15 (q, J = 7,0 Hz, 2H), 3,90 (s, 3H), 1,38 (t, J = 7,0 Hz, 3H). [M+H]+: 377,16. |
| 27 | 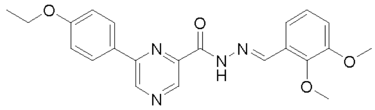<br>*(E)-N'*-(2,3-dimethoxybenzylidene)-6-(4-ethoxyphenyl) pyrazine-2-carbohydrazide. | 1H-RMN, 600 MHz, DMSO-d6 $\delta$ 12,18 (s, 1H), 9,45 (s, 1H), 9,10 (s, 1H), 8,99 (s, 1H), 8,44 - 8,39 (m, 2H), 7,54 (dd, J = 6,8, 2,6 Hz, 1H), 7,20 - 7,10 (m, 4H), 4,16 (p, J = 7,0 Hz, 2H), 3,85 (d, J = 13,1 Hz, 6H), 1,38 (t, J = 7,0 Hz, 3H). [M+H]+: 407,11. |
| 28 | 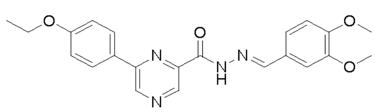<br>*(E)-N'*-(3,4-dimethoxybenzylidene)-6-(4-ethoxyphenyl) pyrazine-2-carbohydrazide. | 1H-RMN, 600 MHz, DMSO-d6 $\delta$ 11,94 (s, 1H), 9,45 (s, 1H), 9,09 (s, 1H), 8,65 (s, 1H), 8,43 - 8,37 (m, 2H), 7,39 (d, J = 1,9 Hz, 1H), 7,27 (dd, J = 8,3, 1,9 Hz, 1H), 7,15 - 7,09 (m, 2H), 7,07 (d, J = 8,3 Hz, 1H), 4,15 (q, J = 7,0 Hz, 2H), 3,84 (d, J = 10,6 Hz, 6H), 1,38 (t, J = 7,0 Hz, 3H). [M+H]+: 407,11. |
| 29 | 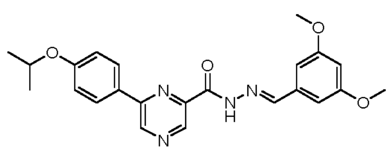<br>*(E)-N'*-(3,5-dimethoxybenzylidene)-6-(4-isopropoxyphenyl)pyrazine-2-carbohydrazide. | 1H-NMR, 400MHz, DMSO-d6 $\delta$ = 12,06 (s, 1H), 9,45 (s, 1H), 9,09 (s, 1H), 8,65 (s, 1H), 8,40 - 8,37 (m, 2H), 7,11 - 7,09 (m, 2H), 6,92 (d, J = 2,3 Hz, 2H), 6,61 (t, J = 2,3 Hz, 1H), 4,77 (sep, J = 6,40 Hz, 1 H), 3,81 (s, 6H), 1,32 (d, J = 6,40 Hz, 6 H). [M+H]+: 421,1. |

(continued)

| Compound No. | Structure | 1H-NMR/ MS m/z [M+H] + |
|---|---|---|
| 30 | 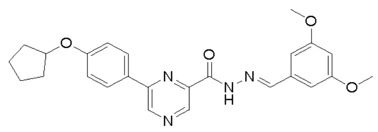 6-[4-(cyclopentyloxy)phenyl]-*N*'-[*(E)*-(3,5-dimethoxyphenyl)methylidene] pyrazine-2-carbohydrazide. | 1H-NMR, 400MHz, DMSO-d6 δ = 12,03 (s, 1H), 9,41 (s, 1H), 9,05 (s, 1H), 8,61 (s, 1H), 8,34 (d, J = 8,8 Hz, 2H), 7,04 (d, J = 8,8 Hz, 2H), 6,88 (d, J = 2,2 Hz, 2H), 6,57 (t, J = 2,2 Hz, 1H), 4,92 (br t, J = 5,8 Hz, 1H), 3,77 (s, 6H), 2,01 - 1,87 (m, 2H), 1,75 - 1,53 (m, 6H). [M+H]+: 447,1. |
| 31 | 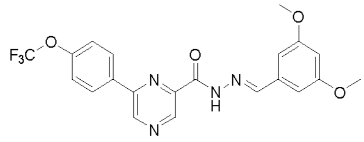 *N*'-[*(E)*-(3,5-dimethoxyphenyl)methylidene]-6-[4-(trifluoromethoxy)phenyl]pyrazine-2-carbohydrazide. | 1H-NMR, 400*MHz,* DMSO-d6 δ = 12,13 (s, 1H), 9,56 (s, 1H), 9,23 (s, 1H), 8,65 (s, 1H), 8,57 (d, J = 8,8 Hz, 2H), 7,60 (d, J = 8,4 Hz, 2H), 6,93 (d, J = 2,2 Hz, 2H), 6,62 (t, J = 2,2 Hz, 1H), 3,82 (s, 6H). [M+H]+: 447,0. |
| 32 | 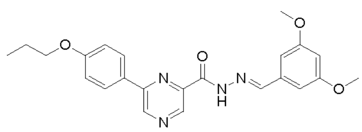 *N*'-[*(E)*-(3,5-dimethoxyphenyl)methylidene]-6-(4-propoxyphenyl)pyrazine-2-carbohydrazide. | 1H-NMR, 400*MHz,* DMSO-d6 δ = 12,06 (s, 1H), 9,47 (s, 1H), 9,13 - 9,09 (m, 1H), 8,69 - 8,64 (m, 1H), 8,44 - 8,37 (m, 2H), 7,13 (d, J = 8,8 Hz, 2H), 6,93 (d, J = 2,3 Hz, 2H), 6,60 (s, 1H), 4,09 - 4,02 (m, 2H), 3,82 (s, 6H), 1,81 - 1,75 (m, 2H), 1,02 (t, J = 7,3 Hz, 3H). [M+H]+: 421,2. |
| 33 | 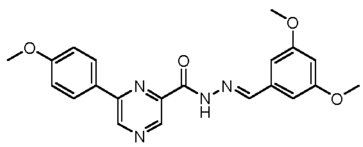 *N*'-[*(E)*-(3,5-dimethoxyphenyl)methylidene]-6-(4-propoxyphenyl)pyrazine-2-carbohydrazide. | 1H-NMR, 400*MHz,* DMSO-d6 δ = 12,07 (s, 1H), 9,47 (s, 1H), 9,10 (s, 1H), 8,65 (s, 1H), 8,46 - 8,37 (m, 2H), 7,14 (d, J = 9,0 Hz, 2H), 6,92 (d, J = 2,2 Hz, 2H), 6,61 (t, J = 2,2 Hz, 1H), 3,87 (s, 3H), 3,81 (s, 6H). [M+H]+: 393,1. |
| 34 | 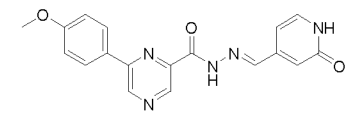 *(E)*-6-(4-methoxyphenyl)-*N*'-((2-oxo-1,2-dihydropyridin-4-yl)methylene) pyrazine-2-carbohydrazide. | 1H-NMR, 400*MHz,* DMSO-d6 δ 12,28 (br s, 1H), 11,63 (br s, 1H), 9,47 (s, 1H), 9,11 (s, 1H), 8,53 (s, 1H), 8,40 (br d, J = 8,6 Hz, 2H), 7,42 (br d, J = 6,7 Hz, 1H), 7,13 (br d, J = 8,6 Hz, 2H), 6,64 (br d, J = 6,6 Hz, 1H), 6,55 (s, 1H), 3,86 ppm (s, 3H). [M+H]+: 350,0. |
| 35 | 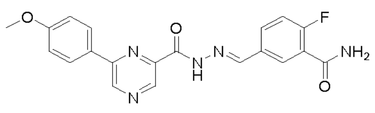 *(E)*-2-fluoro-5-((2-(6-(4-methoxyphenyl)pyrazine-2-carbonyl) hydrazono)methyl)benzamide. | 1H-NMR, 400*MHz,* DMSO-d6 δ 12,14 (s, 1H), 9,46 (s, 1H), 9,11 (s, 1H), 8,74 (s, 1H), 8,40 (d, J = 8,9 Hz, 2H), 8,06 (dd, J = 7,0, 2,0 Hz, 1H), 7,89 - 7,94 (m, 1H), 7,86 (br s, 1H), 7,77 (brs, 1H), 7,41 (t, J = 9,4 Hz, 1H), 7,13 (d, J = 8,8 Hz, 2H), 3,87 (s, 3H). [M+H]+: 394,0. |

(continued)

| Compound No. | Structure | 1H-NMR/ MS m/z [M+H] + |
|---|---|---|
| 36 | 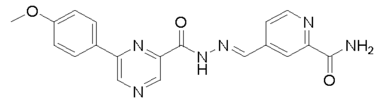 *(E)*-4-((2-(6-(4-methoxyphenyl)pyrazine -2-carbonyl) hydrazono)methyl) picolinamide. | 1H-NMR, 400*MHz,* DMSO-d6 $\delta$ 12,38 (br s, 1H), 9,48 (s, 1H), 9,13 (s, 1H), 8,80 (s, 1H), 8,74 (d, J = 4,9 Hz, 1H), 8,37-8,43 (m, 3H), 8,19 (br d, J = 1,7 Hz, 1H), 7,88 (dd, J = 5,0, 1,6 Hz, 1H), 7,74 (br d, J = 1,8 Hz, 1H), 7,14 (d, J = 8,8 Hz, 2H), 3,87 (s, 3H). [M+H]+: 377,0. |
| 37 | 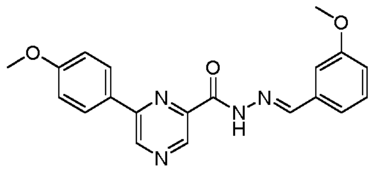 *(E)-N'*-(3-methoxybenzylidene)-6-(4-methoxyphenyl) pyrazine-2-carbohydrazide. | 1H-NMR, 400*MHz,* DMSO-d6 $\delta$ 12,05 (s, 1H), 9,45 (s, 1H), 9,09 (s, 1H), 8,68 (s, 1H), 8,40 (d, J = 8,8 Hz, 2H), 7,43 - 7,37 (m, 1H), 7,35 - 7,29 (m, 2H), 7,12 (d, J = 8,8 Hz, 2H), 7,04 (dd, J = 1,5, 9,3 Hz, 1H), 3,85 (s, 3H), 3,82 (s, 3H). [M+H]+: 363,1. |
| 38 | 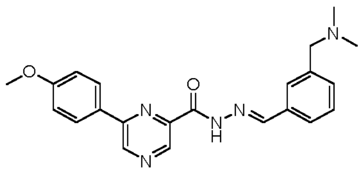 *(E)-N'*-(3-((dimethylamino)methyl) benzylidene)-6-(4-methoxyphenyl) pyrazine-2-carbohydrazide. | 1H-NMR, 400*MHz,* DMSO-d6 $\delta$ 12,02 (s, 1H), 9,48 - 9,41 (m, 1H), 9,09 (s, 1H), 8,70 (s, 1H), 8,39 (d, J = 8,8 Hz, 2H), 7,76 (d, J = 0,9 Hz, 1H), 7,62 - 7,57 (m, 1H), 7,45 - 7,34 (m, 2H), 7,12 (d, J = 8,8 Hz, 2H), 3,85 (s, 3H), 3,44 (s, 2H), 2,16 (s, 6H). [M+H]+: 390,1. |
| 39 | 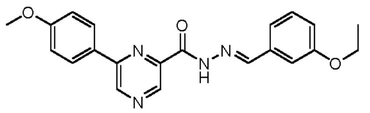 *(E)-N'*-(3-methoxybenzylidene)-6-(4-methoxyphenyl) pyrazine-2-carbohydrazide. | 1H-NMR, 400*MHz,* DMSO-d6 $\delta$ = 12,05 (s, 1H), 9,45 (s, 1H), 9,10 (s, 1H), 8,69 (s, 1H), 8,40 (d, J = 8,8 Hz, 2H), 7,44 - 7,25 (m, 3H), 7,13 (d, J = 8,8 Hz, 2H), 7,03 (dd, J = 1,8, 8,0 Hz, 1H), 4,09 (q, J = 6,9 Hz, 2H), 3,87 (s, 3H), 1,36 (t, J = 7,0 Hz, 3H). [M+H]+: 377,2. |
| 40 | 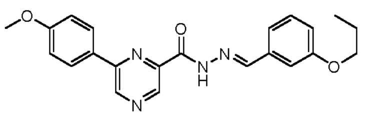 *(E)*-6-(4-methoxyphenyl)-*N'*-(3-propoxybenzylidene) pyrazine-2-carbohydrazide. | 1H-NMR, 400*MHz,* DMSO-d6 $\delta$ = 12,04 (s, 1H), 9,44 (s, 1H), 9,10 (s, 1H), 8,69 (s, 1H), 8,40 (d, J = 8,8 Hz, 2H), 7,43 - 7,27 (m, 3H), 7,18 - 6,97 (m, 3H), 3,98 (t, J = 6,5 Hz, 2H), 3,86 (s, 3H), 1,76 (sxt, J = 7,0 Hz, 2H), 1,00 (t, J = 7,3 Hz, 3H). [M+H]+: 391,2. |
| 41 | 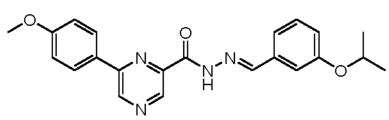 *(E)-N'*-(3-isopropoxybenzylidene)-6-(4-methoxyphenyl) pyrazine-2-carbohydrazide. | 1H-NMR, 400*MHz,* DMSO-d6 $\delta$ = 12,05 (s, 1H), 9,46 (s, 1H), 9,11 (s, 1H), 8,68 (s, 1H), 8,41 (d, J = 8,6 Hz, 2H), 7,43 - 7,25 (m, 3H), 7,13 (br d, J = 8,3 Hz, 2H), 7,02 (br d, J = 8,1 Hz, 1H), 4,67 (spt, J = 5,7 Hz, 1H), 3,87 (s, 3H), 1,30 (d, J = 6,0 Hz, 6H). [M+H]+: 391,2. |

(continued)

| Compound No. | Structure | 1H-NMR/ MS m/z [M+H] + |
|---|---|---|
| 42 | 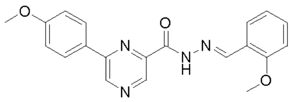 *(E)*-*N*'-(2-methoxybenzylidene)-6-(4-methoxyphenyl) pyrazine-2-carbohydrazide. | 1H-NMR, 400*MHz,* DMSO-d6 $\delta$ = 12,13 (s, 1H), 9,44 (s, 1H), 9,08 (d, J = 16,1 Hz, 2H), 8,42 (br d, J = 8,2 Hz, 2H), 7,93 (br d, J = 7,6 Hz, 1H), 7,45 (br t, J = 7,8 Hz, 1H), 7,20 - 7,00 (m, 4H), 3,88 (d, J = 12,5 Hz, 6H). [M+H] +: 363.1. |
| 43 | 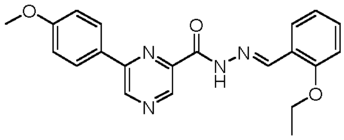 *(E)*-*N*'-(2-methoxybenzylidene)-6-(4-methoxyphenyl) pyrazine-2-carbohydrazide. | 1H-NMR, 400*MHz,* DMSO-d6 $\delta$ = 12.19 (s, 1H), 9.44 (s, 1H), 9.10 (s, 1H), 9.03 (s, 1H), 8.40 (br d, J = 8.8 Hz, 2H), 7.93 (dd, J = 1.5, 7.7 Hz, 1H), 7.46 - 7.39 (m, 1H), 7.20 - 7.09 (m, 3H), 7.04 (t, J = 7.5 Hz, 1H), 4.17 (q, J = 7.0 Hz, 2H), 3.87 (s, 3H), 1.41 (t, J = 6.9 Hz, 3H). [M+H]+: 377,2. |
| 44 | 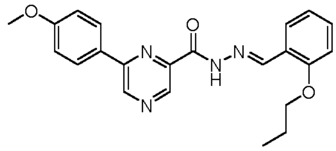 *(E)*-6-(4-methoxyphenyl)-*N'*-(2-propoxybenzylidene) pyrazine-2-carbohydrazide. | 1H-NMR, 400*MHz,* DMSO-d6 $\delta$ = 12,21 (s, 1H), 9,43 (s, 1H), 9,13 - 8,98 (m, 2H), 8,39 (d, J = 8,8 Hz, 2H), 7,93 (dd, J = 1,5, 7,8 Hz, 1H), 7,48 - 7,35 (m, 1H), 7,19 - 6,95 (m, 4H), 4,06 (t, J = 6,5 Hz, 2H), 3,87 (s, 3H), 1,81 (sxt, J = 7,0 Hz, 2H), 1,04 (t, J = 7,4 Hz, 3H). [M+H]+: 377,2. |
| 45 | 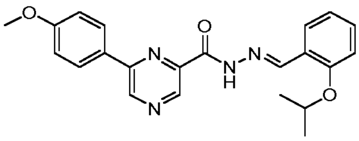 *(E)*-*N*'-(3-methoxybenzylidene)-6-(4-methoxyphenyl) pyrazine-2-carbohydrazide. | 1H-NMR, 400*MHz,* DMSO-d6 $\delta$ = 12,20 (s, 1H), 9,44 (s, 1H), 9,09 (s, 1H), 8,99 (s, 1H), 8,40 (d, J = 8,9 Hz, 2H), 7,93 (dd, J = 1,5, 7,8 Hz, 1H), 7,46 - 7,36 (m, 1H), 7,15 (d, J = 8,8 Hz, 3H), 7,02 (t, J = 7,5 Hz, 1H), 4,74 (td, J = 6,0, 12,1 Hz, 1H), 3,87 (s, 3H), 1,34 (d, J = 6,0 Hz, 6H). [M+H]+: 391,1. |
| 46 | 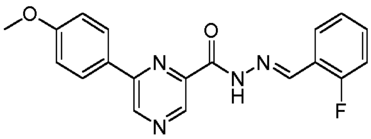 *(E)*-*N*'-(2-fluorobenzylidene)-6-(4-methoxyphenyl) pyrazine-2-carbohydrazide. | 1H-NMR, 400*MHz,* DMSO-d6 $\delta$ = 12,22 (br s, 1H), 9,46 (d, J = 2,4 Hz, 1H), 9,11 (d, J = 2,7 Hz, 1H), 9,00 (s, 1H), 8,42 (br dd, J = 2,2, 8,6 Hz, 2H), 8,01 (br t, J = 6,8 Hz, 1H), 7,58 - 7,48 (m, 1H), 7,38 - 7,28 (m, 2H), 7,14 (brdd, J = 2,2, 8,6 Hz, 2H), 3,87 (d, J = 2,3 Hz, 3H). [M+H]+: 351,1. |
| 47 | 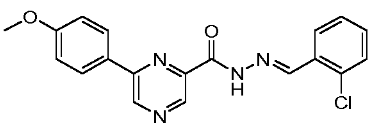 *(E)*-*N*'-(2-chlorobenzylidene)-6-(4-methoxyphenyl) pyrazine-2-carbohydrazide. | 1H-NMR, 400*MHz,* DMSO-d6 $\delta$ = 12,37 (s, 1H), 9,47 (s, 1H), 9,13 (d, J = 5,3 Hz, 2H), 8,42 (d, J = 8,9 Hz, 2H), 8,13 - 8,04 (m, 1H), 7,60 - 7,43 (m, 3H), 7,16 (d, J = 8,8 Hz, 2H), 3,87 (s, 3H). [M+H]+: 367,1. |

(continued)

| Compound No. | Structure | 1H-NMR/ MS m/z [M+H] + |
|---|---|---|
| 48 | 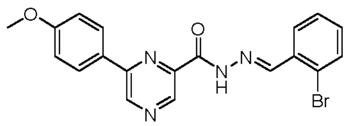 *(E)-N'*-(2-bromobenzylidene)-6-(4-methoxyphenyl) pyrazine-2-carbohydrazide. | 1H-NMR, 400*MHz,* DMSO-d6 $\delta$ = 12,42 (s, 1H), 9,46 (s, 1H), 9,09 (d, J = 17,7 Hz, 2H), 8,41 (d, J = 8,8 Hz, 2H), 8,07 (dd, J = 1,3, 7,8 Hz, 1H), 7,73 (d, J = 7,9 Hz, 1H), 7,55 - 7,36 (m, 2H), 7,15 (d, J = 8,8 Hz, 2H), 3,87 (s, 3H). [M+H]+: 411,0. |
| 49 | 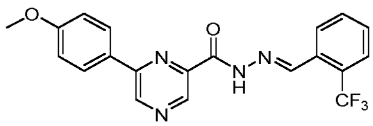 *(E)*-6-(4-methoxyphenyl)-*N'*-(2-(trifluoromethyl) benzylidene)pyrazin e-2-carbohydrazide. | 1H-NMR, 400*MHz,* DMSO-d6 $\delta$ = 12,52 (s, 1H), 9,47 (s, 1H), 9,16 - 9,06 (m, 2H), 8,39 (d, J = 8,9 Hz, 2H), 8,28 (d, J = 7,9 Hz, 1H), 7,87 - 7,77 (m, 2H), 7,74 - 7,61 (m, 1H), 7,16 (d, J = 8,8 Hz, 2H), 3,87 (s, 3H). [M+H]+: 401,1. |
| 50 | 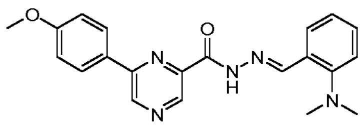 *(E)-N'*-(3-((dimethylamino)methyl) benzylidene)-6-(4-methoxyphenyl) pyrazine-2-carbohydrazide. | 1H-NMR, 400*MHz,* DMSO-d6 $\delta$ = 12,18-12,11 (m, 1H), 9,49-9,41 (m, 1H), 9,10 (s, 1H), 8,99 (s, 1H), 8,44 - 8,37 (m, 2H), 7,88 (dd, J = 1,5, 7,7 Hz, 1H), 7,44 - 7,35 (m, 1H), 7,20 - 7,05 (m, 4H), 3,87 (s, 3H), 2,74 (s, 6H). [M+H]+: 376,1. |
| 51 | 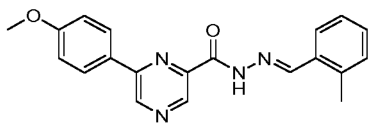 *(E)*-6-(4-methoxyphenyl)-*N'*-(2-methylbenzylidene) pyrazine-2-carbohydrazide. | 1H-NMR, 400*MHz,* DMSO-d6 $\delta$ = 12,11 - 12,03 (m, 1H), 9,51 - 9,42 (m, 1H), 9,16 - 9,09 (m, 1H), 9,05 - 8,97 (m, 1H), 8,40 (d, J = 8,4 Hz, 2H), 7,96 - 7,85 (m, 1H), 7,42 - 7,23 (m, 3H), 7,15 (d, J = 8,4 Hz, 2H), 3,90 - 3,81 (m, 3H), 2,47 (br d, J = 4,6 Hz, 3H). [M+H]+: 347.2. |
| 52 | 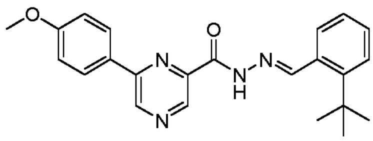 *(E)-N'*-(2-(*tert-butyl*)benzylidene)-6-(4-methoxyphenyl) pyrazine-2-carbohydrazide. | 1H-NMR, 400*MHz,* DMSO-d6 $\delta$ = 12,28 (s, 1H), 9,44 (d, J = 10,8 Hz, 2H), 9,10 (s, 1H), 8,36 (d, J = 8,9 Hz, 2H), 7,92 (dd, J = 1,2, 7,6 Hz, 1H), 7,49 - 7,27 (m, 3H), 7,16 (d, J = 8,9 Hz, 2H), 3,86 (s, 3H), 1,44 (s, 9H). [M+H]+: 389,1. |
| 53 | 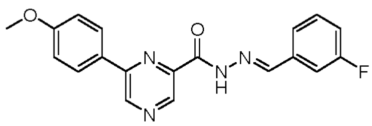 *(E)-N'*-(3-fluorobenzylidene)-6-(4-methoxyphenyl) pyrazine-2-carbohydrazide. | 1H-NMR, 400*MHz,* DMSO-d6 $\delta$ = 12,19 - 12,10 (m, 1H), 9,45 (s, 1H), 9,11 (s, 1H), 8,73 (s, 1H), 8,40 (d, J = 8,6 Hz, 2H), 7,66 - 7,47 (m, 3H), 7,37 - 7,25 (m, 1H), 7,13 (d, J = 8,6 Hz, 2H), 3,86 (s, 3H). [M+H]+: 351,1. |

(continued)

| Compound No. | Structure | 1H-NMR/ MS m/z [M+H] + |
|---|---|---|
| 54 | 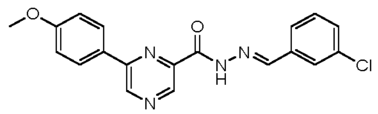 *(E)-N*'-(3-chlorobenzylidene)-6-(4-methoxyphenyl) pyrazine-2-carbohydrazide. | 1H-NMR, 400*MHz,* DMSO-d6 $\delta$ = 12,18 (s, 1H), 9,47 (s, 1H), 9,12 (s, 1H), 8,72 (s, 1H), 8,41 (d, J = 8,7 Hz, 2H), 7,92 - 7,81 (m, 1H), 7,78 -7,69 (m, 1H), 7,59 - 7,49 (m, 2H), 7,15 (d, J = 8,8 Hz, 2H), 3,88 (s, 3H). [M+H]+: 367,1. |
| 55 | 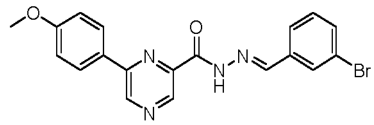 *(E)-N*'-(3-bromobenzylidene)-6-(4-methoxyphenyl) pyrazine-2-carbohydrazide. | 1H-NMR, 400*MHz,* DMSO-d6 $\delta$ = 12,20 (s, 1H), 9,47 (s, 1H), 9,11 (s, 1H), 8,70 (s, 1H), 8,41 (d, J = 8,8 Hz, 2H), 7,97 (s, 1H), 7,77 (d, J = 7,8 Hz, 1H), 7,67 (br d, J = 8,8 Hz, 1H), 7,50 - 7,43 (m, 1H), 7,14 (d, J = 8,8 Hz, 2H), 3,87 (s, 3H). [M+H]+: 411,0. |
| 56 | 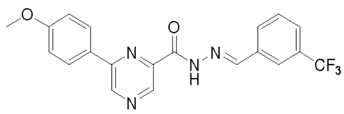 *(E)*-6-(4-methoxyphenyl)-*N*'-(3-(trifluoromethyl) benzylidene)pyrazin e-2-carbohydrazide. | 1H-NMR, 400*MHz,* DMSO-d6 $\delta$ = 12,28 - 12,17 (m, 1H), 9,53 - 9,40 (m, 1H), 9,12 (s, 1H), 8,82 (s, 1H), 8,41 (d, J = 8,7 Hz, 2H), 8,18 - 7,99 (m, 2H), 7,90 - 7,67 (m, 2H), 7,14 (d, J = 8,6 Hz, 2H), 3,95 - 3,79 (m, 3H). [M+H]+: 401,1. |
| 57 | 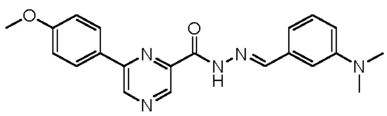 *(E)-N*'-(3-((dimethylamino)methyl) benzylidene)-6-(4-methoxyphenyl) pyrazine-2-carbohydrazide. | 1H-NMR, 400*MHz,* DMSO-d6 $\delta$ = 11,98 (s, 1H), 9,46 (s, 1H), 9,10 (s, 1H), 8,66 (s, 1H), 8,42 (d, J = 8,9 Hz, 2H), 7,32 - 7,26 (m, 1H), 7,16 - 7,03 (m, 4H), 6,85 (dd, J = 2,1, 8,3 Hz, 1H), 3,87 (s, 3H), 2,96 (s, 6H). [M+H]+: 376,1. |
| 58 | 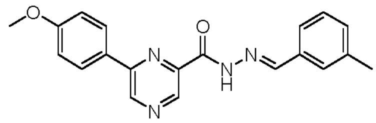 *(E)*-6-(4-methoxyphenyl)-*N*'-(3-methylbenzylidene) pyrazine-2-carbohydrazide. | 1H-NMR, 400*MHz,* DMSO-d6 $\delta$ = 12,03 (s, 1H), 9,46 (s, 1H), 9,11 (s, 1H), 8,68 (s, 1H), 8,41 (br d, J = 8,3 Hz, 2H), 7,65 - 7,52 (m, 2H), 7,42 - 7,25 (m, 2H), 7,14 (br d, J = 7,8 Hz, 2H), 3,87 (s, 3H), 2,38 (s, 3H). [M+H]+: 347,1. |
| 59 | 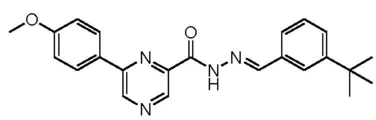 *(E)-N'*-(3-(tert-butyl)benzylidene)-6-(4-methoxyphenyl) pyrazine-2-carbohydrazide. | 1H-NMR, 400*MHz,* DMSO-d6 $\delta$ = 11,98 (s, 1H), 9,46 (s, 1H), 9,10 (s, 1H), 8,66 (s, 1H), 8,42 (d, J = 8,9 Hz, 2H), 7,34 - 7,25 (m, 1H), 7,18 - 7,02 (m, 4H), 6,85 (dd, J = 2,1, 8,3 Hz, 1H), 3,87 (s, 3H), 2,96 (s, 6H). [M+H]+: 389,2. |

(continued)

| Compound No. | Structure | 1H-NMR/ MS m/z [M+H] + |
|---|---|---|
| 60 | 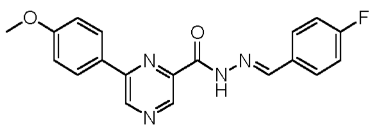 *(E)-N*'-(4-fluorobenzylidene)-6-(4-methoxyphenyl) pyrazine-2-carbohydrazide. | 1H-NMR, 400*MHz,* DMSO-d6 $\delta$ = 12,06 (br s, 1H), 9,48 - 9,40 (m, 1H), 9,10 (s, 1H), 8,72 (s, 1H), 8,40 (br d, J = 8,4 Hz, 2H), 7,84 (dd, J = 5,7, 8,3 Hz, 2H), 7,33 (br t, J = 8,5 Hz, 2H), 7,13 (br d, J = 8,1 Hz, 2H), 3,87 (s, 3H). [M+H]+: 351,1. |
| 61 | 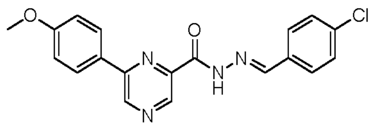 *(E)-N*'-(4-chlorobenzylidene)-6-(4-methoxyphenyl) pyrazine-2-carbohydrazide. | 1H-NMR, 400*MHz,* DMSO-d6 $\delta$ = 12,10 (s, 1H), 9,45 (s, 1H), 9,11 (s, 1H), 8,72 (s, 1H), 8,40 (d, J = 8,9 Hz, 2H), 7,80 (d, J = 8,6 Hz, 2H), 7,55 (d, J = 8,6 Hz, 2H), 7,13 (d, J = 8,9 Hz, 2H), 3,87 (s, 3H). [M+H]+: 367,1. |
| 62 | 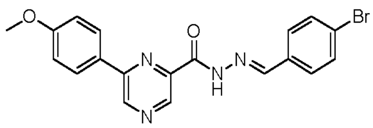 *(E)-N*'-(4-bromobenzylidene)-6-(4-methoxyphenyl) pyrazine-2-carbohydrazide. | 1H-NMR, 400*MHz,* DMSO-d6 $\delta$ = 12,11 (s, 1H), 9,46 (s, 1H), 9,11 (s, 1H), 8,70 (s, 1H), 8,43 - 8,36 (m, 2H), 7,76 - 7,62 (m, 4H), 7,18 - 7,07 (m, 2H), 3,87 (s, 3H). [M+H]+: 411,0. |
| 63 | 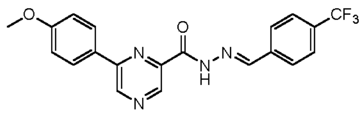 *(E)*-6-(4-methoxyphenyl)-*N*'-(4-(trifluoromethyl) benzylidene)pyrazin e-2-carbohydrazide. | 1H-NMR, 400*MHz,* DMSO-d6 $\delta$ = 12,22 (s, 1H), 9,46 (s, 1H), 9,12 (s, 1H), 8,81 (s, 1H), 8,41 (d, J = 8,8 Hz, 2H), 8,00 (d, J = 8,2 Hz, 2H), 7,85 (d, J = 8,3 Hz, 2H), 7,14 (d, J = 8,8 Hz, 2H), 3,87 (s, 3H). [M+H]+: 401,1. |
| 64 | 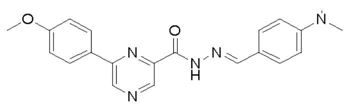 *(E)-N*-(4-((dimethylamino)methyl) benzylidene)-6-(4-methoxyphenyl) pyrazine-2-carbohydrazide. | 1H-NMR, 400*MHz,* DMSO-d6 $\delta$ = 11,78 (s, 1H), 9,44 (s, 1H), 9,08 (s, 1H), 8,55 (s, 1H), 8,41 (d, J = 8,8 Hz, 2H), 7,59 (d, J = 8,7 Hz, 2H), 7,13 (d, J = 8,9 Hz, 2H), 6,79 (d, J = 8,8 Hz, 2H), 3,87 (s, 3H), 2,99 (s, 6H). [M+H]+: 376,1. |
| 65 | 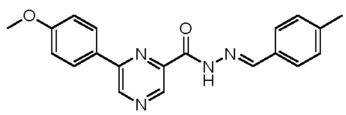 *(E)*-6-(4-methoxyphenyl)-*N*'-(4-methylbenzylidene) pyrazine-2-carbohydrazide. | 1H-NMR, 400*MHz,* DMSO-d6 $\delta$ = 11,98 (s, 1H), 9,45 (s, 1H), 9,10 (s, 1H), 8,68 (s, 1H), 8,40 (br d, J = 7,1 Hz, 2H), 7,68 (d, J = 7,8 Hz, 2H), 7,30 (br d, J = 7,2 Hz, 2H), 7,13 (br d, J = 7,1 Hz, 2H), 3,87 (s, 3H), 2,36 (s, 3H). [M+H]+: 347,1. |
| 66 | 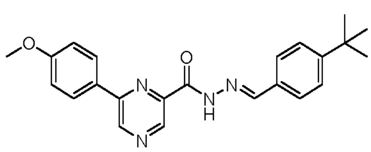 *(E)-N*'-(4-(tert-butyl)benzylidene)-6-(4-methoxyphenyl) pyrazine-2-carbohydrazide. | 1H-NMR, 400*MHz,* DMSO-d6 $\delta$ = 11,99 (s, 1H), 9,45 (s, 1H), 9,10 (s, 1H), 8,70 (s, 1H), 8,41 (d, J = 8,8 Hz, 2H), 7,71 (d, J = 8,4 Hz, 2H), 7,52 (d, J = 8,4 Hz, 2H), 7,14 (d, J = 8,9 Hz, 2H), 3,87 (s, 3H), 1,31 (s, 9H). [M+H] +: 389,2. |

(continued)

| Compound No. | Structure | 1H-NMR/ MS m/z [M+H] + |
|---|---|---|
| 67 | 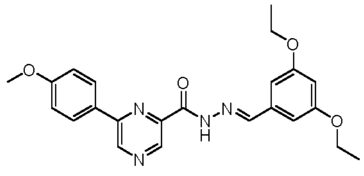 *(E)-N'*-(3,5-diethoxybenzylidene)-6-(4-methoxyphenyl) pyrazine-2-carbohydrazide. | 1H-NMR, 400*MHz,* DMSO-d6 $\delta$ = 12,04 (s, 1H), 9,45 (s, 1H), 9,10 (s, 1H), 8,62 (s, 1H), 8,40 (d, J = 8,8 Hz, 2H), 7,13 (d, J = 8,9 Hz, 2H), 6,89 (d, J = 2,1 Hz, 2H), 6,57 (s, 1H), 4,07 (q, J = 6,9 Hz, 4H), 3,87 (s, 3H), 1,34 (t, J = 6,9 Hz, 6H). [M+H]+: 421,2. |
| 68 | 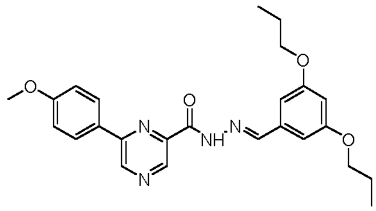 *(E)-N'*-(3,5-diethoxybenzylidene)-6-(4-methoxyphenyl) pyrazine-2-carbohydrazide. | 1H-NMR, 400*MHz,* DMSO-d6 $\delta$ = 12,06 (s, 1H), 9,46 (s, 1H), 9,11 (s, 1H), 8,63 (s, 1H), 8,41 (d, J = 8,7 Hz, 2H), 7,14 (d, J = 8,7 Hz, 2H), 6,91 (d, J = 2,0 Hz, 2H), 6,59 (s, 1H), 3,98 (t, J = 6,5 Hz, 4H), 3,88 (s, 3H), 1,76 (sxt, J = 7,0 Hz, 4H), 1,01 (t, J = 7,3 Hz, 6H). [M+H]+: 449,2. |
| 69 | 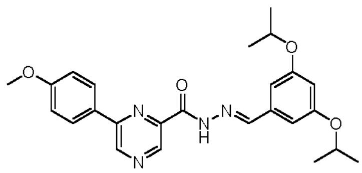 *(E)-N'*-(3,5-diethoxybenzylidene)-6-(4-methoxyphenyl) pyrazine-2-carbohydrazide. | 1H-NMR, 400*MHz,* DMSO-d6 $\delta$ = 12,04 (s, 1H), 9,46 (s, 1H), 9,10 (s, 1H), 8,62 (s, 1H), 8,41 (d, J = 8,7 Hz, 2H), 7,13 (d, J = 8,7 Hz, 2H), 6,87 (d, J = 1,8 Hz, 2H), 6,54 (s, 1H), 4,66 (spt, J = 6,0 Hz, 2H), 3,87 (s, 3H), 1,29 (d, J = 6,0 Hz, 12H). [M+H]+: 449,2. |
| 70 | 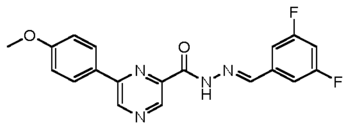 *(E)-N'*-(3,5-diethoxybenzylidene)-6-(4-methoxyphenyl) pyrazine-2-carbohydrazide. | 1H-NMR, 400*MHz,* DMSO-d6 $\delta$ = 12,26 (s, 1H), 9,46 (s, 1H), 9,11 (s, 1H), 8,71 (s, 1H), 8,40 (d, J = 8,9 Hz, 2H), 7,51 - 7,43 (m, 2H), 7,36 (tt, J = 2,2, 9,2 Hz, 1H), 7,13 (d, J = 8,9 Hz, 2H), 3,87 (s, 3H). [M+H]+: 369,1. |
| 71 | 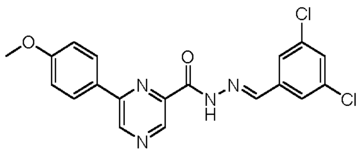 *(E)-N'*-(3,5-diethoxybenzylidene)-6-(4-methoxyphenyl) pyrazine-2-carbohydrazide. | 1H-NMR, 400*MHz,* DMSO-d6 $\delta$ = 12,30 (s, 1H), 9,46 (s, 1H), 9,11 (s, 1H), 8,67 (s, 1H), 8,39 (d, J = 8,7 Hz, 2H), 7,82 - 7,65 (m, 3H), 7,13 (d, J = 8,7 Hz, 2H), 3,87 (s, 3H). [M+H]+: 401,1. |
| 72 | 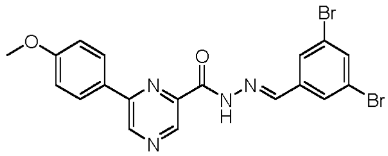 *(E)-N'*-(3,5-diethoxybenzylidene)-6-(4-methoxyphenyl) pyrazine-2-carbohydrazide. | 1H-NMR, 400*MHz,* DMSO-d6 $\delta$ = 12,29 (s, 1H), 9,45 (s, 1H), 9,10 (s, 1H), 8,64 (s, 1H), 8,39 (d, J = 8,8 Hz, 2H), 7,98 - 7,88 (m, 3H), 7,13 (d, J = 8,9 Hz, 2H), 3,86 (s, 3H). [M+H]+: 491,0. |

(continued)

| Compound No. | Structure | 1H-NMR/ MS m/z [M+H] + |
|---|---|---|
| 73 | 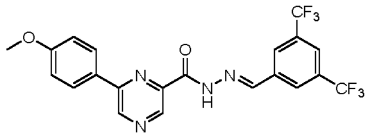<br>*(E)-N'*-(3,5-bis(trifluoromethyl)benzylidene)-6-(4-methoxyphenyl)pyrazine-2-carbohydrazide. | 1H-NMR, 400*MHz,* DMSO-d6<br>$\delta$ = 12,46 - 12,33 (m, 1H), 9,47 (s, 1H), 9,12 (s, 1H), 8,88 (s, 1H), 8,45 - 8,33 (m, 4H), 8,21 (s, 1H), 7,14 (d, J = 8,7 Hz, 2H), 3,87 (s, 3H).<br>[M+H]+: 469,1. |
| 74 | 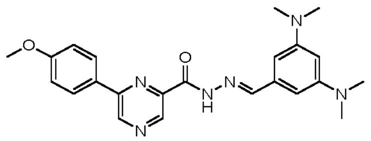<br>*(E)-N'*-(3,5-bis(trifluoromethyl)benzylidene)-6-(4-methoxyphenyl)pyrazine-2-carbohydrazide. | 1H-NMR, 400*MHz,* DMSO-d6<br>$\delta$ = 11,89 (s, 1H), 9,46 (s, 1H), 9,11 - 9,07 (m, 1H), 8,59 (s, 1H), 8,46 - 8,39 (m, 2H), 7,15 - 7,09 (m, 2H), 6,49 (d, J = 2,0 Hz, 2H), 6,13 - 6,06 (m, 1H), 3,87 (s, 3H), 2,94 (s, 12H).<br>[M+H]+: 419,2. |
| 75 | 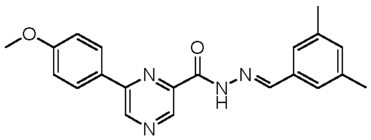<br>*(E)-N'*-(3,5-diethoxybenzylidene)-6-(4-methoxyphenyl) pyrazine-2-carbohydrazide. | 1H-NMR, 400*MHz,* DMSO-d6<br>$\delta$ = 12,02 (s, 1H), 9,49 - 9,36 (m, 1H), 9,15 - 8,92 (m, 1H), 8,64 (s, 1H), 8,41 (d, J = 8,3 Hz, 2H), 7,39 (s, 2H), 7,18 - 7,04 (m, 3H), 3,92 - 3,81 (m, 3H), 2,34 (s, 6H).<br>[M+H]+: 361,2. |
| 76 | 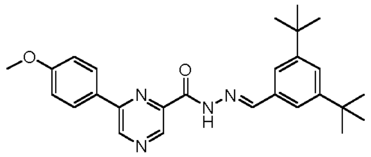<br>*(E)-N'*-(3,5-di-tert-*butylbenzylidene)-6-(4*-methoxyphenyl) pyrazine-2-carbohydrazide. | 1H-NMR, 400*MHz,* DMSO-d6<br>$\delta$ = 11,99 - 11,91 (m, 1H), 9,46 (s, 1H), 9,11 (s, 1H), 8,74 (s, 1H), 8,46 - 8,36 (m, 2H), 7,61 - 7,47 (m, 3H), 7,16 - 7,08 (m, 2H), 3,87 (s, 3H), 1,34 (s, 18H).<br>[M+H]+: 445,3. |
| 77 | 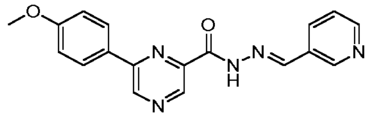<br>*(E)*-6-(4-methoxyphenyl)-*N'*-(pyridin-3-ylmethylene) pyrazine-2-carbohydrazide. | 1H-NMR, 400*MHz,* DMSO-d6<br>$\delta$ = 12,21 (s, 1H), 9,47 (s, 1H), 9,12 (s, 1H), 8,90 (d, J = 1,7 Hz, 1H), 8,79 (s, 1H), 8,65 (dd, J = 1,5, 4,7 Hz, 1H), 8,41 (d, J = 8,9 Hz, 2H), 8,20 (td, J = 1,8, 7,9 Hz, 1H), 7,53 (dd, J = 4,8, 7,9 Hz, 1H), 7,14 (d, J = 8,8 Hz, 2H), 3,87 (s, 3H).<br>[M+H]+: 334,1. |
| 78 | 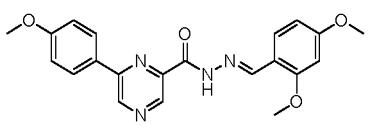<br>*(E)-N'*-(2,4-dimethoxybenzylidene)-6-(4-methoxyphenyl) pyrazine-2-carbohydrazide. | 1H-NMR, 400*MHz,* DMSO-d6<br>$\delta$ = 11,99 (s, 1H), 9,43 (s, 1H), 9,08 (s, 1H), 8,95 (s, 1H), 8,42 (d, J = 8,9 Hz, 2H), 7,90 - 7,83 (m, 1H), 7,13 (d, J = 8,9 Hz, 2H), 6,68 - 6,61 (m, 2H), 3,95 - 3,75 (m, 9H).<br>[M+H]+: 393,1. |

(continued)

| Compound No. | Structure | 1H-NMR/ MS m/z [M+H] + |
|---|---|---|
| 79 | 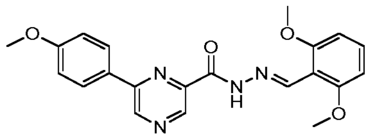 *(E)-N'*-(2,6-dimethoxybenzylidene)-6-(4-methoxyphenyl) pyrazine-2-carbohydrazide. | 1H-NMR, 400*MHz,* DMSO-d6 $\delta$ = 11,96 (s, 1H), 9,44 (s, 1H), 9,08 (s, 1H), 8,81 (s, 1H), 8,42 (d, J = 8,8 Hz, 2H), 7,38 (t, J = 8,4 Hz, 1H), 7,14 (d, J = 8,7 Hz, 2H), 6,75 (d, J = 8,5 Hz, 2H), 3,91 - 3,77 (m, 9H). [M+H]+: 393,1. |
| 80 | 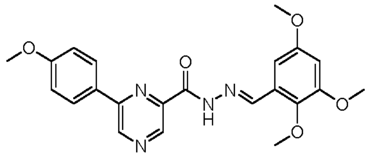 *(E)*-6-(4-ethoxyphenyl)-*N'*-(2,3,5-trimethoxybenzylidene) pyrazine-2-carbohydrazide. | 1H-NMR, 400*MHz,* DMSO-d6 $\delta$ = 12,18 (s, 1H), 9,45 (s, 1H), 9,10 (s, 1H), 8,96 (s, 1H), 8,41 (d, J = 8,8 Hz, 2H), 7,15 (d, J = 8,8 Hz, 2H), 6,99 (d, J = 2,8 Hz, 1H), 6,73 (d, J = 2,7 Hz, 1H), 3,90 - 3,67 (m, 12H). [M+H]+: 423,1. |
| 81 | 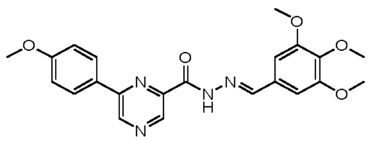 *(E)*-6-(4-ethoxyphenyl)-*N'*-(3,4,5-trimethoxybenzylidene) pyrazine-2-carbohydrazide. | 1H-NMR, 400*MHz,* DMSO-d6 $\delta$ = 12,01 (s, 1H), 9,46 (s, 1H), 9,10 (s, 1H), 8,65 (s, 1H), 8,41 (d, J = 8,9 Hz, 2H), 7,17 - 7,03 (m, 4H), 3,90 - 3,83 (m, 9H), 3,73 (s, 3H). [M+H]+: 423,2. |
| 82 | 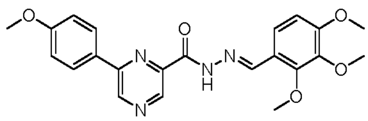 *(E)*-6-(4-methoxyphenyl)-*N'*-(2,3,4-trimethoxybenzylidene) pyrazine-2-carbohydrazide. | 1H-NMR, 400*MHz,* DMSO-d6 $\delta$ = 12,06 (s, 1H), 9,44 (s, 1H), 9,09 (s, 1H), 8,87 (s, 1H), 8,40 (d, J = 8,8 Hz, 2H), 7,68 (d, J = 8,9 Hz, 1H), 7,23 - 6,90 (m, 3H), 3,93 - 3,71 (m, 12H). [M+H]+: 423,2. |
| 83 | 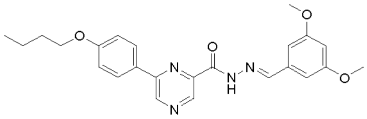 6-(4-butoxyphenyl)-*N'*-[*(E)*-(3,5-*dimethoxyphenyl) methylidene]pyrazine-2-carbohydrazide.* | 1H-NMR, 400MHz, DMSO-d6 $\delta$ = 12,07 (s, 1H), 9,49 - 9,44 (m, 1H), 9,13 - 9,08 (m, 1H), 8,66 (s, 1H), 8,41 (br d, J = 8,7 Hz, 2H), 7,13 (br d, J = 8,7 Hz, 2H), 6,93 (d, J = 2,0 Hz, 2H), 6,62 (s, 1H), 4,10 (t, J = 6,3 Hz, 2H), 3,82 (s, 6H), 1,80 - 1,71 (m, 2H), 1,53 - 1,43 (m, 2H), 0,97 (t, J = 7,4 Hz, 3H). [M+H]+: 435,2. |
| 84 | 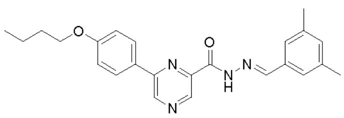 *(E)*-6-(4-butoxyphenyl)-*N'*-(3,5-dimethylbenzylidene) pyrazine-2-carbohydrazide. | 1H-NMR, 400*MHz,* DMSO-d6 $\delta$ 12,02 (s, 1H), 9,45 (s, 1H), 9,10 (s, 1H), 8,64 (s, 1H), 8,39 (d, J = 8,9 Hz, 2H), 7,39 (s, 2H), 7,05-7,17 (m, 3H), 4,08 (t, J = 6,5 Hz, 2H), 2,34 (s, 6H), 1,69-1,81 (m, 2H), 1,47 (dq, J = 14,9, 7,4 Hz, 2H), 0,96 (t, J = 7,4 Hz, 3H). [M+H]+: 403,2. |

(continued)

| Compound No. | Structure | 1H-NMR/ MS m/z [M+H] + |
|---|---|---|
| 85 | 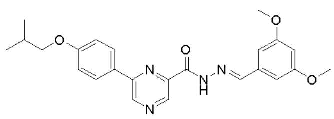<br>(E)-N'-(3,5-dimethoxybenzylidene)-6-(4-isobutoxyphenyl) pyrazine-2-carbohydrazide. | 1H-NMR, 400MHz CDCl3 $\delta$ = 10,77 (s, 1H), 9,36 (s, 1H), 9,19 (s, 1H), 8,41 (s, 1H), 8,01 (br d, J = 8,8 Hz, 2H), 7,09 (br d, J = 8,7 Hz, 2H), 7,00 (d, J = 2,1 Hz, 2H), 6,56 (s, 1H), 3,95 - 3,78 (m, 8H), 2,26- 2,07 (m, 1H), 1,08 (d, J = 6,7 Hz, 6H). [M+H]+: 435,1. |
| 86 | 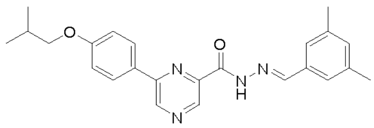<br>(E)-N'-(3,5-dimethylbenzylidene)-6-(4-isobutoxyphenyl) pyrazine-2-carbohydrazide. | 1H-NMR, *400MHz,* DMSO-d6 $\delta$ 12,06 (s, 1H), 9,47 (s, 1H), 9,11 (s, 1H), 8,65 (s, 1H), 8,41 (br d, J = 7,9 Hz, 2H), 7,40 (s, 2H), 7,14 (br d, J = 8,5 Hz, 3H), 3,88 (br d, J = 6,1 Hz, 2H), 2,35 (s, 6H), 2,08 (td, J = 6,4, 12,9 Hz, 1H), 1,03 (br d, J = 6,5 Hz, 6H). [M+H]+: 403,1. |
| 87 | 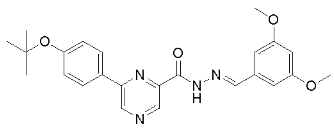<br>*(E)*-6-(4-(tert-butoxy)phenyl)-*N*'-(3,5-dimethoxybenzylidene)pyrazine-2-carbohydrazide. | 1H-NMR, 400MHz CDCl3 $\delta$ = 10,76 (s, 1H), 9,39 (s, 1H), 9,20 (s, 1H), 8,40 (s, 1H), 7,98 (d, J = 8,6 Hz, 2H), 7,19 (d, J = 8,7 Hz, 2H), 6,99 (d, J = 2,2 Hz, 2H), 6,55 (t, J = 2,2 Hz, 1H), 3,86 (s, 6H), 1,45 (s, 9H). [M+H]+: 435,1. |
| 88 | 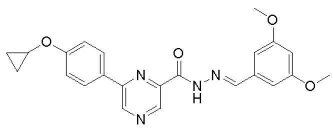<br>*(E)*-6-(4-cyclopropoxyphenyl)-*N*'-(3,5-dimethoxybenzylidene)pyrazine-2-carbohydrazide. | 1H-NMR, 400MHz CDCl3 $\delta$= 10,78 (s, 1H), 9,39 (s, 1H), 9,20 (s, 1H), 8,41 (s, 1H), 8,03 (br d, J = 8,7 Hz, 2H), 7,26 (br d, J = 8,8 Hz, 2H), 7,01 (d, J = 2,2 Hz, 2H), 6,57 (t, J = 2,1 Hz, 1H), 3,88 (s, 6H), 0,94 - 0,81 (m, 4H). [M+H]+: 419,1. |
| 89 | 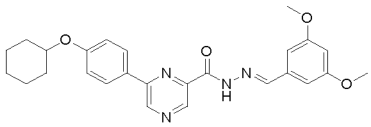<br>*(E)*-6-(4-(cyclohexyloxy)phenyl)-*N*- (3,5-dimethoxybenzylidene)pyrazine-2-carbohydrazide. | 1H-NMR, 400MHz CDCl3 $\delta$ = 10,77 (s, 1H), 9,36 (s, 1H), 9,18 (s, 1H), 8,39 (s, 1H), 8,05 - 7,95 (m, 2H), 7,11 - 7,04 (m, 2H), 6,99 (d, J = 2,3 Hz, 2H), 6,55 (t, J = 2,3 Hz, 1H), 4,45 - 4,27 (m, 1H), 2,09 - 1,98 (m, 2H), 1,92 - 1,79 (m, 2H), 1,67 - 1,58 (m, 3H), 1,50 - 1,29 (m, 3H). [M+H]+: 461,1. |
| 90 | 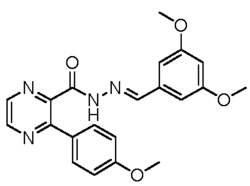<br>*(E)*-*N*'-(3,5-dimethoxybenzylidene)-3-(4-methoxyphenyl) pyrazine-2-carbohydrazide. | 1H-NMR, 400*MHz,* DMSO-d6 $\delta$ 12,18 - 12,04 (m, 1H), 8,86 - 8,77 (m, 1H), 8,64 (dd, J = 2,4, 8,6 Hz, 1H), 8,23 - 7,74 (m, 1H), 7,67 (dd, J = 8,7, 17,3 Hz, 2H), 7,04 (dd, J = 8,8, 17,9 Hz, 2H), 6,87 (d, J = 2,0 Hz, 1H), 6,60 - 6,44 (m, 1H), 6,39 (d, J = 2,0 Hz, 1H), 3,81 - 3,76 (m, 6H), 3,66 (s, 3H). [M+H]+: 393,1. |

(continued)

| Compound No. | Structure | 1H-NMR/ MS m/z [M+H] + |
|---|---|---|
| 91 | 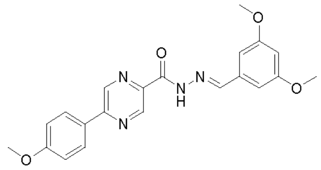 *(E)-N'*-(3,5-dimethoxybenzylidene)-5-(4-methoxyphenyl) pyrazine-2-carbohydrazide. | 1H-NMR, *400MHz,* DMSO-d6 $\delta$ 12,24 (s, 1H), 9,24 (dd, J = 1,3, 17,9 Hz, 2H), 8,57 (s, 1H), 8,23 (d, J = 8,8 Hz, 2H), 7,13 (d, J = 8,8 Hz, 2H), 6,86 (d, J = 2,2 Hz, 2H), 6,58 (t, J = 2,3 Hz, 1H), 3,85 (s, 3H), 3,79 (s, 6H). [M+H]+: 393.0. |
| 92 | 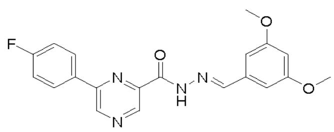 *(E)-N'*-(3,5-dimethoxybenzylidene)-6-(4-fluorophenyl) pyrazine-2-carbohydrazide. | 1H-NMR, *400MHz,* DMSO-d6 $\delta$ 12,09 (s, 1H), 9,52 (s, 1H), 9,17 (s, 1H), 8,63 (s, 1H), 8,51 (dd, J = 5,7, 8,4 Hz, 2H), 7,43 (t, J = 8,6 Hz, 2H), 6,91 (d, J = 2,0 Hz, 2H), 6,60 (s, 1H), 3,80 (s, 6H), 3,28 (s, 1H). [M+H]+: 381,0. |
| 93 | 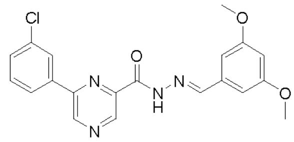 *(E)*-6-(3-chlorophenyl)-*N'*-(3,5-dimethoxybenzylidene) pyrazine-2-carbohydrazide. | 1H-NMR, *400MHz,* DMSO-d6 $\delta$ 12,19 (s, 1H), 9,58 (s, 1H), 9,24 (s, 1H), 8,68 (s, 1H), 8,58 (s, 1H), 8,40 (d, J = 6,1 Hz, 1H), 7,69 - 7,60 (m, 2H), 6,95 (d, J = 2,2 Hz, 2H), 6,62 (t, J = 2,2 Hz, 1H), 3,82 (s, 6H). [M+H]+: 397.0. |
| 94 | 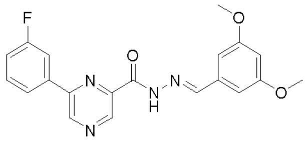 *(E)-N'*-(3,5-dimethoxybenzylidene)-6-(3-fluorophenyl) pyrazine-2-carbohydrazide. | 1H-NMR, 400*MHz,* DMSO-d6 $\delta$ 12,14 (s, 1H), 9,59 (s, 1H), 9,24 (s, 1H), 8,68 (s, 1H), 8,41 (br d, J = 10,5 Hz, 1H), 8,29 (d, J = 7,9 Hz, 1H), 7,70 - 7,60 (m, 1H), 7,43 (dt, J = 2,3, 8,5 Hz, 1H), 6,95 (d, J = 2,2 Hz, 2H), 6,62 (t, J = 2,1 Hz, 1H), 3,82 (s, 6H). [M+H]+: 381,1. |
| 95 | 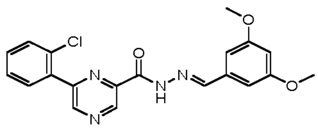 *(E)*-6-(2-chlorophenyl)-*N'*-(3,5-dimethoxybenzylidene) pyrazine-2-carbohydrazide. | 1H-NMR, 400*MHz,* DMSO-d6 $\delta$ 12,05 (s, 1H), 9,25 (s, 1H), 9,20 (s, 1H), 8,53 (s, 1H), 7,90 - 7,81 (m, 1H), 7,72 - 7,63 (m, 1H), 7,60 - 7,55 (m, 2H), 6,86 (d, J = 2,0 Hz, 2H), 6,59 (s, 1H), 3,78 (s, 6H). [M+H]+: 396,9. |
| 96 | 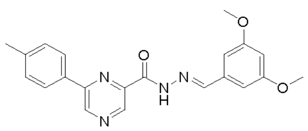 *(E)-N'*-(3,5-dimethoxybenzylidene)-6-(p-tolyl)pyrazine-2-carbohydrazide. | 1H-NMR, 400*MHz,* DMSO-d6 $\delta$ 12,06 (s, 1H), 9,48 (s, 1H), 9,14 (s, 1H), 8,64 (s, 1H), 8,33 (d, J = 8,2 Hz, 2H), 7,39 (d, J = 8,2 Hz, 2H), 6,91 (d, J = 2,2 Hz, 2H), 6,60 (s, 1H), 3,80 (s, 6H), 2,40 (s, 3H). [M+H]+: 377,2. |

(continued)

| Compound No. | Structure | 1H-NMR/ MS m/z [M+H] + |
|---|---|---|
| 97 | 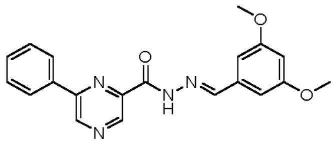 *(E)-N'*-(3,5-dimethoxybenzylidene)-6-phenylpyrazine-2-carbohydrazide. | 1H-NMR, 400*MHz,* DMSO-d6 *δ* 12,10 (s, 1H), 9,54 (s, 1H), 9,20 (s, 1H), 8,66 (s, 1H), 8,48 - 8,41 (m, 2H), 7,65 - 7,56 (m, 3H), 6,94 (d, J = 2,1 Hz, 2H), 6,62 (t, J = 2,2 Hz, 1H), 3,82 (s, 6H). [M+H]+: 363.1. |
| 98 | 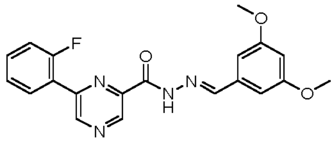 *(E)-N'*-(3,5-dimethoxybenzylidene)-6-(2-fluorophenyl) pyrazine-2-carbohydrazide. | 1H-NMR, 400*MHz,* DMSO-d6 *δ* 12,06 (s, 1H), 9,26 (d, J = 2,6 Hz, 1H), 9,20 (s, 1H), 8,58 (s, 1H), 8,32 (dt, J = 1,9, 7,9 Hz, 1H), 7,67 - 7,56 (m, 1H), 7,49 - 7,39 (m, 2H), 6,89 (d, J = 2,2 Hz, 2H), 6,58 (t, J = 2,3 Hz, 1H), 3,78 (s, 6H). [M+H]+: 381,0. |
| 99 | 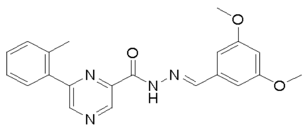 *(E)-N'*-(3,5-dimethoxybenzylidene)-6-(p-tolyl)pyrazine-2-carbohydrazide. | 1H-NMR, 400*MHz,* DMSO-d6 *δ* 12,05 (br s, 1H), 9,23 (s, 1H), 9,09 (s, 1H), 8,57 (s, 1H), 7,65 (d, J = 7,5 Hz, 1H), 7,49 - 7,37 (m, 3H), 6,88 (d, J = 2,1 Hz, 2H), 6,61 (t, J = 2,1 Hz, 1H), 3,80 (s, 6H), 2,44 (s, 3H). [M+H]+: 377,2. |
| 100 | 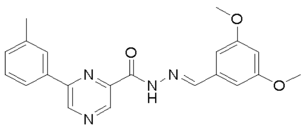 *(E)-N'*-(3,5-dimethoxybenzylidene)-6-(p-tolyl)pyrazine-2-carbohydrazide. | 1H-NMR, 400*MHz,* DMSO-d6 *δ* 12.09 (s, 1H), 9.50 (s, 1H), 9.18 (s, 1H), 8.66 (s, 1H), 8.27 - 8.14 (m, 2H), 7.54 - 7.35 (m, 2H), 6.93 (s, 2H), 6.61 (br s, 1H), 3.81 (s, 6H). [M+H]+: 377.1. |
| 101 | 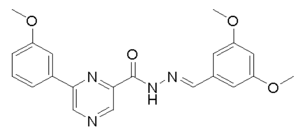 *(E)-N'*-(3,5-dimethoxybenzylidene)-3-(4-methoxyphenyl) pyrazine-2-carbohydrazide. | 1H-NMR, 400*MHz,* DMSO-d6 *δ* 12,12 (br s, 1H), 9,53 (s, 1H), 9,19 (s, 1H), 8,69 - 8,61 (m, 1H), 8,03 - 7,94 (m, 2H), 7,52 (t, J = 7,9 Hz, 1H), 7,15 (dd, J = 2,0, 8,1 Hz, 1H), 6,93 (d, J = 2,2 Hz, 2H), 6,61 (t, J = 2,1 Hz, 1H), 3,90 (s, 3H), 3,81 (s, 6H). [M+H]+: 393,1. |
| 102 | 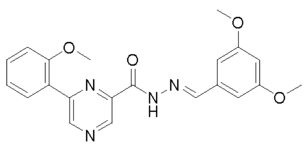 *(E)-N*-(3,5-dimethoxybenzylidene)-6-(2-methoxyphenyl) pyrazine-2-carbohydrazide. | 1H-NMR, 400*MHz,* DMSO-d6 δ 12,48 - 11,44 (m, 1H), 9,37 - 9,32 (m, 1H), 9,16 - 9,11 (m, 1H), 8,59 (s, 1H), 8,11 (dd, J = 1,8, 7,7 Hz, 1H), 7,60 - 7,51 (m, 1H), 7,29 - 7,14 (m, 2H), 6,90 (d, J = 2,4 Hz, 2H), 6,60 (t, J = 2,2 Hz, 1H), 3,92 (s, 3H), 3,80 (s, 6H). [M+H]+: 393,0. |

(continued)

| Compound No. | Structure | 1H-NMR/ MS m/z [M+H] + |
|---|---|---|
| 103 | 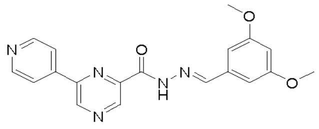 *(E)-N'*-(3,5-dimethoxybenzylidene)-6-(pyridin-4-yl) pyrazine-2-carbohydrazide. | 1H-NMR, 400*MHz,* DMSO-d6 $\delta$ 12,15 (s, 1H), 9,66 (s, 1H), 9,31 (s, 1H), 8,83 (br d, J = 5,0 Hz, 2H), 8,66 (s, 1H), 8,43 (br d, J = 5,1 Hz, 2H), 6,93 (s, 2H), 6,62 (br s, 1H), 3,81 (s, 6H). [M+H]+: 363.9. |
| 104 | 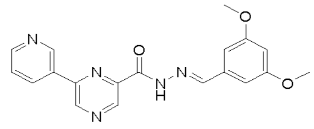 *(E)-N'*-(3,5-dimethoxybenzylidene)-6-(pyridin-3-yl) pyrazine-2-carbohydrazide. | 1H-NMR, 400*MHz,* DMSO-d6 $\delta$ 12.12 (br s, 1H), 9.63 (br d, J = 14.4 Hz, 2H), 9.26 (s, 1H), 8.90 - 8.58 (m, 3H), 7.72 - 7.54 (m, 1H), 6.93 (br s, 2H), 6.62 (br s, 1H), 3.82 (s, 6H). [M+H]+: 364.3. |
| 105 | 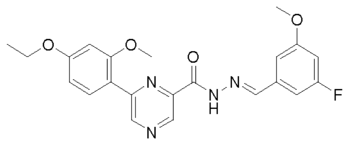 *(E)*-6-(4-ethoxy-2-methoxyphenyl)-*N'*-(3-fluoro-5-methoxybenzylidene) pyrazine-2-carbohydrazide. | 1H-NMR, 400*MHz,* DMSO-d6 $\delta$ 12.10 (s, 1H), 9.32 (s, 1H), 9.04 (s, 1H), 8.61 (s, 1H), 8.15 (br d, J = 8.2 Hz, 1H), 7.14 (br s, 2H), 6.94 (br d, J = 11.2 Hz, 1H), 6.77 - 6.70 (m, 2H), 4.14 (q, J = 6.7 Hz, 2H), 3.91 (s, 3H), 3.85 - 3.80 (m, 3H), 1.36 (t, J = 6.9 Hz, 3H). [M+H] +: 425.1. |
| 106 | 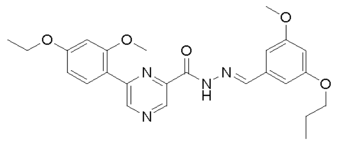 *(E)*-6-(4-ethoxy-2-methoxyphenyl)-N'-(3-methoxy-5-propoxybenzylidene) pyrazine-2-carbohydrazide. | 1H-NMR, 400*MHz,* DMSO-d6 $\delta$ 11.98 (s, 1H), 9.30 (s, 1H), 9.02 (s, 1H), 8.56 (s, 1H), 8.14 (d, J = 9.2 Hz, 1H), 6.89 - 6.85 (m, 2H), 6.74 - 6.70 (m, 2H), 6.56 (t, J = 2.2 Hz, 1H), 4.13 (q, J = 6.8 Hz, 2H), 3.94 (t, J = 6.6 Hz, 2H), 3.89 (s, 3H), 3.77 (s, 3H), 1.72 (sxt, J = 7.0 Hz, 2H), 1.35 (t, J = 6.9 Hz, 3H), 0.97 (t, J = 7.5 Hz, 3H). [M+H]+: 465.1. |
| 107 | 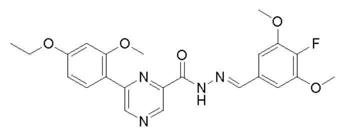 *(E)*-6-(4-ethoxy-2-methoxyphenyl)-*N'*-(4-fluoro-3,5-dimethoxybenzylidene) pyrazine-2-carbohydrazide. | 1H-NMR, 400*MHz,* DMSO-d6 $\delta$ 12,02 (s, 1H), 9,34 (s, 1H), 9,05 (s, 1H), 8,62 (s, 1H), 8,18 (d, J = 8,4 Hz, 1H), 7,15 (d, J = 7,1 Hz, 2H), 6,78 - 6,71 (m, 2H), 4,16 (q, J = 6,9 Hz, 2H), 4,00 - 3,87 (m, 9H), 1,38 (t, J = 6,9 Hz, 3H). [M+H]+: 455.1. |
| 108 | 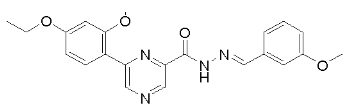 *(E)*-6-(4-ethoxy-2-methoxyphenyl) - *N'*-(3-methoxybenzylidene)pyrazine-2-carbohydrazide. | 1H-NMR, 400*MHz,* DMSO-d6 $\delta$ 12,00 (s, 1H), 9,32 (s, 1H), 9,04 (s, 1H), 8,64 (s, 1H), 8,16 (d, J = 9,3 Hz, 1H), 7,43 - 7,37 (m, 1H), 7,34 - 7,29 (m, 2H), 7,06 - 7,01 (m, 1H), 6,77 - 6,71 (m, 2H), 4,15 (q, J = 7,0 Hz, 2H), 3,91 (s, 3H), 3,81 (s, 3H), 1,37 (t, J = 6,9 Hz, 3H). [M+H]+: 407.2. |

(continued)

| Compound No. | Structure | 1H-NMR/ MS m/z [M+H] + |
|---|---|---|
| 109 | 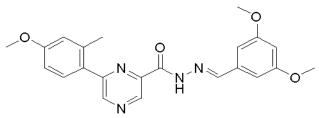 *(E)-N'*-(3,5-dimethoxybenzylidene)-6-(4-methoxy-2-methylphenyl)pyrazine-2-carbohydrazide. | 1H-NMR, 400*MHz,* DMSO-d6 $\delta$ 11,98 (s, 1H), 9,14 (s, 1H), 9,02 (s, 1H), 8,54 (s, 1H), 7,61 (d, J = 8,3 Hz, 1H), 6,98 - 6,91 (m, 2H), 6,85 (s, 2H), 6,57 (s, 1H), 3,82 - 3,75 (m, 9H), 2,42 (s, 3H). [M+H]+: 407,1. |
| 110 | 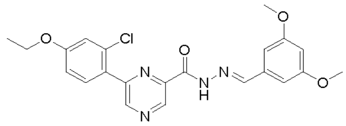 *(E)*-6-(2-chloro-4-ethoxyphenyl)-*N'*-(3,5-dimethoxybenzylidene)pyrazine-2-carbohydrazide. | 1H-NMR, 400*MHz,* DMSO-d6 $\delta$ 12,03 (s, 1H), 9,19 (d, J = 6,5 Hz, 2H), 8,52-8,67 (m, 1H), 7,84 (d, J = 8,7 Hz, 1H), 7,23 (d, J = 2,3 Hz, 1H), 7,14 (dd, J = 8,7, 2,3 Hz, 1H), 7,05 (d, J = 2,1 Hz, 1H), 6,88 (d, J = 2,1 Hz, 2H), 6,57-6,67 (m, 1H), 4,16 (q, J = 6,9 Hz, 2H), 3,80 (s, 6H), 1,37 (t, J = 6,9 Hz, 3H). [M+H]+: 441.0. |
| 111 | 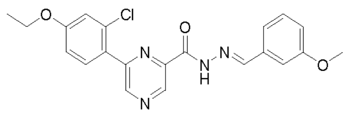 *(E)*-6-(2-chloro-4-ethoxyphenyl)-*N'*-(3-methoxybenzylidene)pyrazine-2-carbohydrazide. | 1H-NMR, 400*MHz,* DMSO-d6 $\delta$ 12,04 (s, 1H), 9,18 (d, J = 8,4 Hz, 2H), 8,59 (s, 1H), 7,83 (d, J = 8,6 Hz, 1H), 7,41 - 7,35 (m, 1H), 7,31 - 7,27 (m, 2H), 7,22 (d, J = 2,4 Hz, 1H), 7,13 (dd, J = 2,5, 8,7 Hz, 1H), 7,03 (dd, J = 1,5, 8,2 Hz, 1H), 4,15 (q, J = 7,0 Hz, 2H), 3,80 (s, 3H), 1,36 (t, J = 6,9 Hz, 3H). [M+H]+: 411,1. |
| 112 | 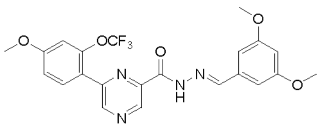 *(E)-N'*-(3,5-dimethoxybenzylidene)-6-(4-methoxy-2-(trifluoromethoxy)phenyl) pyrazine-2-carbohydrazide. | 1H-NMR, 400*MHz,* DMSO-d6 $\delta$ 12,06 (s, 1H), 9,19 (d, J = 11,6 Hz, 2H), 8,58 (s, 1H), 8,19 (d, J = 8,8 Hz, 1H), 7,26 (dd, J = 8,7, 2,5 Hz, 1H), 7,13-7,19 (m, 1H), 6,91 (d, J = 2,2 Hz, 2H), 6,62 (t, J = 2,3 Hz, 1H), 3,92 (s, 3H), 3,81 (s, 6H). [M+H]+: 477,1. |
| 113 | 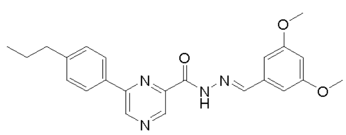 *(E)-N'*-(3,5-dimethoxybenzylidene)-6-(4-propylphenyl) pyrazine-2-carbohydrazide. | 1H-NMR, 400*MHz,* DMSO-d6 $\delta$ 12,09 (s, 1H), 9,49 (s, 1H), 9,15 (s, 1H), 8,68-8,62 (m, 1H), 8,34 (d, J = 8,4 Hz, 2H), 7,42 (d, J = 8,2 Hz, 2H), 6,92 (d, J = 2,4 Hz, 2H), 6,62 (t, J = 2,2 Hz, 1H), 3,81 (s, 6H), 2,71 - 2,62 (m, 2H), 1,65 (sxt, J = 7,3 Hz, 2H), 0,93 (t, J = 7,4 Hz, 3H). [M+H]+: 405,1. |

(continued)

| Compound No. | Structure | 1H-NMR/ MS m/z [M+H] + |
|---|---|---|
| 114 | 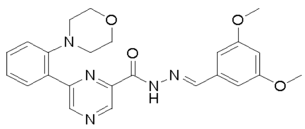 *(E)-N'*-(3,5-dimethoxybenzylidene)-6-(2-methoxyphenyl) pyrazine-2-carbohydrazide. | 1H-NMR, 400*MHz,* DMSO-d6 $\delta$ 12.03 (s, 1H), 9.52 (s, 1H), 9.10 (s, 1H), 8.56 (s, 1H), 7.86 - 7.75 (m, 1H), 7.54 - 7.40 (m, 1H), 7.32 - 7.18 (m, 2H), 6.87 (d, *J* = 2.0 Hz, 2H), 6.63 - 6.52 (m, 1H), 3.78 (s, 6H), 3.53 (br s, 4H), 2.78 (br s, 4H). [M+H]+: 448.2. |
| 115 | 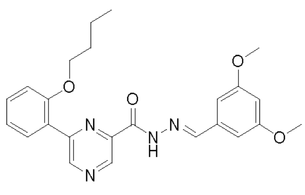 *(E)*-6-(2-chlorophenyl)-*N'*-(3,5-dimethoxybenzylidene) pyrazine-2-carbohydrazide. | 1H-NMR, 400*MHz,* DMSO-d6 $\delta$ 10,79 (s, 1H), 9,39 (d, J = 2,5 Hz, 2H), 8,32 (s, 1H), 7,84 (dd, J = 1,7, 7,6 Hz, 1H), 7,56 - 7,47 (m, 1H), 7,16 (t, J = 7,3 Hz, 1H), 7,08 (d, J = 8,2 Hz, 1H), 6,97 (d, J = 2,3 Hz, 2H), 6,54 (t, J = 2,3 Hz, 1H), 4,11 (t, J = 6,4 Hz, 2H), 3,85 (s, 6H), 3,88 - 3,79 (m, 1H), 1,87 - 1,76 (m, 2H), 1,48 (sxt, J = 7,5 Hz, 2H), 0,96 (t, J = 7,4 Hz, 3H). [M+H]+: 435,2. |
| 116 | 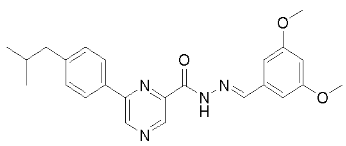 *(E)-N'*-(3,5-dimethoxybenzylidene)-6-(4-isobutylphenyl) pyrazine-2-carbohydrazide. | 1H-NMR, 400*MHz,* DMSO-d6 $\delta$ 12.09 (s, 1H), 9.49 (s, 1H), 9.15 (s, 1H), 8.68-8.62 (m, 1H), 8.34 (d, J = 8.4 Hz, 2H), 7.42 (d, J = 8.2 Hz, 2H), 6.92 (d, J = 2.4 Hz, 2H), 6.62 (t, J = 2.2 Hz, 1H), 3.81 (s, 6H), 2.71 - 2.62 (m, 2H), 1.65 (sxt, J = 7.3 Hz, 2H), 0.93 (t, J = 7.4 Hz, 3H). [M+H]+: 419.3. |
| 117 | 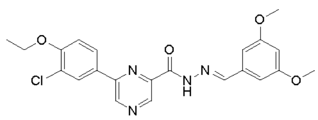 *(E)*-6-(3-chloro-4-ethoxyphenyl)-*N'*-(3,5-dimethoxybenzylidene)pyrazine-2-carbohydrazide. | 1H-NMR, 400*MHz,* DMSO-d6 612.17 (s, 1H), 9.52 (s, 1H), 9.15 (s, 1H), 8.70 - 8.59 (m, 2H), 8.37 (dd, J = 2.3, 8.7 Hz, 1H), 7.33 (d, J = 8.8 Hz, 1H), 6.95 (d, J = 2.3 Hz, 2H), 6.62 (t, J = 2.3 Hz, 1H), 4.25 (q, J = 6.9 Hz, 2H), 3.82 (s, 6H), 1.42 (t, J = 7.0 Hz, 3H). [M+H]+: 441.2. |
| 118 | 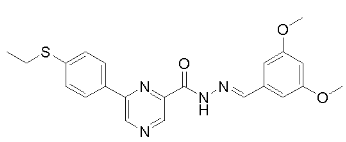 *(E)-N'*-(3,5-dimethoxybenzylidene)-6-(4-(ethylthio)phenyl) pyrazine-2-carbohydrazide. | 1H-NMR, 400*MHz,* DMSO-d6 $\delta$ 12.09 (s, 1H), 9.51 (s, 1H), 9.16 (s, 1H), 8.66 (s, 1H), 8.39 (d, J = 8.6 Hz, 2H), 7.49 (d, J = 8.5 Hz, 2H), 6.93 (d, J = 2.3 Hz, 2H), 6.62 (t, J = 2.3 Hz, 1H), 3.82 (s, 6H), 3.11 (q, J = 7.3 Hz, 2H), 1.31 (t, J = 7.3 Hz, 3H). [M+H]+: 423,2. |

(continued)

| Compound No. | Structure | 1H-NMR/ MS m/z [M+H] + |
|---|---|---|
| 119 | 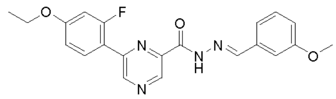 *(E)*-6-(4-ethoxy-2-fluorophenyl)-*N'*-(3-methoxybenzylidene)pyrazine-2-carbohydrazide. | 1H-NMR, 400*MHz,* DMSO-d6 $\delta$ 12.06 (s, 1H), 9.21 (d, J = 2.2 Hz, 1H), 9.14 (s, 1H), 8.65 (s, 1H), 8.35 (t, J = 9.0 Hz, 1H), 7.42 - 7.37 (m, 1H), 7.34 - 7.30 (m, 2H), 7.07 - 6.99 (m, 3H), 4.15 (q, J = 7.1 Hz, 2H), 3.82 (s, 3H), 1.37 (t, J = 6.9 Hz, 3H). [M+H]+: 395.1. |
| 120 | 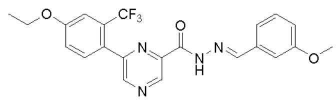 (E)-6-(4-ethoxy-2-(trifluoromethyl)phenyl)-*N'*-(3-methoxybenzylidene)pyrazine-2-carbohydrazide. | 1H-NMR, 400*MHz,* DMSO-d6 $\delta$ 12.06 (s, 1H), 9.43 (s, 1H), 9.19 (s, 1H), 8.62 (s, 1H), 8.31 (d, J = 8.4 Hz, 1H), 7.53 - 7.48 (m, 2H), 7.42 - 7.36 (m, 1H), 7.33 - 7.28 (m, 2H), 7.06 - 7.01 (m, 1H), 4.29 (q, J = 6.9 Hz, 2H), 3.81 (s, 3H), 1.37 (t, J = 6.8 Hz, 3H). [M+H]+: 445.1. |
| 121 | 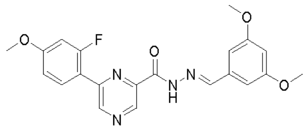 *(E)-N'*-(3,5-dimethoxybenzylidene)-6-(2-fluoro-4-methoxyphenyl)pyrazine-2-carbohydrazide. | 1H-NMR, 400*MHz,* DMSO-d6 $\delta$ 12.05 (s, 1H), 9.33 (s, 1H), 9.12 (s, 1H), 8.59 (s, 1H), 8.22 (br t, J = 7.9 Hz, 1H), 7.18 (br d, J = 11.1 Hz, 1H), 7.03 (br t, J = 7.5 Hz, 1H), 6.90 (s, 2H), 6.60 (br s, 1H), 3.94 (s, 3H), 3.80 ppm (s, 6H). [M+H] +: 411.0. |
| 122 | 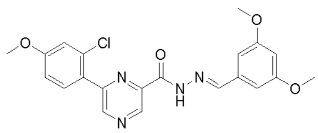 *(E)*-6-(2-chloro-4-ethoxyphenyl)- *N'*-(3,5-dimethoxybenzylidene)pyrazine-2-carbohydrazide. | 1H-NMR, 400*MHz,* DMSO-d6 $\delta$ 12.04 (s, 1H), 9.19 (d, J = 7.0 Hz, 2H), 8.55 (s, 1H), 7.85 (d, J = 8.6 Hz, 1H), 7.26 (d, J = 1.8 Hz, 1H), 7.16 (br d, J = 8.8 Hz, 1H), 6.89 (s, 2H), 6.60 (br s, 1H), 3.88 (s, 3H), 3.80 ppm (s, 6H). [M+H]+: 427.0. |
| 123 | 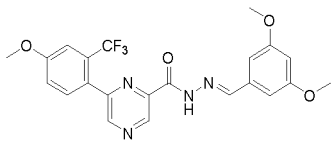 *(E)-N'*-(3,5-dimethoxybenzylidene)-6-(4-methoxy-2-(trifluoromethoxy)phenyl) pyrazine-2-carbohydrazide. | 1H-NMR, 400*MHz,* DMSO-d6 $\delta$ 12.08 (s, 1H), 9.39 (s, 1H), 9.20 (s, 1H), 8.58 (s, 1H), 8.30 (d, J = 7.7 Hz, 1H), 7.50 - 7.56 (m, 2H), 6.90 (d, J = 2.2 Hz, 2H), 6.61 (t, J = 2.2 Hz, 1H), 4.01 (s, 3H), 3.80 ppm (s, 6H). [M+H]+: 461.0. |
| 124 | 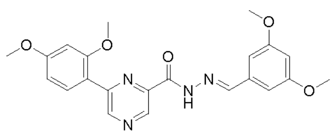 *(E)-N'*-(3,5-dimethoxybenzylidene)-6-(2,4-dimethoxyphenyl)pyrazine-2-carbohydrazide. | 1H-NMR, 400*MHz,* DMSO-d6 $\delta$ 12.01 (br s, 1H), 9.33 (s, 1H), 9.05 (s, 1H), 8.59 (s, 1H), 8.17 (d, J = 8.6 Hz, 1H), 6.90 (d, J = 2.3 Hz, 2H), 6.73-6.78 (m, 2H), 6.60 (t, J = 2.3 Hz, 1H), 3.93 (s, 3H), 3.87 (s, 3H), 3.78-3.82 (m, 1H), 3.80 ppm (s, 5H). [M+H]+: 423,2. |

(continued)

| Compound No. | Structure | 1H-NMR/ MS m/z [M+H] + |
|---|---|---|
| 125 | 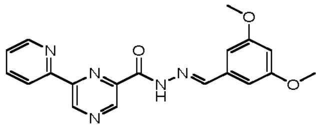 *(E)*-*N*'-(3,5-dimethoxybenzylidene)-6-(pyridin-2-yl) pyrazine-2-carbohydrazide. | 1H-NMR, 400*MHz,* DMSO-d6 $\delta$ 12,18 (s, 1H), 9,76 (s, 1H), 9,30 (s, 1H), 8,88 (br d, J = 7,9 Hz, 1H), 8,80 (br d, J = 4,2 Hz, 1H), 8,68 (s, 1H), 8,10 (br t, J = 7,6 Hz, 1H), 7,62 (br d, J = 5,5 Hz, 1H), 6,94 (s, 2H), 6,63 (br s, 1H), 3,82 (s, 6H). [M+H]+: 364.0. |
| 126 | 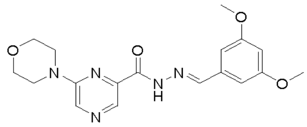 *(E)*-*N*'-(3,5-dimethoxybenzylidene)-6-morpholinopyrazine-2-carbohydrazide. | 1H-NMR, 400*MHz,* DMSO-d6 $\delta$ 11,63 (s, 1H), 8,69 - 8,37 (m, 3H), 7,00 - 6,53 (m, 3H), 3,80 (s, 6H), 3,75 (br d, J = 7,7 Hz, 8H). [M+H]+: 372.0. |
| 127 | 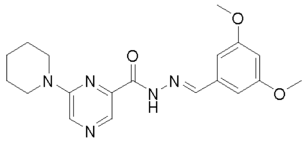 *(E)*-*N*'-(3,5-dimethoxybenzylidene)-6-(piperidin-1-yl) pyrazine-2-carbohydrazide. | 1H-NMR, 400*MHz,* DMSO-d6 $\delta$ 11,58 (s, 1H), 8,56 (d, J = 18,3 Hz, 2H), 8,36 (s, 1H), 6,90 (d, J = 2,3 Hz, 2H), 6,60 (t, J = 2,2 Hz, 1H), 3,80 (s, 6H), 3,77 - 3,70 (m, 4H), 1,72 - 1,56 (m, 6H). [M+H]+: 370.0. |
| 128 | 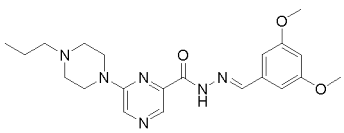 *(E)*-*N*'-(3,5-dimethoxybenzylidene)-6-(4-propylpiperazin-1-yl)pyrazine-2-carbohydrazide. | 1H-NMR, 400*MHz,* DMSO-d6 $\delta$ 8.52 (s, 1H), 8.45 (s, 1H), 8.40 (s, 1H), 7.06 (d, J =2.3 Hz, 2H), 6.56 (t, J =2.3 Hz, 1H), 3.85 (s, 6H), 3.84 - 3.79 (m, 4H), 2.71 - 2.61 (m, 4H), 2.52 - 2.36 (m, 2H), 1.63 (dd, J =7.6, 15.5 Hz, 2H), 0.99 (t, J =7.4 Hz, 3H). [M+H]+: 413.1. |
| 129 | 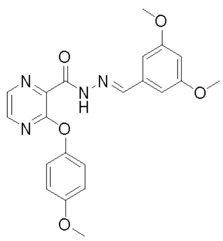 *(E)*-*N*'-(3,5-dimethoxybenzylidene)-3-(4-methoxyphenyl) pyrazine-2-carbohydrazide. | 1H-NMR, 400*MHz,* DMSO-d6 $\delta$ 12,11 - 11,81 (m, 1H), 8,43 - 7,95 (m, 3H), 7,12 (br d, J = 8,2 Hz, 1H), 7,02 - 6,82 (m, 4H), 6,60 - 6,46 (m, 2H), 3,84 - 3,60 (m, 9H). [M+H]+: 409.0. |
| 130 | 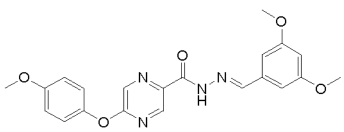 *(E)*-*N*'-(3,5-dimethoxybenzylidene)-5-(4-methoxyphenyl) pyrazine-2-carbohydrazide. | 1H-NMR, 400*MHz,* DMSO-d6 $\delta$ 12,18 - 12,04 (m, 1H), 8,86 - 8,77 (m, 1H), 8,64 (dd, J = 2,4, 8,6 Hz, 1H), 8,23 - 7,74 (m, 1H), 7,67 (dd, J = 8,7, 17,3 Hz, 2H), 7,04 (dd, J = 8,8, 17,9 Hz, 2H), 6,87 (d, J = 2,0 Hz, 1H), 6,60 - 6,44 (m, 1H), 6,39 (d, J = 2,0 Hz, 1H), 3,81 - 3,76 (m, 6H), 3,66 (s, 3H). [M+H]+: 409.0. |

(continued)

| Compound No. | Structure | 1H-NMR/ MS m/z [M+H] + |
|---|---|---|
| 131 | 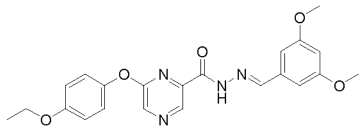 *(E)-N'*-(3,5- dimethoxybenzylidene)-6-(4-ethoxyphenyl) pyrazine-2-carbohydrazide. | 1H-NMR, 400MHz CDCl3 $\delta$ 10,12 (s, 1H), 9,16 (s, 1H), 8,58 (s, 1H), 8,09 (s, 1H), 7,13 (d, J = 8,9 Hz, 2H), 7,01 (d, J = 8,9 Hz, 2H), 6,91 (d, J = 1,9 Hz, 2H), 6,53 (s, 1H), 4,10 (q, J = 7,0 Hz, 2H), 3,88 - 3,81 (m, 6H), 1,48 (t, J = 6,9 Hz, 3H). [M+H]+: 423,2. |
| 132 | 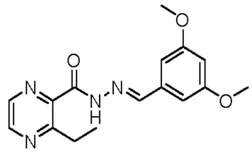 *(E)-N'*-(3,5-dimethoxybenzylidene)-3-ethylpyrazine-2-carbohydrazide. | 1H-NMR, 400*MHz,* DMSO-d6 $\delta$ 11,83 (br s, 1H), 8,76 - 8,59 (m, 1H), 8,54 (br s, 1H), 8,44 - 7,92 (m, 1H), 6,97 - 6,38 (m, 3H), 3,81 - 3,61 (m, 6H), 3,11 (br d, J = 7,1 Hz, 1H), 2,74 (br s, 1H), 1,31 - 1,15 (m, 3H). [M+H]+: 315.1. |
| 133 | 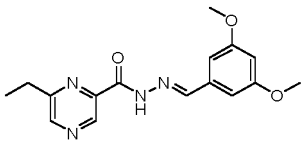 *(E)-N'*-(3,5-dimethoxybenzylidene)-6-ethylpyrazine-2-carbohydrazide. | 1H-NMR, 400*MHz,* DMSO-d6 $\delta$ 11,95 (s, 1H), 9,06 (s, 1H), 8,84 (s, 1H), 8,59 (s, 1H), 6,88 (d, J = 2,3 Hz, 2H), 6,60 (t, J = 2,3 Hz, 1H), 3,80 (s, 6H), 2,93 (q, J = 7,6 Hz, 2H), 1,35 (t, J = 7,6 Hz, 3H). [M+H]+: 315.0. |
| 134 | 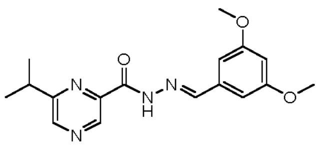 *(E)-N'*-(3,5-dimethoxybenzylidene)-6-isopropylpyrazine-2-carbohydrazide. | 1H-NMR, 400*MHz,* DMSO-d6 $\delta$ 11,81 (s, 1H), 9,05 (s, 1H), 8,85 (s, 1H), 8,59 (s, 1H), 6,88 (d, J = 2,0 Hz, 2H), 6,59 (s, 1H), 3,78 (s, 6H), 3,27 (br s, 1H), 1,35 (d, J = 7,1 Hz, 6H). [M+H]+: 329.1. |
| 135 | 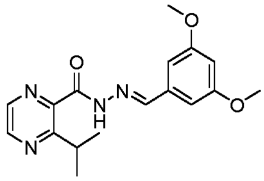 *(E)-N'*-(3,5-dimethoxybenzylidene)-3-isopropylpyrazine-2-carbohydrazide. | 1H-NMR, 400*MHz,* DMSO-d6 $\delta$ 11,88 (br s, 1H), 8,78 - 8,61 (m, 1H), 8,56 - 8,43 (m, 1H), 8,37 (s, 1H), 6,94 - 6,54 (m, 2H), 6,44 (br s, 1H), 3,86 - 3,69 (m, 5H), 3,63 (br s, 2H), 1,29 - 1,17 (m, 6H). [M+H]+: 329.1. |
| 136 | 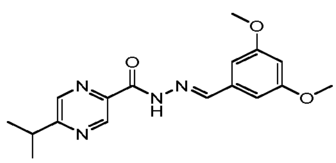 *(E)-N'*-(3,5-dimethoxybenzylidene)-5-isopropylpyrazine-2-carbohydrazide. | 1H-NMR, 400*MHz,* DMSO-d6 $\delta$ 9,26 - 9,17 (m, 1H), 8,95 - 8,58 (m, 1H), 8,34 (s, 1H), 7,04 (d, J = 2,2 Hz, 2H), 6,52 (s, 1H), 4,84 (s, 5H), 4,85 - 4,82 (m, 1H), 3,28 - 3,17 (m, 1H), 2,28 - 1,31 (m, 6H). [M+H]+: 329.2. |

(continued)

| Compound No. | Structure | 1H-NMR/ MS m/z [M+H] + |
|---|---|---|
| 137 | 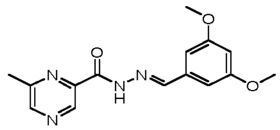 *(E)-N'*-(3,5-dimethoxybenzylidene)-6-methylpyrazine-2-carbohydrazide. | 1H-NMR, 400*MHz,* DMSO-d6 $\delta$ 12,04 (br s, 1H), 9,05 (s, 1H), 8,82 (s, 1H), 8,58 (s, 1H), 6,88 (d, J = 2,2 Hz, 2H), 6,61 (t, J = 2,3 Hz, 1H), 3,79-3,82 (m, 1H), 3,81 (s, 5H), 2,64 (s, 3H). [M+H]+: 301.0. |
| 138 | 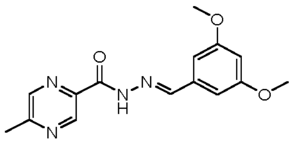 *(E)-N'*-(3,5-dimethoxybenzylidene)-5-methylpyrazine-2-carbohydrazide. | 1H-NMR, 400*MHz,* DMSO-d6 $\delta$ 9,12 (d, J = 0,9 Hz, 1H), 8,68 (s, 1H), 8,56 (s, 1H), 6,85 (d, J = 2,2 Hz, 2H), 6,59 (t, J = 2,1 Hz, 1H), 3,80 (s, 6H), 2,62 (s, 3H). [M+H]+: 301.1. |
| 139 | 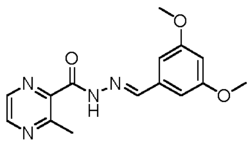 *(E)-N'*-(3,5-dimethoxybenzylidene)-3-methylpyrazine-2-carbohydrazide. | 1H-NMR, 400*MHz,* DMSO-d6 $\delta$ 12,07 - 11,83 (m, 1H), 8,71 (br s, 1H), 8,63 - 7,96 (m, 2H), 6,93 - 6,43 (m, 3H), 3,86 - 3,62 (m, 6H), 2,77 (s, 2H), 2,46 (br s, 1H). [M+H]+: 301.1. |
| 140 | 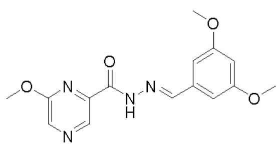 *(E)-N'*-(3,5-dimethoxybenzylidene)-6-methoxypyrazine-2-carbohydrazide. | 1H-NMR, 400*MHz,* DMSO-d6 $\delta$ 11,76 (s, 1H), 8,79 (s, 1H), 8,57 (d, J = 5,4 Hz, 2H), 6,90 (d, J = 2,1 Hz, 2H), 6,60 (t, J = 2,1 Hz, 1H), 4,11 (s, 3H), 3,78 - 3,81 (m, 1H), 3,80 (s, 5H). [M+H]+: 317.0. |
| 141 | 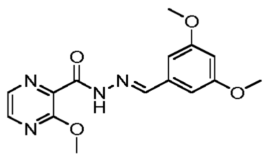 *(E)-N'*-(3,5-dimethoxybenzylidene)-3-methoxypyrazine-2-carbohydrazide. | 1H-NMR, 400*MHz,* DMSO-d6 $\delta$ 11,76 (br s, 1H), 8,52 - 7,81 (m, 3H), 7,05 - 6,72 (m, 1H), 6,69 - 6,27 (m, 2H), 4,01 - 3,88 (m, 3H), 3,82 - 3,62 (m, 6H). [M+H]+: 317.0. |
| 142 | 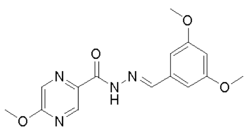 *(E)-N'*-(3,5-dimethoxybenzylidene)-5-methoxypyrazine-2-carbohydrazide. | 1H-NMR, 400*MHz,* DMSO-d6 $\delta$ 12,00 (br s, 1H), 8,86 (d, J = 1,2 Hz, 1H), 8,53 (s, 1H), 8,39 (d, J = 1,2 Hz, 1H), 6,86 (d, J = 2,2 Hz, 2H), 6,58 (t, J = 2,3 Hz, 1H), 4,02 (s, 3H), 3,80 (s, 6H). [M+H]+: 317.1. |

(continued)

| Compound No. | Structure | 1H-NMR/ MS m/z [M+H] + |
|---|---|---|
| 143 | 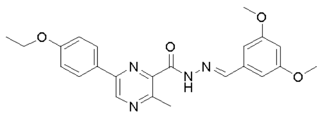 *(E)-N'*-(3,5-dimethoxybenzylidene)-6-(4-ethoxyphenyl)-3-methylpyrazine-2-carbohydrazide. | 1H-NMR, 400MHz CDCl3 $\delta$ 1,47 (t, J = 7,06 Hz, 2 H) 1,42 - 1,50 (m, 1 H) 1,61 (s, 3 H) 3,04 (s, 3 H) 3,84 (s, 6 H) 4,12 (q, J = 6,84 Hz, 2 H) 6,52 (t, J = 2,21 Hz, 1 H) 6,93 - 7,09 (m, 2 H) 6,93 - 7,11 (m, 1 H) 8,00 (br d, J = 8,82 Hz, 1 H) 7,95 (d, J = 8,82 Hz, 1 H) 8,20 - 8,25 (m, 1 H) 8,29 (s, 1 H) 9,02 (s, 1 H) 10,93 (s, 1 H). [M+H] +: 421.2. |
| 144 | 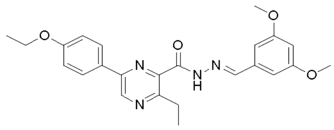 *(E)-N'*-(3,5-dimethoxybenzylidene)-6-(4-ethoxyphenyl)-3-ethylpyrazine-2-carbohydrazide. | 1H-NMR, 400MHz CDCl3 $\delta$ 1,33 - 1,52 (m, 6 H) 3,46 (q, J = 7,50 Hz, 2 H) 3,84 (s, 6 H) 4,12 (q, J = 7,06 Hz, 2 H) 6,52 (t, J = 2,20 Hz, 1 H) 6,90 - 7,11 (m, 4 H) 7,95 (d, J = 8,82 Hz, 2 H) 8,29 (s, 1 H) 9,05 (s, 1 H) 10,94 (s, 1 H). [M+H]+: 435,2. |
| 145 | 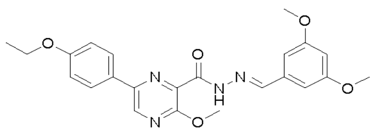 *(E)-N'*-(3,5-dimethoxybenzylidene)-6-(4-ethoxyphenyl)-3-methoxypyrazine-2-carbohydrazide. | 1H-NMR, 400MHz CDCl3 $\delta$ 1,45 (t, J = 6,84 Hz, 3 H) 3,83 (s, 6 H) 3,97 - 4,25 (m, 4 H) 4,05 - 4,16 (m, 1 H) 6,51 (t, J = 2,10 Hz, 1 H) 6,86 - 7,08 (m, 4 H) 7,86 (d, J = 8,38 Hz, 2 H) 8,48 (s, 1 H) 8,67 (s, 1 H) 10,60 (s, 1 H) 10,58 - 10,61 (m, 1 H) 10,58 - 10,62 (m, 1 H). [M+H]+: 437.2. |
| 146 | 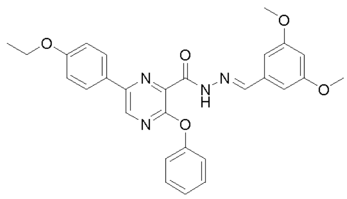 *(E)-N'*-(3,5-dimethoxybenzylidene)-6-(4-ethoxyphenyl)-3-phenoxypyrazine -2-carbohydrazide. | 1H-NMR, 400MHz CDCl3 $\delta$ 1,46 (t, J = 6,95 Hz, 3 H) 3,84 (s, 6 H) 4,11 (q, J = 7,06 Hz, 2 H) 6,52 (t, J = 2,20 Hz, 1 H) 6,93 - 7,07 (m, 4 H) 7,19 - 7,25 (m, 2 H) 7,27 - 7,32 (m, 1 H) 7,39 - 7,47 (m, 2 H) 7,87 (d, J = 8,82 Hz, 2 H) 8,53 (s, 1 H) 8,62 (s, 1 H) 10,68 (s, 1 H). [M+H]+: 499.2. |
| 147 | 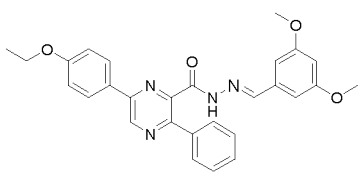 *(E)-N'*-(3,5-dimethoxybenzylidene)-6-(4-ethoxyphenyl)-3-phenylpyrazine-2-carbohydrazide. | 1H-NMR, 400MHz CDCl3 $\delta$ 1,35 (q, J = 6,84 Hz, 3 H) 3,51 - 3,85 (m, 6 H) 4,11 (quin, J = 7,10 Hz, 2 H) 6,36 - 6,61 (m, 1 H) 6,36 - 6,44 (m, 1 H) 6,54 - 6,60 (m, 1 H) 6,87 (d, J = 2,20 Hz, 1 H) 7,09 (br dd, J = 12,78, 9,04 Hz, 2 H) 7,36 - 7,52 (m, 3 H) 7,68 - 7,77 (m, 2 H) 8,18 - 8,28 (m, 2 H) 9,40 (s, 1 H) 12,23 (s, 1 H). [M+H]+: 483.2. |

(continued)

| Compound No. | Structure | 1H-NMR/ MS m/z [M+H] + |
|---|---|---|
| 148 | 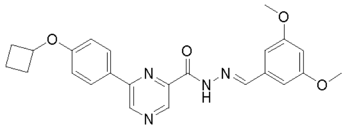 *(E)*-6-(4-cyclopropoxyphenyl)-*N'*-(3,5-dimethoxybenzylidene)pyrazine-2-carbohydrazide. | 1H-NMR, 400*MHz,* DMSO-d6 $\delta$ 12,06 (s, 1H), 9,45 (s, 1H), 9,10 (s, 1H), 8,66 (s, 1H), 8,38 (d, J = 8,8 Hz, 2H), 7,03 (d, J = 8,8 Hz, 2H), 6,93 (d, J = 2,2 Hz, 2H), 6,62 (t, J = 2,3 Hz, 1H), 4,83 (quin, J = 7,1 Hz, 1H), 3,82 (s, 6H), 2,50 - 2,45 (m, 2H), 2,15 - 2,04 (m, 2H), 1,83 (q, J = 10,1 Hz, 1H), 1,76 - 1,61 (m, 1H). [M+H]+: 433.1. |
| 149 | 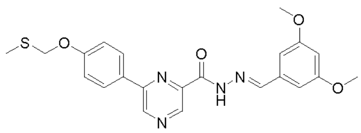 *(E)*-*N*'-(3,5-dimethoxybenzylidene)-6-(4-((methylthio) methoxy)phenyl)pyrazi ne-2-carbohydrazide. | 1H-NMR, 400*MHz,* DMSO-d6 $\delta$ 12,07 (s, 1H), 9,48 (s, 1H), 9,12 (s, 1H), 8,62-8,68 (m, 1H), 8,65 (s, 1H), 8,41 (d, J = 8,9 Hz, 2H), 7,21 (d, J = 8,9 Hz, 2H), 6,93 (d, J = 2,2 Hz, 2H), 6,62 (t, J = 2,3 Hz, 1H), 5,41 (s, 2H), 3,82 (s, 6H), 2,22 (s, 3H). [M+H]+: 439.1. |
| 150 | 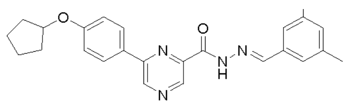 *(E)*-6-(4-(cyclopentyloxy)phenyl)-*N*-(3,5-dimethylbenzylidene)pyrazine-2-carbohydrazide. | 1H-NMR, 400*MHz,* DMSO-d6 $\delta$ 12,03 (s, 1H), 9,45 (s, 1H), 9,48 - 9,42 (m, 1H), 9,10 (s, 1H), 8,64 (s, 1H), 8,42 - 8,35 (m, 2H), 7,39 (s, 2H), 7,14 - 7,03 (m, 3H), 5,02 - 4,92 (m, 1H), 2,34 (s, 6H), 2,07 - 1,94 (m, 2H), 1,82 - 1,70 (m, 4H), 1,67 - 1,57 (m, 2H). [M+H]+: 415.2. |
| 151 | 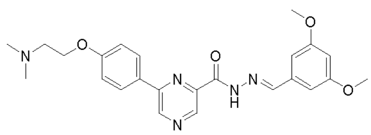 *(E)*-*N*'-(3,5-dimethoxybenzylidene)-6-(4-(2-(dimethylamino)ethoxy)phenyl) pyrazine-2-carbohydrazide. | 1H-NMR, 400*MHz,* DMSO-d6 $\delta$ 12,07 (s, 1H), 9,47 (s, 1H), 9,11 (s, 1H), 8,66 (s, 1H), 8,41 (d, J = 8,9 Hz, 2H), 7,15 (d, J = 8,8 Hz, 2H), 6,93 (s, 1H), 6,62 (t, J = 2,3 Hz, 1H), 4,18 (t, J = 5,7 Hz, 2H), 3,82 (s, 6H), 3,30 (s, 1H), 2,67 (t, J = 5,7 Hz, 2H), 2,25 (s, 6H). [M+H]+: 450.1. |
| 152 | 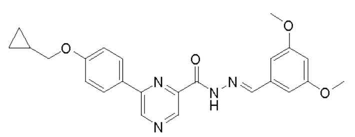 *(E)*-6-(4-(cyclopropylmethoxy)phenyl)-*N*'-(3,5-dimethoxybenzylidene) pyrazine-2-carbohydrazide. | 1H-NMR, 400*MHz,* DMSO-d6 $\delta$ 12,06 (s, 1H), 9,46 (s, 1H), 9,10 (s, 1H), 8,66 (s, 1H), 8,40 (d, J =8,8 Hz, 2H), 7,12 (d, J = 8,9 Hz, 2H), 6,93 (d, J = 2,2 Hz, 2H), 6,62 (t, J = 2,3 Hz, 1H), 3,94 (d, J = 7,0 Hz, 2H), 3,82 (s, 6H), 1,22-1,32 (m, 1H), 0,57 - 0,65 (m, 2H), 0,34-0,40 (m, 2H). [M+H] +: 433.1. |
| 153 | 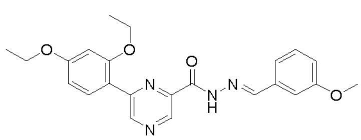 *(E)*-6-(2,4-diethoxyphenyl)-*N*'-(3-methoxybenzylidene) pyrazine-2-carbohydrazide. | 1H-NMR, 400*MHz,* DMSO-d6 $\delta$ 11,98 (s, 1H), 9,36 (s, 1H), 9,02 (s, 1H), 8,69 - 8,60 (m, 1H), 8,16 (d, J = 8,8 Hz, 1H), 7,44 - 7,27 (m, 3H), 7,10 - 6,98 (m, 1H), 6,74 - 6,67 (m, 2H), 4,21 - 4,08 (m, 4H), 3,81 - 3,78 (m, 3H), 1,36 (q, J = 6,8 Hz, 6H). [M+H] +: 421.1. |

(continued)

| Compound No. | Structure | 1H-NMR/ MS m/z [M+H] + |
|---|---|---|
| 154 | 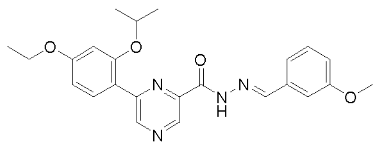 (E)-6-(4-ethoxy-2-isopropoxyphenyl)-N'-(3-methoxybenzylidene)pyrazine-2-carbohydrazide. | 1H-NMR, *400MHz,* DMSO-d6 $\delta$ 12,06 (s, 1H), 9,42 (s, 1H), 9,11 (s, 1H), 8,71 (s, 1H), 8,21 (d, J = 8,9 Hz, 1H), 7,50 - 7,44 (m, 1H), 7,41 - 7,36 (m, 2H), 7,14 - 7,09 (m, 1H), 6,83 - 6,77 (m, 2H), 4,89 (td, J = 5,9, 11,9 Hz, 1H), 4,21 (q, J = 6,8 Hz, 2H), 3,89 (s, 3H), 1,48 - 1,36 (m, 9H). [M+H]+: 435,1. |
| 155 | 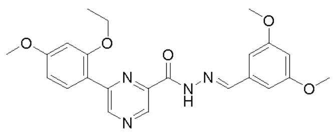 *(E)-N'*-(3,5-dimethoxybenzylidene)-6-(2-methoxyphenyl) pyrazine-2-carbohydrazide. | 1H-NMR, 400*MHz,* DMSO-d6 $\delta$ 11,99 (s, 1H), 9,39 (s, 1H), 9,05 (s, 1H), 8,60 (s, 1H), 8,15-8,21 (m, 1H), 6,91 (d, J = 2,0 Hz, 2H), 6,71-6,78 (m, 2H), 6,61 (s, 1H), 4,21 (q, J = 6,9 Hz, 2H), 3,86 (s, 3H), 3,81 (s, 6H), 1,39 ppm (t, J = 6,9 Hz, 3H). [M+H] +: 437.2. |
| 156 | 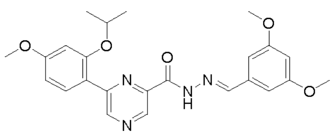 *(E)-N'*-(3,5-dimethoxybenzylidene)-6-(2-isopropoxy-4-methoxyphenyl) pyrazine-2-carbohydrazide. | 1H-NMR, 400*MHz,* DMSO-d6 $\delta$ 11,99 (s, 1H), 9,35 (s, 1H), 9,04 (s, 1H), 8,59 (s, 1H), 8,15 (d, J = 8,4 Hz, 1H), 6,90 (d, J = 2,2 Hz, 2H), 6,71 - 6,77 (m, 2H), 6,60 (t, J = 2,3 Hz, 1H), 4,83 (spt, J = 6,0 Hz, 1H), 3,85 (s, 3H), 3,80 (s, 6H), 1,33 (s, 3H), 1,32 ppm (s, 3H). [[M+H]+: 451.2. |
| 157 | 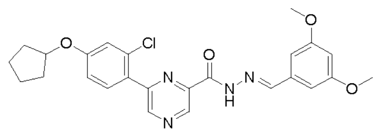 (*E*)-6-(2-chloro-4-(cyclopentyloxy)phenyl)-*N'*-(3,5-dimethoxybenzylidene)pyrazine-2-carbohydrazide. | 1H-NMR, 400M*Hz,* DMSO-d6 $\delta$ 12,01 (s, 1H), 9,17 (d, J = 5,3 Hz, 2H), 8,53 (s, 1H), 7,81 (d, J = 8,6 Hz, 1H), 7,18 (d, J = 2,4 Hz, 1H), 7,10 (dd, J = 2,4, 8,6 Hz, 1H), 6,86 (d, J = 2,2 Hz, 2H), 6,58 (t, J = 2,2 Hz, 1H), 5,07 - 4,84 (m, 1H), 3,78 (s, 6H), 2,02 - 1,88 (m, 2H), 1,79 - 1,68 (m, 4H), 1,66 - 1,44 (m, 2H). [M+H]+: 481.1. |
| 158 | 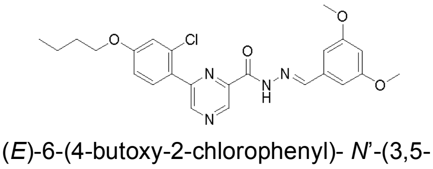 (*E*)-6-(4-butoxy-2-chlorophenyl)- *N'*-(3,5-dimethoxybenzylidene)pyrazine-2-carbohydrazide. | 1H-NMR, 400*MHz,* DMSO-d6 $\delta$ 12,04 (s, 1H), 9,20 (d, J = 6,0 Hz, 2H), 8,56 (s, 1H), 7,85 (d, J = 8,7 Hz, 1H), 7,26 (d, J = 2,3 Hz, 1H), 7,16 (dd, J = 2,4, 8,7 Hz, 1H), 6,89 (d, J = 2,1 Hz, 2H), 6,68 - 6,53 (m, 1H), 4,12 (t, J = 6,5 Hz, 2H), 3,81 (s, 6H), 1,82 - 1,65 (m, 2H), 1,58 - 1,34 (m, 2H), 0,96 (t, J = 7,4 Hz, 3H). [M+H]+: 469,1. |

(continued)

| Compound No. | Structure | 1H-NMR/ MS m/z [M+H] + |
|---|---|---|
| 159 | 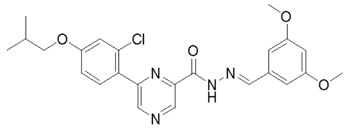 (*E*)-6-(2-chloro-4-isobutoxyphenyl)-*N*'-(3,5-dimethoxybenzylidene)pyrazine-2-carbohydrazide. | 1H-NMR, 400*MHz,* DMSO-d6 $\delta$ 12,04 (s, 1H), 9,20 (d, J = 6,7 Hz, 2H), 8,56 (s, 1H), 7,85 (d, J = 8,7 Hz, 1H), 7,26 (d, J = 2,3 Hz, 1H), 7,16 (dd, J = 2,3, 8,7 Hz, 1H), 6,89 (d, J = 2,1 Hz, 2H), 6,67 - 6,53 (m, 1H), 3,90 (d, J = 6,6 Hz, 2H), 3,81 (s, 6H), 2,07 (td, J = 6,6, 13,3 Hz, 1H), 1,01 (d, J = 6,7 Hz, 6H). [M+H] +: 469.1. |
| 160 | 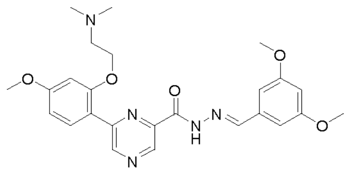 (*E*)-*N*'-(3,5-dimethoxybenzylidene)-6-(2-(2-(dimethylamino)ethoxy)-4-methoxyphenol) pyrazine-2-carbohydrazide. | 1H-NMR, 400*MHz,* DMSO-d6 $\delta$ 12,00 (s, 1H), 9,52 (s, 1H), 9,05 (s, 1H), 8,61 (s, 1H), 8,21 (d, J = 8,7 Hz, 1H), 6,91 (d, J = 2,0 Hz, 2H), 6,82 - 6,71 (m, 2H), 6,61 (s, 1H), 4,24 (t, J = 5,4 Hz, 2H), 3,88 (s, 3H), 3,81 (s, 6H), 2,69 (t, J = 5,4 Hz, 2H), 2,23 (s, 6H). [M+H]+: 480.2. |
| 161 | 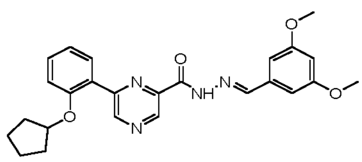 *(E)*-6-(2-(cyclopentyloxy)phenyl)- *N*'-(3,5-dimethoxybenzylidene)pyrazine-2-carbohydrazide. | 1H-NMR, 400*MHz,* DMSO-d6 $\delta$ 12,02 (s, 1H), 9,33 (s, 1H), 9,13 (s, 1H), 8,60 (s, 1H), 8,12 (br d, J =7,2 Hz, 1H), 7,52 (br t, J =7,5 Hz, 1H), 7,28 - 7,13 (m, 2H), 6,91 (br s, 2H), 6,62 (br s, 1H), 5,02 (br s, 1H), 3,82 (s, 6H), 1,94 (br s, 2H), 1,80 (br s, 2H), 1,63 (br d, J = 5,1 Hz, 4H). [M+H] +: 447.1. |
| 162 | 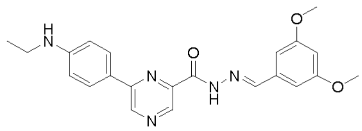 (*E*)-*N*'-(3,5-dimethoxybenzylidene)-6-(4-(ethylthio)phenyl) pyrazine-2-carbohydrazide. | 1H-NMR, 400*MHz,* DMSO-d6 $\delta$ 11.97 (s, 1H), 9.33 (s, 1H), 8.96 (s, 1H), 8.66 (s, 1H), 8.22 (d, J = 8.8 Hz, 2H), 6.93 (d, J = 2.3 Hz, 2H), 6.71 (d, J = 8.8 Hz, 2H), 6.62 (t, J = 2.3 Hz, 1H), 6.25 (t, J = 5.2 Hz, 1H), 3.82 (s, 6H), 3.20 - 3.10 (m, 2H), 1.21 (t, J = 7.2 Hz, 3H) [M+H] +: 406.1. |
| 163 | 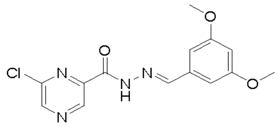 (*E*)-6-chloro-N'-(3,5-dimethoxybenzylidene)pyrazine-2-carbohydrazide. | 1H-NMR, 400*MHz,* DMSO-d6 $\delta$ 12,22 (br s, 1H), 9,21 (s, 1H), 9,06 (s, 1H), 8,58 (s, 1H), 6,84-6,91 (m, 1H), 6,87 (d, J =1,8 Hz, 1H), 6,60 (s, 1H), 3,80 (s, 6H). [M+H]+: 321.0. |
| 164 | 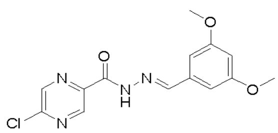 (*E*)-5-chloro-N'-(3,5-dimethoxybenzylidene)pyrazine-2-carbohydrazide. | 1H-NMR, 400*MHz,* DMSO-d6 $\delta$ 12.31 (s, 1H), 9.07 (d, J = 1.1 Hz, 1H), 8.91 (d, J = 1.1 Hz, 1H), 8.54 (s, 1H), 6.84 (d, J = 2.2 Hz, 2H), 6.58 (t, J = 2.2 Hz, 1H), 3.78 (s, 6H). [M+H] +: 321.1. |

(continued)

| Compound No. | Structure | 1H-NMR/ MS m/z [M+H] + |
|---|---|---|
| 165 | 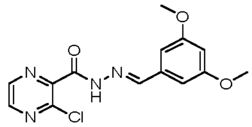 (*E*)-3-chloro-N'-(3,5-dimethoxybenzylidene)pyrazine-2-carbohydrazide. | 1H-NMR, 400*MHz,* DMSO-d6 $\delta$ 12.51 - 12.27 (m, 1H), 8.76 (dd, J = 2.5, 7.2 Hz, 1H), 8.71 - 8.63 (m, 1H), 8.28 - 7.98 (m, 1H), 6.89 (d, J = 2.2 Hz, 1H), 6.59 - 6.45 (m, 2H), 3.78 (s, 3H), 3.64 (s, 3H). [M+H]+: 321.0. |
| 166 | 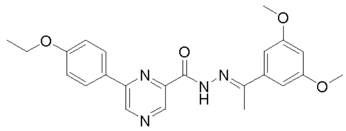 (*E*)-*N*'-(1-(3,5-dimethoxyphenyl)ethylidene)-6-(4-ethoxyphenyl)pyrazine-2-carbohydrazide. | 1H-NMR, 400MHz CDCl3 $\delta$ 10,88 (s, 1 H) 9,37 (s, 1 H) 9,20 (s, 1 H) 7,95 - 8,03 (m, 2 H) 7,04 - 7,13 (m, 4 H) 6,54 (t, J = 2,20 Hz, 1 H) 6,51 - 6,57 (m, 1 H) 4,15 (q, J = 6,97 Hz, 2 H) 3,87 (s, 6 H) 2,45 (s, 3 H) 1,49 (t, J = 6,97 Hz, 3 H). [M+H] +: 421.2. |
| 167 | 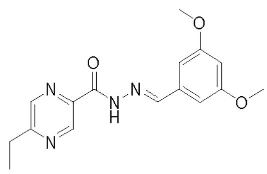 (*E*)-*N*'-(3,5-dimethoxybenzylidene)-5-ethylpyrazine-2-carbohydrazide. | 1H-NMR, 400*MHz,* DMSO-d6 $\delta$ 12,18 (br s, 1H), 9,14 (d, J = 1,1 Hz, 1H), 8,68 (s, 1H), 8,54 (s, 1H), 6,84 (d, J = 2,0 Hz, 2H), 6,57 (t, J = 2,2 Hz, 1H), 3,78 (s, 6H), 2,91 (q, J = 7,6 Hz, 2H), 1,28 (t, J = 7,5 Hz, 3H). [M+H]+: 315.1. |
| 169 | 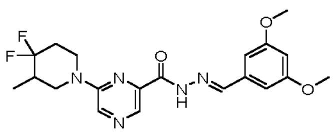 (*E*)-6-(4,4-difluoro-3-methylpiperidin-1-yl)-*N*'-(3,5-dimethoxybenzylidene) pyrazine-2-carbohydrazide. | 1H-NMR, 400*MHz,* DMSO-d6 $\delta$ 11,80 - 11,53 (m, 1H), 8,68 (s, 1H), 8,60 (s, 1H), 8,45 (s, 1H), 8,48 - 8,41 (m, 1H), 6,91 (d, J =2,3 Hz, 2H), 6,60 (t, J = 2,2 Hz, 1H), 4,69 - 4,32 (m, 2H), 3,81 (s, 6H), 3,29 (br d, J = 2,9 Hz, 1H), 3,16 - 2,94 (m, 1H), 2,33 - 1,86 (m, 3H), 1,06 (d, J = 6,8 Hz, 3H). [M+H] +: 420.1. |
| 170 | 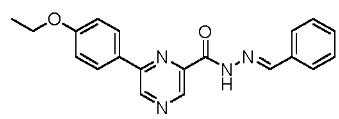 (*E*)-*N*'-benzylidene-6-(4-ethoxyphenyl)pyrazine-2-carbohydrazide. | 1H-NMR, 400*MHz,* DMSO-d6 $\delta$ 12,07 (s, 1H), 9,47 (s, 1H), 9,11 (s, 1H), 8,74 (s, 1H), 8,41 (d, J = 8,9 Hz, 2H), 7,79 (dd, J = 2,0, 7,6 Hz, 2H), 7,54 - 7,46 (m, 3H), 7,13 (d, J = 8,8 Hz, 2H), 4,16 (q, J = 7,0 Hz, 2H), 1,39 (t, J = 7,0 Hz, 3H). [M+H]+: 347.0. |
| 171 | 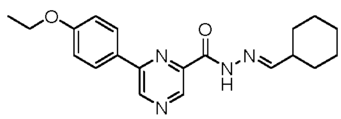 (*E*)-*N*'-(cyclohexylmethylene)-6-(4-ethoxyphenyl)pyrazine-2-carbohydrazide. | 1H-NMR, 400*MHz,* DMSO-d6 $\delta$ 11.62 (s, 1H), 9.43 (s, 1H), 9.05 (s, 1H), 8.37 (br d, J = 8.8 Hz, 2H), 7.91 (br d, J = 5.5 Hz, 1H), 7.10 (br d, J = 8.8 Hz, 2H), 4.14 (q, J = 7.0 Hz, 2H), 2.33 (br d, J = 1.8 Hz, 1H), 1.90 - 1.57 (m, 6H), 1.45 - 1.12 (m, 9H). [M+H]+: 353.1. |

(continued)

| Compound No. | Structure | 1H-NMR/ MS m/z [M+H] + |
|---|---|---|
| 172 | 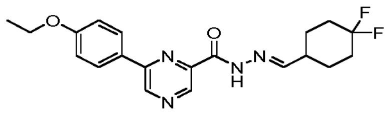 (*E*)-*N*'-((4,4-difluorocyclohexyl) methylene)-6-(4-ethoxyphenyl)pyrazine-2-carbohydrazide. | 1H-NMR, 400*MHz,* DMSO-d6 $\delta$ 11,70 (s, 1H), 9,43 (s, 1H), 9,06 (s, 1H), 8,37 (d, J = 8,8 Hz, 2H), 7,96 (d, J = 5,0 Hz, 1H), 7,10 (d, J = 8,8 Hz, 2H), 4,14 (q, J = 7,0 Hz, 2H), 2,20 - 1,80 (m, 7H), 1,76 - 1,47 (m, 2H), 1,38 (t, J = 6,9 Hz, 3H). [M+H]+: 389,1. |
| 173 | 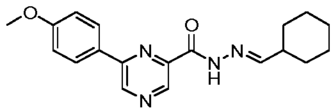 (*E*)-*N*'-(cyclohexylmethylene)-6-(4-methoxyphenyl) pyrazine-2-carbohydrazide. | 1H-NMR, 400*MHz,* DMSO-d6 $\delta$ 11,62 (s, 1H), 9,43 (s, 1H), 9,06 (s, 1H), 8,38 (d, J = 8,8 Hz, 2H), 7,91 (d, J = 5,5 Hz, 1H), 7,12 (d, J = 8,8 Hz, 2H), 3,86 (s, 3H), 2,39 - 2,25 (m, 1H), 1,93 - 1,57 (m, 5H), 1,48 - 1,00 (m, 5H). [M+H]+: 339.1. |
| 174 | 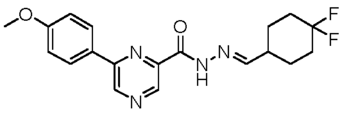 (*E*)-*N*'-((4,4-difluorocyclohexyl) methylene)-6-(4-ethoxyphenyl)pyrazine-2-carbohydrazide. | 1H-NMR, 400*MHz,* DMSO-d6 δ 12,11 (s, 1H), 9,57 (s, 1H), 9,22 (s, 1H), 8,64 (s, 1H), 8,56 (d, J = 8,2 Hz, 2H), 7,78 (d, J = 8,2 Hz, 2H), 7,15 (s, 1H), 6,91 (d, J = 2,2 Hz, 2H), 6,60 (t, J = 2,2 Hz, 1H), 3,80 (s, 6H). [M+H]+: 413.1. |
| 175 | 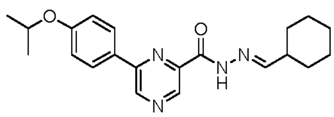 (*E*)-*N*'-(cyclohexylmethylene)-6-(4-isopropoxyphenyl) pyrazine-2-carbohydrazide. | 1H-NMR, 400*MHz,* DMSO-d6 δ 11.62 (s, 1H), 9.42 (s, 1H), 9.05 (s, 1H), 8.35 (br d, J = 8.7 Hz, 2H), 7.91 (br d, J = 5.4 Hz, 1H), 7.09 (br d, J = 8.7 Hz, 2H), 4.77 (td, J = 5.9, 11.9 Hz, 1H), 2.33 (br s, 1H), 1.95 - 1.54 (m, 5H), 1.44 - 1.17 (m, 11H). [M+H]+: 367,1. |
| 176 | 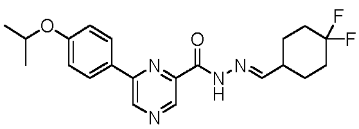 (*E*)-*N*'-((4,4-difluorocyclohexyl) methylene)-6-(4-ethoxyphenyl)pyrazine-2-carbohydrazide. | 1H-RMN, 400 MHz, DMSO-d6 δ 11.70 (s, 1H), 9.43 (s, 1H), 9.05 (s, 1H), 8.35 (d, J = 8.8 Hz, 2H), 7.96 (d, J = 5.0 Hz, 1H), 7.09 (d, J = 8.8 Hz, 2H), 4.89 - 4.63 (m, 1H), 2.47 - 2.39 (m, 1H), 2.19 - 1.81 (m, 6H), 1.69 - 1.48 (m, 2H), 1.32 (d, J = 6.0 Hz, 6H). [M+H] +: 403.1. |
| 177 | 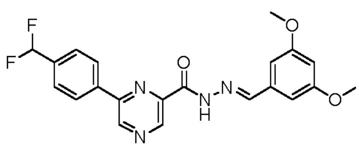 *(E)*-6-(4-(difluoromethyl)pnenyli)- *N*'-(3,5-dimethoxybenzylidene)pyrazine-2-carbohydrazide. | 1H-NMR, 400*MHz,* DMSO-d6 $\delta$ 12,11 (s, 1H), 9,57 (s, 1H), 9,22 (s, 1H), 8,64 (s, 1H), 8,56 (d, J = 8,2 Hz, 2H), 7,78 (d, J = 8,2 Hz, 2H), 7,15 (s, 1H), 6,91 (d, J = 2,2 Hz, 2H), 6,60 (t, J = 2,2 Hz, 1H), 3,80 (s, 6H). [M+H]+: 413.1. |

(continued)

| Compound No. | Structure | 1H-NMR/ MS m/z [M+H] + |
|---|---|---|
| 178 | 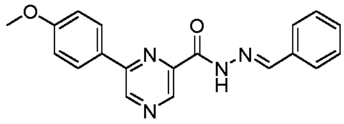 (*E*)-*N*'-benzylidene-6-(4-methoxyphenyl)pyrazine-2-carbohydrazide. | 1H-NMR, 400*MHz,* DMSO-d6 $\delta$ 12.05 (s, 1H), 9.44 (s, 1H), 9.09 (s, 1H), 8.71 (s, 1H), 8.39 (d, J =8.8 Hz, 2H), 7.79 - 7.72 (m, 2H), 7.52 - 7.42 (m, 3H), 7.12 (d, J =8.8 Hz, 2H), 3.84 (s, 3H). [M+H]+: 333.0. |
| 179 | 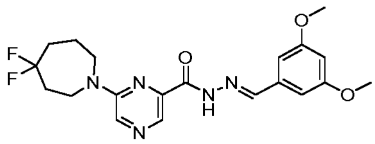 (*E*)-6-(4,4-difluoroazepan-1-yl)-*N*-(3,5-dimethoxybenzylidene)pyrazine-2-carbohydrazide. | 1H-NMR, 400*MHz,* DMSO-d6 $\delta$ 11.52 (s, 1H), 8.58 (s, 1H), 8.41 (d, J = 7.8 Hz, 2H), 6.91 (d, J=2.3 Hz, 2H), 6.60 (t, J = 2.3 Hz, 1H), 3.95 - 3.86 (m, 2H), 3.81 (s, 8H), 2.38 - 2.05 (m, 4H), 2.00 - 1.85 (m, 2H). [M+H] +: 420.1. |
| 180 | 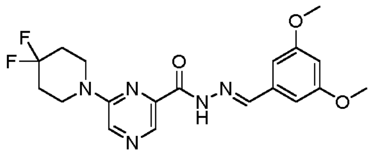 *(E)*-6-(4,4-difluoropiperidin-1-yl)- *N*'-(3,5-dimethoxybenzylidene)pyrazine-2-carbohydrazide. | 1H-NMR, 400*MHz,* DMSO-d6 $\delta$ 11.66 (s, 1H), 8.66 (s, 1H), 8.59 (s, 1H), 8.46 (s, 1H), 6.91 (d, J = 2.3 Hz, 2H), 6.60 (t, J = 2.3 Hz, 1H), 3.99 - 3.87 (m, 4H), 3.81 (s, 6H), 2.20 - 1.97 (m, 4H). [M+H] +: 406.1. |
| 181 | 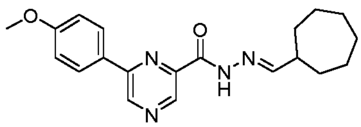 (*E*)-*N*'-(cycloheptylmethylene)-6-(4-methoxyphenyl) pyrazine-2-carbohydrazide. | 1H-NMR, 400*MHz,* DMSO-d6 $\delta$ 11.61 (s, 1H), 9.44 (s, 1H), 9.06 (s, 1H), 8.47 - 8.33 (m, 2H), 7.95 (d, J = 5.7 Hz, 1H), 7.12 (d, J = 8.9 Hz, 2H), 3.86 (s, 3H), 1.98 - 1.78 (m, 2H), 1.76 - 1.46 (m, 10H). [M+H]+: 353.2. |
| 182 | 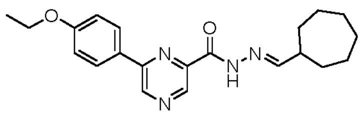 (*E*)-*N*'-(cycloheptylmethylene)-6-(4-ethoxyphenyl) pyrazine-2-carbohydrazide. | 1H-NMR, 400*MHz,* DMSO-d6 $\delta$ 11.60 (s, 1H), 9.49 - 9.33 (m, 1H), 9.05 (s, 1H), 8.46 - 8.29 (m, 2H), 7.95 (d, J = 5.7 Hz, 1H), 7.20 - 7.00 (m, 2H), 4.14 (q, J = 7.0 Hz, 2H), 1.95 - 1.80 (m, 2H), 1.77 - 1.45 (m, 10H), 1.38 (t, J = 7.0 Hz, 3H). [M+H]+: 367.2. |
| 183 | 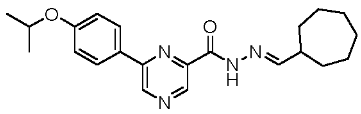 (*E*)-*N*'-(cycloheptylmethylene)-6-(4-isopropoxyphenyl) pyrazine-2-carbohydrazide. EF-00721 | 1H-NMR, 400*MHz,* DMSO-d6 $\delta$ 11.58 (s, 1H), 9.40 (s, 1H), 9.03 (s, 1H), 8.44 - 8.26 (m, 2H), 7.93 (d, J = 5.7 Hz, 1H), 7.15 - 6.98 (m, 2H), 4.75 (spt, J = 6.0 Hz, 1H), 1.92 - 1.78 (m, 2H), 1.77 - 1.39 (m, 10H), 1.30 (d, J = 6.1 Hz, 6H). [M+H]+: 381.3. |

(continued)

| Compound No. | Structure | 1H-NMR/ MS m/z [M+H] + |
|---|---|---|
| 184 | 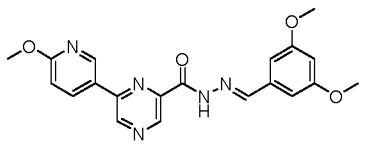<br>(*E*)-*N*'-(3,5-dimethoxybenzylidene)-6-(6-methoxypyridin-3-yl)pyrazine-2-carbohydrazide. | 1H-NMR, 400*MHz,* DMSO-d6<br>$\delta$ 12.08 (s, 1H), 9.56 - 9.52 (m, 1H), 9.30 (d, J = 2.5 Hz, 1H), 9.19 - 9.15 (m, 1H), 8.80 - 8.74 (m, 1H), 8.68 - 8.64 (m, 1H), 7.07 - 7.02 (m, 1H), 6.93 (d, J = 2.3 Hz, 2H), 6.64 - 6.60 (m, 1H), 4.01 - 3.95 (m, 3H), 3.85 - 3.79 (m, 6H).<br>[M+H]+: 394.1. |
| 185 | 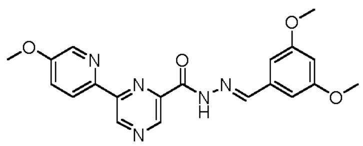<br>(E)-*N*'-(3,5-dimethoxybenzylidene)-6-(6-methoxypyridin-3-yl)pyrazine-2-carbohydrazide. | 1H-RMN, 400 MHz, DMSO-d6 $\delta$ 12.16 (s,<br>1H), 9.68 (s, 1H), 9.22 (s, 1H), 8.84 (d, *J* = 8.8 Hz, 1H), 8.68 (s, 1H), 8.51 (d, *J* = 2.8 Hz, 1H), 7.66 (dd, *J* = 8.8, 3.2 Hz, 1H), 6.94 (d, *J* = 2.4 Hz, 2H), 6.63 (t, *J* = 2.4 Hz, 1H), 3.97 (s, 3H), 3.83 (s, 6H).<br>[M+H]+: 394.1. |
| 186 | 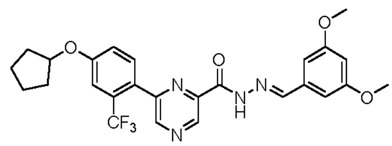<br>(*E*)-6-(4-(cyclopentyloxy)-2-(trifluoromethyl) phenyl)-*N*'-(3,5-dimethoxybenzylidene)pyrazine-2-carbohydrazide. | |
| 187 | 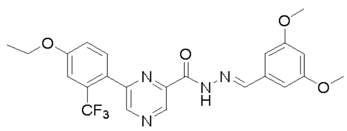<br>(*E*)-*N*'-(3,5-dimethoxybenzylidene)-6-(4-methoxy-2-(trifluoromethoxy)phenyl) pyrazine-2-carbohydrazide. | 1H-NMR, 400*MHz,* DMSO-d6<br>$\delta$ 12.07 (s, 1H), 9.45 (s, 1H), 9.21 (s, 1H), 8.59 (s, 1H), 8.40 - 8.27 (m, 1H), 7.57 - 7.48 (m, 2H), 6.91 (d, J = 2.3 Hz, 2H), 6.62 (t, J = 2.2 Hz, 1H), 4.31 (q, J = 6.9 Hz, 2H), 3.81 (s, 6H), 1.39 (t, J = 6.9 Hz, 3H).<br>[M+H]+: 475.0. |
| 188 | 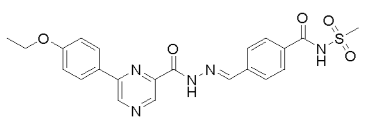<br>(*E*)-4-((2-(6-(4-ethoxyphenyl)pyrazine-2-carbonyl) hydrazinalidene)methyl)-*N*-(methylsulfonyl)benzamide. | 1H-RMN, 400 MHz, DMSO-d6<br>$\delta$ 12.28-12.20 (m, 2H), 9.48 (s, 1H), 9.12 (s, 1H), 8.79 (s, 1H), 8.41 (dd, *J* = 7.2, 2.0 Hz, 2H), 8.05 (d, *J* = 8.4 Hz, 2H), 7.92 (d, *J* = 8.4 Hz, 2H), 7.13 (dd, *J* = 6.8, 2.0 Hz, 2H), 4.16 (q, *J* = 7.2 Hz, 2H), 3.39 (s, 3H), 1.39 (t, *J* = 7.2 Hz, 3H).<br>[M+H]+: 468.1. |
| 189 | 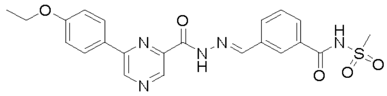<br>*(E)*-4-((2-(6-(4-ethoxyphenyl)pyrazine-2-carbonyl) hydrazinalidene)methyl)- *N*-(methylsulfonyl)benzamide. | 1H-NMR, 400*MHz,* DMSO-d6<br>$\delta$ 12.05 (s, 1H), 9.47 (s, 1H), 9.13 (s, 1H), 8.76 (s, 1H), 8.42-8.37 (m, 3H), 8.03-8.00 (m, 1H), 7.80-7.78 (m, 1H), 7.46 (t, *J*= 7.6 Hz, 1H), 7.15-7.05 (m, 3H), 4.16 (q, *J*= 7.2 Hz, 2H), 2.89 (s, 3H), 1.39 (t, *J* = 7.2 Hz, 3H).<br>[M+H] +: 475.0. |

(continued)

| Compound No. | Structure | 1H-NMR/ MS m/z [M+H] + |
|---|---|---|
| 190 | 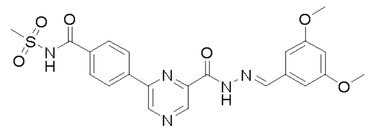 (*E*)-4-(6-(2-(3,5-dimethoxybenzylidene)hydrazine- 1-carbonyl)pyrazin-2-yl)-*N*-(methylsulfonyl)benzamide. | 1H-NMR, 400*MHz,* DMSO-d6 $\delta$ 12.35 (br s, 1H), 12.17 (s, 1H), 9.63 (s, 1H), 9.26 (s, 1H), 8.68 (s, 1H), 8.61 (d, *J* = 8.8 Hz, 2H), 8.15 (d, *J* = 8.4 Hz, 2H), 6.94 (d, *J* = 2.4 Hz, 2H), 6.63 (t, *J* = 2.4 Hz, 1H), 3.82 (s, 6H), 3.39 (s, 3H). [M+H]+: 484.0. |
| 191 | 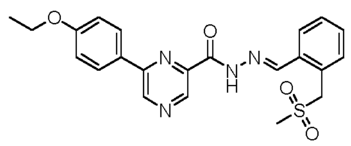 (*E*)-6-(4-ethoxyphenyl)-*N*-(2-((methylsulfonyl)methyl) benzyliden e)pyrazine-2-carbohydrazide. | 1H-NMR, 400*MHz,* DMSO-d6 $\delta$ 12.13 (s, 1H), 9.47 (s, 1H), 9.13 (s, 1H), 8.96 (s, 1H), 8.40-8.36 (m, 2H), 7.94-7.92 (m, 1H), 7.54-7.51 (m, 3H), 7.16-7.14 (m, 2H), 4.91 (s, 2H), 4.16 (q, *J* = 6.8 Hz, 2H), 3.02 (s, 3H), 1.38 (t, *J* = 6.8 Hz, 3H). [M+H]+: 439.2. |
| 192 | 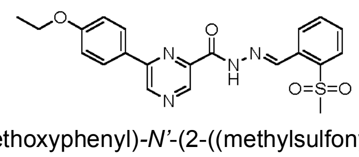 (*E*)-6-(4-ethoxyphenyl)-*N'*-(2-((methylsulfonyl)methyl) benzyliden e)pyrazine-2-carbohydrazide. | 1H-NMR, 400*MHz,* DMSO-d6 $\delta$ 11.02 (s, 1H), 9.38 (s, 2H), 9.22 (s, 1H), 8.40 (d, J = 7.82, 1.22 Hz, 1H), 8.12 (dd, J = 7.95, 1.22 Hz, 1H), 8.00 - 8.08 (m, 2H), 7.75 (td, J = 7.64, 0.86 Hz, 1 H), 7.62 - 7.69 (m, 1 H), 7.06 - 7.15 (m, 2 H), 4.17 (q, J = 6.97 Hz, 2 H), 1.50 (t, J = 7.03 Hz, 3 H) 3.17 (s, 3 H). [M+H]+: 425.0. |
| 193 | 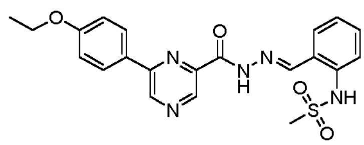 (*E*)-*N*-(2-((2-(6-(4-ethoxyphenyl)pyrazine-2-carbonyl) hydrazinalidene)methyl)phenyl) methanesulfonamide. | 1H-NMR, 400*MHz,* DMSO-d6 $\delta$ 12.34 (s, 1 H) 10.70 (s, 1 H) 9.48 (s, 1 H) 9.13 (s, 1 H) 8.88 - 8.97 (m, 1 H) 8.40 (d, J = 8.88 Hz, 2 H) 7.74 (dd, J = 7.75, 1.13 Hz, 1 H) 7.54 - 7.60 (m, 1 H) 7.46 - 7.54 (m, 1 H) 7.27 - 7.36 (m, 1 H) 7.14 (d, J = 8.88 Hz, 2 H) 4.16 (q, J = 6.88 Hz, 2 H) 3.15 - 3.19 (m, 3 H) 1.39 (t, J = 6.94 Hz, 3 H). [M+H]+: 440,0. |
| 194 | 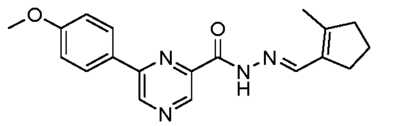 *(E)*-6-(4-methoxyphenyl)-*N'*-((2-methylcyclopent-1-en-1-yl)methylene)pyrazine-2-carbohydrazide. | |
| 195 | 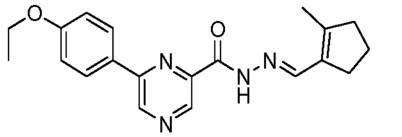 *(E)*-6-(4-ethoxyphenyl)-*N'*-((2-methylcyclopent-1-en-1-yl) methylene)pyrazine-2- carbohydrazide. | |

(continued)

| Compound No. | Structure | 1H-NMR/ MS m/z [M+H] + |
|---|---|---|
| 196 | 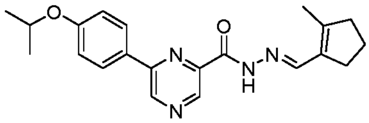 (*E*)-6-(4-isopropoxyphenyl)-/V-((2-methylcyclohept-1-en-1-yl)methylene)pyrazine-2- carbohydrazide. | |
| 197 | 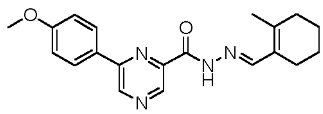 *(E)*-6-(4-methoxyphenyl)-*N*-((2-methylcyclopent-1-en-1-yl) methylene)pyrazine-2-carbohydrazide. | 1H-NMR, 400*MHz,* DMSO-d6 $\delta$ 11.70 (s, 1H), 9.44 (s, 1H), 9.07 (s, 1H), 8.83 (s, 1H), 8.38 (d, J = 8.8 Hz, 2H), 7.14 (d, J = 8.8 Hz, 2H), 3.96 - 3.78 (m, 3H), 3.32 (s, 8H), 2.31 (br s, 2H), 2.16 (br s, 2H), 1.92 (s, 3H), 1.61 (br s, 4H). [M+H]+: 351.0. |
| 198 | 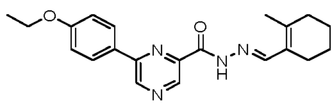 *(E)*-6-(4-ethoxyphenyl)-*N*'-((2-methylcyclopent-1-en-1-yl) methylene)pyrazine-2-carbohydrazide. | 1H-NMR, 400*MHz,* DMSO-d6 $\delta$ 11.71 (s, 1H), 9.43 (s, 1H), 9.07 (s, 1H), 8.83 (s, 1H), 8.37 (d, J = 8.8 Hz, 2H), 7.12 (d, J = 8.8 Hz, 2H), 4.14 (q, J = 7.0 Hz, 2H), 2.31 (br s, 2H), 2.16 (br s, 2H), 1.92 (s, 3H), 1.61 (br s, 4H), 1.38 (t, J = 6.9 Hz, 3H). [M+H]+: 365.0. |
| 199 | 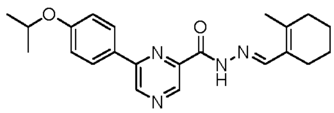 (*E*)-6-(4-isopropoxyphenyl)-*N*-((2-methylcyclohept-1-en-1-yl)methylene)pyrazine-2-carbohydrazide. | 1H-NMR, 400*MHz,* DMSO-d6 $\delta$ 11.70 (s, 1H), 9.42 (s, 1H), 9.07 (s, 1H), 8.83 (s, 1H), 8.35 (d, J = 8.8 Hz, 2H), 7.11 (d, J = 8.8 Hz, 2H), 4.78 (td, J = 5.9, 11.9 Hz, 1H), 2.31 (br s, 2H), 2.16 (br s, 2H), 2.00 - 1.85 (m, 3H), 1.61 (br s, 4H), 1.32 (d, J = 6.0 Hz, 6H). [M+H]+: 379.1. |
| 200 | 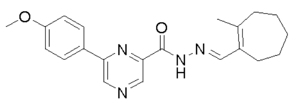 (*E*)-6-(4-methoxyphenyl)-*N*-((2-methylcyclopent-1-en-1-yl) methylene)pyrazine-2-carbohydrazide. | 1H-NMR, 400*MHz,* DMSO-d6 $\delta$ 11.85 - 11.60 (m, 1H), 9.42 (s, 1H), 9.04 (s, 1H), 8.74 (s, 1H), 8.36 (d, J = 8.8 Hz, 2H), 7.12 (d, J = 8.8 Hz, 2H), 3.84 (s, 3H), 2.62 (br d, J = 9.5 Hz, 2H), 2.37 - 2.24 (m, 2H), 2.06 - 1.91 (m, 3H), 1.73 (br d, J = 5.7 Hz, 2H), 1.53 - 1.33 (m, 4H). [M+H]+: 365.1. |
| 201 | 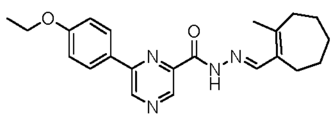 (*E*)-6-(4-ethoxyphenyl)-*N*'-((2-methylcyclopent-1-en-1-yl) methylene)pyrazine-2-carbohydrazide. | 1H-NMR, 400*MHz,* DMSO-d6 $\delta$ 11.70 (s, 1H), 9.41 (s, 1H), 9.04 (s, 1H), 8.75 (s, 1H), 8.34 (d, J = 9.0 Hz, 2H), 7.10 (d, J = 9.0 Hz, 2H), 4.12 (q, J = 6.8 Hz, 2H), 2.62 (br d, J = 9.3 Hz, 2H), 2.37 - 2.25 (m, 2H), 1.98 (s, 3H), 1.73 (br d, J = 5.1 Hz, 2H), 1.53 - 1.27 (m, 7H). [M+H]+: 379.1. |

(continued)

| Compound No. | Structure | 1H-NMR/ MS m/z [M+H] + |
|---|---|---|
| 202 | 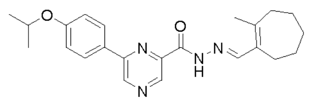 (*E*)-6-(4-isopropoxyphenyl)-*N*-((2-methylcyclohept-1-en-1-yl)methylene)pyrazine-2-carbohydrazide. | 1H-NMR, 400*MHz,* DMSO-d6 $\delta$ 11.72 (s, 1H), 9.49 - 9.36 (m, 1H), 9.16 - 9.01 (m, 1H), 8.77 (s, 1H), 8.42 - 8.30 (m, 2H), 7.22 - 7.01 (m, 2H), 4.77 (spt, J = 6.1 Hz, 1H), 2.74 - 2.59 (m, 2H), 2.39 - 2.28 (m, 2H), 2.01 (s, 3H), 1.82 - 1.71 (m, 2H), 1.54 - 1.37 (m, 4H), 1.32 (d, J = 6.1 Hz, 6H). [M+H] +: 393.1. |
| 203 | 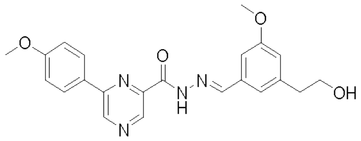 *(E)-N'*-(3-(2-hydroxyethyl)-5-methoxybenzylidene)-6-(4-methoxyphenyl)pyrazine-2-carbohydrazide. | |
| 204 | 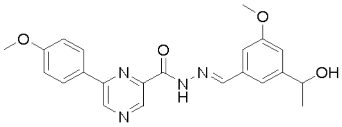 (*E*)-*N*'-(3-(1-hydroxyethyl)-5-methoxybenzylidene)-6-(4-methoxyphenyl)pyrazine-2-carbohydrazide. | |
| 205 | 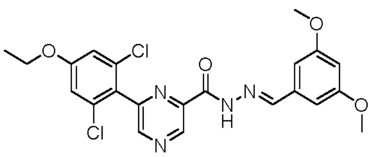 *(E)*-6-(2,6-dichloro-4-ethoxyphenyl)-*N'*-(3,5-dimethoxybenzylidene)pyrazine-2-carbohydrazide. | |
| 206 | 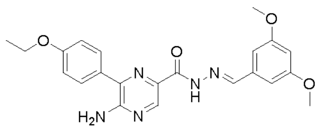 (*E*)-5-amino-N'-(3,5-dimethoxybenzylidene)-6-(4-ethoxyphenyl)pyrazine-2-carbohydrazide. | 1H-NMR, 400*MHz,* DMSO-d6 $\delta$ 11.44 (s, 1H), 8.55 (s, 1H), 8.49 (s, 1H), 7.80 (d, J = 8.8 Hz, 2H), 7.07 (d, J = 8.8 Hz, 2H), 6.94 (br s, 2H), 6.86 (d, J = 2.2 Hz, 2H), 6.57 (t, J = 2.3 Hz, 1H), 4.13 (q, J = 7.0 Hz, 2H), 3.80 (s, 6H), 1.38 (t, J = 7.0 Hz, 3H). [M+H]+: 422.1. |
| 207 | 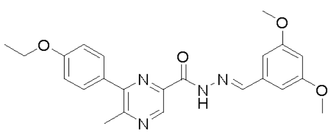 (*E*)-*N*'-(3,5-dimethoxybenzylidene)-6-methylpyrazine-2-carbohydrazide. | 1H-NMR, 400*MHz,* DMSO-d6 $\delta$ 11.91 (s, 1H), 9.05 (s, 1H), 8.56 (s, 1H), 7.84 - 7.75 (m, 2H), 7.10 (d, J = 8.7 Hz, 2H), 6.89 (d, J = 2.3 Hz, 2H), 6.60 (t, J = 2.3 Hz, 1H), 4.14 (q, J = 7.0 Hz, 2H), 3.80 (s, 6H), 2.73 - 2.67 (m, 3H), 1.38 (t, J = 7.0 Hz, 3H). [M+H] +: 421.1. |

(continued)

| Compound No. | Structure | 1H-NMR/ MS m/z [M+H] + |
|---|---|---|
| 208 | 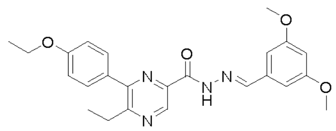 (*E*)-*N*'-(3,5-dimethoxybenzylidene)-6-methylpyrazine-2-carbohydrazide. | 1H-NMR, 400*MHz,* DMSO-d6 $\delta$ 11.92 (s, 1H), 9.13 (s, 1H), 8.56 (s, 1H), 7.77 - 7.68 (m, 2H), 7.14 - 7.07 (m, 2H), 6.89 (d, J = 2.4 Hz, 2H), 6.61 (t, J = 2.3 Hz, 1H), 4.15 (q, J = 7.0 Hz, 2H), 3.81 (s, 6H), 2.99 (q, J = 7.5 Hz, 2H), 1.39 (t, J = 6.9 Hz, 3H), 1.24 (t, J = 7.4 Hz, 3H). [M+H]+: 435,2. |
| 209 | 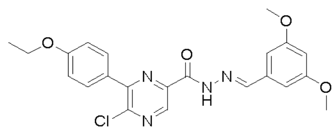 (*E*)-5-chloro-N'-(3,5-dimethoxybenzylidene)-6-(4-ethoxyphenyl)pyrazine-2-carbohydrazide. | 1H-NMR, 400*MHz,* DMSO-d6 $\delta$ 12.02 (s, 1 H), 8.98 (s, 1 H), 8.57 (s, 1 H), 7.92 - 7.99 (m, 2 H), 7.12 (d, J=8.80 Hz, 2 H), 6.90 (d, J=2.20 Hz, 2 H), 6.61 (t, J=2.26 Hz, 1 H), 4.15 (q, J=6.97 Hz, 2 H), 3.81 (s, 6 H), 1.39 (t, J=6.97 Hz, 3 H). [M+H]+: 441.0. |
| 210 | 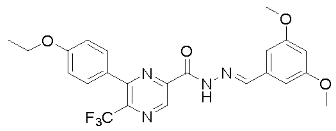 (*E*)-*N*'-(3,5-dimethoxybenzylidene)-6-(4-ethoxyphenyl)-5-(trifluoromethyl)pyrazine-2-carbohydrazide. | 1H-NMR, 400*MHz,* DMSO-d6 $\delta$ 12.16 (s, 1H), 9.32 (s, 1H), 8.59 (s, 1H), 7.70 (d, J = 8.6 Hz, 2H), 7.19 - 7.06 (m, 2H), 6.91 (d, J = 2.3 Hz, 2H), 6.63 (t, J = 2.3 Hz, 1H), 4.21 - 4.10 (m, 2H), 3.82 (s, 6H), 1.40 (t, J = 6.9 Hz, 3H). [M+H] +: 475.0. |
| 211 | 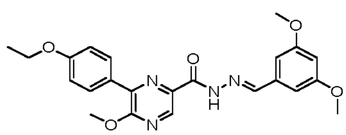 (*E*)-*N*'-(3,5-dimethoxybenzylidene)-6-methylpyrazine-2-carbohydrazide. | 1H-NMR, 400*MHz*, DMSO-d6 $\delta$ 11.54 (s, 1 H), 8.55 - 8.63 (m, 3 H), 8.51 (s, 1 H), 7.03 (d, J=8.94 Hz, 2 H), 6.90 (d, J=2.15 Hz, 2 H), 6.60 (t, J=2.21 Hz, 1 H), 4.10 - 4.17 (m, 2 H), 3.81 (s, 6 H), 3.63 (s, 3 H), 3.18 (d, J=5.25 Hz, 1 H), 1.33 - 1.42 (m, 3 H). [M+H]+: 437.1. |
| 212 | 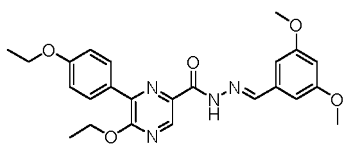 (*E*)-*N*'-(3,5-dimethoxybenzylidene)-5-ethoxy-6-(4-ethoxyphenyl)pyrazine-2-carbohydrazide. | 1H-NMR, 400*MHz,* DMSO-d6 $\delta$ 11.52 (s, 1 H), 8.57 (d, J=9.29 Hz, 3 H), 8.49 (s, 1 H), 7.02 (d, J=9.05 Hz, 2 H), 6.89 (d, J=2.20 Hz, 2 H), 6.59 (t, J=2.20 Hz, 1 H), 4.12 (q, J=6.93 Hz, 4 H), 3.81 (s, 6 H), 1.35 (dt, J=18.49, 7.08 Hz, 6 H). [M+H]+: 451.1. |

(continued)

| Compound No. | Structure | 1H-NMR/ MS m/z [M+H] + |
|---|---|---|
| 213 | 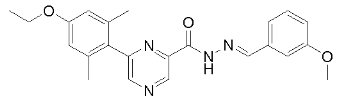 (*E*)-6-(4-ethoxy-2,6-dimethylphenyl)-*N'*-(3-methoxybenzylidene)pyrazine-2-carbohydrazide. | 1H-NMR, 400*MHz,* DMSO-d6 $\delta$ 12.05 (s, 1H), 9.23 (s, 1H), 8.86 (s, 1H), 8.64 (s, 1H), 7.39 (t, *J* = 8.0 Hz, 1H), 7.28-7.26 (m, 2H), 7.05-7.03 (m, 1H), 6.79 (s, 2H), 4.08 (q, *J* = 6.8 Hz, 2H), 3.82 (s, 3H), 2.05 (s, 6H), 1.35 (t, *J* = 7.2 Hz, 3H). [M+H]+: 405.2. |
| 214 | 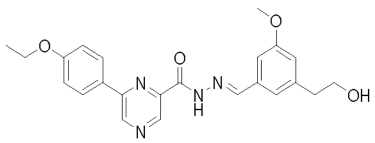 (*E*)-*N*'-(3-(2-hydroxyethyl)-5-methoxybenzylidene)-6-(4-methoxyphenyl)pyrazine-2-carbohydrazide. | 1H-NMR, 400*MHz,* DMSO-d6 $\delta$ 12.04 (s, 1H), 9.47 (s, 1H), 9.11 (s, 1H), 8.67 (s, 1H), 8.41 (dd, J = 6.8, 2.0 Hz, 2H), 7.22 (s, 1H), 7.16-7.11 (m, 3H), 6.92 (dd, J = 2.0, 1.6 Hz, 1H), 4.68 (t, *J* = 5.2 Hz, 1H), 4.16 (q, *J* = 7.2 Hz, 2H), 3.82 (s, 3H), 3.68-3.63 (m, 2H), 2.79-2.75 (m, 2H), 1.39 (t, *J* = 7.2 Hz, 3H). [M+H] +: 421.1. |
| 215 | 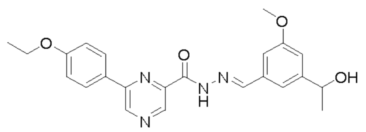 *(E)-N'*-(3-(2-hydroxyethyl)-5-methoxybenzylidene)-6-(4-methoxyphenyl)pyrazine-2-carbohydrazide. | 1H-NMR, 400*MHz,* DMSO-d6 $\delta$ 12.04 (s, 1H), 9.47 (s, 1H), 9.11 (s, 1H), 8.69 (s, 1H), 8.41 (d, *J* = 8.8 Hz, 2H), 7.38 (s, 1H), 7.16-7.12 (m, 3H), 7.02 (s, 1H), 5.29 (d, *J* = 4.4 Hz, 1H), 4.78-4.75 (m, 1H), 4.16 (q, *J* = 6.8 Hz, 2H), 3.83 (s, 3H), 1.41-1.35 (m, 6H). [M+H] +: 421.1. |

**Example 2.** (*E*)-*N*'-(3,5-dimethoxybenzylidene)-6-(4-methoxyphenyl)-N-methylpyrazine-2-carbohydrazide (Compound **168**):

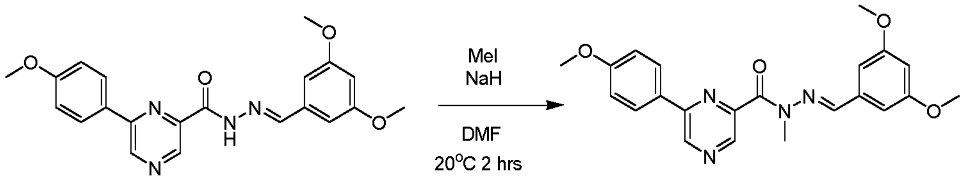


In a flask containing 3.57 mmol of the (E)-N'-(3,5-dimethoxybenzylidene)-6-(4-methoxyphenyl)pyrazine-2-carbohydrazide, dissolved in 50 mL of DMF, 4.28 mmol of NaH (60% purity) was added at 0 °C. The mixture was heated at 20 °C for 2 hours, to subsequently add 9.86 mmol iodomethane. Subsequently, the reaction mixture was allowed to stir at 20 °C for 2 hours. After total consumption of the starting reagent (monitored by CCF), the reaction mixture was diluted with a solution of NH4Cl. The product was filtered and used without subsequent purification. 450 mg (30% yield) of the compound of interest were obtained. 1H-RMN (400 MHz, DMSO-d6) $\delta$ = 9,29 (s, 1H), 8,69 (s, 1H), 8,12 (d, J = 8,8 Hz, 2H), 8,00 (s, 1H), 7,09 (d, J = 8,9 Hz, 2H), 6,53 (d, J = 2,2 Hz, 2H), 6,44 (t, J = 2,2 Hz, 1H), 3,83 (s, 3H), 3,55 (s, 6H), 3,54 (s, 3H). MS: 407.0.

**Example 3. In vitro tests:**

Biological assays were performed on Chinese hamster ovary (CHO) cells expressing the human sodium channels Nav 1.8 and Nav 1.7 stably.

The experimental *voltage-clamp* protocol using the whole cell configuration was established on the automated ScreenPatch® 384P platform (SP384PE, Nanion Technologies, Livingston, NJ) and recording of the results was performed with a Nanion 384-well Patch Clamp chip (NPC) (Nanion Technologies, Livingston, NJ).

Compounds of Formula (I), were diluted in eight concentrations in the extracellular solution composed of physiological saline, buffered with HEPES (mM): NaCl, 137; KCl, 4; CaCl2, 3.8; MgCl2, 1; HEPES, 10; Glucose, 10; pH 7.4. The extracellular solution is composed of (mM) CsCl, 50; CsF, 90;MgCl2, 5; EGTA, 5; HEPES, 10; pH 7.2. The duration of exposure of each compound with the cells expressing Nav 1.7 or Nav 1.8 was at least five minutes and the assays were performed at room temperature.

The measurements of the sodium currents of Nav 1.8 and Nav 1.7 were obtained using the voltage protocols described below:

**Protocol 1:** A holding voltage of -90 mV was established followed by a voltage phase of 200 ms to -120 mV, followed by a voltage step of 10 mV for 1 second for Nav 1.8 or 0 mV for Nav 1.7, followed by a step of -100 mV for 20 ms, followed by a step of 20 ms for 10 mV for Nav 1.8 or 0 mV for Nav 1.7 (TP1A) before returning to the holding voltage of -90 mV.

**Protocol 2:** A holding voltage of -100 mV was established followed by an inactivation voltage step at -40 mV for 8 seconds, followed by a -100 mV step for 20 ms, followed by a 20 ms step for 10 mV for Nav 1.8 or 0 mV for Nav 1.7 (TP1A) before returning to the holding voltage of -100 mV.

Protocols were repeated at a frequency of 0.1 Hz (Protocol 1) or 0.05 Hz (Protocol 2) and current amplitude was quantified over the TP1A phase log. The variation in the peak amplitude of the current was evaluated according to the Formula described below, after exposure of the cells expressing the channels, at each concentration of the different compounds:

$$\% \text{ Block} = (1 - (I_{TP1A,molecule} / I_{TP1A,basal}) \times 100\%,$$

where $I_{TP1A,basal}$, and $I_{TP1A,molecule}$ represent the entry peaks of sodium currents into TP1A prior to exposure to the compound and in the presence of the compound, respectively.

The decrease in peak current amplitude, after exposure of the cells to the compounds, was used to calculate the percent relative blocking of the channels relative to the positive control according to the Formula below:

$$\% \text{ Block'} = 100\% - ((\% \text{ Block} - \% \text{ CP}) * (100\% / (\%V - \% \text{ CP})),$$

where %V and % CP represent the averages of the values of current inhibition with a vehicle (DMSO) and positive controls, respectively. The sodium currents of the positive control were considered as 100%.

$$\% \text{ Block'} = (\% 100 / [1 + ([\text{Test}] / IC_{50})^N],$$

where [Test] represents the concentration of the evaluated molecule, $IC_{50}$ is the concentration of the compound that generates half of the maximum inhibition, N is the Hill's coefficient and % Block' is the percentage of the sodium channel current (Nav 1.8 and Nav 1.7) inhibited at each concentration of the evaluated molecule. Data were obtained by *non linear least squares* regression with XL fit for Excel (Microsoft, Redmond, WA).

Compounds were tested in at least one assay to obtain the $IC_{50}$ value. For compounds that were tested in two or more assays, the results are described as the average of the $IC_{50}$ values.

**Table 16.** $IC_{50}$ values for compounds 1-169 in Nav1.8 and Nav1.7 channels, Protocol 1.

| Compound No. | Nav1.8 $IC_{50}$ (nM) | Nav1.7 $IC_{50}$ (nM) |
|---|---|---|
| 4 | 6016 | 6664 |
| 5 | 5628 | >10000 |
| 7 | 2155 | 2054 |
| 8 | >10000 | >10000 |
| 9 | 9772 | >10000 |

(continued)

| Compound No. | Nav1.8 $IC_{50}$ (nM) | Nav1.7 $IC_{50}$ (nM) |
|---|---|---|
| 11 | 3230 | 4518 |
| 23 | 1901 | 697 |
| 24 | 8093 | >10000 |
| 25 | >10000 | >10000 |
| 28 | 2372 | 1405 |
| 166 | 9179 | 6356 |

**Table 17.** $IC_{50}$ values of compounds 1-169 in Nav 1.8 and Nav 1.7 channels, Protocol 2.

| Compound No. | Nav1.8 $IC_{50}$ (nM) | Nav1.7 $IC_{50}$ (nM) |
|---|---|---|
| 1 | >1000 | >1000 |
| 2 | 32.51 | 239.19 |
| 3 | 152 | 855 |
| 6 | >300 | >300 |
| 10 | >300 | >300 |
| 12 | 25.57 | 97.95 |
| 13 | 72.96 | 189.48 |
| 14 | 156.16 | 308.47 |
| 15 | 25.24 | 242.88 |
| 16 | 93.3 | 224.11 |
| 17 | 232.13 | 7859.84 |
| 18 | 62.56 | 237.47 |
| 19 | 93.49 | 575.34 |
| 20 | 13.3 | 228.29 |
| 21 | 74.51 | 267.63 |
| 22 | 27.18 | 227.83 |
| 26 | 24.86 | 321.81 |
| 27 | 221 | 233 |
| 29 | 51.28 | 63.12 |
| 30 | 101.86 | 55.61 |
| 31 | 132.31 | >10000 |
| 32 | 73.87 | 210.14 |
| 33 | 96.8 | 2174.85 |
| 34 | >1000 | >10000 |
| 35 | >1000 | >10000 |
| 36 | >1000 | >10000 |
| 37 | 114.92 | 1574.23 |
| 38 | >1000 | >10000 |

(continued)

| Compound No. | Nav1.8 $IC_{50}$ (nM) | Nav1.7 $IC_{50}$ (nM) |
|---|---|---|
| 39 | 86.36 | 1520.66 |
| 40 | 277.15 | 3358.75 |
| 41 | 94.49 | 3289.2 |
| 42 | 621.47 | 3904.81 |
| 43 | 187.77 | 3917.82 |
| 44 | 162.62 | 3614.91 |
| 45 | 713.4 | 3764.65 |
| 46 | 177.91 | 1988.12 |
| 47 | 81.97 | 1417.16 |
| 48 | 233.42 | 798.17 |
| 49 | 158.12 | 1311.69 |
| 50 | >1000 | 6230.99 |
| 51 | 131.46 | 1378.03 |
| 52 | >1000 | 3586.88 |
| 53 | 440.9 | >10000 |
| 54 | 311.07 | 2909.85 |
| 55 | 475.74 | 2190.68 |
| 56 | 461.68 | 5477.75 |
| 57 | 626.82 | 3436.67 |
| 58 | 97.03 | 1052.99 |
| 59 | 492.13 | 1849.88 |
| 60 | 339.77 | 5191.52 |
| 61 | 613.57 | 7023.19 |
| 62 | 760.71 | >10000 |
| 63 | >1000 | >10000 |
| 64 | 1000 | 8156.5 |
| 65 | 800.05 | 6597.14 |
| 66 | >1000 | >10000 |
| 67 | 747 | 1930.8 |
| 68 | 289.3 | 1833.44 |
| 69 | 516.59 | 1433.59 |
| 70 | 972.62 | 10000 |
| 71 | 184.51 | 2593.34 |
| 72 | 158.35 | 2286.92 |
| 73 | 776.79 | >10000 |
| 74 | >1000 | >10000 |
| 75 | 51.17 | 464.43 |
| 76 | >1000 | >10000 |

(continued)

| Compound No. | Nav1.8 $IC_{50}$ (nM) | Nav1.7 $IC_{50}$ (nM) |
|---|---|---|
| 77 | >1000 | >10000 |
| 78 | 840.87 | 7151.52 |
| 79 | >1000 | >10000 |
| 80 | 352.13 | 1493.15 |
| 81 | >1000 | >10000 |
| 82 | >1000 | 2773.02 |
| 83 | 102.94 | 135.14 |
| 84 | 152.11 | 178.95 |
| 85 | 31.78 | 158.92 |
| 86 | 208.7 | 141 |
| 87 | 447.42 | 264.45 |
| 88 | 42.63 | 70.39 |
| 89 | 148.72 | 211.58 |
| 90 | >1000 | >10000 |
| 91 | >1000 | >10000 |
| 92 | >1000 | >10000 |
| 93 | >1000 | >10000 |
| 94 | >1000 | >10000 |
| 95 | >1000 | >10000 |
| 96 | 886.51 | >10000 |
| 97 | 394.5 | >10000 |
| 98 | >1000 | >10000 |
| 99 | >1000 | >10000 |
| 100 | >1000 | >10000 |
| 101 | 1000 | 2900.46 |
| 102 | 459.49 | >10000 |
| 103 | >1000 | >10000 |
| 104 | >1000 | >10000 |
| 105 | 33.69 | 44.65 |
| 106 | 90.15 | 82.97 |
| 107 | 1000 | 5164.45 |
| 108 | 17.71 | 30.77 |
| 109 | 148.65 | 566.34 |
| 110 | 351.49 | 282.84 |
| 111 | 119.28 | 541.46 |
| 112 | 965.83 | 2349 |
| 113 | 433.32 | 1004.51 |
| 114 | >1000 | >10000 |

(continued)

| Compound No. | Nav1.8 $IC_{50}$ (nM) | Nav1.7 $IC_{50}$ (nM) |
|---|---|---|
| 115 | >1000 | >10000 |
| 116 | 331.63 | 666 |
| 117 | 238.25 | 736.91 |
| 118 | 79.88 | 416 |
| 119 | 128.46 | >10000 |
| 120 | 133.37 | 115.88 |
| 121 | 116.61 | 499.45 |
| 122 | >1000 | 5124.24 |
| 123 | 802.94 | 49.24 |
| 124 | 73.22 | 180.93 |
| 125 | >1000 | >10000 |
| 126 | >1000 | >10000 |
| 127 | >1000 | >10000 |
| 128 | >1000 | >10000 |
| 129 | >1000 | >10000 |
| 130 | >1000 | >10000 |
| 131 | >1000 | >10000 |
| 132 | >1000 | >10000 |
| 133 | 913.16 | >10000 |
| 134 | >1000 | >10000 |
| 135 | >1000 | >10000 |
| 136 | >1000 | >10000 |
| 137 | >1000 | >10000 |
| 138 | >1000 | >10000 |
| 139 | >1000 | >10000 |
| 140 | >1000 | >10000 |
| 141 | >1000 | >10000 |
| 142 | >1000 | >10000 |
| 143 | 650 | 5150 |
| 144 | >300 | >300 |
| 145 | >300 | >300 |
| 146 | >300 | >300 |
| 147 | >300 | >300 |
| 148 | 48.45 | 69.1 |
| 149 | 60.87 | 122.87 |
| 150 | 117.59 | 121.24 |
| 151 | >1000 | >10000 |
| 152 | 88.42 | 138.35 |

(continued)

| Compound No. | Nav1.8 $IC_{50}$ (nM) | Nav1.7 $IC_{50}$ (nM) |
|---|---|---|
| 153 | 120.86 | 227.84 |
| 154 | >1000 | 1393.03 |
| 155 | 266.29 | 360.91 |
| 156 | >1000 | 4785.15 |
| 157 | 456.05 | 180 |
| 158 | 281.77 | 245 |
| 159 | 293.03 | 217 |
| 160 | >1000 | >10000 |
| 161 | >1000 | >10000 |
| 162 | 899 | 2662 |
| 163 | >1000 | >10000 |
| 164 | >1000 | >10000 |
| 165 | >1000 | >10000 |
| 167 | >1000 | >10000 |
| 168 | >1000 | >10000 |
| 169 | >1000 | >10000 |
| 170 | >1000 | >10000 |
| 171 | >1000 | >10000 |
| 172 | 573 | >10000 |
| 173 | 629 | >10000 |
| 174 | >1000 | >10000 |
| 175 | 361 | 110 |
| 176 | 456 | 400 |
| 177 | 963 | >1000 |
| 178 | 358 | 3191 |
| 179 | >1000 | >10000 |
| 180 | >1000 | >10000 |
| 181 | 320 | 6409 |
| 182 | 300 | >10000 |
| 183 | 231 | 256 |
| 184 | 398 | >10000 |
| 185 | >1000 | >10000 |
| 187 | 496 | 219 |
| 188 | >1000 | >10000 |
| 189 | >1000 | >10000 |
| 190 | >1000 | >10000 |
| 191 | >1000 | 3200 |
| 192 | 952 | 7217 |

(continued)

| Compound No. | Nav1.8 $IC_{50}$ (nM) | Nav1.7 $IC_{50}$ (nM) |
|---|---|---|
| 193 | >1000 | >10000 |
| 197 | 162 | 778 |
| 198 | 24 | 367 |
| 199 | 166 | 1741 |
| 200 | 129 | 2652 |
| 201 | 70 | 425 |
| 202 | 93 | 107 |
| 206 | >1000 | >10000 |
| 207 | 472 | >10000 |
| 208 | 431 | 1887 |
| 209 | 189 | 2470 |
| 210 | 680 | >10000 |
| 211 | >1000 | >10000 |
| 212 | >1000 | >10000 |
| 213 | >1000 | >10000 |
| 214 | >1000 | >10000 |
| 215 | 116 | 1913 |

From these results, the inhibitory activity of Nav 1.7 and/or 1.8 is proven, the application of which is readily carried out by persons skilled in the art in pharmaceutical compositions, which may comprise one or more of said compounds of Formula !, kits, in addition to uses in the treatment of pain-related pathologies.

in particular, such results indicate the possibility of using the compounds of Formula (I) in the preparation of medicaments for treating conditions such as peripheral neuropathic pain, chemotherapy-induced neuropathy, complex regional pain, neuropathy related to viral infection, neuropathy secondary to tumor infiltration, diabetic neuropathy, phantom limb pain, postherpetic neuralgia, trigeminal neuralgia, and postsurgical neuralgia.

It should be understood that the embodiments described above are merely illustrative and that various modifications may be made by one skilled in the art thereto without departing from the scope of the present invention. Accordingly, the present invention should not be considered limited to the exemplary embodiments described in the present application.

## Claims

**1.** A COMPOUND **characterized in that** it comprises Formula (I)

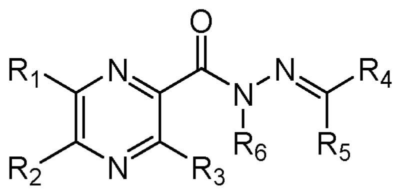

Formula (I)

or a pharmaceutically acceptable salt, hydrate, solvate, and isomer thereof, wherein:

- R1 is selected from hydrogen, halogen, branched or linear C1-6 alkyl branched or linear C1-6 alkoxy, morpholin, piperidinyl or substituted piperidinyl, pyridinyl or substituted pyridinyl, piperazin or substituted piperazin, C3-7

heterocycloalkyl, aryloxy, substituted aryloxy, or R7;
- R2 and R3 are independently selected from the group consisting of hydrogen, halogen, linear or branched C1-6 alkyl; linear or branched C1-6 alkoxy, amine, C1-6haloalkyl, aryl, substituted aryl, aryloxy, or substituted aryloxy;
- R4 is selected from the group consisting of hydrogen, halogen, linear or branched C1-6alkyl; linear or branchedC1-6 alkoxy, aryl, pyridinyl or substituted pyridinyl, pyridone, saturated or unsaturated C3-7cycloalkyl saturated or unsaturated C1-5 alkylsubstituted C3-7cycloalkyl saturated or unsaturated halosubstituted C3-7 cycloalkyl, picolinamide or R13;
- R5 and R6 are independently selected from hydrogen or linear or branched C1-6 alkyl.
- R7 is:

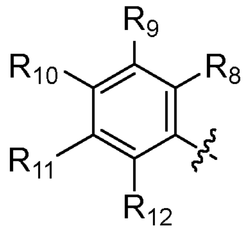

wherein,
- R8 is selected from the group consisting of hydrogen, halogen, linear or branched C1-6 alkyl, linear or branched C1-6 alkoxy, C1-6haloalkyl, morpholin,C1-6 haloalkyloxy, C1-6 alkylamino or (C1-6 alkylamino)C1-6alkoxy;
-R9, R11 andR12 are independently selected from the group consisting of hydrogen, halogen, linear or branched C1-6 alkyl, linear or branched C1-6 alkoxy; C1-6haloalkyl or C3-6cycloalkyloxy;
- R10 is selected from the group consisting of hydrogen, halogen, linear or branched C1-6alkyl, linear or branched C1-6 alkoxy, C3-6 cycloalkyloxy; (C3-6cycloalkyl) C1-6 alkoxy; -S-C1-6 alkyl; C1-6 alkylamino; haloalkyloxy C1-6,haloalkyl C1-6, (C1-6 alkylamino) C1-6 alkoxy, (-S-C1-6alkyl) C1-6alkyloxy, C1-6alkylsulfonylamide, or-OCD3;
- R13 is:

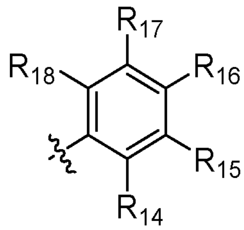

wherein,
- R14 and R15 are independently selected from the group consisting of hydrogen, halogen, linear or branched C1-6 alkyl; linear or branched C1-6 alkoxy, C1-6haloalkyl,C1-6 alkylamino, C1-6 alkylsufonyl,C1-6 alkylsufonylamide, (C1-6 alkylsufonyl) C1-6alkyl; C1-6hydroxyalkyl, (C1-6 alkylamino) C1-6 alkoxy, amide, -OCD3;
or, R14 and R15 are optionally substituted with an OC=N, N=CO or SC=N group forming together a thiazole or an oxazole.
- R16 and R17 are independently selected from the group consisting of hydrogen, halogen, linear or branched C1-6 alkyl; linear or branched C1-6 alkoxy, C1-6haloalkyl, C1-6alkylamino, C1-6alkylsufonylamide, (C1-6alkylamino) C1-6alkyl or-OCD3;
- R18 is selected from the group consisting of hydrogen, halogen, linear or branched C1-6alkyl or linear or branched C1-6 alkoxy.

**2.** The COMPOUND according to claim 1, **characterized in that** it is selected from the group consisting of:

6-(4-chlorophenyl)-N'-[(E)-(3,5-dimethoxyphenyl)methylidene]pyrazine-2-carbohydrazide (Compound 1);
(*E*)-*N*'-(3,5-dimethoxybenzylidene)-6-(4-ethoxyphenyl)pyrazine-2-carbohydrazide (Compound 2);
(*E*)-*N*'-(2,5-dimethoxybenzylidene)-6-(4-ethoxyphenyl)pyrazine-2-carbohydrazide (Compound 3);
(*E*)-*N*'-(3-(2-(dimethylam ino)ethoxy)-5-methoxybenzylidene)-6-(4-ethoxyphenyl)pyrazine-2-carbohydrazide

(Compound 4);
(*E*) -6-(4-ethoxyphenyl) -*N'*-((5-methoxybenzo[d]oxazol-7-yl)methylene)pyrazine-2-carbohydrazide (Compound 5);
(*E*) -6-(4-ethoxyphenyl) -*N'*-((6-methoxybenzo[d]oxazol-4-yl)methylene)pyrazine-2-carbohydrazide (Compound 6);
(*E*) -6-(4-ethoxyphenyl) -*N'*-((5-methoxybenzo[d]thiazol-7-yl)methylene)pyrazine-2-carbohydrazide (Compound 7);
*(E)*-6-(4-ethoxyphenyl)-*N'*-((5-methoxypyridin-3-yl)methylene)pyrazine-2-carbohydrazide (Compound 8);
*(E)*-6-(4-ethoxyphenyl)-*N'*-((4-methoxypyridin-2-yl)methylene)pyrazine-2-carbohydrazide (Compound 9);
*(E)*-6-(4-ethoxyphenyl)-*N'*-((2-methoxypyridin-4-yl)methylene)pyrazine-2-carbohydrazide (Compound 10);
*(E)*-6-(4-ethoxyphenyl)-*N'*-((6-methoxypyridin-2-yl)methylene)pyrazine-2-carbohydrazide (Compound 11);
*(E)*-*N'*-(3,5-dimethylbenzylidene)-6-(4-ethoxyphenyl)pyrazine-2-carbohydrazide (Compound 12);
(*E*)-6-(4-ethoxyphenyl)-N'-(2,3,5-trimethoxybenzylidene)pyrazine-2-carbohydrazide (Compound 13);
(*E*)-*N*'-(2-ethoxy-3,5-dimethoxybenzylidene)-6-(4-ethoxyphenyl)pyrazine-2-carbohydrazide (Compound 14);
(*E*)-6-(4-ethoxyphenyl)-N'-(3-fluoro-5-methoxybenzylidene)pyrazine-2-carbohydrazide (Compound 15);
(*E*)-6-(4-ethoxyphenyl)-N'-(3-methoxy-5-propoxybenzylidene)pyrazine-2-carbohydrazide (Compound 16);
(*E*)-6-(4-ethoxyphenyl)-N'-(4-fluoro-3,5-dimethoxybenzylidene)pyrazine-2-carbohydrazide (Compound 17);
(*E*)-*N*'-(2-chloro-5-methoxybenzylidene)-6-(4-ethoxyphenyl)pyrazine-2-carbohydrazide (Compound 18);
(*E*)-*N*'-(2-chloro-3-methoxybenzylidene)-6-(4-ethoxyphenyl)pyrazine-2-carbohydrazide (Compound 19);
(*E*)-6-(4-ethoxyphenyl)-N'-(2-fluoro-5-methoxybenzylidene)pyrazine-2-carbohydrazide (Compound 20);
(*E*)-6-(4-ethoxyphenyl)-N'-(2-fluoro-3-methoxybenzylidene)pyrazine-2-carbohydrazide (Compound 21);
(E)-6-(4-ethoxyphenyl)-N'-(3-methoxybenzylidene)pyrazine-2-carbohydrazide (Compound 22);
(*E*)-6-(4-ethoxyphenyl)-N'-(4-methoxybenzylidene)pyrazine-2-carbohydrazide (Compound 23);
(*E*)-6-(4-ethoxyphenyl)-N'-(pyridin-4-ylmethylene)pyrazine-2-carbohydrazide (Compound 24);
(*E*)-6-(4-ethoxyphenyl)-N'-(pyridin-2-ylmethylene)pyrazine-2-carbohydrazide (Compound 25);
(*E*)-6-(4-ethoxyphenyl)-N'-(2-methoxybenzylidene)pyrazine-2-carbohydrazide (Compound 26);
(*E*)-*N*'-(2,3-dimethoxybenzylidene)-6-(4-ethoxyphenyl)pyrazine-2-carbohydrazide (Compound 27);
(*E*)-*N*'-(3,4-dimethoxybenzylidene)-6-(4-ethoxyphenyl)pyrazine-2-carbohydrazide (Compound 28);
(E)-N'-(3,5-dimethoxybenzylidene)-6-(4-isopropoxyphenyl)pyrazine-2-carbohydrazide (Compound 29);
6-[4-(cyclopentyloxy)phenyl]-*N'*-[*(E)*-(3,5-dimethoxyphenyl)methylidene]pyrazine-2-carbohydrazide (Compound 30);
*N'*-[*(E)*-(3,5-dimethoxyphenyl)methylidene]-6-[4-(trifluoromethoxy)phenyl]pyrazine-2-carbohydrazide (Compound 31);
*N'*-[*(E)*-(3,5-dimethoxyphenyl)methylidene]-6-(4-propoxyphenyl)pyrazine-2-carbohydrazide (Compound 32);
*N'*-[*(E)*-(3,5-dimethoxyphenyl)methylidene]-6-(4-methoxyphenyl)pyrazine-2-carbohydrazide (Compound 33);
*(E)*-6-(4-methoxyphenyl)-*N'*-((2-oxo-1,2-dihydropyridin-4-yl)methylene)pyrazine-2-carbohydrazide (Compound 34);
(*E*)-2-fluoro-5-((2-(6-(4-methoxyphenyl)pyrazine-2-carbonyl)hydrazono)methyl) benzamide (Compound 35);
(*E*)-4-((2-(6-(4-methoxyphenyl)pyrazine-2-carbonyl)hydrazono)methyl)picolinamide (Compound 36);
(*E*)-*N*'-(3-methoxybenzylidene)-6-(4-methoxyphenyl)pyrazine-2-carbohydrazide (Compound 37);
(*E*)-*N*'-(3-((dimethylamino)methyl)benzylidene)-6-(4-methoxyphenyl)pyrazine-2-carbohydrazide (Compound 38);
(*E*)-*N*'-(3-ethoxybenzylidene)-6-(4-methoxyphenyl)pyrazine-2-carbohydrazide (Compound 39);
(*E*) -6-(4-methoxyphenyl) -*N'*-(3-propoxybenzylidene)pyrazine-2-carbohydrazide (Compound 40);
(*E*)-*N*'-(3-isopropoxybenzylidene)-6-(4-methoxyphenyl)pyrazine-2-carbohydrazide (Compound 41);
(*E*)-*N*'-(2-methoxybenzylidene)-6-(4-methoxyphenyl)pyrazine-2-carbohydrazide (Compound 42);
(*E*)-*N*'-(2-ethoxybenzylidene)-6-(4-methoxyphenyl)pyrazine-2-carbohydrazide (Compound 43);
(*E*) -6-(4-methoxyphenyl) -*N'*-(2-propoxybenzylidene)pyrazine-2-carbohydrazide (Compound 44);
(E)-N'-(2-isopropoxybenzylidene)-6-(4-methoxyphenyl)pyrazine-2-carbohydrazide (Compound 45);
(E)-N'-(2-fluorobenzylidene)-6-(4-methoxyphenyl)pyrazine-2-carbohydrazide (Compound 46);
*(E)*-*N'*-(2-chlorobenzylidene)-6-(4-methoxyphenyl)pyrazine-2-carbohydrazide (Compound 47);
(*E*)-*N*'-(2-bromobenzylidene)-6-(4-methoxyphenyl)pyrazine-2-carbohydrazide (Compound 48);
(*E*)-6-(4-methoxyphenyl)-N'-(2-(trifluoromethyl)benzylidene)pyrazine-2-carbohydrazide (Compound 49);
(*E*)-N'-(2-(dimethylamino)benzylidene)-6-(4-methoxyphenyl)pyrazine-2-carbohydrazide (Compound 50);
(*E*)-6-(4-methoxyphenyl)-N'-(2-methylbenzylidene)pyrazine-2-carbohydrazide (Compound 51);
(*E*)-*N*'-(2-(tert-butyl)benzylidene)-6-(4-methoxyphenyl)pyrazine-2-carbohydrazide (Compound 52);
(*E*)-*N*'-(3-fluorobenzylidene)-6-(4-methoxyphenyl)pyrazine-2-carbohydrazide (Compound 53);
*(E)*-*N'*-(3-chlorobenzylidene)-6-(4-methoxyphenyl)pyrazine-2-carbohydrazide (Compound 54);

(E)-*N*'-(3-bromobenzylidene)-6-(4-methoxyphenyl)pyrazine-2-carbohydrazide (Compound 55);
*(E)*-6-(4-methoxyphenyl)-*N'*-(3-(trifluoromethyl)benzylidene)pyrazine-2-carbohydrazide (Compound 56);
(*E*)-*N*'-(3-(dimethylamino)benzylidene)-6-(4-methoxyphenyl)pyrazine-2-carbohydrazide (Compound 57);
(*E*)-6-(4-methoxyphenyl) -*N'*-(3-methylbenzylidene)pyrazine-2-carbohydrazide (Compound 58);
(*E*)-*N*'-(3-(tert-butyl)benzylidene)-6-(4-methoxyphenyl)pyrazine-2-carbohydrazide (Compound 59);
(*E*)-*N*'-(4-fluorobenzylidene)-6-(4-methoxyphenyl)pyrazine-2-carbohydrazide (Compound 60);
E)-N'-(4-chlorobenzylidene)-6-(4-methoxyphenyl)pyrazine-2-carbohydrazide (Compound 61);
(*E*)-*N*'-(4-bromobenzylidene)-6-(4-methoxyphenyl)pyrazine-2-carbohydrazide (Compound 62);
*(E)*-6-(4-methoxyphenyl)-*N'*-(4-(trifluoromethyl)benzylidene)pyrazine-2-carbohydrazide (Compound 63);
(*E*)-N'-(4-(dimethylamino)benzylidene)-6-(4-methoxyphenyl)pyrazine-2-carbohydrazide (Compound 64);
(*E*)-6-(4-methoxyphenyl)-N'-(4-methylbenzylidene)pyrazine-2-carbohydrazide (Compound 65);
(*E*)-*N*'-(4-(tert-butyl)benzylidene)-6-(4-methoxyphenyl)pyrazine-2-carbohydrazide (Compound 66);
(*E*)-*N*'-(3,5-diethoxybenzylidene)-6-(4-methoxyphenyl)pyrazine-2-carbohydrazide (Compound 67);
(*E*)-*N*'-(3,5-dipropoxybenzylidene)-6-(4-methoxyphenyl)pyrazine-2-carbohydrazide (Compound 68);
(*E*)-*N*'-(3,5-diisopropoxybenzylidene)-6-(4-methoxyphenyl)pyrazine-2-carbohydrazide (Compound 69);
(E)-N'-(3,5-difluorobenzylidene)-6-(4-methoxyphenyl)pyrazine-2-carbohydrazide (Compound 70);
(E)-N'-(3,5-dichlorobenzylidene)-6-(4-methoxyphenyl)pyrazine-2-carbohydrazide (Compound 71);
(*E*)-*N*'-(3,5-dibromobenzylidene)-6-(4-methoxyphenyl)pyrazine-2-carbohydrazide (Compound 72);
*(E)-N'*-(3,5-bis(trifluoromethyl)benzylidene)-6-(4-methoxyphenyl)pyrazine-2-carbohydrazide (Compound 73);
(*E*)-*N*'-(3,5-bis(dimethylamino)benzylidene)-6-(4-methoxyphenyl)pyrazine-2-carbohydrazide (Compound 74);
(*E*)-*N*'-(3,5-dimethylbenzylidene)-6-(4-methoxyphenyl)pyrazine-2-carbohydrazide (Compound 75);
(*E*)-*N*'-(3,5-di-*tert*-butylbenzylidene)-6-(4-methoxyphenyl)pyrazine-2-carbohydrazide (Compound 76);
(*E*)-6-(4-methoxyphenyl)-N'-(pyridin-3-ylmethylene)pyrazine-2-carbohydrazide (Compound 77);
(*E*)-N'-(2,4-dimethoxybenzylidene)-6-(4-methoxyphenyl)pyrazine-2-carbohydrazide (Compound 78);
(*E*)-*N*'-(2,6-dimethoxybenzylidene)-6-(4-methoxyphenyl)pyrazine-2-carbohydrazide (Compound 79);
(*E*)-6-(4-methoxyphenyl)-*N*'-(2,3,5-trimethoxybenzylidene)pyrazine-2-carbohydrazide (Compound 80);
(*E*)-6-(4-methoxyphenyl)-*N*'-(3,4,5-trimethoxybenzylidene)pyrazine-2-carbohydrazide (Compound 81);
(*E*)-6-(4-methoxyphenyl)-*N*'-(2,3,4-trimethoxybenzylidene)pyrazine-2-carbohydrazide (Compound 82);
6-(4-butoxyphenyl)-*N'*-[*(E)*-(3,5-dimethoxyphenyl)methylidene]pyrazine-2-carbohydrazide (Compound 83);
(*E*)-6-(4-butoxyphenyl) -*N'*-(3,5-dimethylbenzylidene)pyrazine-2-carbohydrazide (Compound 84);
(E)-N'-(3,5-dimethoxybenzylidene)-6-(4-isobutoxyphenyl)pyrazine-2-carbohydrazide (Compound 85);
(E)-N'-(3,5-dimethylbenzylidene)-6-(4-isobutoxyphenyl)pyrazine-2-carbohydrazide (Compound 86);
*(E)*-6-(*4-(tert*-butoxy)phenyl)-*N'*-(3,5-dimethoxybenzylidene)pyrazine-2-carbohydrazide (Compound 87);
(*E*)-6-(4-cyclopropoxyphenyl)-*N*'-(3,5-dimethoxybenzylidene)pyrazine-2-carbohydrazide (Compound 88);
(*E*)-6-(4-(cyclohexyloxy)phenyl)-N'-(3,5-dimethoxybenzylidene)pyrazine-2-carbohydrazide (Compound 89);
(*E*)-*N*'-(3,5-dimethoxybenzylidene)-3-(4-methoxyphenyl)pyrazine-2-carbohydrazide (Compound 90);
(*E*)-*N*'-(3,5-dimethoxybenzylidene)-5-(4-methoxyphenyl)pyrazine-2-carbohydrazide (Compound 91);
(*E*)-*N*'-(3,5-dimethoxybenzylidene)-6-(4-fluorophenyl)pyrazine-2-carbohydrazide (Compound 92);
(*E*)-6-(3-chlorophenyl)-N'-(3,5-dimethoxybenzylidene)pyrazine-2-carbohydrazide (Compound 93);
(*E*)-*N*'-(3,5-dimethoxybenzylidene)-6-(3-fluorophenyl)pyrazine-2-carbohydrazide (Compound 94);
(*E*)-6-(2-chlorophenyl)-N'-(3,5-dimethoxybenzylidene)pyrazine-2-carbohydrazide (Compound 95);
(E)-N'-(3,5-dimethoxybenzylidene)-6-(p-tolyl)pyrazine-2-carbohydrazide (Compound 96);
(E)-N'-(3,5-dimethoxybenzylidene)-6-phenylpyrazine-2-carbohydrazide (Compound 97);
(*E*)-*N*'-(3,5-dimethoxybenzylidene)-6-(2-fluorophenyl)pyrazine-2-carbohydrazide (Compound 98);
(*E*)-*N*'-(3,5-dimethoxybenzylidene)-6-(o-tolyl)pyrazine-2-carbohydrazide (Compound 99);
(*E*)-*N*'-(3,5-dimethoxybenzylidene)-6-(m-tolyl)pyrazine-2-carbohydrazide (Compound 100);
(*E*)-*N*'-(3,5-dimethoxybenzylidene)-6-(3-methoxyphenyl)pyrazine-2-carbohydrazide (Compound 101);
(*E*)-*N*'-(3,5-dimethoxybenzylidene)-6-(2-methoxyphenyl)pyrazine-2-carbohydrazide (Compound 102);
(*E*)-*N*'-(3,5-dimethoxybenzylidene)-6-(pyridin-4-yl)pyrazine-2-carbohydrazide (Compound 103);
(*E*)-*N*'-(3,5-dimethoxybenzylidene)-6-(pyridin-3-yl)pyrazine-2-carbohydrazide (Compound 104);
(*E*)-6-(4-ethoxy-2-methoxyphenyl)-*N*'-(3-fluoro-5-methoxybenzylidene)pyrazine-2-carbohydrazide (Compound 105);
(*E*)-6-(4-ethoxy-2-methoxyphenyl)-*N*'-(3-methoxy-5-propoxybenzylidene)pyrazine-2-carbohydrazide (Compound 106);
*(E)*-6-(4-ethoxy-2-methoxyphenyl)-*N'*-(4-fluoro-3,5-dimethoxybenzylidene)pyrazine-2-carbohydrazide (Compound 107);
(*E*)-6-(4-ethoxy-2-methoxyphenyl)-*N*'-(3-methoxybenzylidene)pyrazine-2-carbohydrazide (Compound 108);
*(E)-N'*-(3,5-dimethoxybenzylidene)-6-(4-methoxy-2-methylphenyl)pyrazine-2-carbohydrazide (Compound

109);
(*E*)-6-(2-chloro-4-ethoxyphenyl)-N'-(3,5-dimethoxybenzylidene)pyrazine-2-carbohydrazide (Compound 110);
(*E*)-6-(2-chloro-4-ethoxyphenyl)-N'-(3-methoxybenzylidene)pyrazine-2-carbohydrazide (Compound 111);
(*E*)-*N*'-(3,5-dimethoxybenzylidene)-6-(4-methoxy-2-(trifluoromethoxy)phenyl)pyrazine-2-carbohydrazide (Compound 112);
(*E*)-*N*'-(3,5-dimethoxybenzylidene)-6-(4-propylphenyl)pyrazine-2-carbohydrazide (Compound 113);
*(E)-N'*-(3,5-dimethoxybenzylidene)-6-(2-morpholinophenyl)pyrazine-2-carbohydrazide (Compound 114);
*(E)*-6-(2-butoxyphenyl)-*N'*-(3,5-dimethoxybenzylidene)pyrazine-2-carbohydrazide (Compound 115);
(*E*)-*N*'-(3,5-dimethoxybenzylidene)-6-(4-isobutylphenyl)pyrazine-2-carbohydrazide (Compound 116);
(*E*)-6-(3-chloro-4-ethoxyphenyl)-N'-(3,5-dimethoxybenzylidene)pyrazine-2-carbohydrazide (Compound 117);
(*E*)-*N*'-(3,5-dimethoxybenzylidene)-6-(4-(ethylthio)phenyl)pyrazine-2-carbohydrazide (Compound 118);
(*E*)-6-(4-ethoxy-2-fluorophenyl)-N'-(3-methoxybenzylidene)pyrazine-2-carbohydrazide (Compound 119);
(*E*)-6-(4-ethoxy-2-(trifluoromethyl)phenyl)-N'-(3-methoxybenzylidene)pyrazine-2-carbohydrazide (Compound 120);
(*E*)-*N*'-(3,5-dimethoxybenzylidene)-6-(2-fluoro-4-methoxyphenyl)pyrazine-2-carbohydrazide (Compound 121);
(*E*)-6-(2-chloro-4-methoxyphenyl)-N'-(3,5-dimethoxybenzylidene)pyrazine-2-carbohydrazide (Compound 122);
(*E*)-*N*'-(3,5-dimethoxybenzylidene)-6-(4-methoxy-2-(trifluoromethyl)phenyl)pyrazine-2-carbohydrazide (Compound 123);
(*E*)-*N*'-(3,5-dimethoxybenzylidene)-6-(2,4-dimethoxyphenyl)pyrazine-2-carbohydrazide (Compound 124);
(*E*)-*N*'-(3,5-dimethoxybenzylidene)-6-(pyridin-2-yl)pyrazine-2-carbohydrazide (Compound 125);
(*E*)-*N*'-(3,5-dimethoxybenzylidene)-6-morpholinopyrazine-2-carbohydrazide (Compound 126);
(E)-*N*'-(3,5-dimethoxybenzylidene)-6-(piperidin-1-yl)pyrazine-2-carbohydrazide (Compound 127);
(*E*)-*N*'-(3,5-dimethoxybenzylidene)-6-(4-propylpiperazin-1-yl)pyrazine-2-carbohydrazide (Compound 128);
(*E*)-*N*'-(3,5-dimethoxybenzylidene)-3-(4-methoxyphenoxy)pyrazine-2-carbohydrazide (Compound 129);
(*E*)-*N*'-(3,5-dimethoxybenzylidene)-5-(4-methoxyphenoxy)pyrazine-2-carbohydrazide (Compound 130);
(*E*)-*N*'-(3,5-dimethoxybenzylidene)-6-(4-ethoxyphenoxy)pyrazine-2-carbohydrazide (Compound 131);
(*E*)-*N*'-(3,5-dimethoxybenzylidene)-3-ethylpyrazine-2-carbohydrazide (Compound 132);
(*E*)-*N*'-(3,5-dimethoxybenzylidene)-6-ethylpyrazine-2-carbohydrazide (Compound 133);
(*E*)-*N*'-(3,5-dimethoxybenzylidene)-6-isopropylpyrazine-2-carbohydrazide (Compound 134);
(*E*)-*N*'-(3,5-dimethoxybenzylidene)-3-isopropylpyrazine-2-carbohydrazide (Compound 135);
(*E*)-*N*'-(3,5-dimethoxybenzylidene)-5-isopropylpyrazine-2-carbohydrazide (Compound 136);
*(E)-N'*-(3,5-dimethoxybenzylidene)-6-methylpyrazine-2-carbohydrazide. (Compound 137);
*(E)-N'*-(3,5-dimethoxybenzylidene)-5-methylpyrazine-2-carbohydrazide (Compound 138);
*(E)-N'*-(3,5-dimethoxybenzylidene)-3-methylpyrazine-2-carbohydrazide (Compound 139);
(*E*)-*N*'-(3,5-dimethoxybenzylidene)-6-methoxypyrazine-2-carbohydrazide (Compound 140);
(*E*)-*N*'-(3,5-dimethoxybenzylidene)-3-methoxypyrazine-2-carbohydrazide (Compound 141);
(*E*)-N'-(3,5-dimethoxybenzylidene)-5-methoxypyrazine-2-carbohydrazide (Compound 142);
*(E)-N'*-(3,5-dimethoxybenzylidene)-6-(4-ethoxyphenyl)-3-methylpyrazine-2-carbohydrazide (Compound 143);
(*E*)-*N*'-(3,5-dimethoxybenzylidene)-6-(4-ethoxyphenyl)-3-ethylpyrazine-2-carbohydrazide (Compound 144);
(*E*)-*N*'-(3,5-dimethoxybenzylidene)-6-(4-ethoxyphenyl)-3-methoxypyrazine-2-carbohydrazide (Compound 145);
(*E*)-*N*'-(3,5-dimethoxybenzylidene)-6-(4-ethoxyphenyl)-3-phenoxypyrazine-2-carbohydrazide (Compound 146);
(*E*)-*N*'-(3,5-dimethoxybenzylidene)-6-(4-ethoxyphenyl)-3-phenylpyrazine-2-carbohydrazide (Compound 147);
(*E*)-6-(4-cyclobutoxyphenyl)-N'-(3,5-dimethoxybenzylidene)pyrazine-2-carbohydrazide (Compound 148);
(*E*)-*N*'-(3,5-dimethoxybenzylidene)-6-(4-((methylthio)methoxy)phenyl)pyrazine-2-carbohydrazide (Compound 149);
(*E*)-6-(4-(cyclopentyloxy)phenyl)-N'-(3,5-dimethylbenzylidene)pyrazine-2-carbohydrazide (Compound 150);
*(E)-N'*-(3,5-dimethoxybenzylidene)-6-(4-(2-(dimethylamino)ethoxy)phenyl)pyrazine-2-carbohydrazide (Compound 151);
(*E*)-6-(4-(cyclopropylmethoxy)phenyl)-*N*'-(3,5-dimethoxybenzylidene)pyrazine-2-carbohydrazide (Compound 152);
(*E*)-6-(2,4-diethoxyphenyl)-N'-(3-methoxybenzylidene)pyrazine-2-carbohydrazide (Compound 153);
(*E*)-6-(4-ethoxy-2-isopropoxyphenyl)-N'-(3-methoxybenzylidene)pyrazine-2-carbohydrazide (Compound 154);
(*E*)-*N*'-(3,5-dimethoxybenzylidene)-6-(2-methoxyphenyl)pyrazine-2-carbohydrazide (Compound 155);
(*E*)-*N*'-(3,5-dimethoxybenzylidene)-6-(2-isopropoxy-4-methoxyphenyl)pyrazine-2-carbohydrazide (Compound 156);

(*E*)-6-(2-chloro-4-(cyclopentyloxy)phenyl)-N'-(3,5-dimethoxybenzylidene)pyrazine-2-carbohydrazide (Compound 157);
(*E*)-6-(4-butoxy-2-chlorophenyl)-N'-(3,5-dimethoxybenzylidene)pyrazine-2-carbohydrazide (Compound 158);
(*E*)-6-(2-chloro-4-isobutoxyphenyl)-N'-(3,5-dimethoxybenzylidene)pyrazine-2-carbohydrazide (Compound 159);
(*E*)-*N*'-(3,5-dimethoxybenzylidene)-6-(2-(2-(dimethylamino)ethoxy)-4-methoxyphenol) pyrazine-2-carbohydrazide (Compound 160);
(*E*)-6-(2-(cyclopentyloxy)phenyl)-N'-(3,5-dimethoxybenzylidene)pyrazine-2-carbohydrazide (Compound 161);
(*E*)-*N*'-(3,5-dimethoxybenzylidene)-6-(4-(ethylamino)phenyl)pyrazine-2-carbohydrazide (Compound 162);
(*E*) -6-chloro-N'- (3,5-dimethoxybenzylidene)pyrazine-2-carbohydrazide (Compound 163);
(*E*) -5-chloro-N'- (3,5-dimethoxybenzylidene)pyrazine-2-carbohydrazide (Compound 164);
(*E*) -3-chloro-N'- (3,5-dimethoxybenzylidene)pyrazine-2-carbohydrazide (Compound 165);
(*E*)-*N*'-(1-(3,5-dimethoxyphenyl)ethylidene)-6-(4-ethoxyphenyl)pyrazine-2-carbohydrazide (Compound 166);
(*E*)-*N*'-(3,5-dimethoxybenzylidene)-5-ethylpyrazine-2-carbohydrazide (Compound 167);
*(E)-N'*-(3,5-dimethoxybenzylidene)-6-(4-methoxyphenyl)-N-methylpyrazine-2-carbohydrazide (Compound 168);
(*E*)-6-(4,4-difluoro-3-methylpiperidin-1-yl)-N'-(3,5-dimethoxybenzylidene)pyrazine-2-carbohydrazide (Compound 169);
(*E*)-*N*'-benzylidene-6-(4-ethoxyphenyl)pyrazine-2-carbohydrazide (Compound 170);
(*E*)-*N*'-(cyclohexylmethylene)-6-(4-ethoxyphenyl)pyrazine-2-carbohydrazide (Compound 171);
(*E*)-*N*'-((4,4-difluorocyclohexyl)methylene)-6-(4-ethoxyphenyl)pyrazine-2-carbohydrazide (Compound 172);
(*E*)-*N*'-(cyclohexylmethylene)-6-(4-methoxyphenyl)pyrazine-2-carbohydrazide (Compound 173);
(*E*)-*N*'-((4,4-difluorocyclohexyl)methylene)-6-(4-methoxyphenyl)pyrazine-2-carbohydrazide (Compound 174);
*(E)-N'*-(cyclohexylmethylene)-6-(4-isopropoxyphenyl)pyrazine-2-carbohydrazide (Compound 175);
(*E*)-*N*'-((4,4-difluorocyclohexyl)methylene)-6-(4-isopropoxyphenyl)pyrazine-2-carbohydrazide (Compound 176);
*(E)*-6-(4-(difluoromethyl)phenyl)-*N'*-(3,5-dimethoxybenzylidene)pyrazine-2-carbohydrazide (Compound 177);
(*E*)-*N*'-benzylidene-6-(4-methoxyphenyl)pyrazine-2-carbohydrazide (Compound 178);
(*E*)-6-(4,4-difluoroazepan-1-yl)-N'-(3,5-dimethoxybenzylidene)pyrazine-2-carbohydrazide (Compound 179);
(*E*)-6-(4,4-difluoropiperidin-1-yl)-N'-(3,5-dimethoxybenzylidene)pyrazine-2-carbohydrazide (Compound 180);
(*E*)-*N*'-(cycloheptylmethylene)-6-(4-methoxyphenyl)pyrazine-2-carbohydrazide (Compound 181);
(*E*)-*N*'-(cycloheptylmethylene)-6-(4-ethoxyphenyl)pyrazine-2-carbohydrazide (Compound 182);
(*E*)-*N*'-(cycloheptylmethylene)-6-(4-isopropoxyphenyl)pyrazine-2-carbohydrazide (Compound 183);
(*E*)-*N*'-(3,5-dimethoxybenzylidene)-6-(6-methoxypyridin-3-yl)pyrazine-2-carbohydrazide (Compound 184);
(*E*)-*N*'-(3,5-dimethoxybenzylidene)-6-(5-methoxypyridin-2-yl)pyrazine-2-carbohydrazide (Compound 185);
(*E*)-6-(4-(cyclopentyloxy)-2-(trifluoromethyl)phenyl)-N'-(3,5-dimethoxybenzylidene) pyrazine-2-carbohydrazide (Compound 186);
(*E*)-N'-(3,5-dimethoxybenzylidene)-6-(4-ethoxy-2-(trifluoromethyl)phenyl)pyrazine-2-carbohydrazide (Compound 187);
(*E*)-4-((2-(6-(4-ethoxyphenyl)pyrazine-2-carbonyl)hydrazinalidene)methyl) -*N*-(methyl sulfonyl)benzamide (Compound 188);
(E)-3-((2-(6-(4-ethoxyphenyl)pyrazine-2-carbonyl)hydrazinaylidene)methyl)-N-(methyl sulfonyl)benzamide (Compound 189);
(E)-4-(6-(2-(3,5-dimethoxybenzylidene)hydrazine-1-carbonyl)pyrazin-2-yl)-N-(methyl sulfonyl)benzamide (Compound 190);
(*E*)-6-(4-ethoxyphenyl)-N'-(2-((methylsulfonyl)methyl)benzylidene)pyrazine-2-carbohydrazide (Compound 191);
(*E*)-6-(4-ethoxyphenyl)-*N*'-(2-(methylsulfonyl)benzylidene)pyrazine-2-carbohydrazide (Compound 192);
(*E*)-*N*-(2-((2-(6-(4-ethoxyphenyl)pyrazine-2-carbonyl)hydrazinalidene)methyl)phenyl) methanesulfonamide (Compound 193);
(*E*) -6-(4-methoxyphenyl) -*N'*-((2-methylcyclopent-1-en-1-yl)methylene)pyrazine-2-carbohydrazide (Compound 194);
(*E*)-6-(4-ethoxyphenyl)-A/-((2-methylcyclopent-1-en-1-yl)methylene)pyrazine-2-carbohydrazide (Compound 195);
(*E*)-6-(4-isopropoxyphenyl)-N'-((2-methylcyclopent-1 -en-1-yl)methylene)pyrazine-2-carbohydrazide (Compound 196);
(*E*)-6-(4-methoxyphenyl) -*N'*-((2-methylcyclohex-1-en-1-yl)methylene)pyrazine-2-carbohydrazide (Compound 197);

(*E*)-6-(4-ethoxyphenyl) -*N'*-((2-methylcyclohex-1-en-1-yl)methylene)pyrazine-2-carbohydrazide (Compound 198);
(*E*)-6-(4-isopropoxyphenyl)-N'-((2-methylcyclohex-1 -en-1-yl)methylene)pyrazine-2-carbohydrazide (Compound 199);
(*E*) -6-(4-methoxyphenyl) -*N'*-((2-methylcyclohept-1-en-1-yl)methylene)pyrazine-2-carbohydrazide (Compound 200);
(*E*) -6-(4-ethoxyphenyl) -*N'*-((2-methylcyclohept-1-en-1-yl)methylene)pyrazine-2-carbohydrazide (Compound 201);
(*E*)-6-(4-isopropoxyphenyl)-N'-((2-methylcyclohept-1 -en-1-yl)methylene)pyrazine-2-carbohydrazide (Compound 202);
(*E*)-*N*'-(3-(2-hydroxyethyl)-5-methoxybenzylidene)-6-(4-methoxyphenyl)pyrazine-2-carbohydrazide (Compound 203);
(*E*)-*N*'-(3-(1-hydroxyethyl)-5-methoxybenzylidene)-6-(4-methoxyphenyl)pyrazine-2-carbohydrazide (Compound 204);
(*E*) -6-(2,6-dichloro-4-ethoxyphenyl) -*N'*-(3,5-dimethoxybenzylidene)pyrazine-2-carbohydrazide (Compound 205);
(*E*)-5-amino-N'-(3,5-dimethoxybenzylidene)-6-(4-ethoxyphenyl)pyrazine-2-carbohydrazide (Compound 206);
*(E)-N'*-(3,5-dimethoxybenzylidene)-6-(4-ethoxyphenyl)-5-methylpyrazine-2-carbohydrazide (Compound 207);
(*E*)-*N*'-(3,5-dimethoxybenzylidene)-6-(4-ethoxyphenyl)-5-ethylpyrazine-2-carbohydrazide (Compound 208);
(*E*)-5-chloro-N'-(3,5-dimethoxybenzylidene)-6-(4-ethoxyphenyl)pyrazine-2-carbohydrazide (Compound 209);
(*E*)-*N*'-(3,5-dimethoxybenzylidene)-6-(4-ethoxyphenyl)-5-(trifluoromethyl)pyrazine-2-carbohydrazide (Compound 210);
(*E*)-*N*'-(3,5-dimethoxybenzylidene)-6-(4-ethoxyphenyl)-5-methoxypyrazine-2-carbohydrazide (Compound 211);
(*E*)-*N*'-(3,5-dimethoxybenzylidene)-5-ethoxy-6-(4-ethoxyphenyl)pyrazine-2-carbohydrazide (Compound 212);
(*E*)-6-(4-ethoxy-2,6-dimethylphenyl)-*N'*-(3-methoxybenzylidene)pyrazine-2-carbohydrazide (Compound 213);
(*E*)-*N*'-(3-(2-hydroxyethyl)-5-methoxybenzylidene)-6-(4-ethoxyphenyl)pyrazine-2-carbohydrazide (Compound 214); and,
*(E)-N'*-(3-(1-hydroxyethyl)-5-methoxybenzylidene)-6-(4-ethoxyphenyl)pyrazine-2-carbohydrazide (Compound 215).

**3.** The COMPOUND according to claim 1 or 2, **characterized in that** it is a selective inhibitor of the voltage-gated sodium channels Nav 1.7 and/or Nav 1.8.

**4.** A PHARMACEUTICAL COMPOSITION **characterized in that** it comprises a therapeutically effective amount of one or more compounds of Formula (I) or a pharmaceutically acceptable salt, hydrate, solvate, and isomer thereof as defined in any one of claims 1 to 3; and one or more pharmaceutically acceptable excipients.

**5.** The PHARMACEUTICAL COMPOSITION according to claim 4, **characterized in that** it is formulated as an oral, sublingual, nasal, parenteral, injectable, submuscular, topical, transdermal, ocular or rectal composition.

**6.** USE OF COMPOUND(S) OF FORMULA (I), as defined in any one of claims 1 to 3, **characterized in that** it is for preparing a medicament for treating pathologies related to neuropathic pain.

**7.** The USE, according to claim 6, **characterized in that** said pathologies are selected from the group consisting of peripheral neuropathic pain, chemotherapy-induced neuropathy, complex regional pain, neuropathy related to viral infection, neuropathy secondary to tumor infiltration, diabetic neuropathy, phantom limb pain, post-herpetic neuralgia, trigeminal neuralgia and post-surgical neuralgia.

**8.** A METHOD OF TREATING, PREVENTING, ALLEVIATING, SUPPRESSING, AND/OR CONTROLLING neuropathic pain-related pathologies **characterized by** administering an effective amount of at least one compound of Formula (I) or a pharmaceutically acceptable salt, hydrate, solvate, and isomer thereof as defined in any one of claims 1 to 3.

**9.** The METHOD, according to claim 8, **characterized in that** it is for the treatment or prophylaxis of peripheral neuropathic pain, chemotherapy-induced neuropathy, complex regional pain, neuropathy related to viral infection, neuropathy secondary to tumor infiltration, diabetic neuropathy, phantom limb pain, post-herpetic neuralgia, trigeminal neuralgia and post-surgical neuralgia.

**10.** The METHOD according to claim 8 or 9, **characterized in that** the administration of the at least one compound of Formula (I) is selected from the group comprising oral, sublingual, nasal, parenteral, injectable, submuscular, topical, transdermal, ocular and rectal routes.

**11.** A PROCESS FOR OBTAINING A COMPOUND OF GENERAL FORMULA (I) as defined in any one of claims 1 to 3, **characterized in that** it comprises the steps:

(a) Forming the intermediate of Formula III:

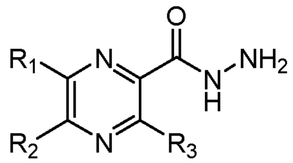

Formula (III)

from the hydrazinolysis reaction of an intermediate of Formula IV:

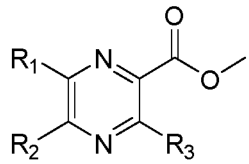

Formula IV

(b) obtaining a Formula I compound wherein R6 is hydrogen;

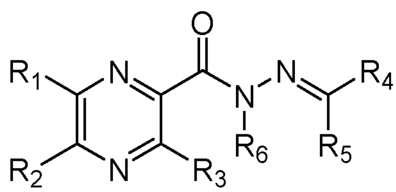

Formula (I)

from condensation of intermediates of Formula II:

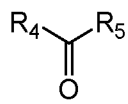

Formula II

and Formula III, with or without presence of catalyst and a suitable solvent; wherein,
- R1 is selected from hydrogen, halogen, branched and linear C1-6alkyl branched and/or linearC1-6 alkoxy, morpholin, piperidinyl or substituted piperidinyl, pyridinyl or substituted pyridinyl, piperazin or substituted piperazin, C3-7 heterocycloalkyl, aryloxy, substituted aryloxy, orR7;
- R7 is:

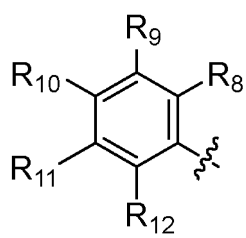

Wherein,
- R8 is selected from the group consisting of hydrogen, halogen, linear or branched C1-6 alkyl, linear or branched C1-6 alkoxy, C1-6haloalkyl, morpholin, C1-6 haloalkyloxy, C1-6 alkylamino or (C1-6 alkylamino) C1-6 alkoxy;
- R9, R11 andR12 are independently selected from the group consisting of hydrogen, halogen, linear or branched C1-6 alkyl, linear or branched C1-6 alkoxy; C1-6haloalkyl or C3-6cycloalkyloxy;
- R10 is selected from the group consisting of hydrogen, halogen, linear or branched C1-6alkyl, linear or branched C1-6 alkoxy, C3-6 cycloalkyloxy; (C3-6cycloalkyl) C1-6 alkoxy; -S-C1-6 alkyl; C1-6 alkylamino; haloalkyloxy C1-6,haloalkyl C1-6, (C1-6 alkylamino) C1-6 alkoxy, (-S-C1-6alkyl) C1-6alkyloxy, C1-6alkylsulfonylamide, or -OCD3;
- R2 and R3 are independently selected from the group consisting of hydrogen, halogen, linear or branched C1-6 alkyl; linear or branched C1-6 alkoxy, amine, C1-6 haloalkyl, aryl, substituted aryl, aryloxy, or substituted aryloxy;
- R4 is selected from the group consisting of hydrogen, halogen, linear or branched C1-6alkyl; linear or branched C1-6 alkoxy, aryl, pyridinyl or substituted pyridinyl, pyridone, saturated or unsaturated C3-7 cycloalkyl saturated or unsaturated C1-5 alkylsubstituted C3-7 cycloalkyl saturated or unsaturated halosubstituted C3-7 cycloalkyl, picolinamide or R13;
- R13 is:

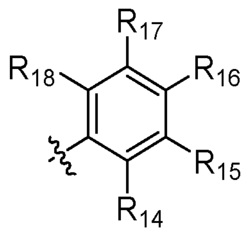

- R14 and R15 are independently selected from the group consisting of hydrogen, halogen, linear or branched C1-6 alkyl; linear or branched C1-6 alkoxy, C1-6haloalkyl, C1-6alkylamino, C1-6 alkylsufonyl, C1-6 alkylsufonylamide, (C1-6 alkylsufonyl) C1-6 alkyl; C1-6 hydroxyalkyl, (C1-6 alkylamino) C1-6 alkoxy, amide, -OCD3, or R14 and R15 are optionally substituted with an OC=N, N=CO or SC=N group forming together a thiazole or an oxazole.
- R16 and R17 are independently selected from the group consisting of hydrogen, halogen, linear or branched C1-6 alkyl; linear or branched C1-6 alkoxy, C1-6haloalkyl, C1-6alkylamino, C1-6 alkylsufonylamide, (C1-6alkylamino) C1-6alkyl or -OCD3;
- R18 is selected from the group consisting of hydrogen, halogen, linear or branched C1-6 alkyl, linear or branched C1-6 alkoxy;
- R5 is selected from hydrogen and linear or branched C1-5 alkyl.

**12.** The PROCESS according to claim 11, **characterized in that** it further comprises a step (c) for forming a compound of Formula (I) in which R6 is linear or branched C1-6 alkyl from the nucleophilic substitution reaction of a compound obtained in step (b), with linear or branched C1-6 alkyl halides in the presence of an inorganic base and polar apoptotic solvents.

**13.** The PROCESS, according to claim 11 or 12, **characterized in that** in step (b) said catalyst is selected from concentrated hydrochloric acid, acetic acid, trifluoroacetic acid, formic acid, or combinations thereof and said solvent is selected from dimethylformamide, dimethylsulfoxide, alcohols, or combinations thereof.

**14.** The PROCESS according to claim 12 or 13, **characterized in that** in step (c) said inorganic base is selected from K2CO3 or NaH.

**15.** A KIT **characterized in that** it comprises a pharmaceutical composition as defined in claim 4 and an application device.

## INTERNATIONAL SEARCH REPORT

International application No. **PCT/BR2022/050303**

A. CLASSIFICATION OF SUBJECT MATTER

**IPC: A61K 31/175 (2006.01), A61K 31/4965 (2006.01), A61P 25/02 (2006.01), A61P 29/00 (2006.01);**
**CPC: A61K 31/175, A61K 31/4965, A61P 25/02, A61P 29/00**

According to International Patent Classification (IPC) or to both national classification and IPC

B. FIELDS SEARCHED

Minimum documentation searched (classification system followed by classification symbols)

**A61K 31, A61P 25, A61P 29**

Documentation searched other than minimum documentation to the extent that such documents are included in the fields searched

**Banco de dados INPI-BR; Periódicos Capes**

Electronic data base consulted during the international search (name of data base and, where practicable, search terms used)

**ESPACENET; CASLINK (REGISTRY, MARPAT, CAPLUS: STN); GOOGLE PATENTS; PUBMED.**

C. DOCUMENTS CONSIDERED TO BE RELEVANT

| Category* | Citation of document, with indication, where appropriate, of the relevant passages | Relevant to claim No. |
|---|---|---|
| X | Da Silva Y.K. et al. Synthesis and pharmacological evaluation of pyrazine N-acylhydrazone derivatives designed as novel analgesic and anti-inflammatory drug candidates. Bioorg Med Chem. 2010 Jul 15;18(14):5007-15. doi: 10.1016/j.bmc.2010.06.002. Epub 2010 Jun 8. PMID: 20598893. **See whole document, particularly abstract and pages 5007-5008, 5014.** | 1-7, 11-13, 15 |
| Y | | 14 |
| X | US 2008300240 A1 (OMEROS CORP [US]) **04 December 2008 (2008-12-04)** **See whole document, particularly p. 2-10, Example 7 and Claims 1, 2 and 10.** | 1-7, 11- 15 |

[X] Further documents are listed in the continuation of Box C. [X] See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **23/09/2022** | **28/09/2022** |
| Name and mailing address of the ISA/**BR** | Authorized officer |
| Facsimile No. | Telephone No. |

Form PCT/ISA/210 (second sheet) (January 2015)

**INTERNATIONAL SEARCH REPORT**

International application No. PCT/BR2022/050303

C (Continuation). DOCUMENTS CONSIDERED TO BE RELEVANT

| Category* | Citation of document, with indication, where appropriate, of the relevant passages | Relevant to claim No. |
|---|---|---|
| X | WO 2011069039 A1 ( SHINKRE BIDHAN A [IN]) 09 June 2011 (2011-06-09) See whole document, particularly paragraphs [0011]-[0027 ], [0065], [0072]-[0085]; claims 11 and 23. | 1-7, 11-13, 15 |
| Y | | 14 |
| X | Rodrigues F.A. *et al.* Biological evaluation of isoniazid derivatives as an anticancer class. Sci Pharm. 2013 Sep 22;82(1):21-8. doi: 10.3797/scipharm.1307-25. PMID: 24634839; PMCID: PMC3951230. See whole document | 1-7, 15 |
| Y | | 11-14 |
| X | EP 1514544 A1 (INST MED MOLECULAR DESIGN INC [JP]) 16 March 2005 (2005-03-06) See abstract, paragraphs [0003] and [0009]; claim 1. | 1-6, 15 |
| Y | | 7, 11-14 |
| X | Milczarska, Barbara & Gobis, Katarzyna & Foks, Henryk & Golunski, Lukasz & Sowinski, Pawel. (2012). The Synthesis of 3-Amino-pyrazine-2-carbohydrazide and 3-Amino-N′-methylpyrazine-2-carbohydrazide Derivatives. Journal of Heterocyclic Chemistry. 49. 10.1002/jhet.877. See whole document | 1-5, 11-15 |
| Y | | 6-7 |
| X | Howie, R. Alan ; da Silva Lima, Camilo H. ; Kaiser, Carlos R. ; de Souza, Marcus V. N. ; Wardell, James L. ; Wardell, Solange M. S. V. . Structures of (pyrazinecarbonyl)hydrazones of substituted benzaldehydes: different supramolecular arrangements from C—H···X (X = O, N, π) hydrogen bonds. Zeitschrift für Kristallographie , v. 225, p. 158-166, 2010. https://doi.org/10.1524/zkri.2010.1235 See whole document | 1-5, 15 |
| Y | | 6-7, 11-14 |
| X | Gomes, L.R., Low, J.N., Rodrigues, A.S., Wardell, J.L., Lima, C.H., & de Souza, M.V. (2013). Five N'-benzylidene-N-methylpyrazine-2-carbohydrazides. Acta crystallographica. Section C, Crystal structure communications, 69 Pt 5, 549-55. doi: 10.1107/s0108270113010056 See whole document | 1-5, 15 |
| Y | | 6-7, 11-14 |

Form PCT/ISA/210 (continuation of second sheet) (January 2015)

**INTERNATIONAL SEARCH REPORT**

International application No. PCT/BR2022/050303

C (Continuation). DOCUMENTS CONSIDERED TO BE RELEVANT

| Category* | Citation of document, with indication, where appropriate, of the relevant passages | Relevant to claim No. |
|---|---|---|
| X | Gregory M. Shutske. Synthesis of anhydro-3-mercapto-5-pyrazinyl-1,2,4-triazolium hydroxides. Journal of Heterocyclic Chemistry 1981, 18 (5) , 1017-1023. https://doi.org/10.1002/jhet.5570180533<br>See whole document | 1-5, 11-15 |
| Y | | 6-7 |
| X | WO 2004105488 A1 ( MARAVETZ LESTER L [US]) 09 December 2004 (2004-12-09)<br>See abstract, pages 1-4; claim 1. | 1-5, 11-15 |
| Y | | 6-7 |
| X | US 2010035932 A1 (SCHEPETKIN, I.A. *et al.* [US]) 11 February 2010 (2010-02-11)<br>See paragraphs [0008]-[0012], [0060]-[0081], [0116], [0120], Claims 1, 26, 27. | 1-6, 15 |
| Y | | 7, 11-14 |
| X | WO 2020123675 A1 (UNIV DUKE [US]) 18 June 2020 (2020-06-18)<br>See paragraphs [008], [0029], claim 1. | 1-6, 15 |
| Y | | 7, 11-14 |
| X | WO 2020023802 A1 (HOPE CITY [US]) 30 January 2020 (2020-01-30)<br>See abstract, paragraphs [0115]; [0116]-[0161]; claim 1. | 1-6, 15 |
| Y | | 7, 11-14 |
| X | CN 105481765 A ( NANJING DRUM TOWER HOSPITAL) 13 April 2016 (2016-04-13)<br>See abstract, claim 1. | 1-5, 15 |
| Y | | 6-7, 11-14 |
| X | CN 103880707 A (SHANGHAI JIAOTONG UNIV SCHOOL MEDICINE) 25 June 2014 (2014-06-25)<br>See abstract, claim 1. | 1-6, 15 |
| Y | | 7, 11-14 |
| X | WO 2013123071 A1 (CLEAVE BIOSCIENCES INC [US]) 22 August 2013 (2013-08-22)<br>See abstract; page 80; claim 1. | 1-6, 15 |
| Y | | 7, 11-14 |

Form PCT/ISA/210 (continuation of second sheet) (January 2015)

**INTERNATIONAL SEARCH REPORT**

International application No.
PCT/BR2022/050303

C (Continuation). DOCUMENTS CONSIDERED TO BE RELEVANT

| Category* | Citation of document, with indication, where appropriate, of the relevant passages | Relevant to claim No. |
|---|---|---|
| X | CN 110128343 A (SICHUAN PROVINCIAL PEOPLES HOSPITAL) 16 August 2019 (2019-08-16) See whole document, particularly the abstract and claim 1. | 1-6, 11-13, 15 |
| Y | | 7, 14 |
| X | de L. Ferreira Marcelle, Candea L.P. Andre, de O. Henriques M. Maria das Gracas, Kaiser R. Carlos, da S. Lima H. Camilo and de Souza V.N. Marcus, Synthesis and Cytotoxic Evaluation of Disubstituted N-Acylhydrazones Pyrazinecarbohydrazide Derivatives, Letters in Drug Design & Discovery 2010; 7(4) . https://dx.doi.org/10.2174/157018010790945814 See abstract obtain based Caplus® (STN®). | 1-5, 15 |
| Y | | 6-7, 11-14 |
| X | US 3573306 A (MERCK CO INC) 30 March 1971 (1971-03-30) See whole document, particularly claim 1. | 1-5, 15 |
| Y | | 6-7, 11-14 |
| Y | Sharma M. *et al.* Discovery of novel 1,2,4-triazol-5-ones as tumor necrosis factor-alpha inhibitors for the treatment of neuropathic pain. Chem Biol Drug Des. 2012 Dec;80(6):961-70. doi: 10.1111/cbdd.12049. Epub 2012 Oct 9. PMID: 22958416. See whole document | 1-7-11-15 |

Form PCT/ISA/210 (continuation of second sheet) (January 2015)

**INTERNATIONAL SEARCH REPORT**

International application No.
PCT/BR2022/050303

**Box No. II Observations where certain claims were found unsearchable (Continuation of item 2 of first sheet)**

This international search report has not been established in respect of certain claims under Article 17(2)(a) for the following reasons:

1. [X] Claims Nos.: **8-10**
because they relate to subject matter not required to be searched by this Authority, namely:
Claims 8-10 contain subject matter that falls under the provisions of PCT Rule 39.1(iv), relating to methods for treatment of the human or animal body by surgery or therapy.

2. [ ] Claims Nos.:
because they relate to parts of the international application that do not comply with the prescribed requirements to such an extent that no meaningful international search can be carried out, specifically:

3. [ ] Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

1. [ ] As all required additional search fees were timely paid by the applicant, this international search report covers all searchable claims.

2. [ ] As all searchable claims could be searched without effort justifying additional fees, this Authority did not invite payment of additional fees.

3. [ ] As only some of the required additional search fees were timely paid by the applicant, this international search report covers only those claims for which fees were paid, specifically claims Nos.:

4. [ ] No required additional search fees were timely paid by the applicant. Consequently, this international search report is restricted to the invention first mentioned in the claims; it is covered by claims Nos.:

**Remark on Protest**

[ ] The additional search fees were accompanied by the applicant's protest and, where applicable, the payment of a protest fee.

[ ] The additional search fees were accompanied by the applicant's protest but the applicable protest fee was not paid within the time limit specified in the invitation.

[ ] No protest accompanied the payment of additional search fees.

Form PCT/ISA/210 (continuation of first sheet (2)) (January 2015)

INTERNATIONAL SEARCH REPORT
Information on patent family members

International application No.
PCT/BR2022/050303

| | | | |
|---|---|---|---|
| US 2008300240 A1 | 2008-12-04 | US 7786139 B2 | 2010-08-31 |
| | | EP 2083819 A2 | 2009-08-05 |
| | | ES 2624791 T3 | 2017-07-17 |
| | | US 2011021509 A1 | 2011-01-27 |
| | | US 8278327 B2 | 2012-10-02 |
| | | WO 2008064342 A2 | 2008-05-29 |
| WO 2011069039 A1 | 2011-06-09 | US 2012316198 A1 | 2012-12-13 |
| | | US 8518968 B2 | 2013-08-27 |
| EP 1514544 A1 | 2005-03-16 | EP 1514544 A4 | 2009-01-07 |
| | | AU 2003242103 A1 | 2003-12-22 |
| | | CA 2488367 A1 | 2003-12-18 |
| | | CN 1658872 A | 2005-08-24 |
| | | EA 200401612 A1 | 2005-08-25 |
| | | EA 009701 B1 | 2008-02-28 |
| | | JP WO2003103665 A1 | 2005-10-06 |
| | | JP 4660674 B2 | 2011-03-30 |
| | | JP 2010215644 A | 2010-09-30 |
| | | KR 20050023287 A | 2005-03-09 |
| | | KR 101054562 B1 | 2011-08-04 |
| | | TW 200402291 A | 2004-02-16 |
| | | US 2007185110 A1 | 2007-08-09 |
| | | US 7700655 B2 | 2010-04-20 |
| | | US 2006122243 A1 | 2006-06-08 |
| | | US 2007185059 A1 | 2007-08-09 |
| | | US 2008090779 A1 | 2008-04-17 |
| | | WO 03103665 A1 | 2003-12-18 |
| WO 2004105488 A1 | 2004-12-09 | AR 045688 A1 | 2005-11-09 |
| | | AU 2004243492 A1 | 2004-12-09 |
| | | BR PI0410626 A | 2006-06-20 |
| | | CL 2004001296 A1 | 2005-04-08 |
| | | CN 1842272 A | 2006-10-04 |
| | | EP 1631143 A1 | 2006-03-08 |
| | | JP 2006528955 A | 2006-12-28 |
| | | KR 20060019558 A | 2006-03-03 |
| | | MX PA05012226 A | 2006-02-10 |
| | | US 2007066627 A1 | 2007-03-22 |
| | | ZA 200510406 B | 2008-12-31 |
| US 2010035932 A1 | 2010-02-11 | None | |
| WO 2020123675 A1 | 2020-06-18 | EP 3894392 A1 | 2021-10-20 |
| | | US 2022106265 A1 | 2022-04-07 |
| WO 2020023802 A1 | 2020-01-30 | US 2021355103 A1 | 2021-11-18 |
| CN 105481765 A | 2016-04-13 | None | |
| CN 103880707 A | 2014-06-25 | CN 103880707 B | 2016-04-13 |
| WO 2013123071 A1 | 2013-08-22 | None | |

**INTERNATIONAL SEARCH REPORT**
Information on patent family members

International application No.
PCT/BR2022/050303

| | | | |
|---|---|---|---|
| CN 110128343 A | 2019-08-16 | None | |
| US 3573306 A | 1971-03-30 | BE 746816 A | 1970-09-04 |
| | | IL 33940 D0 | 1970-04-20 |
| | | NL 7001141 A | 1970-09-08 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 10550080 B
- US 9765029 B
- US 10000475 B
- WO 2020261114 A
- WO 2020092667 A
- US 9163042 B
- WO 2014120808 A
- WO 2014120815 A
- WO 2018213426 A
- WO 2019014352 A
- WO 2015006280 A
- US 7928107 B
- WO 2018235851 A
- US 8629149 B
- JP 2017001991 B

### Non-patent literature cited in the description


- *Schaible. Langenbecks Arch. Surg.,* 2004, vol. 389, 237
- **SMITH.** *Pain,* 2020, vol. 161 (1), S127
- **CAVALLI.** *Int. J. Immunopathol. Pharmacol.,* 2019, vol. 33, 2058738419838383
- **BOUHASSIRA.** *Rev Neurol (Paris).,* 2019, vol. 175 (1-2), 16
- **SCHOLZ.** *Nature Neurosci.,* 2002, vol. 5, 1062
- **COSTIGAN.** *Annu. Rev. Neurosci.,* 2009, vol. 32, 1
- **DWORKIN.** *Clin. J. Pain,* 2002, vol. 18 (6), 343
- **DUCREUX.** *Brain,* 2006, vol. 129, 963
- *Pak. Curr. Pain Headache Rep.,* 2018, vol. 22 (2), 9
- **KUSHNAREV.** *Expert Opinion. Investig. Drugs,* 2020, vol. 29 (3), 259
- *Emery. Expert Opin. Ther. Targets,* 2016, vol. 20 (8), 975
- **CATTERALL.** *Nat. Chem. Biol.,* 2020, vol. 16, 1314
- **WISEDCHAISRI.** *Cell,* 2019, vol. 178 (4), 993
- **CLAIRFEUILLE.** *Science,* 2019, vol. 363, 1302
- **LERA-RUIZ.** *J. Med. Chem.,* 2015, vol. 58 (18), 7093
- **BAGAL.** *J. Med. Chem.,* 2013, vol. 56 (3), 593
- **BAGAL.** *J. Med. Chem.,* 2013, vol. 56 (3), 593
- **BAGAL.** *Channels,* 2015, vol. 9 (6), 360
- **LAW.** *Drug Discovery Today,* 2019, vol. 24 (7), 1389
- **BAGAL.** *Channels (Austin),* 2015, vol. 9 (6), 360
- **VETTER.** *Pharmacology & Therapeutics,* 2017, vol. 172, 73
- **AHUJA.** *Science,* 2015, vol. 350 (6267), 1491
- **KINGWELL.** *Nat. Rev. Drug Discov.,* 2019, vol. 18, 321
- **SAFINA.** *J. Med. Chem.,* 2021, vol. 64, 2953
- **LUO.** *J. Med. Chem.,* 2019, vol. 62, 831
- **BANKAR.** *Cell Reports,* 2018, vol. 24, 3133
- **BROWN.** *Bioorg. Med. Chem.,* 2019, vol. 27 (1), 230
- **PAYNE.** *Br. J. Pharmacol.,* 2015, vol. 172 (10), 2654
- **BAGAL.** *Med. Chem. Lett.,* 2015, vol. 6 (6), 650
- **KORT.** *J. Med. Chem.,* 2008, vol. 51, 407
- **ZHANG.** *Neuropharmacology,* 2010, vol. 59, 201-207
- **LAW.** *Drug Discovery Today,* 2019, vol. 24 (7), 1389
- **BAGAL.** *Channels (Austin),* 2015, vol. 9 (6), 360
- **KORNECOOK.** *J. Pharmacol. Agency No. Ther.,* 2017, vol. 362, 146
- **DEUIS.** *Neuropharmacology,* 2017, vol. 127, 87-108
- **KUSHNAREV.** *Expert Opin. Investig. Drugs,* 2020, vol. 29 (3), 259
- **MCKERRALL.** *Bioorganic & Medicinal Chemistry Lett.,* 2018, vol. 28, 3141
- **BAGAL.** *Bioorganic & Medicinal Chemistry Lett.,* 2014, vol. 24, 3690